# EUROPÄISCHE PATENTSCHRIFT

(11) **EP 3 638 665 B1**
(45) Veröffentlichungstag und Bekanntmachung des Hinweises auf die Patenterteilung: **21.07.2021**
(21) Anmeldenummer: 18728668.7
(22) Anmeldetag: 11.06.2018
(51) Int. Cl.: C07D 413/12, A01N 43/80

(54) **HERBIZID WIRKSAME 3-PHENYLISOXAZOLIN-5-CARBOXAMIDE VON TETRAHYDRO- UND DIHYDROFURANCARBONSÄUREN UND -ESTERN**
HERBICIDAL 3-PHENYLISOXAZOLINE-5-CARBOXAMIDES OF TETRAHYDRO AND DIHYDROFURAN CARBOXYLIC ACIDS AND ESTERS
HERBICIDES EFFICACES A BASE DE 3-PHENYLISOXAZOLINE-5-CARBOXAMIDES D'ACIDES ET D'ESTERS CARBOXYLIQUES DE TETRAHYDRO ET DIHYDROFURANE

(30) Priorität: 13.06.2017 EP 17175777
(43) Veröffentlichungstag der Anmeldung: 22.04.2020
(73) Patentinhaber: Bayer Aktiengesellschaft, 51373 Leverkusen (DE); Bayer CropScience Aktiengesellschaft, 40789 Monheim am Rhein (DE)
(72) Erfinder: PETERS, Olaf, 99891 Tabarz (DE); HAAF, Klaus, Bernhard, 65779 Kelkheim (DE); LINDELL, Stephen, David, 65817 Eppstein (DE); BOJACK, Guido, 65207 Wiesbaden-Naurod (DE); LAW, Katherine, Rose, 65812 Bad Soden (DE); MACHETTIRA, Anu, Bheemaiah, 60326 Frankfurt am Main (DE); DIETRICH, Hansjörg, 65835 Liederbach am Taunus (DE); GATZWEILER, Elmar, 61231 Bad Nauheim (DE); ROSINGER, Christopher, Hugh, 65719 Hofheim (DE)
(74) Vertreter: BIP Patents
(86) Internationale Anmeldenummer: PCT/EP2018/065333
(87) Internationale Veröffentlichungsnummer: WO 2018/228985

(56) Entgegenhaltungen:
- WO-A1-2012/130798

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Speziell betrifft sie substituierte 3-Phenylisoxazolin-5-carboxamide und -5-thioamide von Tetrahydro- und Dihydrofurancarbonsäuren und -estern, Verfahren zu ihrer Herstellung und ihre Verwendung als Herbizide.

WO1995/014681 A1, WO1995/014680 A1, WO 2008/035315 A1, WO2005/051931 A1 und WO2005/021515 A1 beschreiben unter anderen jeweils 3-Phenylisoxazolin-5-carboxamide, die am Phenylring in 3- und 4-Position durch Alkoxy-Reste substiuiert sind. WO1998/057937 A1 beschreibt unter anderem Verbindungen, die am Phenylring in 4-Position durch einen Alkoxy-Rest substiuiert sind. WO2006/016237 A1 beschreibt unter anderen jeweils solche Verbindungen, die am Phenylring durch einen Amido-Rest substiuiert sind. Die in vorstehend genannten Dokumenten beschriebenen Verbindungen werden darin als pharmakologisch wirksam offenbart.

In WO2005/021516 A1 werden 3-(([3-(3-tert-Butylphenyl)-5-ethyl-4,5-dihydro-1,2-oxazol-5-yl]carbonyl)amino)-5-fluor-4-oxopentansäure und 3-(([3-(3-tert-Butylphenyl)-5-isopropyl-4,5-dihydro-1,2-oxazol-5-yl]carbonyl)amino)-5-fluor-4-oxopentansäure als pharmakologisch wirksame Verbindungen offenbart.

Aus DE 4026018 A1, EP 0 520 371 A2 und DE 4017665 sind 3-Phenylisoxazolin-5-carboxamide bekannt, die in 5-Position des Isoxazolin-Rings ein Wasserstoffatom tragen. Diese Verbindungen werden dort als agrochemisch wirksame Safener beschrieben, d.h. als Verbindungen, die die unerwünschte herbizide Wirkung von Herbiziden gegenüber Kulturpflanzen aufheben. Eine herbizide Wirkung dieser Verbindungen ist nicht offenbart. Die prioritätsältere nicht vorveröffentlichte europäische Patentanmeldung Nr. 10170238 offenbart herbizid und fungizid wirksame 3-Phenylisoxazolin-5-carboxamide und 3-Phenylisoxazolin-5-thioamide, die in 5-Position des Isoxazolin-Rings ein Wasserstoffatom tragen. Aus Monatshefte Chemie (2010) 141, 461 und Letters in Organic Chemistry (2010), 7, 502 sind ebenfalls 3-Phenylisoxazolin-5-carboxamide bekannt, die in 5-Position des Isoxazolin-Rings ein Wasserstoffatom tragen. Für einige der genannten Verbindungen wird eine fungizide, jedoch keine herbizide Wirkung offenbart.

WO 2014/048827 beschreibt die herbizide Wirkung von 3-Phenylisoxazolin-5-carbonsäuren, -5-carbonsäureestern, 5-carbaldehyden und 5-nitrilen.

WO 2014/048853 offenbart Isoxazolin-5-carboxamide und -5-thioamide mit Heterocyclen in 3-Position, die eine herbizide und fungizide Wirkung zeigen.

WO 2014/048940 offenbart fungizide wirksame Isoxazolin-carboxamide mit einem Chinolin als speziellem Heterocyclus in der 3-Position.

WO 2014/048882 offenbart Isoxazolin-carboxamide mit Alkoxy als speziellen Rest in der 5-Position.

In WO 2012/130798 werden herbizid und fungizid aktive 3-Phenylisoxazolin-5-carboxamide und -5-thioamide von substituierten Heterocyclen beschrieben.

Die herbizide Wirkung dieser bekannten Verbindungen, insbesondere bei niedrigen Aufwandmengen, bzw. deren Verträglichkeit gegenüber Kulturpflanzen bleiben verbesserungswürdig.

Aus den genannten Gründen besteht weiterhin ein Bedarf nach wirkungsstarken Herbiziden und/oder Pflanzenwachstumsregulatoren für die selektive Anwendung in Pflanzenkulturen oder die Anwendung auf Nichtkulturland, wobei diese Wirkstoffe vorzugsweise weitere vorteilhafte Eigenschaften in der Anwendung haben sollten, wie zum Beispiel eine verbesserte Verträglichkeit gegenüber Kulturpflanzen.

Ein Gegenstand der vorliegenden Erfindung ist daher die Bereitstellung von Verbindungen mit herbizider Wirkung (Herbizide), die bereits bei relativ niedrigen Aufwandmengen gegen wirtschaftlich wichtige Schadpflanzen hochwirksam sind und vorzugsweise bei guter Wirksamkeit gegen Schadpflanzen selektiv in Kulturpflanzen eingesetzt werden können und dabei vorzugsweise eine gute Verträglichkeit gegenüber Kulturpflanzen zeigen. Bevorzugt sollten diese herbiziden Verbindungen insbesondere effektiv und effizient gegen ein breites Spektrum an Ungräsern sein, und vorzugsweise zusätzlich eine gute Wirksamkeit gegen viele Unkräuter aufweisen.

Neben einer herbiziden Wirkung weisen zahlreiche Verbindung der Formel (I) auch fungizide Wirkung auf, die jedoch nur gering ausgeprägt ist.

Überraschenderweise wurde nun gefunden, dass die nachfolgend definierten 3-Phenylisoxazolin-5-carboxamide von Tetrahydro- und Dihydrofurancarbonsäuren und -estern der Formel (I) und deren Salze eine ausgezeichnete herbizide Wirksamkeit gegen ein breites Spektrum wirtschaftlich wichtiger mono- und dikotyler annueller Schadpflanzen aufweisen.

Gegenstand der vorliegenden Erfindung sind daher Verbindungen der allgemeinen Formel (I) 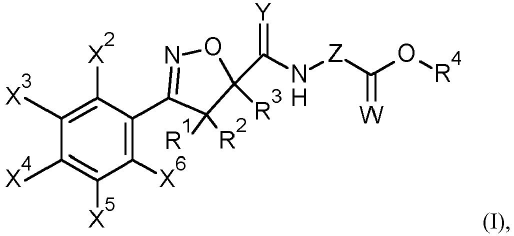 und deren agrochemisch verträglichen Salze, in welchen
- R¹ und R²: unabhängig voneinander jeweils Wasserstoff, Fluor, Chlor oder Cyano,
oder
durch jeweils m Reste aus der Gruppe bestehend aus Fluor, Chlor, Brom und Cyano substituiertes (C₁-C₃)-Alkyl oder (C₁-C₃)-Alkoxy bedeuten;
- R³: durch jeweils m Reste aus der Gruppe bestehend aus Fluor, Chlor, Brom, Cyano, (C₁-C₄)-Alkoxy und Hydroxy substituiertes (C₁-C₄)-Alkyl, (C₃-C₅)-Cycloalkyl, (C₂-C₄)-Alkenyl, (C₂-C₄)-Alkinyl oder (C₁-C₄)-Alkoxy bedeutet;
- R⁴: Wasserstoff,
oder
durch jeweils m Reste aus der Gruppe bestehend aus Halogen, Cyano, (C₁-C₆)-Alkoxy, Hydroxy und Aryl substituiertes (C₁-C₁₂)-Alkyl, (C₃-C₇)-Cycloalkyl, (C₃-C₇)-Cycloalkyl-(C₁-C₈)-Alkyl, (C₂-C₈)-Alkenyl, (C₅-C₆)-Cycloalkenyl oder (C₂-C₈)-Alkinyl bedeutet;
- Y: Sauerstoff oder Schwefel bedeutet;
- W: Sauerstoff oder Schwefel bedeutet;
- Z: für einen vollständig gesättigten oder teilweise gesättigten Furanring steht, der mit k Resten der Gruppe R⁵ substituiert ist 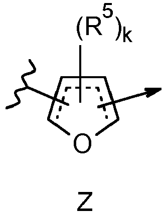 worin der Pfeil jeweils für eine Bindung zur Gruppe C=W der Formel (I) steht;
- R⁵: Fluor, Chlor, Cyano oder CO₂R⁷,
oder
durch jeweils m Reste aus der Gruppe bestehend aus Fluor und Chlor substituiertes (C₁-C₂)-Alkyl oder (C₁-C₂)-Alkoxy bedeutet;
- X², X⁴ und X⁶: unabhängig voneinander jeweils Wasserstoff, Fluor, Chlor, Brom oder Cyano,
oder
jeweils durch m Reste aus der Gruppe bestehend aus Fluor, Chlor, Brom und (C₁-C₂)-Alkoxy substituiertes (C₁-C₂)-Alkyl bedeuten;
- X³ und: X⁵ unabhängig voneinander Wasserstoff, Fluor, Chlor, Brom, Iod, Hydroxy, Cyano, Nitro, S(O)ₙR⁶ oder CO₂R⁷,
oder
jeweils durch m Reste aus der Gruppe bestehend aus Fluor, Chlor und Brom substituiertes (C₁-C₃)-Alkyl, (C₁-C₃)-Alkoxy, (C₃-C₄)-Cycloalkyl, (C₂-C₃)-Alkenyl oder (C₂-C₃)-Alkinyl bedeuten;
- R⁶: durch jeweils m Reste aus der Gruppe bestehend aus Fluor und Chlor substituiertes (C₁-C₂)-Alkyl oder (C₃-C₄)-Cycloalkyl bedeutet;
- R⁷: Wasserstoff,
oder
durch jeweils m Reste aus der Gruppe bestehend aus Fluor, Chlor und (C₁-C₂)-Alkoxy substituiertes (C₁-C₃)-Alkyl oder (C₃-C₅)-Cycloalkyl bedeutet;
- k: die Laufzahl 0, 1 oder 2 beträgt;
- m: die Laufzahl 0, 1, 2, 3, 4 oder 5 beträgt; und
- n: die Laufzahl 0, 1 oder 2 beträgt.

Alkyl bedeutet gesättigte, geradkettige oder verzweigte Kohlenwasserstoffreste mit der jeweils angegebenen Anzahl von Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Di-methylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl.

Durch Halogen substitiertes Alkyl bedeutet geradkettige oder verzweigte Alkylgruppen, wobei in diesen Gruppen teilweise oder vollständig die Wasserstoffatome durch Halogenatome ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl.

Alkenyl bedeutet ungesättigte, geradkettige oder verzweigte Kohlenwasserstoffreste mit der jeweils angegebenen Anzahl von Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

Alkinyl bedeutet geradkettige oder verzweigte Kohlenwasserstoffreste mit der jeweils angegebenen Anzahl von Kohlenstoffatomen und einer Dreifachbindung in einer beliebigen Position, z.B. C₂-C₆-Alkinyl wie Ethinyl, 1-Propinyl, 2-Propinyl (oder Propargyl), 1-Butinyl, 2-Butinyl, 3-Butinyl, 1-Methyl-2-propinyl, 1-Pentinyl, 2-Pentinyl, 3-Pentinyl, 4-Pentinyl, 3-Methyl-1-butinyl, 1-Methyl-2-butinyl, 1-Methyl-3-butinyl, 2-Methyl-3-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 3-Methyl-1-pentinyl, 4-Methyl-1-pentinyl, 1-Methyl-2-pentinyl, 4-Methyl-2-pentinyl, 1-Methyl-3-pentinyl, 2-Methyl-3-pentinyl, 1-Methyl-4-pentinyl, 2-Methyl-4-pentinyl, 3-Methyl-4-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl.

Cycloalkyl bedeutet ein carbocyclisches, gesättigtes Ringsystem mit vorzugsweise 3-8 Ring-C-Atomen, z.B. Cyclopropyl, Cyclobutyl, Cyclopentyl oder Cyclohexyl. Im Falle von gegebenenfalls substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind.
Im Falle von gegebenenfalls substituiertem Cycloalkyl werden auch mehrcyclische aliphatische Systeme umfaßt, wie beispielsweise Bicyclo[1.1.0]butan-1-yl, Bicyclo[1.1.0]butan-2-yl, Bicyclo[2.1.0]pentan-1-yl, Bicyclo[2.1.0]pentan-2-yl, Bicyclo[2.1.0]pentan-5-yl, Bicyclo[2.2.1]hept-2-yl (Norbornyl), Adamantan-1 -yl und Adamantan-2-yl.

Im Falle von substituiertem Cycloalkyl werden auch spirocyclische aliphatische Systeme umfaßt, wie beispielsweise Spiro[2.2]pent-1-yl, Spiro[2.3]hex-1-yl und Spiro[2.3]hex-4-yl, 3-Spiro[2.3]hex-5-yl.

Cycloalkenyl bedeutet ein carbocyclisches, nicht aromatisches, partiell ungesättigtes Ringsystem mit vorzugsweise 4-8 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl, wobei auch Substituenten mit einer Doppelbindung am Cycloalkenylrest, z. B. eine Alkylidengruppe wie Methyliden, umfasst sind. Im Falle von gegebenenfalls substituiertem Cycloalkenyl gelten die Erläuterungen für substituiertes Cycloalkyl entsprechend.

Alkoxy bedeutet gesättigte, geradkettige oder verzweigte Alkoxyreste mit der jeweils angegebenen Anzahl von Kohlenstoffatomen, z.B. C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy. Durch Halogen substitiertes Alkoxy bedeutet geradkettige oder verzweigte Alkoxyreste mit der jeweils angegebenen Anzahl von Kohlenstoffatomen, wobei in diesen Gruppen teilweise oder vollständig die Wasserstoffatome durch Halogenatome wie vorstehend genannt ersetzt sein können, z.B. C₁-C₂-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlor-fluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor-1,2-difluorethoxy, 2,2-Dichlor-2-fluorethoxy, 2,2,2-Trichlorethoxy, Pentafluor-ethoxy und 1,1,1-Trifluorprop-2-oxy.

Aryl bedeutet ein gegebenenfalls durch 0 - 5 Reste aus der Gruppe Fluor, Chlor, Brom, Iod, Cyano, Hydroxy, (C₁- C₃)-Alkyl, (C₁-C₃)-Alkoxy, (C₃- C₄)-Cycloalkyl, (C₂- C₃)-Alkenyl oder (C₂- C₃)-Alkinyl substituiertes Phenyl.

Die Bezeichnung "Halogen" bedeutet Fluor, Chlor, Brom oder Iod. Wird die Bezeichnung für einen Rest verwendet, dann bedeutet "Halogen" ein Fluor-, Chlor-, Brom- oder Iodatom.

Die Verbindungen der Formel (I) können je nach Art und Verknüpfung der Substituenten als Stereoisomere vorliegen. Sind beispielsweise ein oder mehrere asymmetrisch substiuierte Kohlenstoffatome und/oder Sulfoxide vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden.

Die Erfindung betrifft auch alle Stereoisomeren und deren Gemische, die von der Formel (I) umfaßt, jedoch nicht spezifisch definiert sind. Im Folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Je nach Art der oben definierten Substituenten weisen die Verbindungen der Formel (I) saure Eigenschaften auf und können mit anorganischen oder organischen Basen oder mit Metallionen Salze, gegebenenfalls auch innere Salze oder Addukte bilden. Tragen die Verbindungen der Formel (I) Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und teritäre Amine mit (C₁-C₄-)-Alkyl-Gruppen, Mono-, Di- und Trialkanolamine von (C₁-C₄)-Alkanolen, Cholin sowie Chlorcholin, sowie organische Amine, wie Trialkylamine, Morpholin, Piperidin oder Pyridin. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel [NRR'R"R"']⁺, worin R bis R'" jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Die Verbindungen der Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion.

Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden.

Ist eine Gruppe mehrfach durch Reste substituiert, so bedeutet dies, dass diese Gruppe durch einen oder mehrere gleiche oder verschiedene der genannten Reste substituiert ist.

In allen nachfolgend genannten Formeln haben die Substituenten und Symbole, sofern nicht anders definiert, dieselbe Bedeutung wie unter Formel (I) beschrieben. Pfeile in einer chemischen Formel bedeuten die Verknüpfungsorte zum restlichen Molekül.

Im Folgenden werden, jeweils für die einzelnen Substituenten, bevorzugte, besonders bevorzugte und ganz besonders bevorzugte Bedeutungen beschrieben. Die übrigen Substituenten der allgemeinen Formel (I), welche nachfolgend nicht genannt werden, weisen die oben genannte Bedeutung auf.

In einer ersten Ausführungsform der vorliegenden Erfindung bedeuten

R¹ und R² bevorzugt unabhängig voneinander jeweils Wasserstoff, Fluor, Chlor oder Cyano, oder durch jeweils m Reste aus der Gruppe bestehend aus Fluor und Chlor substituiertes Methyl oder Methoxy.

Besonders bevorzugt bedeuten R¹ und R² jeweils Wasserstoff.

In einer zweiten Ausführungsform der vorliegenden Erfindung bedeutet

R³ bevorzugt durch jeweils m Reste aus der Gruppe bestehend aus Fluor, Chlor, Brom, Cyano, (C₁-C₂)-Alkoxy und Hydroxy substituiertes (C₁-C₃)-Alkyl, (C₃-C₄)-Cycloalkyl, (C₂-C₃)-Alkenyl, (C₂-C₃)-Alkinyl oder (C₁-C₃)-Alkoxy.

In einer dritten Ausführungsform der vorliegenden Erfindung bedeutet

R⁴ bevorzugt Wasserstoff, oder durch jeweils m Reste aus der Gruppe bestehend aus Fluor, Chlor, Brom, Cyano, (C₁-C₄)-Alkoxy, Hydroxy und Aryl substituiertes (C₁-C₆)-Alkyl, (C₃-C₆)-Cycloalkyl, (C₃-C₆)-Cycloalkyl-(C₁-C₆)-Alkyl, (C₂-C₆)-Alkenyl, (C₅-C₆)-Cycloalkenyl oder (C₂-C₆)-Alkinyl.

In einer vierten Ausführungsform der vorliegenden Erfindung bedeutet

Y bevorzugt Sauerstoff.

In einer fünften Ausführungsform der vorliegenden Erfindung bedeutet

W bevorzugt Sauerstoff.

In einer sechsten Ausführungsform der vorliegenden Erfindung steht

Z bevorzugt für eine Gruppe Z-1 bis Z-33, wobei Z-1 bis Z-33 folgende Bedeutung haben: 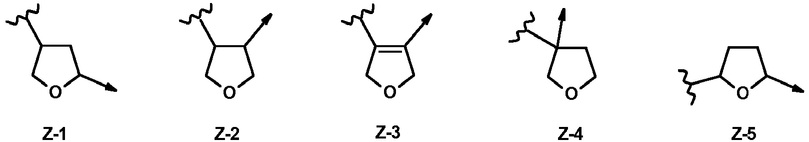 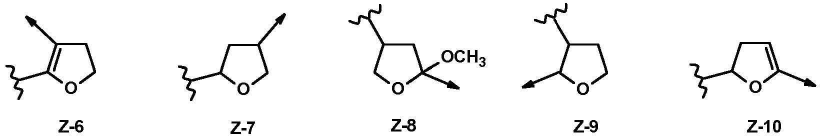 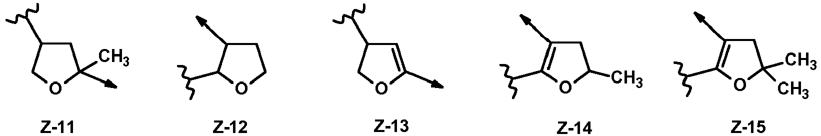 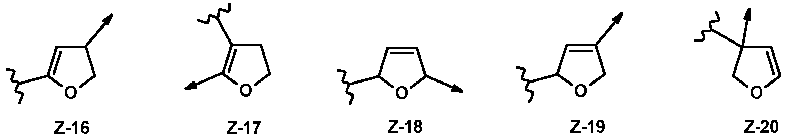 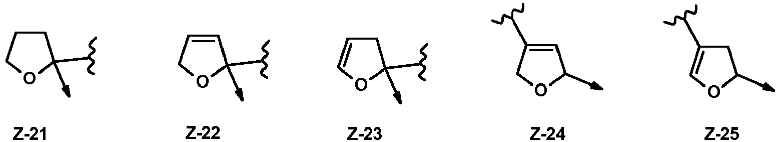 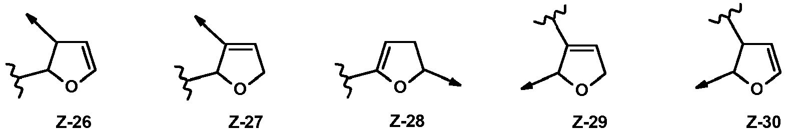 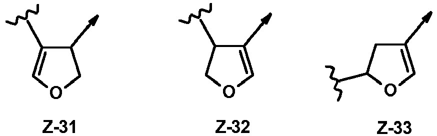 worin der Pfeil jeweils für eine Bindung zur Gruppe C=W der Formel (I) steht.

Besonders bevorzugt steht Z für eine Gruppe Z-1 bis Z-15, wobei Z-1 bis Z-15 folgende Bedeutung haben: 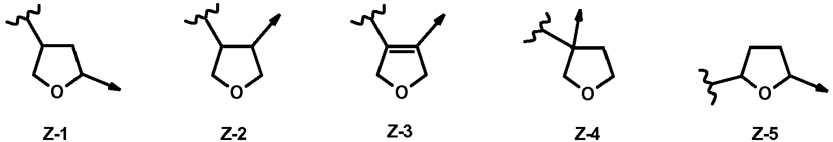 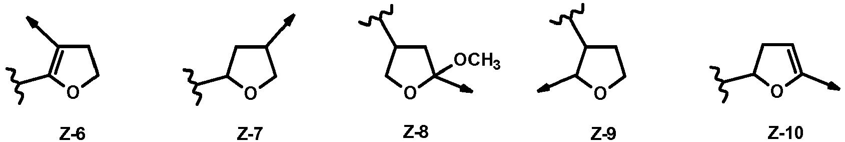 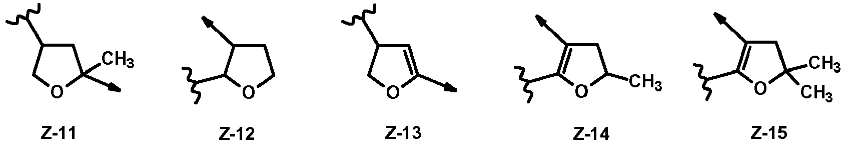 worin der Pfeil jeweils für eine Bindung zur Gruppe C=W der Formel (I) steht.

Am meisten bevorzugt steht Z für Z-1 bis Z-4 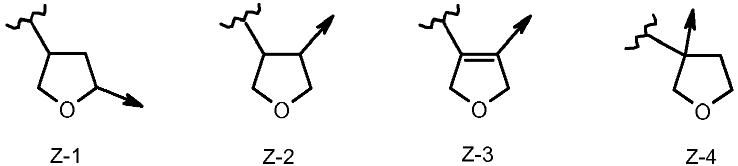 worin der Pfeil jeweils für eine Bindung zur Gruppe C=W der Formel (I) steht.

In einer siebten Ausführungsform der vorliegenden Erfindung bedeutet

R⁵ bevorzugt Fluor, Chlor, Cyano, CO₂H, CO₂CH₃ oder CO₂CH₂CH₃, oder jeweils durch m Reste aus der Gruppe bestehend aus Fluor und Chlor substituiertes (C₁-C₂)-Alkyl oder (C₁-C₂)-Alkoxy.

In einer achten Ausführungsform der vorliegenden Erfindung bedeuten

X², X⁴ und X⁶ bevorzugt unabhängig voneinander jeweils Wasserstoff, Fluor, Chlor, Brom oder Cyano, oder jeweils durch m Reste aus der Gruppe bestehend aus Fluor und Chlor substituiertes Methyl oder Methoxy.

Besonders bevorzugt bedeuten X², X⁴ und X⁶ unabhängig voneinander Wasserstoff oder Fluor.

In einer neunten Ausführungsform der vorliegenden Erfindung bedeutet

X³ und X⁵ bevorzugt unabhängig voneinander Wasserstoff, Fluor, Chlor, Brom, Hydroxy oder Cyano, oder jeweils durch m Reste aus der Gruppe bestehend aus Fluor, Chlor und Brom substituiertes (C₁-C₃)-Alkyl, (C₁-C₃)-Alkoxy, (C₃-C₄)-Cycloalkyl, (C₂-C₃)-Alkenyl oder (C₂-C₃)-Alkinyl.

In einer zehnten Ausführungsform der vorliegenden Erfindung bedeutet

R⁷ bevorzugt Wasserstoff, oder durch jeweils m Reste aus der Gruppe bestehend aus Fluor und Chlor substituiertes (C₁-C₃)-Alkyl.

In einer elften Ausführungsform der vorliegenden Erfindung beträgt die Laufzahl

m bevorzugt 0, 1, 2 oder 3.

Im Rahmen der vorliegenden Erfindung ist es möglich, die einzelnen bevorzugten, besonders bevorzugten und am meisten bevorzugten Bedeutungen für die Substituenten R¹ bis R⁷, X² bis X⁶, W, Y und Z, sowie die Laufzahlen k, m, und n beliebig miteinander zu kombinieren.

Das heißt, dass Verbindungen der allgemeinen Formel (I) von der vorliegenden Erfindung umfasst sind, in welchen beispielsweise der Substituent R¹ eine bevorzugte Bedeutung aufweist und die Substituenten R² bis R⁷ die allgemeine Bedeutung aufweisen oder aber der Substituent R² eine bevorzugte Bedeutung aufweist, der Substituent R³ eine besonders bevorzugte, bzw. eine ganz besonders bevorzugte, Bedeutung aufweist und die übrigen Substituenten eine allgemeine Bedeutung aufweisen.

Drei dieser Kombinationen der oben für die Substituenten R¹ bis R⁷, X² bis X⁶, W, Y und Z, sowie die Laufzahlen k, m, und n angegebenen Definitionen werden nachfolgend beispielhaft erläutert und jeweils als weitere Ausführungsformen offenbart:

In einer zwölften Ausführungsform der vorliegenden Erfindung bedeuten
- R¹ und R²: unabhängig voneinander jeweils Wasserstoff, Fluor, Chlor oder Cyano,
oder
durch jeweils m Reste aus der Gruppe bestehend aus Fluor, Chlor, Brom und Cyano substituiertes (C₁-C₃)-Alkyl oder (C₁-C₃)-Alkoxy;
- R³: bedeutet durch jeweils m Reste aus der Gruppe bestehend aus Fluor, Chlor, Brom, Cyano, (C₁-C₄)-Alkoxy und Hydroxy substituiertes (C₁-C₄)-Alkyl, (C₃-C₅)-Cycloalkyl, (C₂-C₄)-Alkenyl, (C₂-C₄)-Alkinyl oder (C₁-C₄)-Alkoxy;
- R⁴: bedeutet Wasserstoff,
oder
durch jeweils m Reste aus der Gruppe bestehend aus Fluor, Chlor, Brom, Cyano, (C₁-C₄)-Alkoxy, Hydroxy und Aryl substituiertes (C₁-C₆)-Alkyl, (C₃-C₆)-Cycloalkyl, (C₃-C₆)-Cycloalkyl-(C₁-C₆)-Alkyl, (C₂-C₆)-Alkenyl, (C₅-C₆)-Cycloalkenyl oder (C₂-C₆)-Alkinyl;
- Y: bedeutet Sauerstoff;
- W: bedeutet Sauerstoff;
- Z: steht für eine Gruppe Z-1 bis Z-33, wobei Z-1 bis Z-33 folgende Bedeutung haben: 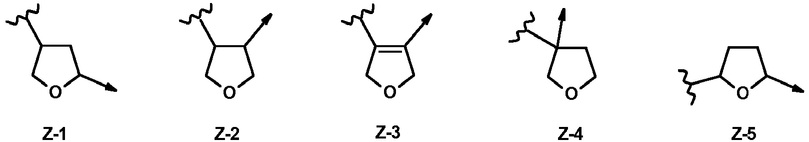 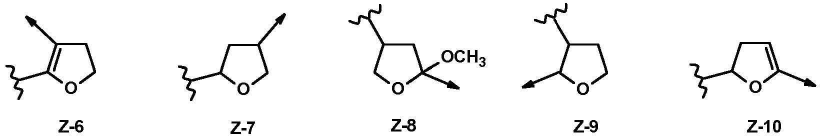 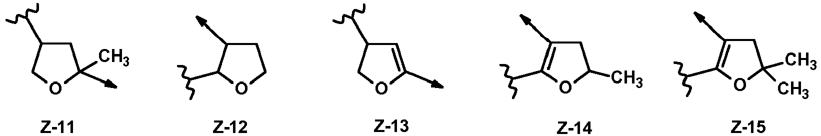 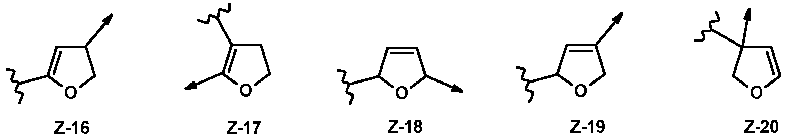 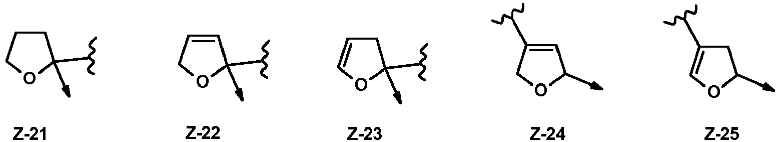 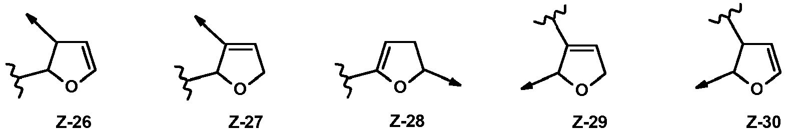 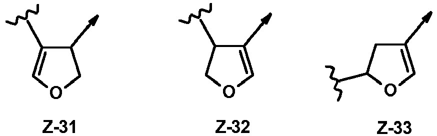
worin der Pfeil jeweils für eine Bindung zur Gruppe C=W der Formel (I) steht;
- X², X⁴ und X⁶: bedeuten unabhängig voneinander jeweils Wasserstoff, Fluor, Chlor, Brom oder Cyano,
oder
jeweils durch m Reste aus der Gruppe bestehend aus Fluor, Chlor, Brom und (C₁-C₂)-Alkoxy substituiertes (C₁-C₂)-Alkyl;
- X³ und X⁵: bedeuten unabhängig voneinander Wasserstoff, Fluor, Chlor, Brom, Iod, Hydroxy, Cyano, Nitro, S(O)ₙR⁶ oder CO²R⁷,
oder
jeweils durch m Reste aus der Gruppe bestehend aus Fluor, Chlor und Brom substituiertes (C₁-C₃)-Alkyl, (C₁-C₃)-Alkoxy, (C₃-C₄)-Cycloalkyl, (C₂-C₃)-Alkenyl oder (C₂-C₃)-Alkinyl;
- R⁶: bedeutet durch jeweils m Reste aus der Gruppe bestehend aus Fluor und Chlor substituiertes (C₁-C₂)-Alkyl oder (C₃-C₄)-Cycloalkyl;
- R⁷: bedeutet Wasserstoff,
oder
durch jeweils m Reste aus der Gruppe bestehend aus Fluor, Chlor und (C₁-C₂)-Alkoxy substituiertes (C₁-C₃)-Alkyl oder (C₃-C₅)-Cycloalkyl;
- m: die Laufzahl 0, 1, 2 oder 3 beträgt; und
- n: die Laufzahl 0, 1 oder 2 beträgt.

In einer dreizehnten Ausführungsform der vorliegenden Erfindung bedeuten
- R¹ und R²: unabhängig voneinander jeweils Wasserstoff, Fluor, Chlor oder Cyano,
oder
durch jeweils m Reste aus der Gruppe bestehend aus Fluor und Chlor substituiertes Methyl oder Methoxy;
- R³: bedeutet durch jeweils m Reste aus der Gruppe bestehend aus Fluor, Chlor, Brom, Cyano, (C₁-C₂)-Alkoxy und Hydroxy substituiertes (C₁-C₃)-Alkyl, (C₃-C₄)-Cycloalkyl, (C₂-C₃)-Alkenyl, (C₂-C₃)-Alkinyl oder (C₁-C₃)-Alkoxy;
- R⁴: bedeutet Wasserstoff,
oder
durch jeweils m Reste aus der Gruppe bestehend aus Fluor, Chlor, Brom, Cyano, (C₁-C₄)-Alkoxy, Hydroxy und Aryl substituiertes (C₁-C₆)-Alkyl, (C₃-C₆)-Cycloalkyl, (C₃-C₆)-Cycloalkyl-(C₁-C₆)-Alkyl, (C₂-C₆)-Alkenyl, (C₅-C₆)-Cycloalkenyl oder (C₂-C₆)-Alkinyl;
- Y: bedeutet Sauerstoff;
- W: bedeutet Sauerstoff;
- Z: steht für eine Gruppe Z-1 bis Z-15, wobei Z-1 bis Z-15 folgende Bedeutung haben: 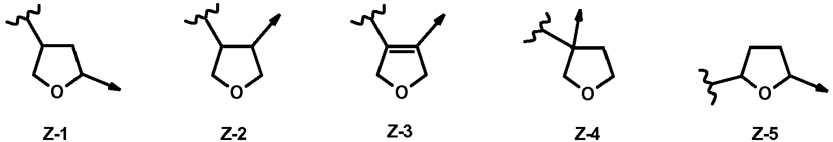 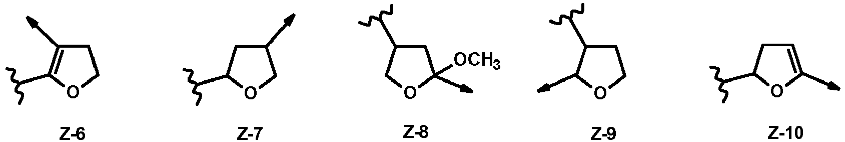 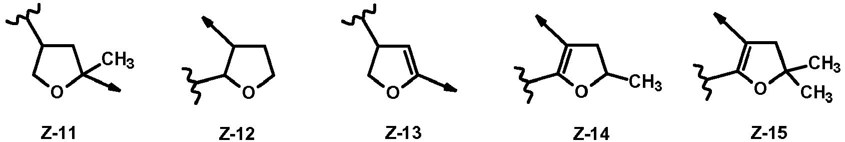
worin der Pfeil jeweils für eine Bindung zur Gruppe C=W der Formel (I) steht;
- X², X⁴ und X⁶: bedeuten unabhängig voneinander jeweils Wasserstoff, Fluor, Chlor, Brom oder Cyano,
oder
jeweils durch m Reste aus der Gruppe bestehend aus Fluor und Chlor substituiertes Methyl oder Methoxy;
- X³ und X⁵: bedeuten unabhängig voneinander Wasserstoff, Fluor, Chlor, Brom, Iod, Hydroxy oder Cyano,
oder
jeweils durch m Reste aus der Gruppe bestehend aus Fluor, Chlor und Brom substituiertes (C₁-C₃)-Alkyl, (C₁-C₃)-Alkoxy, (C₃-C₄)-Cycloalkyl, (C₂-C₃)-Alkenyl oder (C₂-C₃)-Alkinyl; und
- m: die Laufzahl 0, 1, 2 oder 3 beträgt.

In einer vierzehnten Ausführungsform der vorliegenden Erfindung bedeuten
- R¹ und R²: jeweils Wasserstoff;
- R³: bedeuten durch jeweils m Reste aus der Gruppe bestehend aus Fluor, Chlor, Brom, Cyano, (C₁-C₂)-Alkoxy und Hydroxy substituiertes (C₁-C₃)-Alkyl, (C₃-C₄)-Cycloalkyl, (C₂-C₃)-Alkenyl, (C₂-C₃)-Alkinyl oder (C₁-C₃)-Alkoxy;
- R⁴: bedeutet Wasserstoff,
oder
durch jeweils m Reste aus der Gruppe bestehend aus Fluor, Chlor, Brom, Cyano, (C₁-C₄)-Alkoxy, Hydroxy und Aryl substituiertes (C₁-C₆)-Alkyl, (C₃-C₆)-Cycloalkyl, (C₃-C₆)-Cycloalkyl-(C₁-C₆)-Alkyl, (C₂-C₆)-Alkenyl, (C₅-C₆)-Cycloalkenyl oder (C₂-C₆)-Alkinyl;
- Y: bedeutet Sauerstoff;
- W: bedeutet Sauerstoff;
- Z: steht für eine Gruppe Z-1 bis Z-4, wobei Z-1 bis Z-4 folgende Bedeutung haben: 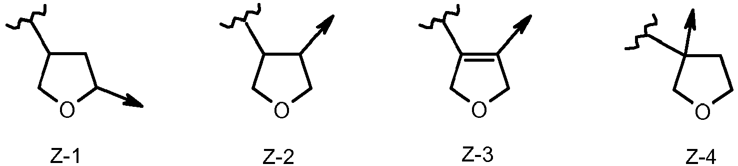
worin der Pfeil jeweils für eine Bindung zur Gruppe C=W der Formel (I) steht;
- X², X⁴ und X⁶: bedeuten unabhängig voneinander Wasserstoff oder Fluor;
- X³ und X⁵: bedeuten unabhängig voneinander Wasserstoff, Fluor, Chlor, Brom, Hydroxy oder Cyano, oder jeweils durch m Reste aus der Gruppe bestehend aus Fluor, Chlor und Brom substituiertes (C₁-C₃)-Alkyl, (C₁-C₃)-Alkoxy, (C₃-C₄)-Cycloalkyl, (C₂-C₃)-Alkenyl oder (C₂-C₃)-Alkinyl; und
- m: die Laufzahl 0, 1, 2 oder 3 beträgt.

Im Folgenden werden in tabellarischer Form Beispiele der Verbindungen der allgemeinen Formel (I) wiedergegeben. In der nachfolgenden Tabelle 1 werden die in Formel (I) allgemein definerten Substituenten spezifiziert.

**Tabelle 1: Verbindungen der allgemeinen Formel (I), worin X² = X⁴ = X⁶ = R¹ = R² = H und für Y = W = O steht**

| Beispiel - Nr. | X³ | X⁵ | R³ | R⁴ | Z | Kommentar |
|---|---|---|---|---|---|---|
| I - 1 | F | F | CH₃ | CH₃ | Z-1 | Z-1 mit Konfiguration 2,4-cis |
| I - 2 | Cl | Cl | OCH₃ | CH₃ | Z-1 | Z-1 mit Konfiguration 2,4-cis |
| I - 3 | Cl | Cl | CH=CH₂ | CH₃ | Z-1 | Z-1 mit Konfiguration 2,4-cis |
| I - 4 | Br | CH₃ | CH₃ | CH₃ | Z-1 | Z-1 mit Konfiguration 2,4-cis |
| I - 5 | F | F | OCH₃ | CH₃ | Z-1 | Z-1 mit Konfiguration 2,4-cis |
| I - 6 | F | F | CF₃ | CH₃ | Z-1 | Z-1 mit Konfiguration 2,4-cis |
| I - 7 | F | H | CH=CH₂ | CH₃ | Z-1 | Z-1 mit Konfiguration 2,4-cis |
| I - 8 | Br | CH₃ | OCH₃ | CH₃ | Z-1 | Z-1 mit Konfiguration 2,4-cis |
| I - 9 | F | F | (R) - CH₃ | CH₃ | Z-1 | Z-1 mit Konfiguration 2,4-cis |
| I - 10 | F | F | (S) - CH=CH₂ | CH₃ | Z-1 | Z-1 mit Konfiguration 2,4-cis; 2 Diastereomere |
| I - 11 | F | F | (S) - CH=CH₂ | CH₃ | Z-1 | Z-1 mit Konfiguration 2,4-cis; Diastereomer 1 von I-10 (einzelnes Enantiomer) |
| I - 12 | F | F | (S) - CH=CH₂ | CH₃ | Z-1 | Z-1 mit Konfiguration 2,4-cis; Diastereomer 2 von I-10 (einzelnes Enantiomer) |
| I - 13 | F | H | CH₃ | CH₃ | Z-1 | Z-1 mit Konfiguration 2,4-cis |
| I - 14 | H | H | OCH₃ | CH₃ | Z-1 | Z-1 mit Konfiguration 2,4-cis |
| I - 15 | F | F | (R) - CH₃ | CH₃ | Z-1 | Z-1 mit Konfiguration 2,4-cis; Diastereomer 1 (einzelnes Enantiomer) |
| I - 16 | F | F | (R) - CH₃ | CH₃ | Z-1 | Z-1 mit Konfiguration 2,4-cis; Diastereomer 2 (einzelnes Enantiomer) |
| I - 17 | F | F | CH₃ | CH₃ | Z-4 | |
| I - 18 | F | F | CH₃ | CH₂CH₃ | Z-4 | |
| I - 19 | F | F | CH₃ | CH₃ | Z-2 | |
| I - 20 | F | H | CH₃ | H | Z-1 | Z-1 mit Konfiguration 2,4-cis |
| I - 21 | F | F | CH=CH₂ | H | Z-1 | Z-1 mit Konfiguration 2,4-cis |
| I - 22 | F | F | (R)-CH=CH₂ | CH₃ | Z-1 | Z-1 mit Konfiguration 2,4-cis |
| I - 23 | F | F | CH=CH₂ | CH₂CH₃ | Z-3 | |
| I - 24 | F | F | CH₃ | CH₂CH₃ | Z-3 | |
| I - 25 | F | F | CH₃ | CH₃ | Z-3 | |
| I - 26 | F | F | (S) - CH=CH₂ | CH₃ | Z-2 | |
| I - 27 | F | F | OCH₃ | CH₃ | Z-2 | |
| I - 28 | F | F | (R) - CH₃ | CH₃ | Z-2 | |
| I - 29 | Cl | Cl | OCH₃ | CH₃ | Z-2 | |
| I - 30 | F | H | CH₃ | CH₂CH₃ | Z-1 | Z-1 mit Konfiguration 2,4-cis |
| I - 31 | F | F | (S) - CH=CH₂ | H | Z-1 | Z-1 mit Konfiguration 2,4-cis |
| I - 32 | F | H | CH₃ | CH(CH₃)₂ | Z-1 | Z-1 mit Konfiguration 2,4-cis |
| I - 33 | F | H | CH₃ | C(CH₃)₃ | Z-1 | Z-1 mit Konfiguration 2,4-cis |
| I - 34 | F | H | CH₃ | CH₂Ph | Z-1 | Z-1 mit Konfiguration 2,4-cis |
| I - 35 | F | F | (S) - CH=CH₂ | CH₂CH₃ | Z-1 | Z-1 mit Konfiguration 2,4-cis |
| I - 36 | F | F | (S) - CH=CH₂ | CH(CH₃)₂ | Z-1 | Z-1 mit Konfiguration 2,4-cis |
| I - 37 | F | F | (S) - CH=CH₂ | CH₂Ph | Z-1 | Z-1 mit Konfiguration 2,4-cis |
| I - 38 | F | F | (S) - CH=CH₂ | C(CH₃)₃ | Z-1 | Z-1 mit Konfiguration 2,4-cis |
| I - 39 | F | H | CH₃ | CH₃ | Z-13 | (4S)-4-amino-4,5-dihydrofuran-2-carboxylate |
| I - 40 | F | H | CH₃ | CH₃ | Z-8 | (2S,4S)-4-amino-tetrahydrofuran-2-carboxylate |
| I - 41 | F | F | (R) - CH₃ | H | Z-1 | Z-1 mit Konfiguration 2,4-cis |
| I - 42 | F | F | (R) - CH₃ | C(CH₃)₃ | Z-1 | Z-1 mit Konfiguration 2,4-cis |
| I - 43 | F | F | (R) - CH₃ | CH₂CH₃ | Z-1 | Z-1 mit Konfiguration 2,4-cis |
| I - 44 | F | F | (R) - CH₃ | CH₂(CH₃)₂ | Z-1 | Z-1 mit Konfiguration 2,4-cis |
| I - 45 | F | F | (R) - CH₃ | CH₂Ph | Z-1 | Z-1 mit Konfiguration 2,4-cis |
| I - 46 | F | H | CH₃ | CH₃ | Z-13 | (4R)-4-amino-4,5-dihydrofuran-2-carboxylate |
| I - 47 | F | H | CH₃ | CH₃ | Z-8 | (2R,4R)-4-amino-tetrahydrofuran-2-carboxylate |
| I - 48 | F | F | (R) - CH₃ | CH₃ | Z-1 | Z-1 mit Konfiguration 2,4-cis |
| I - 49 | F | F | (R) - CF₂CH₃ | CH₃ | Z-1 | Z-1 mit Konfiguration 2,4-cis |
| I - 50 | F | F | (R) - CF₂CH₃ | H | Z-1 | Z-1 mit Konfiguration 2,4-cis |
| I - 51 | F | F | (R) - CF₂CH₃ | CH(CH₃)₂ | Z-1 | Z-1 mit Konfiguration 2,4-cis |
| I - 52 | F | F | (S) - CH=CH₂ | CH₂(3-F-Ph) | Z-1 | Z-1 mit Konfiguration 2,4-cis |
| I - 53 | F | F | (R) - CH₃ | CH₂(3-F-Ph) | Z-1 | Z-1 mit Konfiguration 2,4-cis |
| I - 54 | F | F | (S) - CH=CH₂ | CH₂(4-OMe-Ph) | Z-1 | Z-1 mit Konfiguration 2,4-cis |
| I - 55 | F | F | (R) - CH₃ | CH₃ | Z-1 | Z-1 mit Konfiguration 2,4-trans |
| I - 56 | F | F | (S) - CH=CH₂ | CH₃ | Z-1 | Z-1 mit Konfiguration 2,4-trans |

Die erfindungsgemäßen Verbindungen können nach verschiedenen Verfahren hergestellt werden, die im Folgenden aufgeführt werden:

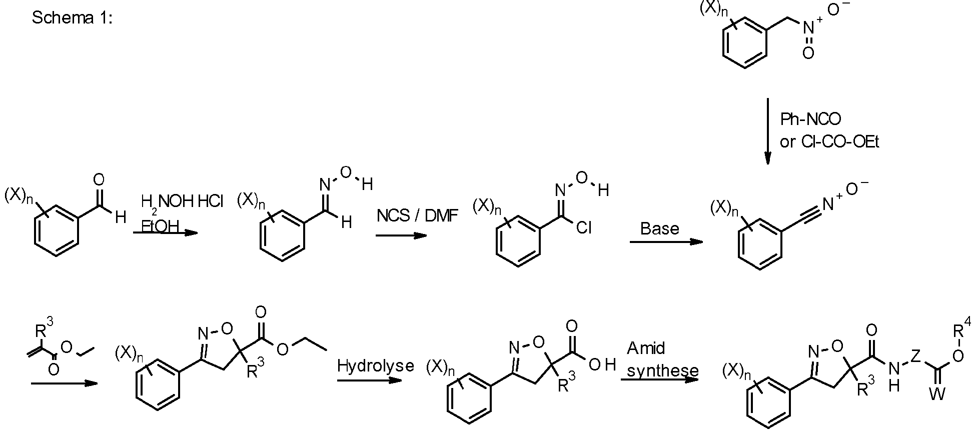

In Schema 1 und den nachfolgenden Schemata steht (X)ₙ für die Substituenten X², X³, X⁴, X⁵ und X⁶. Solche 1,3-dipolaren Cycloadditionen von Nitriloxiden mit geeigneten Dipolarophilen sind beispielsweise beschrieben in Reviews: 1,3 dipolar Cycloaddition Chemistry, Padwa, ed. Wiley, New York, 1984; Kanemasa and Tsuge, Heterocycles 1990, 30, 719. Zur Darstellung von Chloroximen siehe Kim, Jae N., Ryu, Eung K. J. Org. Chem. 1992, 57, 6649).

Erfindungsgemäße Verbindungen, die in der 4- und 5- Position des Isoxazolin-Ringsystems substituiert sind, lassen sich ebenfalls durch 1,3-dipolare Cycloaddition herstellen, indem geeignete 1,2-disubstituierte Olefine als Dipolarophile eingesetzt werden. Zumeist entstehen bei dieser Reaktion Diastereomerengemische, die durch Säulenchromatographie getrennt werden können. Optisch aktive Isoxazoline können durch chirale HPLC geeigneter Vorstufen oder Endstufen erhalten werden, ebenso auch durch enantioselektive Reakionen wie z.B. enzymatische Ester- oder Amidspaltungen oder durch den Einsatz von chiralen Hilfsreagenzien am Dipolarophil wie von Olssen beschrieben, (J. Org.Chem. 1988, 53, 2468).

Zur Herstellung der erfindungsgemäßen Verbindungen können auch geeignet substituierte 2-Alkoxyacrylsäureamide eingesetzt werden (Schema 3). Diese sind aus den in Schema 2 beschriebenen Acrylsäureestern nach Hydrolyse und Amidbildung zugänglich.

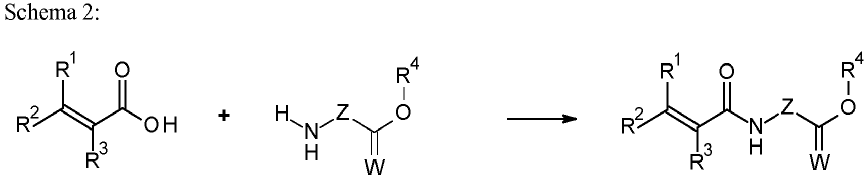

Zur Aktivierung der Acrylsäure bieten sich dabei Carbodiimide wie zum Beispiel EDCI an (Chen, F. M. F.; Benoiton, N. L. Synthesis 1979, 709). Zur Herstellung von Acrylsäureamiden siehe US 2,521,902, JP60112746, J. of Polymer Science 1979, 17 (6), 1655. Geeignet substituierte Acrylsäureamide können in einer 1,3-Cycloadditionsreaktion mit Nitriloxiden zu den erfindungsgemäßen Verbindungen umgesetzt werden (Schema 3).

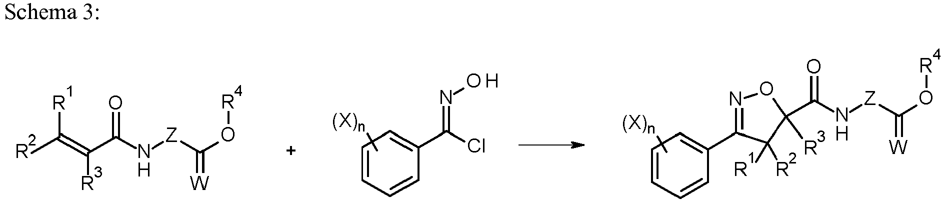

Transformationen der funktionellen Gruppen R³ sind sowohl auf der Stufe der Alkene als auch auf der Stufe der Isoxazoline möglich.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Die erfindungsgemäßen Verbindungen können in Nutzkulturen Selektivitäten aufweisen und können auch als nichtselektive Herbizide eingesetzt werden.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten in der Agrarindustrie verwendeten Wirkstoff, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften liegen in einer Toleranz oder Resistenz gegen abiotische Stressoren z.B. Hitze, Kälte, Trockenheit, Salz und ultraviolette Strahlung.

Bevorzugt ist die Anwendung der erfindungsgemäßen Verbindungen der Formel (I) oder deren Salze in wirtschaftlich bedeutenden transgenen Kulturen von Nutz-und Zierpflanzen,
Die Verbindungen der Formel (I) können als Herbizide in Nutzpflanzenkulturen eingesetzt werden, welche gegenüber den phytotoxischen Wirkungen der Herbizide resistent sind bzw. gentechnisch resistent gemacht wurden.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP 0221044, EP 0131624). Beschrieben wurden beispielsweise in mehreren Fällen gentechnische Veränderungen von Kulturpflanzen zwecks Modifikation der in den Pflanzen synthetisierten Stärke (z.B. WO 92/011376 A, WO 92/014827 A, WO 91/019806 A), transgene Kulturpflanzen, welche gegen bestimmte Herbizide vom Typ Glufosinate (vgl. z.B. EP 0242236 A, EP 0242246 A) oder Glyphosate (WO 92/000377 A) oder der Sulfonylharnstoffe (EP 0257993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum™ GAT™ (Glyphosate ALS Tolerant).
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924 A, EP 0193259 A).
- transgene Kulturpflanzen mit modifizierter Fettsäurezusammensetzung (WO 91/013972 A).
- gentechnisch veränderte Kulturpflanzen mit neuen Inhalts- oder Sekundärstoffen z.B. neuen Phytoalexinen, die eine erhöhte Krankheitsresistenz verursachen (EP 0309862 A, EP 0464461 A)
- gentechnisch veränderte Pflanzen mit reduzierter Photorespiration, die höhere Erträge und höhere Stresstoleranz aufweisen (EP 0305398 A)
- transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z.B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z.B. I. Potrykus und G. Spangenberg (eds.) Gene Transfer to Plants, Springer Lab Manual (1995), Springer Verlag Berlin, Heidelberg, oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen. So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z.B. 2,4-D, Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, oder gegen beliebige Kombinationen dieser Wirkstoffe, resistent sind.

Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung OptimumTM GATTM (Glyphosate ALS Tolerant) eingesetzt werden.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxid-addukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen Wirkstoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II oder Protoporphyrinogen-Oxidase beruhen, einsetzbar, wie sie z.B. aus Weed Research 26 (1986) 441-445 oder "The Pesticide Manual", 16th edition, The British Crop Protection Council und the Royal Soc. of Chemistry, 2006 und dort zitierter Literatur beschrieben sind. Nachfolgend werden beispielhaft bekannte Herbizide oder Pflanzenwachstumsregulatoren genannt, die mit den erfindungsgemäßen Verbindungen kombiniert werden können, wobei diese Wirkstoffe entweder mit ihrem "common name" in der englischsprachigen Variante gemäß International Organization for Standardization (ISO) oder mit dem chemischen Namen bzw. mit der Codenummer bezeichnet sind. Dabei sind stets sämtliche Anwendungsformen wie beispielsweise Säuren, Salze, Ester sowie auch alle isomeren Formen wie Stereoisomere und optische Isomere umfaßt, auch wenn diese nicht explizit erwähnt sind.

Beispiele für solche herbiziden Mischungspartner sind:
Acetochlor, acifluorfen, acifluorfen-sodium, aclonifen, alachlor, allidochlor, alloxydim, alloxydimsodium, ametryn, amicarbazone, amidochlor, amidosulfuron, 4-amino-3-chloro-6-(4-chloro-2-fluoro-3-methylphenyl)-5-fluoropyridine-2-carboxylic acid, aminocyclopyrachlor, aminocyclopyrachlorpotassium, aminocyclopyrachlor-methyl, aminopyralid, amitrole, ammoniumsulfamate, anilofos, asulam, atrazine, azafenidin, azimsulfuron, beflubutamid, benazolin, benazolin-ethyl, benfluralin, benfuresate, bensulfuron, bensulfuron-methyl, bensulide, bentazone, benzobicyclon, benzofenap, bicyclopyron, bifenox, bilanafos, bilanafos-sodium, bispyribac, bispyribac-sodium, bromacil, bromobutide, bromofenoxim, bromoxynil, bromoxynil-butyrate, -potassium, -heptanoate und -octanoate, busoxinone, butachlor, butafenacil, butamifos, butenachlor, butralin, butroxydim, butylate, cafenstrole, carbetamide, carfentrazone, carfentrazone-ethyl, chloramben, chlorbromuron, chlorfenac, chlorfenacsodium, chlorfenprop, chlorflurenol, chlorflurenol-methyl, chloridazon, chlorimuron, chlorimuron-ethyl, chlorophthalim, chlorotoluron, chlorthal-dimethyl, chlorsulfuron, cinidon, cinidon-ethyl, cinmethylin, cinosulfuron, clacyfos, clethodim, clodinafop, clodinafop-propargyl, clomazone, clomeprop, clopyralid, cloransulam, cloransulam-methyl, cumyluron, cyanamide, cyanazine, cycloate, cyclopyrimorate, cyclosulfamuron, cycloxydim, cyhalofop, cyhalofop-butyl, cyprazine, 2,4-D, 2,4-D-butotyl, -butyl, - dimethylammonium, -diolamin, -ethyl, 2-ethylhexyl, -isobutyl, -isooctyl, -isopropylammonium, - potassium, -triisopropanolammonium und -trolamine, 2,4-DB, 2,4-DB-butyl, -dimethylammonium, isooctyl, -potassium und -sodium, daimuron (dymron), dalapon, dazomet, n-decanol, desmedipham, detosyl-pyrazolate (DTP), dicamba, dichlobenil, 2-(2,4-dichlorobenzyl)-4,4-dimethyl-1,2-oxazolidin-3-one, 2-(2,5-dichlorobenzyl)-4,4-dimethyl-1,2-oxazolidin-3-one, dichlorprop, dichlorprop-P, diclofop, diclofop-methyl, diclofop-P-methyl, diclosulam, difenzoquat, diflufenican, diflufenzopyr, diflufenzopyrsodium, dimefuron, dimepiperate, dimethachlor, dimethametryn, dimethenamid, dimethenamid-P, dimetrasulfuron, dinitramine, dinoterb, diphenamid, diquat, diquat-dibromid, dithiopyr, diuron, DNOC, endothal, EPTC, esprocarb, ethalfluralin, ethametsulfuron, ethametsulfuron-methyl, ethiozin, ethofumesate, ethoxyfen, ethoxyfen-ethyl, ethoxysulfuron, etobenzanid, F-9600, F-5231, i.e. N-[2-Chlor-4-fluor-5-[4-(3-fluorpropyl)-4,5-dihydro-5-oxo-1H-tetrazol-1-yl]-phenyl]-ethansulfonamid, F-7967, i.e. 3-[7-Chlor-5-fluor-2-(trifluormethyl)-1H-benzimidazol-4-yl]-1-methyl-6-(trifluormethyl)pyrimidin-2,4(1H,3H)-dion, fenoxaprop, fenoxaprop-P, fenoxaprop-ethyl, fenoxaprop-P-ethyl, fenoxasulfone, fenquinotrione, fentrazamide, flamprop, flamprop-M-isopropyl, flamprop-M-methyl, flazasulfuron, florasulam, fluazifop, fluazifop-P, fluazifop-butyl, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, flurenol, flurenol-butyl, - dimethylammonium und -methyl, fluoroglycofen, fluoroglycofen-ethyl, flupropanate, flupyrsulfuron, flupyrsulfuron-methyl-sodium, fluridone, flurochloridone, fluroxypyr, fluroxypyr-meptyl, flurtamone, fluthiacet, fluthiacet-methyl, fomesafen, fomesafen-sodium, foramsulfuron, fosamine, glufosinate, glufosinate-ammonium, glufosinate-P-sodium, glufosinate-P-ammonium, glufosinate-P-sodium, glyphosate, glyphosate-ammonium, -isopropylammonium, -diammonium, -dimethylammonium,-potassium, -sodium und -trimesium, H-9201, i.e. O-(2,4-Dimethyl-6-nitrophenyl)-O-ethyl-isopropylphosphoramidothioat, halauxifen, halauxifen-methyl, halosafen, halosulfuron, halosulfuronmethyl, haloxyfop, haloxyfop-P, haloxyfop-ethoxyethyl, haloxyfop-P-ethoxyethyl, haloxyfop-methyl, haloxyfop-P-methyl, hexazinone, HW-02, i.e. 1-(Dimethoxyphosphoryl)-ethyl-(2,4-dichlorphenoxy)acetat, imazamethabenz, Imazamethabenz-methyl, imazamox, imazamox-ammonium, imazapic, imazapic-ammonium, imazapyr, imazapyr-isopropylammonium, imazaquin, imazaquinammonium, imazethapyr, imazethapyr-immonium, imazosulfuron, indanofan, indaziflam, iodosulfuron, iodosulfuron-methyl-sodium, ioxynil, ioxynil-octanoate, -potassium und sodium, ipfencarbazone, isoproturon, isouron, isoxaben, isoxaflutole, karbutilate, KUH-043, i.e. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, ketospiradox, lactofen, lenacil, linuron, MCPA, MCPA-butotyl, -dimethylammonium, -2-ethylhexyl, - isopropylammonium, -potassium und -sodium, MCPB, MCPB-methyl, -ethyl und -sodium, mecoprop, mecoprop-sodium, und -butotyl, mecoprop-P, mecoprop-P-butotyl, -dimethylammonium, -2-ethylhexyl und -potassium, mefenacet, mefluidide, mesosulfuron, mesosulfuron-methyl, mesotrione, methabenzthiazuron, metam, metamifop, metamitron, metazachlor, metazosulfuron, methabenzthiazuron, methiopyrsulfuron, methiozolin, methyl isothiocyanate, metobromuron, metolachlor, S-metolachlor, metosulam, metoxuron, metribuzin, metsulfuron, metsulfuron-methyl, molinat, monolinuron, monosulfuron, monosulfuron-ester, MT-5950, i.e. N-[3-chlor-4-(1-methylethyl)-phenyl]-2-methylpentanamid, NGGC-011, napropamide, NC-310, i.e. 4-(2,4-Dichlorbenzoyl)-1-methyl-5-benzyloxypyrazol, neburon, nicosulfuron, nonanoic acid (Pelargonsäure), norflurazon, oleic acid (fatty acids), orbencarb, orthosulfamuron, oryzalin, oxadiargyl, oxadiazon, oxasulfuron, oxaziclomefon, oxyfluorfen, paraquat, paraquat dichloride, pebulate, pendimethalin, penoxsulam, pentachlorphenol, pentoxazone, pethoxamid, petroleum oils, phenmedipham, picloram, picolinafen, pinoxaden, piperophos, pretilachlor, primisulfuron, primisulfuron-methyl, prodiamine, profoxydim, prometon, prometryn, propachlor, propanil, propaquizafop, propazine, propham, propisochlor, propoxycarbazone, propoxycarbazone-sodium, propyrisulfuron, propyzamide, prosulfocarb, prosulfuron, pyraclonil, pyraflufen, pyraflufen-ethyl, pyrasulfotole, pyrazolynate (pyrazolate), pyrazosulfuron, pyrazosulfuronethyl, pyrazoxyfen, pyribambenz, pyribambenz-isopropyl, pyribambenz-propyl, pyribenzoxim, pyributicarb, pyridafol, pyridate, pyriftalid, pyriminobac, pyriminobac-methyl, pyrimisulfan, pyrithiobac, pyrithiobac-sodium, pyroxasulfone, pyroxsulam, quinclorac, quinmerac, quinoclamine, quizalofop, quizalofop-ethyl, quizalofop-P, quizalofop-P-ethyl, quizalofop-P-tefuryl, rimsulfuron, saflufenacil, sethoxydim, siduron, simazine, simetryn, SL-261, sulcotrion, sulfentrazone, sulfometuron, sulfometuron-methyl, sulfosulfuron, , SYN-523, SYP-249, i.e. 1-Ethoxy-3-methyl-1-oxobut-3-en-2-yl-5-[2-chlor-4-(trifluormethyl)phenoxy]-2-nitrobenzoat, SYP-300, i.e. 1-[7-Fluor-3-oxo-4-(prop-2-in-1-yl)-3,4-dihydro-2H-1,4-benzoxazin-6-yl]-3-propyl-2-thioxoimidazolidin-4,5-dion, 2,3,6-TBA, TCA (Trifluoressigsäure), TCA-sodium, tebuthiuron, tefuryltrione, tembotrione, tepraloxydim, terbacil, terbucarb, terbumeton, terbuthylazin, terbutryn, thenylchlor, thiazopyr, thiencarbazone, thiencarbazonemethyl, thifensulfuron, thifensulfuron-methyl, thiobencarb, tiafenacil, tolpyralate, topramezone, tralkoxydim, triafamone, tri-allate, triasulfuron, triaziflam, tribenuron, tribenuron-methyl, triclopyr, trietazine, trifloxysulfuron, trifloxysulfuron-sodium, trifludimoxazin, trifluralin, triflusulfuron, triflusulfuron-methyl, tritosulfuron, urea sulfate, vernolate, XDE-848, ZJ-0862, i.e. 3,4-Dichlor-N-{2-[(4,6-dimethoxypyrimidin-2-yl)oxy]benzyl}anilin, sowie die folgenden Verbindungen:

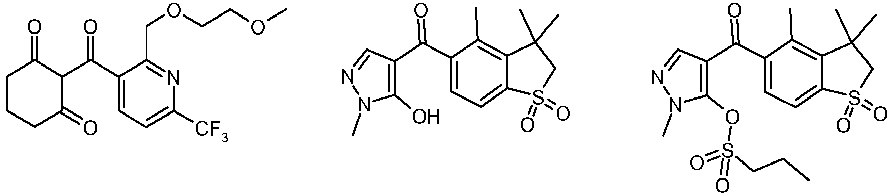

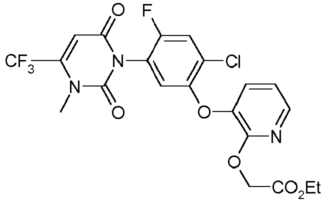

Beispiele für Pflanzenwachstumsregulatoren als mögliche Mischungspartner sind: Acibenzolar, acibenzolar-S-methyl, 5-Aminolävulinsäure, ancymidol, 6-benzylaminopurine, Brassinolid, Catechin, chlormequat chloride, cloprop, cyclanilide, 3-(Cycloprop-1-enyl)propionsäure, daminozide, dazomet, n-decanol, dikegulac, dikegulac-sodium, endothal, endothaldipotassium, -disodium, und mono(N,N-dimethylalkylammonium), ethephon, flumetralin, flurenol, flurenol-butyl, flurprimidol, forchlorfenuron, gibberellic acid, inabenfide, indol-3-acetic acid (IAA), 4-indol-3-ylbutyric acid, isoprothiolane, probenazole, Jasmonsäure, Jasmonsäuremethylester, maleic hydrazide, mepiquat chloride, 1-methylcyclopropene, 2-(1-naphthyl)acetamide, 1-naphthylacetic acid, 2- naphthyloxyacetic acid, nitrophenolate-mixture, 4-Oxo-4[(2-phenylethyl)amino]buttersäure, paclobutrazol, N-phenylphthalamic acid, prohexadione, prohexadione-calcium, prohydrojasmone, Salicylsäure, Strigolacton, tecnazene, thidiazuron, triacontanol, trinexapac, trinexapac-ethyl, tsitodef, uniconazole, uniconazole-P.

Safener, die in Kombination mit den erfindungsgemäßen Verbindungen der Formel (I) und ggf. in Kombinationen mit weiteren Wirkstoffen wie z.B. Insektiziden, Akariziden, Herbiziden, Fungiziden wie oben aufgelistet, eingesetzt werden können, sind vorzugsweise ausgewählt aus der Gruppe bestehend aus:
S1) Verbindungen der Formel (S1), 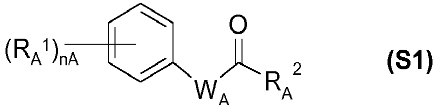 wobei die Symbole und Indizes folgende Bedeutungen haben:
   n_{A} ist eine natürliche Zahl von 0 bis 5, vorzugsweise 0 bis 3;
   R_{A}¹ ist Halogen, (C₁-C₄)Alkyl, (C₁-C₄)Alkoxy, Nitro oder (C₁-C₄)Haloalkyl;
   W_{A} ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist, vorzugsweise ein Rest aus der Gruppe (W_{A}¹) bis (W_{A}⁴), 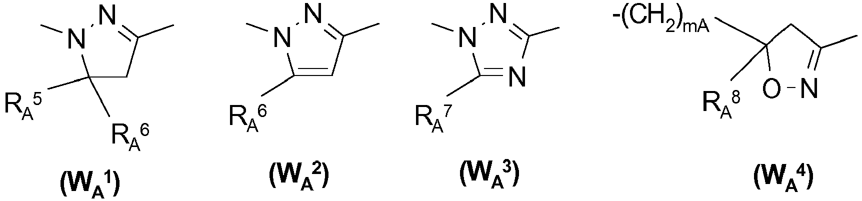
   m_{A} ist 0 oder 1;
   R_{A}² ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
   R_{A}³ ist Wasserstoff oder ein unsubstituierter oder substituierter aliphatischer Kohlenwasserstoffrest, vorzugsweise mit insgesamt 1 bis 18 C-Atomen;
   R_{A}⁴ ist Wasserstoff, (C₁-C₆)Alkyl, (C₁-C₆)Alkoxy oder substituiertes oder unsubstituiertes Phenyl;
   R_{A}⁵ ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
   R_{A}⁶, R_{A}⁷, R_{A}⁸ sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
   vorzugsweise:
   a) Verbindungen vom Typ der Dichlorphenylpyrazolin-3-carbonsäure (S1^{a}), vorzugsweise Verbindungen wie 1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäureethylester (S1-1) ("Mefenpyr-diethyl"), und verwandte Verbindungen, wie sie in der WO-A-91/07874 beschrieben sind;
   b) Derivate der Dichlorphenylpyrazolcarbonsäure (S1^{b}), vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4) und verwandte Verbindungen, wie sie in EP-A-333 131 und EP-A-269 806 beschrieben sind;
   c) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
   d) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-7), und verwandte Verbindungen wie sie in EP-A-174 562 und EP-A-346 620 beschrieben sind;
   e) Verbindungen vom Typ der 5-Benzyl- oder 5-Phenyl-2-isoxazolin-3- carbonsäure oder der 5,5-Diphenyl-2-isoxazolin-3-carbonsäure (S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-3-carbonsäure (S1-10) oder 5,5-Diphenyl-2-isoxazolin-3-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.
S2) Chinolinderivate der Formel (S2), 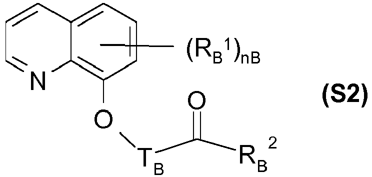 wobei die Symbole und Indizes folgende Bedeutungen haben:
   R_{B}¹ ist Halogen, (C₁-C₄)Alkyl, (C₁-C₄)Alkoxy, Nitro oder (C₁-C₄)Haloalkyl;
   n_{B} ist eine natürliche Zahl von 0 bis 5, vorzugsweise 0 bis 3;
   R_{B}² ist OR_{B}³, SR_{B}³ oder NR_{B}³R_{B}⁴ oder ein gesättigter
   oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
   R_{B}³ ist Wasserstoff oder ein unsubstituierter oder substituierter aliphatischer Kohlenwasserstoffrest, vorzugsweise mit insgesamt 1 bis 18 C-Atomen;
   R_{B}⁴ ist Wasserstoff, (C₁-C₆)Alkyl, (C₁-C₆)Alkoxy oder substituiertes oder unsubstituiertes Phenyl;
   T_{B} ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
   vorzugsweise:
   a) Verbindungen vom Typ der 8-Chinolinoxyessigsäure (S2^{a}), vorzugsweise (5-Chlor-8-chinolinoxy)essigsäure-(1-methylhexyl)ester ("Cloquintocet-mexyl") (S2-1), (5-Chlor-8-chinolinoxy)essigsäure-(1,3-dimethyl-but-1-yl)ester (S2-2), (5-Chlor-8-chinolinoxy)essigsäure-4-allyloxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium-Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
   b) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
S3) Verbindungen der Formel (S3) 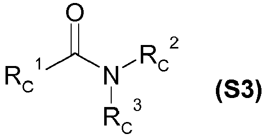 wobei die Symbole und Indizes folgende Bedeutungen haben:
   R_{C}¹ ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
   R_{C}², R_{C}³ sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
   vorzugsweise:
   Wirkstoffe vom Typ der Dichloracetamide, die häufig als Vorauflaufsafener (bodenwirksame Safener) angewendet werden, wie z. B.
   "Dichlormid" (N,N-Diallyl-2,2-dichloracetamid) (S3-1),
   "R-29148" (3-Dichloracetyl-2,2,5-trimethyl-1,3-oxazolidin) der Firma Stauffer (S3-2),
   "R-28725" (3-Dichloracetyl-2,2,-dimethyl-1,3-oxazolidin) der Firma Stauffer (S3-3),
   "Benoxacor" (4-Dichloracetyl-3,4-dihydro-3-methyl-2H-1,4-benzoxazin) (S3-4),
   "PPG-1292" (N-Allyl-N-[(1,3-dioxolan-2-yl)-methyl]-dichloracetamid) der Firma PPG Industries (S3-5),
   "DKA-24" (N-Allyl-N-[(allylaminocarbonyl)methyl]-dichloracetamid) der Firma Sagro-Chem (S3-6), "AD-67" oder "MON 4660" (3-Dichloracetyl-1-oxa-3-aza-spiro[4,5]decan) der Firma Nitrokemia bzw. Monsanto (S3-7),
   "TI-35" (1-Dichloracetyl-azepan) der Firma TRI-Chemical RT (S3-8),
   "Diclonon" (Dicyclonon) oder "BAS145138" oder "LAB145138" (S3-9) ((RS)-1-Dichloracetyl-3,3,8a-trimethylperhydropyrrolo[1,2-a]pyrimidin-6-on) der Firma BASF, "Furilazol" oder "MON 13900" ((RS)-3-Dichloracetyl-5-(2-furyl)-2,2-dimethyloxazolidin) (S3-10); sowie dessen (R)-Isomer (S3-11).
S4) N-Acylsulfonamide der Formel (S4) und ihre Salze, 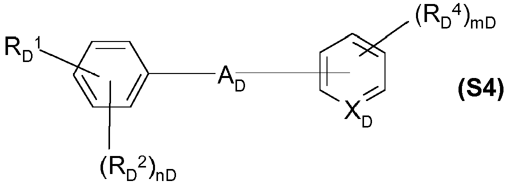 worin die Symbole und Indizes folgende Bedeutungen haben:
   A_{D} ist SO₂-NR_{D}³-CO oder CO-NR_{D}³-SO₂
   X_{D} ist CH oder N;
   R_{D}¹ ist CO-NR_{D}⁵R_{D}⁶ oder NHCO-R_{D}⁷;
   R_{D}² ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
   R_{D}³ ist Wasserstoff, (C₁-C₄)Alkyl, (C₂-C₄)Alkenyl oder (C₂-C₄)Alkinyl;
   R_{D}⁴ ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;
   R_{D}⁵ ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend v_{D} Heteroatome aus der Gruppe Stickstoff, Sauerstoff und Schwefel, wobei die sieben letztgenannten Reste durch v_{D} Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
   R_{D}⁶ ist Wasserstoff, (C₁-C₆)Alkyl, (C₂-C₆)Alkenyl oder (C₂-C₆)Alkinyl, wobei die drei letztgenannten Reste durch v_{D} Reste aus der Gruppe Halogen, Hydroxy, (C₁-C₄)Alkyl, (C₁-C₄)Alkoxy und (C₁-C₄)Alkylthio substituiert sind, oder
   R_{D}⁵ und R_{D}⁶ gemeinsam mit dem dem sie tragenden Stickstoffatom einen Pyrrolidinyl- oder Piperidinyl-Rest bilden;
   R_{D}⁷ ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   n_{D} ist 0, 1 oder 2;
   m_{D} ist 1 oder 2;
   v_{D} ist 0, 1, 2 oder 3;
   davon bevorzugt sind Verbindungen vom Typ der N-Acylsulfonamide, z.B. der nachfolgenden Formel (S4^{a}), die z. B. bekannt sind aus WO-A-97/45016 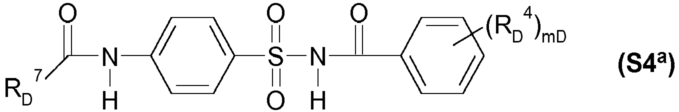
   worin
   R_{D}⁷ (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   R_{D}⁴ Halogen, (C₁-C₄)Alkyl, (C₁-C₄)Alkoxy, CF₃;
   m_{D} 1 oder 2;
   v_{D} ist 0, 1, 2 oder 3 bedeutet;
   sowie
   Acylsulfamoylbenzoesäureamide, z.B. der nachfolgenden Formel (S4^{b}), die z.B. bekannt sind aus WO-A-99/16744, 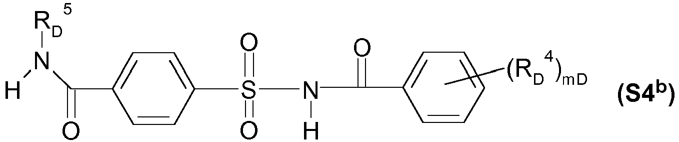
   z.B. solche worin
   R_{D}⁵ = Cyclopropyl und (R_{D}⁴) = 2-OMe ist("Cyprosulfamide", S4-1),
   R_{D}⁵ = Cyclopropyl und (R_{D}⁴) = 5-Cl-2-OMe ist (S4-2),
   R_{D}⁵ = Ethyl und (R_{D}⁴) = 2-OMe ist (S4-3),
   R_{D}⁵ = Isopropyl und (R_{D}⁴) = 5-Cl-2-OMe ist (S4-4) und
   R_{D}⁵ = Isopropyl und (R_{D}⁴) = 2-OMe ist (S4-5).
   sowie
   Verbindungen vom Typ der N-Acylsulfamoylphenylharnstoffe der Formel (S4^{c}), die z.B. bekannt sind aus der EP-A-365484, 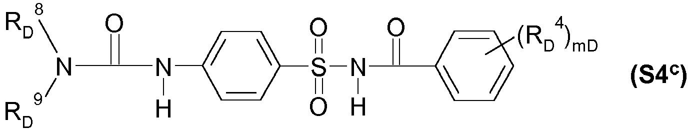
   worin
   R_{D}⁸ und R_{D}⁹ unabhängig voneinander Wasserstoff, (C₁-C₈)Alkyl, (C₃-C₈)Cycloalkyl, (C₃-C₆)Alkenyl, (C₃-C₆)Alkinyl,
   R_{D}⁴ Halogen, (C₁-C₄)Alkyl, (C₁-C₄)Alkoxy, CF₃
   m_{D} 1 oder 2 bedeutet;
      beispielsweise
      1-[4-(N-2-Methoxybenzoylsulfamoyl)phenyl]-3-methylharnstoff,
      1-[4-(N-2-Methoxybenzoylsulfamoyl)phenyl]-3,3-dimethylharnstoff,
      1-[4-(N-4,5-Dimethylbenzoylsulfamoyl)phenyl]-3-methylharnstoff,
      sowie
      N-Phenylsulfonylterephthalamide der Formel (S4^{d}), die z.B. bekannt sind aus CN 101838227, 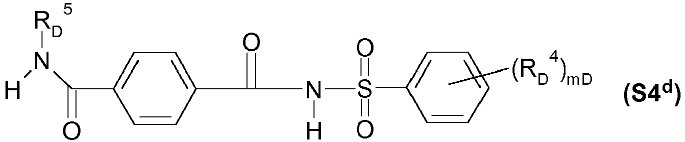 z.B. solche worin
   R_{D}⁴ Halogen, (C₁-C₄)Alkyl, (C₁-C₄)Alkoxy, CF₃;
   m_{D} 1 oder 2;
   R_{D}⁵ Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl bedeutet.
S5) Wirkstoffe aus der Klasse der Hydroxyaromaten und der aromatisch-aliphatischen Carbonsäurederivate (S5), z.B.
   3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-(2-Methylsulfonylaminoethyl)-3-(2-thienyl)-1,2-dihydro-chinoxalin-2-on, wie sie in der WO-A-2005/112630 beschrieben sind.
S7) Verbindungen der Formel (S7),wie sie in der WO-A-1998/38856 beschrieben sind 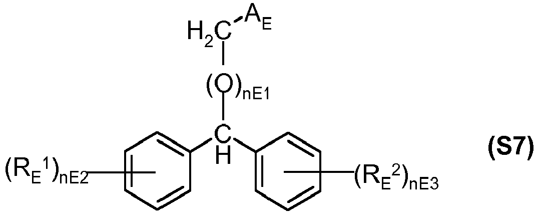 worin die Symbole und Indizes folgende Bedeutungen haben:
   R_{E}¹, R_{E}² sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
   A_{E} ist COOR_{E}³ oder COSR_{E}⁴
   R_{E}³, R_{E}⁴ sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
   n_{E}¹ ist 0 oder 1
   n_{E}², n_{E}³ sind unabhängig voneinander 0, 1 oder 2,
   vorzugsweise:
   Diphenylmethoxyessigsäure,
   Diphenylmethoxyessigsäureethylester,
   Diphenylmethoxyessigsäuremethylester (CAS-Reg.Nr. 41858-19-9) (S7-1).
S8) Verbindungen der Formel (S8),wie sie in der WO-A-98/27049 beschrieben sind 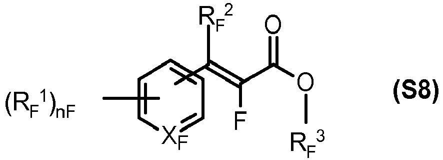 worin
   X_{F} CH oder N,
   n_{F} für den Fall, dass X_{F}=N ist, eine ganze Zahl von 0 bis 4 und
   für den Fall, dass X_{F}=CH ist, eine ganze Zahl von 0 bis 5 ,
   R_{F}¹ Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,
   R_{F}² Wasserstoff oder (C₁-C₄)Alkyl
   R_{F}³ Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; bedeuten, oder deren Salze,
   vorzugsweise Verbindungen worin
   X_{F} CH,
   n_{F} eine ganze Zahl von 0 bis 2 ,
   R_{F}¹ Halogen, (C₁-C₄)Alkyl, (C₁-C₄)Haloalkyl, (C₁-C₄)Alkoxy, (C₁-C₄)Haloalkoxy,
   R_{F}² Wasserstoff oder (C₁-C₄)Alkyl,
   R_{F}³ Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten,
   oder deren Salze.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
S10) Verbindungen der Formeln (S10^{a}) oder (S10^{b})
   wie sie in der WO-A-2007/023719 und WO-A-2007/023764 beschrieben sind 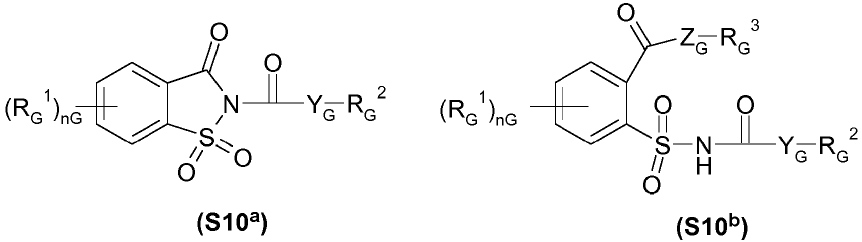 worin
   - R_{G}¹: Halogen, (C₁-C₄)Alkyl, Methoxy, Nitro, Cyano, CF₃, OCF₃
   - Y_{G}, Z_{G}: unabhängig voneinander O oder S,
   - n_{G}: eine ganze Zahl von 0 bis 4,
   - R_{G}²: (C₁-C₁₆)Alkyl, (C₂-C₆)Alkenyl, (C₃-C₆)Cycloalkyl, Aryl; Benzyl, Halogenbenzyl,
   - R_{G}³: Wasserstoff oder (C₁-C₆)Alkyl bedeutet.
S11) Wirkstoffe vom Typ der Oxyimino-Verbindungen (S11), die als Saatbeizmittel bekannt sind, wie z. B.
   "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist,
   "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1H-2-benzothiopyran-4(3H)-yliden)methoxy]acetat (CAS-Reg.Nr. 205121-04-6) (S12-1) und verwandte Verbindungen aus WO-A-1998/13361.
S13) Eine oder mehrere Verbindungen aus Gruppe (S13):
   "Naphthalic anhydrid" (1,8-Naphthalindicarbonsäureanhydrid) (S13-1), das als Saatbeiz-Safener für Mais gegen Schäden von Thiocarbamatherbiziden bekannt ist,
   "Fenclorim" (4,6-Dichlor-2-phenylpyrimidin) (S13-2), das als Safener für Pretilachlor in gesätem Reis bekannt ist,
   "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
   "CL 304415" (CAS-Reg.Nr. 31541-57-8)
   (4-Carboxy-3,4-dihydro-2H-1-benzopyran-4-essigsäure) (S13-4) der Firma American Cyanamid, das als Safener für Mais gegen Schäden von Imidazolinonen bekannt ist,
   "MG 191" (CAS-Reg.Nr. 96420-72-3) (2-Dichlormethyl-2-methyl-1,3-dioxolan) (S13-5) der Firma Nitrokemia, das als Safener für Mais bekannt ist,
   "MG 838" (CAS-Reg.Nr. 133993-74-5)
   (2-propenyl l-oxa-4-azaspiro[4.5]decan-4-carbodithioat) (S13-6) der Firma Nitrokemia,
   "Disulfoton" (O,O-Diethyl S-2-ethylthioethyl phosphordithioat) (S13-7),
   "Dietholate" (O,O-Diethyl-O-phenylphosphorothioat) (S13-8),
   "Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen, wie z. B.
   "Dimepiperate" oder "MY 93" (*S*-1-Methyl-1-phenylethyl-piperidin-1-carbothioat), das als Safener für Reis gegen Schäden des Herbizids Molinate bekannt ist,
   "Daimuron" oder "SK 23" (1-(1-Methyl-1-phenylethyl)-3-p-tolyl-harnstoff), das als Safener für Reis gegen Schäden des Herbizids Imazosulfuron bekannt ist,
   "Cumyluron" = "JC 940" (3-(2-Chlorphenylmethyl)-1-(1-methyl-1-phenyl-ethyl)harnstoff, siehe JP-A-60087254), das als Safener für Reis gegen Schäden einiger Herbizide bekannt ist,
   "Methoxyphenon" oder "NK 049" (3,3'-Dimethyl-4-methoxy-benzophenon), das als Safener für Reis gegen Schäden einiger Herbizide bekannt ist,
   "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Verbindungen der Formel (S15) oder deren Tautomere 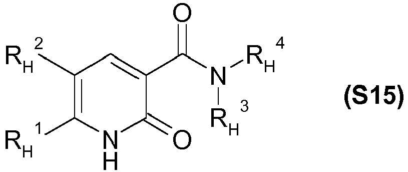 wie sie in der WO-A-2008/131861 und WO-A-2008/131860 beschrieben sind, worin
   R_{H}¹ einen (C₁-C₆)Haloalkylrest bedeutet und
   R_{H}² Wasserstoff oder Halogen bedeutet und
   R_{H}³, R_{H}⁴ unabhängig voneinander Wasserstoff, (C₁-C₁₆)Alkyl, (C₂-C₁₆)Alkenyl oder (C₂-C₁₆)Alkinyl,
   wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
   oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist,
   wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-_{C4})Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
   bedeutet oder
   R_{H}³ (C₁-C₄)-Alkoxy, (C₂-C₄)Alkenyloxy, (C₂-C₆)Alkinyloxy oder (C₂-C₄)Haloalkoxy bedeutet und
   R_{H}⁴ Wasserstoff oder (C₁-C₄)-Alkyl bedeutet oder
   R_{H}³ und R_{H}⁴ zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet.
S16) Wirkstoffe, die vorrangig als Herbizide eingesetzt werden, jedoch auch Safenerwirkung auf Kulturpflanzen aufweisen, z.B.
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy) buttersäure,
   3,6-Dichlor-2-methoxybenzoesäure (Dicamba),
   1-(Ethoxycarbonyl)ethyl-3,6-dichlor-2-methoxybenzoat (Lactidichlor-ethyl).

Besonders bevorzugte Safener sind Mefenpyr-diethyl, Cyprosulfamid, Isoxadifen-ethyl, Cloquintocetmexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolether-sulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepoly-glykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfett-säureester oder Polyoxethylensorbitanester wie z.B. Polyoxyethylensorbitan-fettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen. In Spritzpulvern beträgt die Wirkstoff-konzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasser-dispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Trägerstoff bedeutet eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste oder flüssige Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse, feste Düngemittel, Wasser, Alkohole, besonders Butanol, organische Solventien, Mineral- und Pflanzenöle sowie Derivate hiervon. Mischungen solcher Trägerstoffe können ebenfalls verwendet werden. Als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als verflüssigte gasförmige Streckmittel oder Trägerstoffe kommen solche Flüssigkeiten infrage, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabikum, Polyvinylalkohol, Polyvinylacetat, sowie natürliche Phospholipide, wie Kephaline und Lecithine, und synthetische Phospholipide. Weitere Additive können mineralische und vegetabile Öle sein. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Dichlormethan, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels. Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden. Im Allgemeinen enthalten die erfindungsgemäßen Mittel und Formulierungen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent. Die erfindungsgemäßen Wirkstoffe bzw. Mittel können als solche oder in Abhängigkeit von ihren jeweiligen physikalischen und/oder chemischen Eigenschaften in Form ihrer Formulierungen oder den daraus bereiteten Anwendungsformen, wie Aerosole, Kapselsuspensionen, Kaltnebelkonzentrate, Heißnebelkonzentrate, verkapselte Granulate, Feingranulate, fließfähige Konzentrate für die Behandlung von Saatgut, gebrauchsfertige Lösungen, verstäubbare Pulver, emulgierbare Konzentrate, Öl-in-Wasser-Emulsionen, Wasser-in-Öl-Emulsionen, Makrogranulate, Mikrogranulate, Öl dispergierbare Pulver, Öl mischbare fließfähige Konzentrate, Öl mischbare Flüssigkeiten, Schäume, Pasten, Pestizid ummanteltes Saatgut, Suspensionskonzentrate, Suspensions-Emulsions-Konzentrate, lösliche Konzentrate, Suspensionen, Spritzpulver, lösliche Pulver, Stäubemittel und Granulate, wasserlösliche Granulate oder Tabletten, wasserlösliche Pulver für Saatgut-behandlung, benetzbare Pulver, Wirkstoff-imprägnierte Natur- und synthetische Stoffe sowie Feinstverkapselungen in polymeren Stoffen und in Hüllmassen für Saatgut, sowie ULV-Kalt- und Warmnebel-Formulierungen eingesetzt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen. Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus oder Gliocladium stammen. Bevorzugt stammt dieses heterologe Gen aus Bacillus sp., wobei das Genprodukt eine Wirkung gegen den Maiszünsler (European corn borer) und/oder Western Corn Rootworm besitzt. Besonders bevorzugt stammt das heterologe Gen aus Bacillus thuringiensis. Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430, US 5,876,739, US 2003/0176428 A1, WO 2002/080675 A1, WO 2002/028186 A2.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser. Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Die erfindungsgemäß verwendbaren Beizmittel-Formulierungen können entweder direkt oder nach vorherigem Verdünnen mit Wasser zur Behandlung von Saatgut der verschiedensten Art, auch von Saatgut transgener Pflanzen, eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes. Sie können vorzugsweise als Pflanzenschutzmittel eingesetzt werden. Sie sind gegen normal sensible und resistente Arten sowie gegen alle oder einzelne Entwicklungsstadien wirksam.

Als Pflanzen, welche erfindungsgemäß behandelt werden können, seien folgende Hauptanbaupflanzen erwähnt: Mais, Sojabohne, Baumwolle, Brassica Ölsaaten wie Brassica napus (z.B. Canola), Brassica rapa, B. juncea (z.B. (Acker-)Senf) und Brassica carinata, Reis, Weizen Zuckerrübe, Zurckerrohr, Hafer, Roggen, Gerste, Hirse, Triticale, Flachs, Wein und verschiedene Früchte und Gemüse von verschiedenen botanischen Taxa wie z.B. Rosaceae sp. (beispielsweise Kernfrüchte wie Apfel und Birne, aber auch Steinfrüchte wie Aprikosen, Kirschen, Mandeln und Pfirsiche und Beerenfrüchte wie Erdbeeren), Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp. (beispielsweise Bananenbäume und -plantagen), Rubiaceae sp. (beispielsweise Kaffee), Theaceae sp., Sterculiceae sp., Rutaceae sp. (beispielsweise Zitronen, Organen und Grapefruit); Solanaceae sp. (beispielsweise Tomaten, Kartoffeln, Pfeffer, Auberginen), Liliaceae sp., Compositae sp. (beispielsweise Salat, Artischocke and Chicoree - einschließlich Wurzelchicoree, Endivie oder gemeinen Chicoree), Umbelliferae sp. (beispielsweise Karrotte, Petersilie, Stangensellerie und Knollensellerie), Cucurbitaceae sp. (beispielsweise Gurke - einschließlich Gewürzgurke, Kürbis, Wassermelone, Flaschenkürbis und Melonen), Alliaceae sp. (beispielsweise Lauch und Zwiebel), Cruciferae sp. (beispielsweise Weißkohl, Rotkohl, Brokkoli, Blumenkohl, Rosenkohl, Pak Choi, Kohlrabi, Radieschen, Meerrettich, Kresse und Chinakohl), Leguminosae sp. (beispielsweise Erdnüsse, Erbsen, und Bohnen - wie z.B. Stangenbohne und Ackerbohne), Chenopodiaceae sp. (beispielsweise Mangold, Futterrübe, Spinat, Rote Rübe), Malvaceae (beispielsweise Okra), Asparagaceae (beispielsweise Spargel); Nutzpflanzen und Zierpflanzen in Garten und Wald; sowie jeweils genetisch modifizierte Arten dieser Pflanzen.

Wie oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Mitochondriengenom der transformierten Pflanze dadurch neue oder verbesserte agronomische oder sonstige Eigenschaften verleiht, dass es ein interessierendes Protein oder Polypeptid exprimiert oder dass es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Beispiele für Nematoden-resistente Pflanzen sind z.B. folgenden US Patentanmeldungen beschrieben: 11/765,491, 11/765,494, 10/926,819, 10/782,020, 12/032,479, 10/783,417, 10/782,096, 11/657,964, 12/192,904, 11/396,808, 12/166,253, 12/166,239, 12/166,124, 12/166,209, 11/762,886, 12/364,335, 11/763,947, 12/252,453, 12/209,354, 12/491,396 und 12/497,221.

Pflanzen, die erfindungsgemäß behandelt werden können, sind Hybridpflanzen, die bereits die Eigenschaften der Heterosis bzw. des Hybrideffekts exprimieren, was im Allgemeinen zu höherem Ertrag, höherer Wüchsigkeit, besserer Gesundheit und besserer Resistenz gegen biotische und abiotische Stressfaktoren führt. Solche Pflanzen werden typischerweise dadurch erzeugt, dass man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d.h. mechanischem Entfernen der männlichen Geschlechtsorgane bzw. der männlichen Blüten), produziert werden; es ist jedoch üblicher, dass die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, dass die Pollenfertilität in Hybridpflanzen, die die für die Pollensterilität verantwortlichen genetischen Determinanten enthalten, völlig restoriert wird. Dies kann erreicht werden, indem sichergestellt wird, dass die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. Pflanzen können mit verschiedenen Methoden tolerant gegenüber Glyphosate gemacht werden. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium Salmonella typhimurium (Comai et al., 1983, Science 221, 370-371), das CP4-Gen des Bakteriums Agrobacterium sp. (Barry et al., 1992, Curr. Topics Plant Physiol. 7, 139-145), die Gene, die für eine EPSPS aus der Petunie (Shah et al., 1986, Science 233, 478-481), für eine EPSPS aus der Tomate (Gasser et al., 1988, J. Biol. Chem. 263, 4280-4289) oder für eine EPSPS aus Eleusine (WO 01/66704) kodieren. Es kann sich auch um eine mutierte EPSPS handeln. Glyphosate-tolerante Pflanzen können auch dadurch erhalten werden, dass man ein Gen exprimiert, das für ein Glyphosate-Oxidoreduktase-Enzym kodiert. Glyphosate-tolerante Pflanzen können auch dadurch erhalten werden, dass man ein Gen exprimiert, das für ein Glyphosateacetyltransferase-Enzym kodiert. Glyphosatetolerante Pflanzen können auch dadurch erhalten werden, dass man Pflanzen, die natürlich vorkommende Mutationen der oben erwähnten Gene enthalten, selektiert. Pflanzen, die EPSPS Gene, welche Glyphosate-Toleranz verleihen, exprimieren, sind beschrieben. Pflanzen, welche andere Gene, die Glyphosate-Toleranz verleihen, z.B. Decarboxylase-Gene, sind beschrieben.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, dass man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes oder chimäres HPPD-Enzym kodiert, transformiert werden, wie in WO 96/38567, WO 99/24585, WO 99/24586, WO 2009/144079, WO 2002/046387 oder US 6,768,044 beschrieben. Eine Toleranz gegenüber HPPD-Hemmern kann auch dadurch erzielt werden, dass man Pflanzen mit Genen transformiert, die für gewisse Enzyme kodieren, die die Bildung von Homogentisat trotz Hemmung des nativen HPPD-Enzyms durch den HPPD-Hemmer ermöglichen. Solche Pflanzen sind in WO 99/34008 und WO 02/36787 beschrieben. Die Toleranz von Pflanzen gegenüber HPPD-Hemmern kann auch dadurch verbessert werden, dass man Pflanzen zusätzlich zu einem Gen, das für ein HPPD-tolerantes Enzym kodiert, mit einem Gen transformiert, das für ein Prephenatdehydrogenase-Enzym kodiert, wie in WO 2004/024928 beschrieben ist. Außerdem können Pflanzen noch toleranter gegen HPPD-Hemmern gemacht werden, indem man ein Gen in ihr Genom einfügt, welches für ein Enzym kodiert, das HPPD-Hemmer metabolisiert oder abbaut, wie z.B. CYP450 Enzyme (siehe WO 2007/103567 und WO 2008/150473).

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, dass verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen wie z.B. in Tranel und Wright (Weed Science 2002, 50, 700-712) beschrieben ist. Die Herstellung von sulfonylharnstofftoleranten Pflanzen und imidazolinontoleranten Pflanzen ist beschrieben. Weitere sulfonylharnstoff- und imidazolinontolerante Pflanzen sind auch beschrieben.

Weitere Pflanzen, die gegenüber Imidazolinonen und/oder Sulfonylharnstoffen tolerant sind, können durch induzierte Mutagenese, Selektion in Zellkulturen in Gegenwart des Herbizids oder durch Mutationszüchtung erhalten werden (vgl. z.B. für Sojabohne US 5,084,082, für Reis WO 97/41218, für Zuckerrübe US 5,773,702 und WO 99/057965, für Salat US 5,198,599 oder für Sonnenblume WO 01/065922).

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Stressfaktoren tolerant. Solche Pflanzen können durch genetische Transformation oder durch Selektion von Pflanzen, die eine Mutation enthalten, die solch eine Stressresistenz verleiht, erhalten werden. Zu besonders nützlichen Pflanzen mit Stresstoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein stresstoleranzförderndes Transgen enthalten, das die Expression und/oder Aktivität der für PARG kodierenden Gene der Pflanzen oder Pflanzenzellen zu reduzieren vermag;
c. Pflanzen, die ein stresstoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.
Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so dass sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.
4) Transgene Pflanzen oder Hybridpflanzen wie Zwiebeln mit bestimmten Eigenschaften wie "hohem Anteil an löslichen Feststoffen" (,high soluble solids content'), geringe Schärfe (,low pungency', LP) und/oder lange Lagerfähigkeit (,long storage', LS).
Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten, wie Baumwollpflanzen mit einer erhöhten Expression der Saccharosephosphatsynthase;
c) Pflanzen wie Baumwollpflanzen mit einer erhöhten Expression der Saccharosesynthase;
d) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
e) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren. Pflanzen oder Pflanzensorten (die nach Methoden der pflanzlichen Biotechnologie, wie der Gentechnik, erhalten werden können), die ebenfalls erfindungsgemäß behandelt werden können, sind Pflanzen wie Kartoffeln, welche Virus-resistent sind z.B. gegen den Kartoffelvirus Y (Event SY230 und SY233 von Tecnoplant, Argentinien), oder welche resistent gegen Krankheiten wie die Kraut- und Knollenfäule (potato late blight) (z.B. RB Gen), oder welche eine verminderte kälteinduzierte Süße zeigen (welche die Gene Nt-Inh, II-INV tragen) oder welche den Zwerg-Phänotyp zeigen (Gen A-20 Oxidase). Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften im Samenausfall (seed shattering). Solche Pflanzen können durch genetische Transformation oder durch Selektion von Pflanzen, die eine Mutation enthalten, die solche veränderten Eigenschaften verleihen, und umfassen Pflanzen wie Raps mit verzögertem oder vermindertem Samenausfall.

Besonders nützliche transgene Pflanzen, die erfindungsgemäß behandelt werden können, sind Pflanzen mit Transformationsevents oder Kombinationen von Transformationsevent, welche in den USA beim Animal and Plant Health Inspection Service (APHIS) of the United States Department of Agriculture (USDA) Gegenstand von erteilten oder anhängigen Petitionen für den nicht-regulierten Status sind. Die Information hierzu ist jederzeit beim APHIS (4700 River Road Riverdale, MD 20737, USA) erhältlich, z.B. über die Internetseite http://www.aphis.usda.gov/brs/not_reg.html. Am Anmeldetag dieser Anmeldung waren beim APHIS die Petitionen mit folgenden Informationen entweder erteilt oder anhängig:
- Petition: Identifikationsnummer der Petition. Die Technische Beschreibung des Transformationsevents kann im einzelnen Petitionsdokument erhältlich von APHIS auf der Website über die Petitionsnummer gefunden werden. Diese Beschreibungen sind hiermit per Referenz offenbart.
- Erweiterung einer Petition: Referenz zu einer frühere Petition, für die eine Erweiterung oder Verlängerung beantragt wird.
- Institution: Name der die Petition einreichenden Person.
- Regulierter Artikel: die betroffen Pflanzenspecies.
- Transgener Phänotyp: die Eigenschaft ("Trait"), die der Pflanze durch das Transformationsevent verliehen wird.
- Transformationevent oder -linie: der Name des oder der Events (manchmal auch als Linie(n) bezeichnet), für die der nicht-regulierte Status beantragt ist.
- APHIS Documente: verschiedene Dokumente, die von APHIS bzgl. der Petition veröffentlicht warden oder von APHIS auf Anfrage erhalten werden können.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und
http://cera-gmc.org/index.php?evidcode=&hstIDXCode=&gType=&AbbrCode=&atCode=&stCode=&coID Code=&action=gm_crop_database&mode=Submit).

### A. Chemische Beispiele

Die NMR-Daten offenbarter Beispiele werden entweder in klassischer Form (δ-Werte, Anzahl der H-Atome, Multiplettaufspaltung) oder als sogenannte NMR-Peak-Listen aufgefuhrt. Beim NMR-Peak-Listenverfahren werden die NMR-Daten ausgewahlter Beispiele in Form von NMR-Peaklisten notiert, wobei zu jedem Signalpeak erst der δ-Wert in ppm und dann die Signalintensität durch ein Leerzeichen getrennt aufgeführt wird. Die δ-Wert-Signalintensitäts - Zahlenpaare von verschiedenen Signalpeaks werden durch Semikolons voneinander getrennt aufgelistet.

Die Peakliste eines Beispieles hat daher die Form:
δ₁ (Intensität₁); δ₂ (Intensität₂);.......,; δᵢ (Intensitätᵢ);......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von ¹H-NMR-Spektren wird Tetramethylsilan benutzt und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden.

Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von ¹H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu ¹H-NMR-Peaklisten können der Research Disclosure Database Number 564025 entnommen werden.

Die nachstehenden Beispiele erläutern die Erfindung näher.

### Intermediat 1

### Herstellung von 3,5-Difluor-N-hydroxybenzolcarboximidoylchlorid

Analog der Vorschrift in WO2012/130798 für 3,5-Dichlor-N-hydroxybenzolcarboximidoylchlorid wurde 3,5-Difluor-N-hydroxybenzolcarboximidoylchlorid ausgehend von 3,5-Difluorbenzaldehyd in zwei Stufen hergestellt.

### Intermediat 2

### Herstellung von 3-(3,5-Difluorphenyl)-5-methyl-4H-isoxazol-5-carbonsäuremethylester

Analog der Vorschrift in WO2012/130798 für 3-(3,5-Difluorphenyl)-5-methyl-4H-isoxazol-5-carbonsäuremethylester wurde 3-(3,5-Difluorphenyl)-5-methyl-4H-isoxazol-5-carbonsäuremethylester ausgehend von 3,5-Difluorbenzaldehyd in drei Stufen hergestellt.

### Intermediat 3

### Herstellung von 3-(3,5-Difluorphenyl)-5-methyl-4H-isoxazol-5-carbonsäure

Analog der Vorschrift in WO2012/130798 für 3-(3,5-Dichlorphenyl)-5-methyl-4H-isoxazol-5-carbonsäure wurde 3-(3,5-Difluorphenyl)-5-methyl-4H-isoxazol-5-carbonsäure durch Verseifung von 3-(3,5-Difluorphenyl)-5-methyl-4H-isoxazol-5-carbonsäuremethylester hergestellt.

### Intermediat 4

### Herstellung von 3-(3,5-Difluorphenyl)-5-methyl-4H-isoxazol-5-carbonsäurechlorid

Analog der Vorschrift in WO2012/130798 für N-tert-Butyl-3-(3,5-dichlorphenyl)-5-methyl-4, 5-dihydro-1,2-oxazol-5-carboxamid wurde aus 3-(3,5-Difluorphenyl)-5-methyl-4H-isoxazol-5-carbonsäure durch Umsetzung mit Oxalylchlorid 3-(3,5-Difluorphenyl)-5-methyl-4H-isoxazol-5-carbonsäurechlorid hergestellt und ohne weitere Reinigung als Rohprodukt eingesetzt.

### Intermediat 5

### Herstellung von 3-(3,5-Difluorphenyl)-5-(1-hydroxyethyl)-4H-isoxazol-5-carbonsäuremethylester

19.9 g (104 mmol) 3,5-Difluor-N-hydroxybenzimidoylchlorid (s. Intermediat 1) wurden in 330 ml 2-Propanol gelöst und mit 15.0 g (104 mmol) 3-Hydroxy-2-methylenbutansäuremethylester versetzt. Nach Zugabe von 43.8 g (522 mmol) Natriumhydrogencarbonat wurde die Suspension auf 50°C geheizt und die Temperatur für 2 h, bis zum vollständigen Umsatz des Ausgangsmaterials, gehalten. Die Suspension wurde filtriert und das Filtrat im Vakuum eingeengt. Der erhaltene Rückstand wurde in Dichlormethan aufgenommen, danach mit gesättigter Natriumchloridlösung gewaschen, die organische Phase mit Natriumsulfat getrocknet und nach Filtrieren im Vakuum eingedampft. Das so erhaltene rohe Produkt wurde in Toluol aufgenommen und durch Zugabe von n-Heptan zum Kristallisieren gebracht. Auf diese Weise erhielt man 25.5 g (86 %) 3-(3,5-Difluorphenyl)-5-(1-hydroxyethyl)-4H-isoxazol-5- carbonsäuremethylester in Form farbloser Kristalle.
Diastereomer 1: ¹H NMR (CDCl3): δ = 1.20 (d, 3H), 2.36 (d, 1H), 3.52 (d, 1H), 3.72 (d, 1H), 3.83 (s, 3H), 4.34 (m, 1H), 6.88 (m, 1H), 7.20 (m, 2H).
Diastereomer 2: ¹H NMR (CDCl3): δ = 1.29 (d, 3H), 2.12 (d, 1H), 3.58 (d, 1H), 3.68 (d, 1H), 3.83 (s, 3H), 4.23 (m, 1H), 6.88 (m, 1H), 7.20 (m, 2H).

### Intermediat 6

### Herstellung von 3-(3,5-Difluorphenyl)-5-[1-(trifluormethylsulfonyloxy)ethyl]-4H-isoxazol-5-carbonsäuremethylester

29.9 (105 mmol) 3-(3,5-Difluorphenyl)-5-(1-hydroxyethyl)-4H-isoxazol-5-carbonsäuremethylester in 660 ml Dichlormethan wurden auf 0°C gekühlt und mit 16.3 g (210 mmol) Pyridin versetzt. Dann wurde langsam eine Lösung aus 38.6 g (137 mmol) Trifluormethansulfonsäureanhydrid in 80 ml Dichloromethan hinzugefügt. Nach 30 Minuten bei 0°C wurde mit 300 ml Dichlormethan versetzt und die organische Phase dreimal mit je 200 ml einer Lösung aus gesättigter Natriumchloridlösung und 1 N Salzsäure (3:1) gewaschen. Danach wurde die organische Phase zweimal mit gesättigter Natriumchloridlösung gewaschen, mit Natriumsulfat getrocknet und das Lösungsmittel im Vakuum entfernt. Das so erhaltene Rohprodukt wurde ohne weitere Reinigung in die nächste Stufe eingesetzt.
Diastereomer 1: ¹H NMR (CDCl3): δ = 1.54 (d, 3H), 3.44 (d, 1H), 3.89 (s, 3H), 3.94 (d, 1H), 5.49 (q, 1H), 6.91 (m, 1H), 7.20 (m, 2H).
Diastereomer 2: ¹H NMR (CDCl3): δ = 1.59 (d, 3H), 3.53 (d, 1H), 3.89 (s, 3H), 3.90 (d, 1H), 5.57 (q, 1H), 6.91 (m, 1H), 7.20 (m, 2H).

### Intermediat 7

### Herstellung von 3-(3,5-Difluorphenyl)-5-vinyl-4H-isoxazol-5-carbonsäuremethylester

43.0 g (103 mmol) Rohes Produkt der letzten Stufe (3-(3,5-Difluorphenyl)-5-[1-(trifluormethylsulfonyloxy)ethyl]-4H-isoxazol-5-carbonsäuremethylester) wurden in 500 ml Dimethylacetamid gelöst und innerhalb von 20 Minuten tropfenweise mit einer Lösung aus 18.8 g (124 mmol) DBU in 50 ml Dimethylacetamid versetzt. Die Reaktionsmischung wurde bei Raumtemperatur 2 h gerührt, dann auf 11 eisgekühlte 2 N Salzsäure gegeben und zweimal mit je 500 ml Diethylether extrahiert. Die vereinigten organischen Phasen wurden mit Natriumsulfat getrocknet, filtriert und im Vakuum eingedampft. Nach chromatographischer Reinigung an Kieselgel mit Dichlormethan als Eluent wurde das Rohprodukt aus Cyclohexan kristalllisiert. Auf diese Weise erhielt man 23.4 g (85 %) farblose Kristalle.
¹H NMR (CDCl3): δ = 3.34 (d, 1H), 3.84 (s, 3H),3.93 (d, 1H), 5.38 (d, 1H), 5.55 (d, 1H), 6.14 (dd, 1H), 6.88 (m, 1H), 7.19 (m, 2H).

### Intermediat 8

### Herstellung von 3-(3,5-Difluorphenyl)-5-vinyl-4H-isoxazol-5-carbonsäure

7.5 g (28.0 mmol) 3-(3,5-Difluorphenyl)-5-vinyl-4H-isoxazol-5-carbonsäuremethylester wurden mit 21 ml 2 N Natronlauge versetzt und 8 h zum Rückfluss erhitzt. Nach dem Abkühlen wurde die Reaktionsmischung mit Ethylacetat gewaschen, die wässrige Phase mit 2 N Salzsäure auf pH 1 angesäuert, der farblose Niederschlag abfiltriert und an der Luft getrocknet. Die Ausbeute betrug 6.8 g, (96 %).
¹H NMR (CDCl3): δ = 3.40 (d, 1H), 3.92 (d, 1H), 5,00 (dd, 1H), 5.45 (d, 1H), 5.63 (d, 1H), 6.16 (dd, 1H), 6.87-6.93 (m, 1H), 7.16-7.21 (m, 2H).

### Intermediat 9

### Herstellung von 3-(3,5-Difluorphenyl)-5-vinyl-4H-isoxazol-5-carbonsäurechlorid

2.70 g (10.6 mmol) 3-(3,5-Difluorphenyl)-5-vinyl-4H-isoxazol-5-carbonsäure wurden in 45 ml Dichlormethan gegeben, dann drei Tropfen Dimethylformamid (DMF) und anschließend 2.03 g (15.9 mmol) Oxalylchlorid hinzugefügt. Eine lebhafte Gasentwicklung war sichtbar. Die Mischung wurde 6 h bei Raumtemperatur gerührt und dann das Lösungsmittel und überschüssiges Oxallylchlorid im Vakuum abgedampft. Das so erhaltene Rohprodukt wurde ohne weitere Reinigung in die nächste Stufe eingesetzt.

### Intermediat 10

### Herstellung von 3-(3,5-Dichlorphenyl)-5-methoxy-4H-isoxazol-5-carbonsäurechlorid

282 mg (0.97 mmol) 3-(3,5-Dichlorphenyl)-5-methoxy-4H-isoxazol-5-carbonsäure wurden in 10 ml Dichlormethan gegeben, dann drei Tropfen Dimethylformamid (DMF) und anschließend 185 mg (1.85 mmol) Oxalylchlorid glöst in wenig Dichlormethan hinzugefügt. Die Mischung wurde 6 h bei Raumtemperatur gerührt und das Reaktionsgemisch dann im Vakuum eingedampft. Danach wurde der Rückstand noch zweimal mit Dichlormethan versetzt und im Vakuum eingedampft. Das so erhaltene Rohprodukt wurde ohne weitere Reinigung in die nächste Stufe eingesetzt.

### Intermediat 11

### Herstellung von 4-Amino-2,5-dihydrofuran-3-carbonsäuremethylester

4-Amino-2,5-dihydrofuran-3-carbonsäuremethylester kann analog der von F. Cohen et al., J. Med. Chem. 2011, 54, 3426-3435 beschriebenen Methode hergestellt werden.

### Intermediat 12

### Herstellung von 4-Amino-2,5-dihydrofuran-3-carbonsäureethylester

4-Amino-2,5-dihydrofuran-3-carbonsäureethylester kann analog der von F. Cohen et al., J. Med. Chem. 2011, 54, 3426-3435 beschriebenen Methode hergestellt werden.

### Intermediat 13

### Herstellung von 4-Aminotetrahydrofuran-3-carbonsäuremethylester

4-Aminotetrahydrofuran-3-carbonsäuremethylester kann nach der von G.R. Ott et al.; Bioorg. Med. Chem. Lett. 2008, 694-699 beschriebenen Methode hergestellt werden.

### Intermediat 14

### Herstellung von Cis-4-Aminotetrahydrofuran-2-carbonsäuremethylester Hydrochlorid

Cis-4-Aminotetrahydrofuran-2-carbonsäuremethylester Hydrochlorid kann nach der von D.P. Walker et al., Synthesis 2011, 1113-1119 beschriebenen Methode hergestellt werden.

### Intermediat 15

### Herstellung von Tert-Butyl-N,N'-diisopropylcarbamimidat

2.33 g tert-Butanol und 3.44 g N-N'-Diisopropylcarbodiimid wurden in einem ausgeheizten Kolben unter Stickstoff mit 27 mg wasserfreiem Kupfer-(I)-chlorid versetzt, bei Raumtemperatur 2 h lang gerührt und drei Tage lang stehen gelassen.
Dieses Rohprodukt wurde so für die Veresterungen zum tert-Butylester verwendet und über einen Spritzenfilter zudosiert.

### Intermediat 16

### Herstellung von Trans-4-Aminotetrahydrofuran-2-carbonsäuremethylester Hydrochlorid

Trans-4-Aminotetrahydrofuran-2-carbonsäuremethylester Hydrochlorid kann entweder aus der kommerziell verfügbaren Trans-aminocarbonsäure durch Veresterung mittels Thionylchlorid/Methanol oder nach der von Allan, Robin D.; Tran, Hue W. Australian Journal of Chemistry (1984), 37(5), 1123-6 beschriebenen Methode aus kommerziell erhältlichem 2,5-Anhydro-3-desoxypentonsäure hergestellt werden.

### Intermediat 17

### Herstellung von (4S)-4-Amino-4,5-dihydrofuran-2-carbonsäuremethylester

(4S)-4-Amino-4,5-dihydrofuran-2-carbonsäuremethylester kann nach der von Joseph P. Burkhart, Gene W. Holbert, Brian W. Metcalf, Tetrahedron Lett., 25 (46), 1984, pp. 5267-5270 beschriebenen Methode aus kommerziell erhältlichem L-Glutaminsäuremethylester hergestellt werden.

### Intermediat 18

### Herstellung von (4R)-4-Amino-4,5-dihydrofuran-2-carbonsäuremethylester

(4R)-4-Amino-4,5-dihydrofuran-2-carbonsäuremethylester kann nach der von Joseph P. Burkhart, Gene W. Holbert, Brian W. Metcalf, Tetrahedron Lett., 25 (46), 1984, pp. 5267-5270 beschriebenen Methode aus kommerziell erhältlichem D-Glutaminsäuremethylester hergestellt werden.

### Beispiel I-01

### Herstellung von Cis-4-[[3-(3,5-Difluorphenyl)-5-methyl-4H-isoxazol-5-carbonyl] amino]tetrahydrofuran-2-carbonsäuremethylester

Zu 262 mg (1.44 mmol) Intermediat 14 in 5 ml Dichlormethan wurden 292 mg (2.88 mmol) Triethylamin gegeben, dann bei 0°C 250 mg (0.96 mmol) Carbonsäurechlorid (Intermediat 4) in 6 ml Dichlormethan hinzugefügt, 6 h unter Erwärmung auf Raumtemperatur gerührt und schließlich Wasser hinzugegeben. Die organische Phase wurde abgetrennt, mit Natriumsulfat getrocknet und im Vakuum eingedampft. Nach Chromatographie des Eindampfrückstandes an Kieselgel (Eluent n-Heptan / Ethylacetat) wurden 75 mg (21 %) cis-4-[[3-(3,5-Difluorphenyl)-5-methyl-4H-isoxazol-5-carbonyl]amino]tetrahydrofuran-2-carbonsäuremeethylester erhalten.

### Beispiel I-02

### Herstellung von Cis-4-[[3-(3,5-Dichlorphenyl)-5-methoxy-4H-isoxazol-5-carbonyl]amino]tetrahydrofuran-2-carbonsäuremethylester

Zu 249 mg (1.37 mmol) Intermediat 14 in 5 ml Dichlormethan wurden 277 mg (2.74 mmol) Triethylamin gegeben, dann bei 0°C 282 mg (0.91 mmol) 3-(3,5-Dichlorphenyl)-5-methoxy-4H-isoxazol-5-carbonsäurechlorid in 5 ml Dichlormethan hinzugefügt, 6 h unter Erwärmung auf Raumtemperatur gerührt und schließlich Wasser hinzugegeben. Die organische Phase wurde abgetrennt, mit Natriumsulfat getrocknet und im Vakuum eingedampft. Nach Chromatographie des Eindampfrückstandes an Kieselgel (Eluent n-Heptan / Ethylacetat) wurden 41 mg (11 %) cis-4-[[3-(3,5-Dichlorphenyl)-5-methoxy-4H-isoxazol-5-carbonyl]amino]tetrahydrofuran-2-carbonsäuremeethylester erhalten.

### Beispiel I-03

### Herstellung von Cis-4-[[3-(3,5-Dichlorphenyl)-5-vinyl-4H-isoxazol-5-carbonyl]amino]tetrahydrofuran-2- carbonsäuremethylester

Ausgehend von 3,5-Dichlorbenzaldehyd wurde zunächst in Analogie zur Herstellung von Intermediat 9 das entsprechende Carbonsäurechlorid hergestellt. Dieses wurde dann analog Beispiel I-02 zur Zielverbindung umgesetzt.

Auf diese Weise wurden aus 317 mg (1.74 mmol) Intermediat 14 und 400 mg (1.39 mmol) Carbonsäurechlorid 10 mg (2 %) cis-4-[[3-(3,5-Dichlorphenyl)-5-vinyl-4H-isoxazol-5-carbonyl]amino]-tetrahydrofuran-2-carbonsäuremethylester erhalten.

### Beispiel I-04

### Herstellung von Cis-4-[[3-(3-Brom-5-methyl-phenyl)-5-methyl-4H-isoxazol-5-carbonyl] amino]tetrahydrofuran-2- carbonsäuremethylester

Ausgehend von 3-Brom-5-methylbenzaldehyd wurde zunächst in Analogie zur Herstellung von Intermediat 4 das entsprechende Carbonsäurechlorid hergestellt. Dieses wurde dann analog Beispiel I-02 zur Zielverbindung umgesetzt.
Auf diese Weise wurden aus 129 mg (0.71 mmol) Intermediat 14 und 150 mg (0.47 mmol) Carbonsäurechlorid 22 mg (11 %) cis-4-[[3-(3-Brom-5-methyl-phenyl)-5-methyl-4H-isoxazol-5-carbonyl] amino]tetrahydrofuran-2- carbonsäuremethylester erhalten.

### Beispiel I-05

### Herstellung von Cis-4-[[3-(3,5-Difluorphenyl)-5-methoxy-4H-isoxazol-5-carbonyl]amino]tetrahydrofuran-2-carbonsäuremethylester

Ausgehend von 3,5-Difluorbenzaldehyd wurde zunächst in Analogie zur Herstellung von Intermediat 4 das entsprechende Carbonsäurechlorid hergestellt, welches dann analog Beispiel I-02 zur Zielverbindung umgesetzt wurde.
Auf diese Weise wurden aus 247 mg (1.36 mmol) Intermediat 14 und 250 mg (0.90 mmol) Carbonsäurechlorid 63 mg (18 %) cis-4-[[3-(3,5-Difluorphenyl)-5-methoxy-4H-isoxazol-5-carbonyl]amino]tetrahydrofuran-2-carbonsäuremethylester erhalten.

### Beispiel I-06

### Herstellung von Cis-4-[[3-(3,5-Difluorphenyl)-5-(trifluormethyl)-4H-isoxazol-5-carbonyl]amino]tetrahydrofuran-2-carbonsäuremethylester

In Analogie zur Herstellung von Intermediat 4 wurde zunächst das entsprechende Carbonsäurechlorid hergestellt, welches dann analog Beispiel I-02 zur Zielverbindung umgesetzt wurde.
Auf diese Weise wurden aus 184 mg (1.01 mmol) Intermediat 14 und 212 mg (0.67 mmol) Carbonsäurechlorid 39 mg (13 %) cis-4-[[3-(3,5-Difluorphenyl)-5-(trifluormethyl)-4H-isoxazol-5-carbonyl]amino]tetrahydrofuran-2-carbonsäuremethylester erhalten.

### Beispiel I-07

### Herstellung von Cis-4-[[3-(3-Fluorphenyl)-5-vinyl-4H-isoxazol-5-carbonyl]amino]tetrahydrofuran-2-carbonsäuremethylester

In Analogie zur Herstellung von Intermediat 9 wurde zunächst das entsprechende Carbonsäurechlorid hergestellt, welches analog Beispiel I-02 zur Zielverbindung umgesetzt wurde.
Auf diese Weise wurden aus 184 mg (1.01 mmol) Intermediat 14 und 212 mg (0.67 mmol) Carbonsäurechlorid 64 mg (26 %) cis-4-[[3-(3-Fluorphenyl)-5-vinyl-4H-isoxazol-5-carbonyl]amino]tetrahydrofuran-2-carbonsäuremethylester erhalten.

### Beispiel I-08

### Herstellung von Cis-4-[[3-(3-Brom-5-methyl-phenyl)-5-methoxy-4H-isoxazol-5-carbonyl] amino]tetrahydrofuran-2- carbonsäuremethylester

In Analogie zur Herstellung von Intermediat 4 wurde zunächst das entsprechende Carbonsäurechlorid hergestellt, welches analog Beispiel I-02 zur Zielverbindung umgesetzt wurde.
Auf diese Weise wurden aus 184 mg (1.01 mmol) Intermediat 14 und 212 mg (0.67 mmol) Carbonsäurechlorid 64 mg (26 %) cis-4-[[3-(3-Fluorphenyl)-5-vinyl-4H-isoxazol-5-carbonyl]amino]tetrahydrofuran-2-carbonsäuremethylester erhalten.

### Beispiel I-09

### Herstellung von Cis-4-[[(5R)-3-(3,5-Difluorphenyl)-5-methyl-4H-isoxazol-5-carbonyl] amino]tetrahydrofuran-2- carbonsäuremethylester

In Analogie zur Herstellung von Intermediat 4 wurde zunächst das entsprechende Carbonsäurechlorid hergestellt, welches analog Beispiel I-01 zur Zielverbindung umgesetzt wurde.
Auf diese Weise wurden aus 227 mg (1.24 mmol) Intermediat 14 und 216 mg (0.83 mmol) Carbonsäurechlorid 79 mg (25 %) cis-4-[[(5R)-3-(3,5-Difluorphenyl)-5-methyl-4H-isoxazol-5-carbonyl] amino]tetrahydrofuran-2- carbonsäuremethylester erhalten.
Das Gemisch der einzelnen Diastereomere konnte mittels HPLC in I-15 und I-16 aufgetrennt werden.

### Beispiel I-10

### Herstellung von Cis-4-[[(5S)-3-(3,5-Difluorphenyl)-5-vinyl-4H-isoxazol-5-carbonyl] amino]tetrahydrofuran-2- carbonsäuremethylester

In Analogie zur Herstellung von Intermediat 9 wurde zunächst das entsprechende Carbonsäurechlorid hergestellt, welches analog Beispiel I-01 zur Zielverbindung umgesetzt wurde.
Auf diese Weise wurden aus 246 mg (1.35 mmol) Intermediat 14 und 228 mg (0.90 mmol) Carbonsäurechlorid 110 mg (31 %) cis-4-[[(5S)-3-(3,5-Difluorphenyl)-5-vinyl-4H-isoxazol-5-carbonyl]amino]tetrahydrofuran-2-carbonsäuremethylester erhalten.
Das Gemisch der einzelnen Diastereomere wurde mittels HPLC in I-11 und I-12 aufgetrennt.

### Beispiel I-11: Diastereomer 1 von I-10

### Herstellung von Cis-4-[[(5S)-3-(3,5-Difluorphenyl)-5-vinyl-4H-isoxazol-5-carbonyl]amino]tetrahydrofuran-2-carbonsäuremethylester

H-NMR Daten siehe Tabelle zu den analytischen Daten.

### Beispiel I-12: Diastereomer 2 von I-10

### Herstellung von Cis-4-[[(5S)-3-(3,5-Difluorphenyl)-5-vinyl-4H-isoxazol-5-carbonyl] amino]tetrahydrofuran-2- carbonsäuremethylester

H-NMR Daten siehe Tabelle zu den analytischen Daten.

### Beispiel I-13

### Herstellung von Cis-4-[[3-(3-Fluorphenyl)-5-methyl-4H-isoxazol-5-carbonyl]amino]tetrahydrofuran-2-carbonsäuremethylester

In Analogie zur Herstellung von Intermediat 4 wurde zunächst das entsprechende Carbonsäurechlorid hergestellt, welches analog Beispiel I-01 zur Zielverbindung umgesetzt wurde.
Auf diese Weise wurden aus 169 mg (0.93 mmol) Intermediat 14 und 150 mg (0.62 mmol) Carbonsäurechlorid 171 mg (75 %) cis-4-[[3-(3-Fluorphenyl)-5-methyl-4H-isoxazol-5-carbonyl]amino]tetrahydrofuran-2-carbonsäuremethylester erhalten.

### Beispiel I-14

### Herstellung von Cis-4-[(5-Methoxy-3-phenyl-4H-isoxazol-5-carbonyl)amino]tetrahydrofuran-2-carbonsäuremethylester

In Analogie zur Herstellung von Intermediat 10 wurde zunächst das entsprechende Carbonsäurechlorid hergestellt, welches analog Beispiel I-03 zur Zielverbindung umgesetzt wurde.
Auf diese Weise wurden aus 247 mg (1.35 mmol) Intermediat 14 und 217 mg (0.90 mmol) Carbonsäurechlorid 75 mg (23 %) Cis-4-[(5-Methoxy-3-phenyl-4H-isoxazol-5-carbonyl)amino]tetrahydrofuran-2-carbonsäuremethylester erhalten.

### Beispiel I-15: Diastereomer 1 von I-09

### Herstellung von Cis-4-[[(5R)-3-(3,5-Difluorphenyl)-5-methyl-4H-isoxazol-5-carbonyl]amino]tetrahydrofuran-2-carbonsäuremethylester

H-NMR Daten siehe Tabelle zu den analytischen Daten.

### Beispiel I-16: Diastereomer 2 von I-09

### Cis-4-[[(5R)-3-(3,5-Difluorphenyl)-5-methyl-4H-isoxazol-5-carbonyl]amino]tetrahydrofuran-2-carbonsäuremethylester

H-NMR Daten siehe Tabelle zu den analytischen Daten.

### Beispiel I-17

### Herstellung von 3-[[3-(3,5-Difluorphenyl)-5-methyl-4H-isoxazol-5-carbonyl]amino]tetrahydrofuran-3-carbonsäuremethylester

Zunächst wurde das entsprechende Carbonsäurechlorid hergestellt, welches analog Beispiel I-01 zur Zielverbindung umgesetzt wurde.
Auf diese Weise wurden aus 1.57 g (8.66 mmol) 3-Aminotetrahydrofuran-3-carbonsäuremethylester - Hydrochlorid und 1.50 g (5.77 mmol) Carbonsäurechlorid 477 mg (22 %) 3-[[3-(3,5-Difluorphenyl)-5-methyl-4H-isoxazol-5-carbonyl]amino]tetrahydrofuran-3-carbonsäuremethylester erhalten.

### Beispiel 1-18

### Herstellung von 3-[[3-(3,5-Difluorphenyl)-5-methyl-4H-isoxazol-5-carbonyl]amino]tetrahydrofuran-3-carbonsäureethylester

Die Titelverbindung kann anaolog Beispiel I-17 aus hergestellt werden.

### Beispiel I-19

### Herstellung von 4-[[3-(3,5-Difluorphenyl)-5-methyl-4H-isoxazol-5-carbonyl]amino]tetrahydrofuran-3-carbonsäuremethylester

Zunächst wurde das entsprechende Carbonsäurechlorid hergestellt, welches analog Beispiel I-01 mit 4-Aminotetrahydrofuran-3-carbonsäuremethylester zur Zielverbindung umgesetzt wurde.
Auf diese Weise wurden aus 419 mg (8.66 mmol) 4-Aminotetrahydrofuran-3-carbonsäuremethylester und 500 mg (1.92 mmol) Carbonsäurechlorid 657 mg (91 %) 4-[[3-(3,5-Difluorphenyl)-5-methyl-4H-isoxazol-5-carbonyl]amino]tetrahydrofuran-3-carbonsäuremethylester erhalten.

### Beispiel I-20

### Herstellung von Cis-4-[[3-(3-Fluorphenyl)-5-methyl-4H-isoxazol-5-carbonyl]amino]tetrahydrofuran-2-carbonsäure

85 mg (0.24 mmol) Cis-4-[[3-(3-Fluorphenyl)-5-methyl-4H-isoxazol-5-carbonyl]amino]tetrahydrofuran-2-carbonsäuremethylester wurden mit 1 ml 2 N Salzsäure versetzt und 3 Tage bei Raumtemperatur belassen. Danach wurde die Reaktionsmischung am Rotationsverdampfer eingedampft. Auf diese Weise wurden 33 mg (40 %) cis-4-[[3-(3-Fluorphenyl)-5-methyl-4H-isoxazol-5-carbonyl] amino]tetrahydrofuran-2-carbonsäure erhalten.

### Beispiel I-21

### Herstellung von Cis-4-[[3-(3,5-Difluorphenyl)-5-vinyl-4H-isoxazol-5-carbonyl]amino]tetrahydrofuran-2-carbonsäure

650 mg (1.70 mmol) Cis-4-[[3-(3,5-Difluorphenyl)-5-vinyl-4H-isoxazol-5-carbonyl]amino]tetrahydrofuran-2-carbonsäuremethylester wurden in 10 ml Tetrahydrofuran gegeben, bei Raumtemperatur mit 0.64 ml einer 2 N Natronlauge versetzt und 6 h gerührt. Dann wurde mit konz. Salzsäure angesäuert, mit Ethylacetat extrahiert und die organische Phase mit Natriumsulfat getrocknet. Durch Eindampfen der organischen Phase erhielt man
530 mg (83 %) cis-4-[[3-(3,5-Difluorphenyl)-5-vinyl-4H-isoxazol-5-carbonyl]amino]tetrahydrofuran-2-carbonsäure.

### Beispiel I-22

### Herstellung von Cis-4-[[(5R)-3-(3,5-Difluorphenyl)-5-vinyl-4H-isoxazol-5-carbonyl]amino]tetrahydrofuran-2-carbonsäuremethylester

Die Titelverbindung wurde in Analogie zu Beispiel 1-01 aus 268 mg (0.98 mmol) des entsprechenden Carbonsäurechlorid und 215 mg (1.48 mmol) des Amins (Intermediat 14) hergestellt.
Auf diese Weise wurden 64 mg (17 %) cis-4-[[(5R)-3-(3,5-Difluorphenyl)-5-vinyl-4H-isoxazol-5-carbonyl]amino]tetrahydrofuran-2-carbonsäuremethylester erhalten.

### Beispiel I-23

### Herstellung von 4-[[3-(3,5-Difluorphenyl)-5-vinyl-4H-isoxazol-5-carbonyl]amino]-2,5-dihydrofuran-3-carbonsäureethylester

Zuerst wurde 4-Amino-2,5-dihydrofuran-3-carbonsäureethylester analog F. Cohen et al., J. Med. Chem. 2011, 54, 3426-3435 aus 4-Oxotetrahydrofurancarbonsäureethylester hergestellt, welcher dann mit dem entsprechenden Carbonsäurechlorid (Intermediat 9) analog Vorschrift I-01 umgesetzt wurde.
Auf diese Weise wurden aus 230 mg (0.84 mmol) Carbonsäurechlorid und 166 mg (1.05 mmol) Amin 52 mg (15 %) 4-[[3-(3,5-Difluorphenyl)-5-vinyl-4H-isoxazol-5-carbonyl]amino]-2,5-dihydrofuran-3-carbonsäureethylester erhalten.

### Beispiel I-24

### Herstellung von 4-[[3-(3,5-Difluorphenyl)-5-methyl-4H-isoxazol-5-carbonyl]amino]-2,5-dihydrofuran-3- carbonsäureethylester

Zuerst wurde 4-Amino-2,5-dihydrofuran-3-carbonsäureethylester analog F. Cohen et al., J. Med. Chem. 2011, 54, 3426-3435 aus 4-Oxotetrahydrofurancarbonsäureethylester hergestellt, welcher dann mit dem entsprechenden Carbonsäurechlorid (Intermediat 4) analog Vorschrift I-01 umgesetzt wurde.
Auf diese Weise wurden aus 220 mg (0.84 mmol) Carbonsäurechlorid und 166 mg (1.05 mmol) Amin 175 mg (53 %) 4-[[3-(3,5-Difluorphenyl)-5-methyl-4H-isoxazol-5-carbonyl]amino]-2,5-dihydrofuran-3-carbonsäureethylester erhalten.

### Beispiel I-25

### Herstellung von 4-[[3-(3,5-Difluorphenyl)-5-methyl-4H-isoxazol-5-carbonyl]amino]-2,5-dihydrofuran-3- carbonsäuremethylester

Zuerst wurde 4-Amino-2,5-dihydrofuran-3-carbonsäuremethylester analog F. Cohen et al., J. Med. Chem. 2011, 54, 3426-3435 aus 4-Oxotetrahydrofurancarbonsäuremethylester hergestellt, welcher dann mit dem entsprechenden Carbonsäurechlorid (Intermediat 4) analog Vorschrift I-01 umgesetzt wurde. Auf diese Weise wurden aus 200 mg (0.77 mmol) Carbonsäurechlorid und 165 mg (1.15 mmol) Amin 94 mg (33 %) 4-[[3-(3,5-Difluorphenyl)-5-methyl-4H-isoxazol-5-carbonyl]amino]-2,5-dihydrofuran-3-carbonsäuremethylester erhalten.

### Beispiel I-26

### Herstellung von 4-[[(5S)-3-(3,5-Difluorphenyl)-5-vinyl-4H-isoxazol-5-carbonyl]amino]tetrahydrofuran-3-carbonsäuremethylester

Zunächst wurde das entsprechende Carbonsäurechlorid hergestellt, welches analog Beispiel I-01 mit 4-Aminotetrahydrofuran-3-carbonsäuremethylester zur Zielverbindung umgesetzt wurde.
Auf diese Weise wurden aus 110 mg (0.40 mmol) Carbonsäurechlorid und 88 mg (0.60 mmol) cis-4-Aminotetrahydrofuran-3-carbonsäuremethylester 7 mg (4 %) 4-[[(5S)-3-(3,5-Difluorphenyl)-5-vinyl-4H-isoxazol-5-carbonyl]amino]tetrahydrofuran-3-carbonsäuremethylester erhalten.

### Beispiel I-27

### Herstellung von 4-[[3-(3,5-Difluorphenyl)-5-methoxy-4H-isoxazol-5-carbonyl]amino]tetrahydrofuran-3 -carbonsäuremethylester

Zunächst wurde das entsprechende Carbonsäurechlorid hergestellt, welches analog Beispiel I-02 mit 4-Aminotetrahydrofuran-3-carbonsäuremethylester zur Zielverbindung umgesetzt wurde.
Auf diese Weise wurden aus 112 mg (0.40 mmol) Carbonsäurechlorid und 88 mg (0.60 mmol) 4-Aminotetrahydrofuran-3-carbonsäuremethylester 28 mg (17 %) 4-[[3-(3,5-Difluorphenyl)-5-methoxy-4H-isoxazol-5-carbonyl]amino]tetrahydrofuran-3-carbonsäuremethylester erhalten.

### Beispiel I-28

### Herstellung von 4-[[(5R)-3-(3,5-Difluorphenyl)-5-methyl-4H-isoxazol-5-carbonyl]amino]tetrahydrofuran-3-carbonsäuremethylester

In Analogie zur Herstellung von Intermediat 4 wurde zunächst das entsprechende Carbonsäurechlorid hergestellt, welches nach Beispiel I-01 mit mit 4-Aminotetrahydrofuran-3-carbonsäuremethylester zur Zielverbindung umgesetzt wurde.
Auf diese Weise wurden aus 110 mg (0.42 mmol) Carbonsäurechlorid und 92 mg (0.63 mmol) 4-Aminotetrahydrofuran-3-carbonsäuremethylester 36 mg (23 %) 4-[[(5R)-3-(3,5-Difluorphenyl)-5-methyl-4H-isoxazol-5-carbonyl]amino]tetrahydrofuran-3-carbonsäuremethylester erhalten.

### Beispiel I-29

### Herstellung von 4-[[3-(3,5-Dichlorphenyl)-5-methoxy-4H-isoxazol-5-carbonyl]amino]tetrahydrofuran-3-carbonsäuremethylester

Zunächst wurde das entsprechende Carbonsäurechlorid (Intermediat 10) hergestellt, welches analog Beispiel I-02 mit 4-Aminotetrahydrofuran-3-carbonsäuremethylester zur Zielverbindung umgesetzt wurde.
Auf diese Weise wurden aus 110 mg (0.35 mmol) Carbonsäurechlorid und 78 mg (0.53 mmol) 4-Aminotetrahydrofuran-3-carbonsäuremethylester 80 mg (53 %) 4-[[3-(3,5-Dichlorphenyl)-5-methoxy-4H-isoxazol-5-carbonyl]amino]tetrahydrofuran-3-carbonsäuremethylester erhalten.

### Beispiel I-30

### Herstellung von Cis-4-[[(5RS)-3-(3-Fluorphenyl)-5-methyl-4H-isoxazol-5-carbonyl]amino]tetrahydrofuran-2-carbonsäureethylester

0.10 g Carbonsäure, 85 mg 1-(3-Dimethylaminopropyl)-3-ethylcarbodiimid hydrochlorid und 4 mg 4-Dimethylaminopyridin wurden in 5 ml Dichlormethan gelöst.
Daraufhin wurde unter Rühren bei Raumtemperatur 68 mg Ethanol zugegeben und das Reaktionsgemisch 8 h bei Raumtemperatur und dann über Nacht rühren gelassen.
Das Reaktionsgemisch wurde mit 0.5 M wässriger Salzsäure gewaschen, getrocknet und im Vakuum eingeengt. Das Rohprodukt wurde dann mittels Chromatographie gereinigt.
Man erhielt 55 mg Ethylester (49 % Ausbeute).

### Beispiel I-31

### Herstellung von Cis-4-[[(5S)-3-(3,5-Difluorphenyl)-5-vinyl-4H-isoxazol-5-carbonyl]amino]tetrahydrofuran-2-carbonsäure

0.52 g des Methylesters wurden in 8 ml Tetrahydrofuran gelöst und auf 0°C gekühlt. Zu dieser Lösung wurde eine Lösung von 98 mg Lithiumhydroxid in 4 ml Wasser tropfenweise unter Rühren zugegeben. Die Reaktionsmischung wurde unter Rühren innerhalb 1 h auf Raumtemperatur gebracht. Das Reaktionsgemisch wurde mit Wasser verdünnt, mit 0.5 M wässriger Salzsäure angesäuert und mit Ethylacetat extrahiert. Die so erhaltene Ethylacetat-Phase wurde dann getrocknet und im Vakuum eingeengt.
Das Rohprodukt wurde zur weiteren Reinigung in 2 M wässriger Natronlauge aufgenommen und mit Ethylacetat gewaschen. Die wässrige Phase wurde daraufhin mit 2 M wässriger Salzsäure angesäuert und mit Dichlormethan extrahiert. Die Dichlormethan-Phase wurde getrocknet und eingeengt. Man erhielt 0.53 g Carbonsäure (84 % Ausbeute).

### Beispiel I-32

### Herstellung von Cis-4-[[(5RS)-3-(3-Fluorphenyl)-5-methyl-4H-isoxazol-5-carbonyl]amino]tetrahydrofuran-2-carbonsäureisopropylester

0.12 g Carbonsäure, 98 mg 1-(3-Dimethylaminopropyl)-3-ethylcarbodiimid hydrochlorid und 4 mg 4-Dimethylaminopyridin wurden in 5 ml Dichlormethan gelöst.
Daraufhin wurde unter Rühren bei Raumtemperatur 0.41 g 2-Propanol zugegeben und das Reaktionsgemisch 1.5 h bei Raumtemperatur und dann drei Tage rühren gelassen.
Das Reaktionsgemisch wurde mit 0.5 M wässriger Salzsäure gewaschen, getrocknet und im Vakuum eingeengt. Das Rohprodukt wurde dann mittels Chromatographie gereinigt.
Man erhielt 82 mg 2-Propylester (61% Ausbeute).

### Beispiel I-33

### Herstellung von Cis-4-[[(5RS)-3-(3-Fluorphenyl)-5-methyl-4H-isoxazol-5-carbonyl]amino]tetrahydrofuran-2-carbonsäuretert.-butylester

0.11 g Carbonsäure wurde in 2 ml Tetrahydrofuran gelöst und unter Stickstoff gesetzt.
Durch einen Spritzenfilter wurden 0.2 ml tert-Butyl-N,N'-diisopropylcarbamimidat hinzugegeben. Die Reaktionsmischung wurde 3 h lang unter Rückfluss erhitzt, abgekühlt, und es wurden weitere 0.2 ml tert-Butyl-N,N'-diisopropylcarbamimidat hinzugefügt. Das Reaktionsgemisch wurde weitere 2 h refluxiert und anschließend auf Raumtemperatur abgekühlt.
Die Reaktionsmischung wurde dann mit Ethylacetat verdünnt und über Celite filtriert, wobei mit zusätzlichem Ethylacetat nachgespült wurde. Die vereinigten Filtrate wurden im Vakuum eingeengt und durch Säulenchromatographie gereinigt. Nach einer weiteren Reinigung via HPLC erhielt man 61 mg tert-Butylester (48 % Ausbeute).

### Beispiel I-34

### Herstellung von Cis-4-[[(5RS)-3-(3-Fluorphenyl)-5-methyl-4H-isoxazol-5-carbonyl] amino]tetrahydrofuran-2-carbonsäurebenzylester

0,10 g Carbonsäure, 89 mg 1-(3-Dimethylaminopropyl)-3-ethylcarbodiimid hydrochlorid und 4 mg 4-Dimethylaminopyridin wurden in 5 ml Dichlormethan gelöst.
Daraufhin wurde unter Rühren bei Raumtemperatur 0.67 g Benzylalkohol zugegeben und das Reaktionsgemisch 1.5 h bei Raumtemperatur und dann drei Tage rühren gelassen.
Das Reaktionsgemisch wurde mit 0.5 M wässriger Salzsäure gewaschen, getrocknet und im Vakuum eingeengt. Das Rohprodukt wurde dann mittels Chromatographie gereinigt.
Man erhielt 84 mg (61 %) Benzylester.

### Beispiel I-35

### Herstellung von Cis-4-[[(5S)-3-(3,5-Difluorphenyl)-5-vinyl-4H-isoxazol-5-carbonyl]amino]tetrahydrofuran-2-carbonsäureethylester

0.12 g Carbonsäure und 8 mg 4-Dimethylaminopyridin wurden in 5ml Dichlormethan gelöst. Daraufhin wurde unter Rühren bei Raumtemperatur 92 mg 1-(3-Dimethylaminopropyl)-3-ethylcarbodiimid hydrochlorid gefolgt von 0.22 g Ethanol zugegeben, und das Reaktionsgemisch wurde 1.5 h bei Raumtemperatur und dann 36h bei dieser Temperatur rühren gelassen.
Das Reaktionsgemisch wurde mit 0.5 M wässriger Salzsäure gewaschen, getrocknet und im Vakuum eingeengt. Das Rohprodukt wurde dann mittels Chromatographie gereinigt.
Man erhielt 82 mg Ethylester (64 % Ausbeute).

### Beispiel I-36

### Herstellung von Cis-4-[[(5S)-3-(3,5-Difluorphenyl)-5-vinyl-4H-isoxazol-5-carbonyl] amino]tetrahydrofuran-2-carbonsäureisopropylester

0.13 g Carbonsäure und 9 mg 4-Dimethylaminopyridin wurden in 5 ml Dichlormethan gelöst.Daraufhin wurde unter Rühren bei Raumtemperatur 104 mg 1-(3-Dimethylaminopropyl)-3-ethylcarbodiimid hydrochlorid gefolgt von 0.33 g 2-Propanol zugegeben, und das Reaktionsgemisch wurde 1.5 h bei Raumtemperatur und dann 36 h bei dieser Temperatur rühren gelassen.
Das Reaktionsgemisch wurde mit 0.5 M wässriger Salzsäure gewaschen, getrocknet und im Vakuum eingeengt. Das Rohprodukt wurde dann mittels Chromatographie gereinigt.
Man erhielt 106 mg 2-Propylester (71 % Ausbeute).

### Beispiel I-37

### Herstellung von Cis-4-[[(5S)-3-(3,5-Difluorphenyl)-5-vinyl-4H-isoxazol-5-carbonyl]amino]tetrahydrofuran-2-carbonsäurebenzylester

0.10 g Carbonsäure und 0.04 g Benzylalkohol wurden in 5 ml Dichlormethan gelöst. Zu dieser Mischung wurden unter Rühren bei Raumtemperatur 0.07 g N,N-Diisopropylethylamin gefolgt von 0.26 g einer Lösung von Propylphosphonsäureanhydrid 50 %ig in Tetrahydrofuran zugegeben und die Reaktionsmischung 1h bei Raumtemperatur gerührt.
Das Reaktionsgemisch wurde mit gesättigter wässriger Natriumhydrogencarbonat-Lösung gewaschen, getrocknet und im Vakuum eingeengt.
Man erhielt 100 mg Benzylester (73 % Ausbeute).

### Beispiel I-38

### Herstellung von Cis-4-[[(5S)-3-(3,5-Difluorphenyl)-5-vinyl-4H-isoxazol-5-carbonyl] amino]tetrahydrofuran-2-carbonsäure-tert. butylester

0.13 g Carbonsäure wurde in 2 ml Tetrahydrofuran gelöst und unter Stickstoff gesetzt. Durch einen Spritzenfilter wurden 0.2 ml tert-Butyl-N,N'-diisopropylcarbamimidat hinzugegeben. Die Reaktionsmischung wurde 3 Stunden lang unter Rückfluss erhitzt, abgekühlt, und es wurden weitere 0.2 ml tert-Butyl-N,N'-diisopropylcarbamimidat hinzugefügt. Das Reaktionsgemisch wurde weitere 2 h refluxiert und anschließend auf Raumtemperatur abgekühlt.
Die Reaktionsmischung wurde dann mit Ethylacetat verdünnt und über Celite filtriert, wobei mit zusätzlichem Ethylacetat nachgespült wurde. Die vereinigten Filtrate wurden im Vakuum eingeengt und durch Säulenchromatographie gereinigt. Nach einer weiteren Reinigung mittels HPLC erhielt man 84 mg tert-Butylester (54 % Ausbeute).

### Beispiel I-39 und Beispiel I-40

### Herstellung von (3S)-3-[[3-(3-Fluorphenyl)-5-methyl-4H-isoxazol-5-carbonyl]amino]-2,3-dihydrofuran-5-carbonsäuremethylester und (2S,4S)-4-[[3-(3-Fluorphenyl)-5-methyl-4H-isoxazol-5-carbonyl]amino]-2-methoxy-tetrahydrofuran-2-carbonsäuremethylester

0.43 g Intermediat 17 (als Hydrochlorid) und 0.42 g Carbonsäure wurden in 15 ml Dichlormethan gelöst. Zu dieser Mischung wurden unter Rühren bei Raumtemperatur 0.78 ml Triethylamin gefolgt von 3.34 ml einer Lösung von Propylphosphonsäureanhydrid 50 %ig in Tetrahydrofuran zugegeben und die Reaktionsmischung 3 h bei Raumtemperatur gerührt. Das Reaktionsgemisch wurde mit gesättigter wässriger Natriumhydrogencarbonat-Lösung gewaschen, getrocknet und im Vakuum eingeengt. Das Rohprodukt wurde dann mittels Chromatographie gereinigt.
Man erhielt 40 mg (5 % Ausbeute) von (3S)-3-[[3-(3-Fluorphenyl)-5-methyl-4H-isoxazol-5-carbonyl]amino]-2,3-dihydrofuran-5-carbonsäuremethylester sowie 96 mg (13 % Ausbeute) von (2S,4S)-4-[[3-(3-Fluorphenyl)-5-methyl-4H-isoxazol-5-carbonyl]amino]-2-methoxy-tetrahydrofuran-2-carbonsäuremethylester.

### Beispiel I-46 und Beispiel I-47

### Herstellung von (3R)-3-[[3-(3-Fluorphenyl)-5-methyl-4H-isoxazol-5-carbonyl]amino]-2,3-dihydrofuran-5-carbonsäuremethylester und (2R,4R)-4-[[3-(3-Fluorphenyl)-5-methyl-4H-isoxazol-5-carbonyl]amino]-2-methoxy-tetrahydrofuran-2-carbonsäuremethylester

Die Herstellung erfolgte analog aus Intermediat 18 (als Hydrochlorid) unter den unter Beispiel I-39 und Beispiel I-40 angegebenen Bedingungen.

### Beispiel I-55

### Herstellung von Trans-4-[[(5R)-3-(3,5-Difluorphenyl)-5-methyl-4H-isoxazol-5-carbonyl]amino]tetrahydrofuran-2-carbonsäuremethylester

0.08 g Intermediat 16 und 0.11 g Carbonsäure wurden in 5 ml Dichlormethan gelöst. Zu dieser Mischung wurden unter Rühren bei Raumtemperatur 0.18 ml Triethylamin, gefolgt von 0.79 ml einer Lösung von Propylphosphonsäureanhydrid 50 %ig in Tetrahydrofuran zugegeben und die Reaktionsmischung 2 h bei Raumtemperatur gerührt. Das Reaktionsgemisch wurde mit gesättigter wässriger Natriumhydrogencarbonat-Lösung gewaschen, getrocknet und im Vakuum eingeengt. Das Rohprodukt wurde dann mittels Chromatographie gereinigt. Man erhielt 90 mg (54 % Ausbeute).

### Beispiel I-56

### Herstellung von Trans-4-[[(5S)-3-(3,5-Difluorphenyl)-5-vinyl-4H-isoxazol-5-carbonyl]amino]tetrahydrofuran-2-carbonsäuremethylester

Die Herstellung erfolgte analog Beispiel I-55 unter Einsatz von 0.08 g Intermediat 16 und 0.11 g Carbonsäure. Man erhielt 110 mg (64 % Ausbeute).

Analytische Daten der Beispiele I-01 - I-56

| Nr. | NMR |
|---|---|
| I-01 | ¹H-NMR(400.0 MHz, CDCl3): |
| | δ= 7.518(1.0);7.260(181.8);7.171(1.8);7.166(2.2);7.162(1.3);7.155(1.2);7.151(2.2);7.146 (1.8);6.996(1.0);6.898(0.6);6.883(0.7);6.882(0.7);6.878(1.2);6.876(1.2);6.870(0.6);6.854 (0.6);4.578(1.1);4.573(0.7);4.569(1.1);4.560(0.5);4.554(1.6);4.545(1.3);4.530(1.0);4.521 (0.8);4.065(0.8);4.052(0.8);4.042(1.3);4.029(1.7);4.017(0.8);4.006(1.1);3.993(1.0);3.967 (0.7);3.962(0.7);3.900(0.6);3.896(0.6);3.834(14.5);3.785(16.0);3.773(2.0);3.756(1.8);3.7 30(2.2);3. 712(2.0);3.190(1.9);3.182(2.0);3.146(1.6);3.138(1.8);2.558(0.7);2.541(0.9);2.5 24(0.8);2.506(0.5);2.019(0.5);1.710(11.3);1.687(10.3);1.541(35.4);0.008(2.1);0.000(68.8 );-0.008(1.9) |
| I-02 | ¹H-NMR(400.0 MHz, CDCl3): |
| | δ=7.556(5.0);7.552(6.9);7.552(6.5);7.548(6.3);7.518(1.0);7.438(1.8);7.437(1.6);7.433(3. 1);7.432(2.8);7.429(1.5);7.427(1.4);7.268(0.6);7.267(0.7);7.260(105.5);6.996(0.6);5.298 (5.7);4.677(0.6);4.671 (0.6);4.590(0.8);4.582(1.2);4.574(0. 7);4.567(0.8);4.558(1.3);4.551 (0.7);4.061(0.6);4.048(0.7);4.045(1.3);4.037(1.4);4.033(1.2);4.024(1.3);4.014(0.6);4.010 (0.7);4.000(0.7);3.994(0.8);3.890(1.5);3.848(1.9);3.844(1.9);3.810(15.9);3.802(2.2);3.79 2(13.4);3.359(13.3);3.355(16.0);3.337(2.2);3.320(1.8);3.291(1.8);3.274(1.6);2.591(0.5); 2.573(0.6);2.567(0.5);2.126(0.5);1.538(20.2);0.008(1.3);0.000(42.8);-0.008(1.2) |
| I-03 | ¹H-NMR(400.0 MHz, CDCl3): |
| | δ= 7.595(2.6);7.590(2.9);7.526(7.9);7.521(8.3);7.518(2.3);7.448(1.1);7.445(1.4);7.443(1. 4);7.419(2.0);7.414(3.2);7.409(1.7);7.259(347.5);6.995(1.9);6.172(1.3);6.145(1.6);6.129 (1.6);6.102(1.7);5.556(1.6);5.555(1.8);5.540(1.0);5.513(1.5);5.512(1.5);5.497(0.8);5.355 (1.6);5.341(0.8);5.328(1.4);5.314(0.8);5.298(3 .5);4.594(0.6);4.587(0.7);4.572(0.8);4.564 (0.9);4.556(1.0);4.548(1.0);4.541(0.5);4.533(0. 8);4.524(0. 8);4. 053(1.3);4. 040(1.6);4.029 (1.4);4.016(1.7);4.008(0.6);4.003(0.8);3.949(0.8);3.924(2.5);3.903(1.3);3.881(2.1);3.860 (1.4);3.857(5.2);3.827(5.8);3.814(9.6);3.803(16.0);3.326(1.0);3.318(1.1);3.315(1.0);3.30 8(1.8);3.301(1.0);3.290(1.0);3.274(0.8);3.265(1.5);2.837(0. 7);2.835(0.7);2.526(0.6);2.50 8(0.5);2.039(0.6);2.004(0.8);1.532(84.5);1.284(0.5);1.256(0.5);0.899(2.5);0.896(2.3);0.8 84(2.5);0.880(2.3);0.008(4.3);0.000(146.4);-0.008(3.9) |
| I-04 | ¹H-NMR(400.0 MHz, CDCl3): |
| | δ= 8.018(1.1);7.569(1.2);7.518(6.4);7.384(2.3);7.359(2.0);7.322(2.1);7.308(2.4);7.295(2. 9);7.259(1071.8);7.252(5.2);7.251(1.9);7.250(1.7);7.249(1.6);7.248(1.4);7.248(1.5);7.24 7(1.5);7.246(1.5);7.245(1.3);7.244(1.2);7.244(1.2);7.242(1.1);7.241(1.2);7.240(1.2);7.23 9(1.1);7.236(1.4);7.232(1.6);7.227(1.8);7.221(1.6);7.217(1.8);7.209(2.1);7.194(1.1);7.15 0(0.6);6.995(5.9);5.298(16.0);4.565(0.8);4.541(0.8);4.419(0.6);4.392(0.6);4.113(0.6);4.0 43(0.8);3.991(0.9);3.947(0.9);3.825(3.9);3. 786(4.3);3. 772(4.7);3. 760(4.6);3.746(1.4);3.7 06(1.6);3.633(1.5);3.589(0.6);3.424(2.1);3.203(2.3);3.166(0.9);3.142(0.9);3.129(1.8);3.1 24(2.2);3.040(2.4);2.992(2.0);2.986(1.8);2.955(11.0);2.884(9.4);2.882(9.4);2.553(0.6);2. 352(6.6);2.340(2.6);2.321(1.4);2.221(3.9);2.043(2.2);2.003(1.2);1.714(6.2);1.704(6.4);1. 686(6.9);1.676(5.7);1.664(6.0);1.658(6.2);1.495(2.1);1.434(2.5);1.333(1.0);1.284(1.1);1. 258(1.9);0.146(1.4);0.008(13.6);0.000(458.9);-0.008(12.8);-0.051(0.7);-0.149(1.4) |
| I-05 | ¹H-NMR(400.0 MHz, CDCl3): |
| | δ= 7.518(1.6);7.310(0.5);7.259(217.2);7.227(0.5);7.211(1.3);7.208(1.9);7.205(2.0);7.202 (2.0);7.191(2.0);7.188(1.8);7.186(1.9);7.182(1.2);6.995(1.2);6.920(0.7);6.904(0.8);6.898 (1.3);6.894(0.7);6.878(0.7);4.678(0.6);4.673(0.6);4.667(0.5);4.591(0.7);4.583(1.3);4.575 (0.9);4.568(0.8);4.560(1.4);4.552(0.8);4.071 (0.5);4.059(0.7);4.047(1.6);4.037(1.6);4.035 (1.6);4.025(1.3);4.015(0.8);4.012(0.8);4.002(0.7);3.998(0.7);3.884(1.8);3.841(1.9);3.838 (2.3);3.810(14.7);3.796(2.4);3.790(16.0);3.366(15.8);3.362(15.0);3.343(2.1);3.326(2.2); 3.298(1.8);3.280(1.8);2.593(0.5);2.580(0.5);2.573(0.6);2.562(0.6);2.556(0.5);2.552(0.5); 2.545(0.5);2.146(0.5);2.128(0.5);2.119(0.5);1.539(11.2);0.008(2.6);0.000(88.3);-0.008(2.4) |
| I-06 | ¹H-NMR(400.0 MHz, CDCl3): |
| | δ= 7.261(34.4);7.205(1.5);7.200(2.0);7.196(1.2);7.189(1.2);7.185(1.9);7.180(1.6);6.960( 0.8);6.945(0.8);6.939(1.5);6.933(0.7);6.917(0.7);5.298(5.5);4.678(0.5);4.675(0.6);4.663( 0.6);4.658(0.5);4.606(0.6);4.598(0.7);4.585(0.8);4.582(0.8);4.577(1.0);4.575(0.8);4.561 ( 0.8);4.554(0.8);4.074(0.7);4.061(0.7);4.050(1.4);4.04 7(0.6);4.037(1.3);4.035(0.6);4.022( 1.2);4.010(1.2);4.000(0.7);3.998(0.8);3.993(1.2);3.987(0.8);3.985(0.7);3.980(0.9);3.969( 0.6);3.955(0.6);3.934(1.1);3.910(0.9);3.829(12.4);3.798(16.0);3.752(2.0);3.746(2.3);3.70 7(1.3);3.701(1.6);2.521(0.5);2.104(0.6);2.099(0.6);2.096(0.6);1.556(6.0);0.000(14.2) |
| I-07 | ¹H-NMR(400.0 MHz, CDCl3): |
| | δ= 7.405(0.7);7.404(0.8);7.402(1.0);7.400(1.0);7.398(1.0);7.394(1.4);7.393(1.4);7.388(0. 9);7.385(1.5);7.380(5.0);7.377(2.9);7.375(2.0);7.372(2.8);7.368(1.5);7.261(32.6);7.149( 0.6);7.143(0.6);7.135(0.8);7.133(0.7);7.127(0.8);7.114(0.5);6.189(1.3);6.163(1.4);6.146( 1.5);6.119(1.6);5.562(1.7);5.561(1.7);5.547(1.4);5.545(1.4);5.519(1.5);5.518(1.5);5.504( 1.2);5.502(1.2);5.346(1.4);5.345(1.4);5.334(1.2);5.332(1.2);5.320(1.4);5.318(1.4);5.307( 1.1);5.305(1.1);5.298(2.9);4.572(0.9);4.564(0.8);4.554(0.9);4.549(0.9);4.545(0.9);4.540( 0.7);4.531(0.8);4.521(0.8);4.062(0.8);4.049(0.8);4.044(0.7);4.039(1.2);4.031(0.7);4.026( 1.2);4.020(1.0);4.007(0.9);3.952(0. 7);3.950(0.7);3.946(2.5);3.926(2.6);3.921(1.0);3.919( 0.9);3.903(2.6);3.883(1.9);3.815(13.3);3.796(16.0);3.357(1.6);3.348(1.9);3.314(1.4);3.30 5(1.6);2.542(0.6);2.531(0.6);2.513(0.5);2.042(0.5);1.572(4.6);0.000(12.8) |
| I-08 | ¹H-NMR(400.0 MHz, CDCl3): |
| | δ= 7.613(0.7);7.611(0.8);7.607(1.5);7.605(1.4);7.603(1.4);7.598(0.7);7.596(0.6);7.413(2. 8);7.411(3.9);7.409(4.0);7.407(3.5);7.262(34.4);5.298(11.2);4.675(0.6);4.670(0.5);4.667 (0.5);4.654(0.5);4.589(0.7);4.580(1.5);4.572(0.8);4.565(0.8);4.557(1.5);4.548(0.8);4.074 (0.6);4.063(0.7);4.062(0.7);4.050(1.8);4.039(1.6);4.038(1.6);4.027(1.2);4.014(0.7);4.012 (0.7);4.009(0.8);4.006(0.7);3.998(0.6);3.996(0.7);3.992(1.0);3.989(0.9);3.881(1.7);3.841 (1.7);3.836(2.1);3.810(14.9);3.795(2.1);3.789(16.0);3.360(2.5);3.355(15.3);3.351(14.7); 3.343(2.2);3.315(1.7);3.297(1.8);2.594(0.5);2.576(0.5);2.574(0.5);2.562(0.6);2.363(11.5 );2.362(11.5);2.143(0.5);2.136(0.6);2.130(0.5);1.568(4.3);0.000(14.6) |
| I-09 | ¹H-NMR(400.0 MHz, CDCl3): |
| | δ= 7.263(29.0);7.172(1.9);7.166(2.3);7.163(1.3);7.155(1.3);7.152(2.2);7.146(1.9);6.900( 0.6);6.898(0.6);6.884(0.7);6.882(0.7);6.878(1.3);6.876(1.2);6.872(0.7);6.870(0.6);6.856( 0.6);6.854(0.6);5.299(4.4);4.579(1.2);4.574(0.7);4.569(1.2);4.560(0.6);4.554(1.7);4.545( 1.4);4.530(0.9);4.521(0.8);4.066(0.7);4.053(0.7);4.042(1.2);4.029(1.7);4.017(0.8);4.006( 1.2);3.993(1.1);3.967(0.7);3.963(0.6);3.961(0.6);3.902(0.6);3.900(0.6);3.896(0.6);3.894( 0.6);3.834(16.0);3.785(15.8);3.779(0.7);3.774(2.3);3.757(1.9);3.730(2.2);3.714(2.2);3.19 1(2.0);3.183(1.9);3.148(1.7);3.140(1.7);2.565(0.6);2.559(0.7);2.541(0.9);2.524(0.8);2.12 2(0.5);2.020(0.5);1.710(10.7);1.687(10.9);1.585(5.6);0.000(11.5) |
| I-10 | ¹H-NMR(400.0 MHz, CDCl3): |
| | δ= 7.262(28.6);7.180(2.2);7.174(2.6);7.171(1.5);7.163(1.5);7.160(2.6);7.154(2.1);6.903( 0.8);6.887(0.9);6.882(1.6);6.876(0.8);6.860(0.8);6.176(2.0);6.150(2.3);6.133(2.4);6.106( 2.5);5.560(1.8);5.558(1.8);5.544(1.7);5.543(1.7);5.517(1.5);5.515(1.5);5.501(1.5);5.500( 1.4);5.357(1.6);5.356(1.6);5.343(1.5);5.342(1.5);5.330(1.5);5.329(1.5);5.316(1.4);5.315( 1.4);5.299(9.2);4.603(0.5);4.596(0.6);4.591(0.6);4.585(0.6);4.579(0.6);4.574(1.1);4.565( 1.2);4.558(1.1);4.550(1.4);4.542(0.9);4.534(0.9);4.525(0.8);4.056(0.8);4.043(0.9);4.041 ( 0.9);4.033(1.3);4.028(0.8);4.020(1.3);4.017(1.4);4.004(1.1);3.950(0.7);3.946(0.7);3.944( 0.7);3.929(1.1);3.922(2.7);3.905(0.5);3.899(2.3);3.878(2.2);3.856(2.2);3.814(15.9);3.800 (16.0);3.322(1.9);3.313(1.9);3.279(1.6);3.270(1.7);2.566(0.5);2.564(0.5);2.555(0.6);2.54 9(0.5);2.546(0.5);2.540(0.6);2.538(0.6);2.531(0.6);2.529(0.6);2.514(0.5);2.511(0.6);2.09 4(0.6);2.060(0.5);2.052(0.5);2.044(0.6);1.577(5.9);0.000(11.4) |
| I-11 | ¹H-NMR(400.6 MHz, CDCl3): |
| | δ= 7.494(0.6);7.474(0.6);7.278(0.9);7.184(1.5);7.178(2.0);7.176(1.4);7.167(1.3);7.164(2. 0);7.159(1.7);6.906(0.6);6.890(0.7);6.884(1.3);6.879(0.7);6.863(0.6);6.180(1.1);6.153(1. 2);6.137(1.4);6.110(1.4);5.560(2.2);5.559(2.2);5.516(1.9);5.516(1.9);5.358(2.1);5.331 (2. 0);4.622(0.5);4.615(0.6);4.611(0.6);4.608(0.6);4.602(0.6);4.597(0.6);4.562(1.0);4.553(1. 2);4.539(1.2);4.530(1.1);4.060(0.9);4.047(1.0);4.036(1.5);4.023(1.4);3.955(1.1);3.951(1. 2);3.928(2.9);3.885(2.5);3.801(16.0);3.322(2.3);3.279(2.0);2.569(0.5);2.551(0.6);2.546( 0.6);2.534(0.7);2.528(0.6);2.517(0.7);2.511(0.7);2.493(0.6);2.056(0.5);2.050(0.8);2.043( 0.6);2.015(0.8);2.009(0.5);0.000(0.8) |
| I-12 | ¹H-NMR(400.6 MHz, CDCl3): |
| | δ= 7.461(0.7);7.442(0.7);7.278(0.9);7.184(1.6);7.178(2.0);7.164(2.1);7.159(1.6);6.907(0. 6);6.891 (0.8);6.885(1.3);6.880(0.7);6.864(0.7);6.179(1.1);6.152(1.3);6.136(1.4);6.109(1. 4);5.543(2.3);5.500(2.0);5.344(2.2);5.317(2.1);4.593(0.6);4.587(0.7);4.579(1.6);4.570(1. 8);4.563(0.6);4.555(1.5);4.546(1.2);4.043(1.0);4.030(1.0);4.019(1.6);4.006(1.4);3.935(1. 2);3.930(1.2);3.905(3.0);3.862(2.6);3.814(16.0);3.331(2.4);3.288(2.1);2.596(0.5);2.579( 0.6);2.573(0.6);2.562(0.7);2.555(0.6);2.544(0.7);2.538(0.7);2.521(0.6);2.107(0.6);2.101( 0.9);2.095(0.6);2.073(0.5);2.067(0.8);2.060(0.5);1.257(0.5);0.000(0.8) |
| I-13 | ¹H-NMR(400.0 MHz, CDCl3): |
| | δ= 7.518(1.2);7.394(1.4);7.389(2.4);7.374(5.0);7.365(4.2);7.358(0.6);7.310(0.7);7.268(1. 5);7.265(3.0);7.259(207.2);7.253(1.0);7.252(0.8);7.251(0.6);7.145(0.8);7.140(0.6);7.130 (1.0);7.121(0.9);7.113(0.6);7.110(0.6);6.995(1.2);4.577(1.2);4.567(1.1);4.557(0.6);4.553 (1.2);4.550(1.2);4.544(1.0);4.540(1.1);4.526(0.9);4.517(0.8);4.072(0.8);4.059(0.8);4.048 (1.3);4.035(1.6);4.021(0.8);4.010(1.1);3.997(0.9);3.966(0.7);3.961(0.8);3.896(0.6);3.890 (0.6);3.835(14.3);3.800(2.0);3.785(2.1);3.778(16.0);3.762(0.6);3.757(2.3);3.742(2.1);3.2 23(1.9);3.215(2.1);3.180(1.7);3.172(1.9);2.561(0.8);2.549(0.5);2.543(0.9);2.526(0.9);2.5 20(0.5);2.509(0.5);2.503(0.5);2.018(0.6);1.983(0.5);1.725(0.6);1.714(1.4);1.708(11.3);1. 697(0.7);1.686(10.3);1.666(0.6);1.606(2.7);0.008(2.2);0.000(86.6);-0.008(2.6) |
| I-14 | ¹H-NMR(400.0 MHz, CDCl3): |
| | δ= 7.684(1.6);7.680(2.9);7.677(2.1);7.670(1.0);7.666(1.6);7.665(1.5);7.663(1.6);7.660(2. 4);7.656(1.9);7.520(0.6);7.454(0.6);7.452(0.7);7.451(0.8);7.450(0.8);7.442(1.4);7.440(1. 6);7.437(5.2);7.432(2.3);7.427(1.0);7.422(1.3);7.418(2.4);7.404(0.8);7.261(43.3);5.298( 3.7);4.680(0.5);4.672(0.5);4.590(0.7);4.581(1.3);4.571(0.8);4.567(0.8);4.557(1.3);4.548( 0.8);4.081(0.6);4.068(1.0);4.057(1.4);4.055(0.8);4.044(2.0);4.031(1.2);4.018(0.7);4.016( 0.7);4.012(0.7);4.009(0.7);3.999(0.6);3.997(0.7);3.992(1.0);3.904(1.9);3.866(1.8);3.858( 2.2);3.821(2.2);3.813(14.8);3.783(15.8);3.438(2.2);3.420(2.3);3.393(1.9);3.375(2.5);3.37 0(16.0);3.364(15.0);2.600(0.8);2.583(0.7);2.577(0.8);2.566(0.6);2.560(0.7);2.146(0.8);2. 143(0.6);2.140(0.6);2.137(0.7);2.111(0.7);2.108(0.5);2.105(0.5);2.102(0.6);1.563(9.6);0. 008(0.5);0.000(18.3);-0.008(0.5) |
| I-15 | ¹H-NMR(400.0 MHz, CDCl3): |
| | δ= 7.518(1.2);7.259(207.4);7.210(0.6);7.170(1.4);7.165(1.8);7.150(1.7);7.145(1.4);6.995 (1.2);6.898(0.6);6.882(0.7);6.876(1.2);6.870(0.6);6.854(0.6);4.581(0.6);4.554(1.2);4.545 (1.0);4.530(1.2);4.521(1.0);4.066(0.9);4.052(0.9);4.042(1.5);4.029(1.4);3.968(1.0);3.962 (1.1);3.945(0.7);3.786(16.0);3.773(2.3);3.729(2.5);3.182(2.4);3.138(2.1);2.559(0.5);2.54 0(0.5);2.536(0.6);2.524(0.7);2.518(0.6);2.506(0.7);2.501(0.6);2.483(0.5);2.019(0.8);1.98 5(0.6);1.710(13.0);1.530(23.7);0.008(2.5);0.000(73.7);-0.008(2.3) |
| I-16 | ¹H-NMR(400.0 MHz, CDCl3): |
| | δ= 7.519(0.5);7.426(0.5);7.260(91.8);7.172(1.4);7.166(1.8);7.152(1.7);7.146(1.4);6.900(0.6);6.884(0.7);6.878(1.2);6.872(0.6);6.856(0.6);4.580(1.3);4.570(1.5);4.556(1.6);4.547(1.3);4.030(1.0);4.017(1.0);4.006(1.4);3.993(1.3);3.901(1.0);3.895(0.9);3.878(0.7);3.871 (0.7);3.834(16.0);3.756(2.2);3.713(2.5);3.190(2.4);3.147(2.0);2.599(0.5);2.581(0.6);2.575(0.5);2.564(0.6);2.558(0.6);2.547(0.6);2.541(0.6);2.524(0.6);2.122(0.8);2.087(0.6);1.688(12.8);1.536(5.7);0.008(1.4);0.000(33.1);-0.008(1.1) |
| I-17 | ¹H-NMR(400.0 MHz, CDCl3): |
| | δ= 7.260(62.3);7.217(0.8);7.186(1.3);7.181(2.3);7.178(1.4);7.175(1.5);7.171(1.1);7.167(1.6);7.164(1.3);7.161(2.3);7.155(1.1);6.919(0.5);6.913(0.9);6.897(1.1);6.891(1.8);6.886(0.9);6.876(0.5);6.870(0.9);4.239(1.7);4.215(2.1);4.203(1.6);4.179(1.9);4.012(1.6);4.002(2.5);3.996(1.5);3.993(2.0);3.988(1.1);3.982(2.1);3.977(2.8);3.968(0.9);3.964(0.9);3.946(1.3);3.922(1.1);3.769(1.9);3.760(1.7);3.724(14.8);3.716(2.1);3.706(16.0);3.208(3.1);3.165(2.8);2.624(0.6);2.603(0.5);2.590(0.9);2.584(0.6);2.570(0.6);2.551(0.8);2.252(0.6);2.236(0.5);2.219(0.5);2.190(0.6);2.174(0.6);1.723(14.0);1.547(5.9);0.008(0.7);0.000(23.6);-0.008(0.7) |
| I-18 | ¹H-NMR(400.0 MHz, CDCl3): |
| | δ= 7.262(30.8);7.221(0.9);7.184(1.4);7.178(2.2);7.173(1.6);7.168(1.1);7.164(1.8);7.158(2.1);7.153(1.0);6.917(0.6);6.911(1.0);6.905(0.5);6.895(1.2);6.889(2.0);6.884(1.0);6.874(0.6);6.868(1.0);5.299(2.7);4.230(1.9);4.206(2.9);4.198(1.6);4.190(1.3);4.188(2.9);4.173(4.5);4.170(3.3);4.155(3.8);4.152(1.2);4.137(1.2);4.009(1.5);4.006(1.9);3.997(1.9);3.993(2.0);3.990(1.4);3.982(2.8);3.978(2.6);3.974(1.7);3.962(1.8);3.953(1.2);3.928(1.0);3.776(2.0);3.766(1.6);3.733(2.3);3.723(1.8);3.207(3.2);3.164(2.8);2.625(0.7);2.610(0.5);2.608(0.5);2.592(1.0);2.589(0.9);2.575(0.6);2.573(0.5);2.570(0.5);2.556(1.0);2.245(0.6);2.229(0.5);2.184(0.7);2.168(0.6);2.151(0.6);1.726(16.0);1.215(3.2);1.197(9.0);1.180(10.3);1.162(3.9);0.000(12.2) |
| I-19 | ¹H-NMR(400.0 MHz, CDCl3): |
| | δ= 7.263(33.4);7.258(0.6);7.179(0.5);7.174(1.4);7.168(1.8);7.165(2.3);7.159(2.1);7.157( 1.5);7.154(2.0);7.148(2.2);7.145(1.7);7.140(1.3);7.040(0.7);7.021(0.7);6.916(0.8);6.910( 0.8);6.900(0.8);6.894(1.7);6.888(1.5);6.882(0.6);6.879(0.7);6.872(1.0);6.866(0.8);4.715( 0.8);4.710(0.6);4.706(0.8);4.700(0.6);4.696(0.8);4.690(0.5);4.225(0.7);4.203(1.7);4.181( 1.8);4.159(0.9);4.149(0.6);4.131(1.6);4.113(1.6);4.095(0.6);4.038(0.9);4.023(0.9);4.014( 1.8);4.000(1.8);3.991(1.0);3.976(1.1);3.950(1.0);3.934(1.0);3.927(0.9);3.922(1.0);3.911( 0.9);3.907(1.1);3.899(0.9);3.884(0.9);3.792(2.0);3.780(2.0);3.774(0.6);3.772(0.5);3.757( 0.9);3.746(16.0);3.741(1.2);3.737(6.0);3.731(1.2);3.725(1.0);3.710(0.6);3.704(0.6);3.691 (16.0);3.680(1.1);3.665(0.9);3.656(0.8);3.541(3.0);3.211(2.8);3.188(0.6);3.168(2.4);3.14 5(0.5);3.007(1.0);2.045(7.1);1.722(10.8);1.715(13.2);1.688(2.5);1.277(2.6);1.264(1.8);1. 260(5.3);1.242(2.3);0.899(0.9);0.882(3.1);0.864(1.2);0.000(13.7) |
| I-20 | ¹H-NMR(400.0 MHz, d₆-DMSO): |
| | δ= 8.147(0.6);8.130(0.6);8.087(0.6);8.069(0.6);7.526(2.2);7.524(2.3);7.517(3.2);7.512(2. 2);7.498(2.4);7.469(1.1);7.465(0.9);7.349(0.6);7.341(0.6);7.334(0.7);7.326(1.0);7.322(0. 9);7.315(0.6);7.308(0.6);7.303(0.7);5.756(5.0);4.380(0.6);4.365(1.4);4.359(0.8);4.349(1. 0);4.343(1.5);4.327(0.9);4.318(0.8);4.303(0.8);3.911(0.7);3.895(0.7);3.889(0.9);3.873(0. 8);3.868(0.7);3.853(0.7);3.847(0.8);3.831(0.7);3.761(1.3);3.740(1.5);3.717(1.6);3.696(1. 9);3.678(0.8);3.671(0.7);3.660(0.8);3.64 7(0.7);3.638(0.6);3.625(0.6);3.390(1.8);3.385(1. 7);3.346(1.9);3.341(2.0);3.322(43.8);2.670(0.6);2.544(0.8);2.524(1.3);2.519(2.0);2.510( 32.8);2.506(72.0);2.501(100.7);2.497(70.3);2.492(31.4);2.468(0.8);2.456(1.2);2.451(1.0) ;2.447(1.1);2.436(0.5);2.425(0.7);2.328(0.6);2.048(0.5);1.979(0.6);1.963(0.6);1.947(0.6) ;1.567(1.4);1.534(16.0);0.000(1.8) |
| I-21 | ¹H-NMR(400.0 MHz, d₆-DMSO): |
| | δ= 7.428(4.5);7.421(1.4);7.410(3.4);7.404(2.6);7.386(0.6);7.381(0.5);6.160(1.0);6.153(0. 9);6.134(1.1);6.126(1.0);6.117(1.2);6.110(1.1);6.091(1.2);6.083(1.0);5.398(2.3);5.396(1. 5);5.355(2.1);5.316(2.1);5.288(2.0);4.376(0.6);4.367(0.8);4.360(1.0);4.354(1.0);4.351(1. 2);4.346(1.3);4.339(1.0);4.330(1.3);4.314(0.5);4.056(1.0);4.038(3.2);4.020(3.2);4.003(1. 1);3.908(0.7);3.893(1.6);3.887(1.0);3.880(1.5);3.871(0.8);3.862(0.6);3.856(0.8);3.849(1. 5);3.840(0.8);3.835(1.7);3.667(1.4);3.653(1.3);3.645(1.2);3.631(1.1);3.601(0.6);3.574(3. 0);3.530(2.6);3.431(1.9);2.670(0.7);2.524(2.1);2.519(3.0);2.510(39.7);2.506(85.1);2.501 (118.3);2.496(82.4);2.492(36.6);2.476(0.6);2.464(0.6);2.453(0.8);2.432(0.6);2.332(0.5); 2.328(0.7);2.323(0.5);2.014(0.6);2.005(0.7);1.996(0.6);1.988(16.0);1.973(0.6);1.908(1.7 );1.760(0.5);1.356(0.8);1.192(4.1);1.175(8.3);1.157(4.0);0.000(3.8) |
| I-22 | ¹H-NMR(400.0 MHz, CDCl3): |
| | δ= 7.262(46.3);7.180(2.1);7.174(2.4);7.171(1.4);7.164(1.4);7.160(2.4);7.154(1.9);6.904( 0.8);6.888(0.9);6.882(1.5);6.877(0.7);6.860(0.7);6.176(2.0);6.150(2.2);6.133(2.4);6.106( 2.4);5.560(1.8);5.559(1.8);5.544(1.7);5.543(1.7);5.517(1.6);5.516(1.6);5.501(1.5);5.500( 1.4);5.358(1.6);5.356(1.6);5.344(1.5);5.342(1.4);5.331(1.4);5.330(1.4);5.317(1.4);5.316( 1.4);5.300(2.9);4.586(0.5);4.579(0.6);4.576(1.1);4.566(1.1);4.559(1.1);4.552(1.3);4.543( 0.8);4.535(0.9);4.526(0.8);4.056(0.8);4.043(0.9);4.041(0.8);4.033(1.3);4.028(0.8);4.020( 1.3);4.017(1.3);4.004(1.1);3.953(0.6);3.951(0.7);3.947(0.7);3.945(0.6);3.930(1.0);3.927( 1.0);3.921(2.4);3.899(2.0);3.878(2.2);3.856(2.1);3.814(15.6);3.801(16.0);3.323(1.8);3.31 3(1.9);3.280(1.6);3 .270(1.6);2.556(0.5);2.549(0.5);2.547(0.5);2.541(0.5);2.539(0.6);2.53 2(0.6);2.530(0.6);2.515(0.5);2.512(0.6);2.095(0.6);2.052(0.6);2.044(0.6);1.569(13.8);0.0 08(0.6);0.000(18.2);-0.008(0.5) |
| I-23 | ¹H-NMR(400.0 MHz, CDCl3): |
| | δ= 7.519(0.6);7.269(0.5);7.268(0.6);7.267(0.7);7.266(0.9);7.266(1.1);7.265(1.4);7.264(1. 9);7.263(2.5);7.260(105.9);7.256(1.7);7.255(1.3);7.254(1.0);7.253(0.7);7.252(0.6);7.210 (0.9);7.200(1.1);7.195(1.2);7.192(0.7);7.184(0.7);7.181(1.3);7.175(1.1);6.996(0.6);6.896 (0.5);6.890(0.9);6.189(0.9);6.162(1.1);6.146(1.2);6.119(1.2);5.647(1.7);5.646(1.7);5.604 (1.5);5.603(1.5);5.428(1.6);5.401(1.4);5.299(1.3);5.217(1.1);5.206(2.1);5.194(1.2);4.754 (1.1);4.742(2.3);4.732(0.8);4.730(0.9);4.318(0.6);4.301(1.9);4.300(1.9);4.283(2.0);4.283 (2.0);4.265(0.7);3.942(1.7);3.898(1.9);3.367(1.6);3.324(1.4);1.539(16.0);1.371(3.2);1.35 3(6.7);1.335(3.1);0.008(1.1);0.000(41.6);-0.005(0.7);-0.006(0.6);-0.008(1.3) |
| I-24 | ¹H-NMR(400.0 MHz, CDCl3): |
| | δ= 10.908(1.1);7.260(57.2);7.210(0.8);7.194(2.0);7.189(2.4);7.178(1.7);7.175(2.4);7.169 (1.8);6.907(0.8);6.891(1.0);6.885(1.6);6.879(0.8);6.869(0.5);6.863(0.8);5.299(0.6);5.222 (1.3);5.211(2.4);5.205(2.5);5.200(1.6);5.194(1.3);4.757(1.6);4.751(1.9);4.745(2.2);4.741 (2.3);4.738(2.0);4.728(1.3);4.324(0.8);4.319(0.8);4.306(2.4);4.301(2.4);4.288(2.5);4.283 (2.4);4.270(0.9);4.265(0.8);4.189(0.7);4.171(2.0);4.153(2.0);4.135(0.8);4.121(5.1);3.852 (1.4);3.836(2.9);3.820(1.5);3.793(2.8);3.756(8.4);3.750(3.5);3.254(3.0);3.211(2.6);2.655 (1.4);2.638(2.6);2.622(1.3);1.777(16.0);1.554(4.9);1.370(5.0);1.352(10.2);1.335(5.0);1.2 85(2.5);1.267(4.9); 1.257(1.0);1.249(2.5);0.008(1.3);0.000(23.4);-0.008(1.1) |
| I-25 | ¹H-NMR(400.0 MHz, CDCl3): |
| | δ= 7.266(0.5);7.263(16.6);7.200(1.2);7.196(0.8);7.194(1.3);7.190(0.7);7.183(0.7);7.180( 1.4);7.177(0.7);7.174(1.1);6.907(0.5);6.891(0.6);6.885(1.1);6.879(0.5);6.863(0.5);5.300( 1.8);5.218(0.6);5.211(0.8);5.207(1.1);5.200(1.3);5.196(0.8);5.189(0.7);4.753(1.0);4.746( 1.0);4.743(1. 1);4.741(1.3);4.736(1.2);4.734(1.0);4.731(0.9);4.724(0.8);4.120(3.7);3.854( 1.3);3.838(2.9);3.822(2.0);3.818(16.0);3.794(1.9);3.756(6.7);3.751(2.3);3.706(7.2);3.257 (1.9);3.214(1.7);2.672(1.0);2.656(1.9);2.640(0.9);1.779(10.8);1.574(2.0);0.000(7.0) |
| I-26 | ¹H-NMR(400.0 MHz, CDCl3): |
| | δ= 7.520(1.9);7.311(0.9);7.291(0.8);7.261(352.0);7.255(0.8);7.254(0.7);7.210(2.5);7.183 (1.3);7.177(1.5);7.174(1.6);7.169(1.5);7.166(1.5);7.163(1.5);7.160(1.3);7.157(1.6);7.155 (1.4);7.149(1.1);6.997(2.5);6.977(0.6);6.920(0.8);6.915(0.8);6.904(0.6);6.899(1.6);6.893 (1.5);6.887(0.6);6.877(0.8);6.871(0.8);6.181(1.1);6.174(1.0);6.154(1.2);6.147(1.2);6.138 (1.2);6.131(1.3);6.111(1.2);6.104(1.2);5.562(1.8);5.561(1.7);5.552(1.7);5.550(1.8);5.519 (1.5);5.517(1.6);5.508(1.5);5.507(1.6);5.372(1.5);5.360(1.6);5.344(1.4);5.333(1.5);5.300 (2.3);4.729(0.6);4.720(0.7);4.212(1.1);4.190(1.8);4.167(5.4);4.026(0.8);4.019(0.8);4.011 (0.8);4.002(1.1);3.995(1.0);3.987(1.1);3.980(0.8);3.943(0.9);3.936(1.9);3.925(2.0);3.921 (1.8);3.905(1.6);3.897(0.8);3.893(2.4);3.882(2.8);3.739(16.0);3.717(0.7);3.711(1.3);3.69 8(15.8);3.687(0.7);3.678(0.6);3.580(1.9);3.336(2.1);3.294(1.8);3.146(0.8);3.128(2.6);3.1 10(2.6);3.091(0.8);1.628(2.0);1.382(2.6);1.363(5.4);1.345(2.5);1.284(0.6);1.254(0.9);0.0 08(4.1);0.000(145.8);-0.006(1.2);-0.008(4.2);-0.050(1.2) |
| I-27 | ¹H-NMR(400.0 MHz, CDCl3): |
| | δ= 7.262(46.2);7.212(0.8);7.210(1.0);7.204(1.3);7.200(1.1);7.194(0.9);7.190(1.3);7.184( 1.1);7.180(0.6);6.909(0.9);6.903(0.8);5.300(3.9);4.258(0.6);4.236(1.0);4.214(0.8);4.035( 0.6);4.026(0.8);4.011(0.8);3.936(0.5);3.920(1.0);3.901(1.2);3.874(1.1);3.856(1.4);3.796( 0.9);3.788(0.6);3.775(0.6);3.765(0.6);3.758(7.8);3.744(8.3);3.736(3.6);3.719(3.6);3.362( 7.7);3.356(6.1);3.354(8.9);3.345(1.2);3.343(0.7);3.337(1.2);3.321(0.5);3.300(1.0);3.292( 1.0);1.558(16.0);0.008(0.5);0.000(18.3) |
| I-28 | ¹H-NMR(400.0 MHz, CDCl3): |
| | δ= 7.262(57.6);7.179(0.6);7.174(1.0);7.169(1.5);7.165(1.8);7.160(1.6);7.154(1.3);7.149( 1.6);7.146(1.2);7.144(0.9);7.140(0.8);6.916(0.6);6.910(0.5);6.901(0.7);6.895(1.2);6.889( 1.0);6.879(0.8);6.873(0.8);6.867(0.5);5.300(8.2);4.704(0.5);4.695(0.5);4.202(1.1);4.181( 1.2);4.158(0.5);4.038(0.6);4.023(0.6);4.021(0.6);4.014(1.1);3.999(1.2);3.990(0.7);3.976( 0.9);3.949(0.6);3.934(0.7);3.926(0.6);3.922(0.7);3.910(0.6);3.906(0.7);3.899(0.5);3.883( 0.5);3.790(1.4);3.778(1.3);3.773(0.8);3.755(0.7);3.746(12.8);3.737(6.4);3.730(1.3);3.723 (0.5);3.703(0.8);3.695(0.8);3.691(11.2);3.678(0.5);3.662(0.5);3.542(5.0);3.211(1.9);3.18 8(0.9);3.167(1.6);3.144(0.8);1.722(7.6);1.715(10.9);1.688(4.0);1.563(16.0);0.008(0.7);0. 000(23.2);-0.008(0.6) |
| I-29 | ¹H-NMR(400.0 MHz, CDCl3): |
| | δ= 7.558(1.3);7.553(7.2);7.549(10.9);7.544(5.8);7.445(1.8);7.444(1.8);7.440(3.4);7.439( 3.2);7.436(2.2);7.263(38.3);7.005(0.6);6.999(0.6);6.990(0.6);5.300(15.8);4.798(0.6);4.78 9(0.7);4.783(0.8);4.778(0.7);4.774(0.7);4.770(0.5);4.764(0.7);4.257(1.3);4.235(2.0);4.21 4(1.5);4.050(0.8);4.047(0.9);4.035(0.9);4.033(0.9);4.025(1.2);4.023(1.2);4.011(1.2);3.96 1(0.9);3.951(1.0);3.945(1.0);3.938(0.9);3.936(1.1);3.927(1.0);3.924(2.0);3.912(0.9);3.90 6(1.9);3.879(2.1);3.860(2.2);3.806(0.8);3.798(1.1);3.787(1.0);3.783(0.7);3.774(0.9);3.76 3(0.9);3.757(15.3);3.744(16.0);3.736(3.1);3.722(3.0);3.354(14.7);3.350(4.7);3.347(16.0) ;3.340(2.4);3.337(0.8);3.332(2.2);3.294(1.9);3.287(1.9);3.077(0.6);3.066(0.8);3.061(0.6) ;3.056(0.6);3.050(0.8);2.045(0.8);1.575(11.7);1.259(0.6);0.000(15.0) |
| I-30 | ¹H-NMR(400.0 MHz, CDCl3): |
| | δ= 7.394(0.9);7.390(1.4);7.388(1.2);7.374(3.6);7.371(2.6);7.368(2.0);7.366(2.7);7.261(6 8.0);7.144(0.7);7.128(0.7);7.121(0.7);4.548(0.7);4.538(0.7);4.522(0.8);4.512(0.8);4.498( 0.7);4.489(0.7);4.296(1.3);4.293(1.3);4.278(1.4);4.275(1.3);4.248(0.9);4.230(2.8);4.213( 2.9);4.195(1.0);4.079(0.6);4.065(0.6);4.055(1.0);4.042(0.9);4.034(0.6);4.021(0.6);4.010( 0.8);3.997(0.8);3.968(0.6);3.963(0.6);3.802(1.6);3.789(1.4);3.759(1.9);3.746(1.6);3.221( 1.5);3.213(1.7);3.178(1.3);3.170(1.5);2.561(0.5);2.526(0.5);1.708(9.2);1.684(8.0);1.554( 16.0);1.360(2.8);1.342(5.8);1.325(2.7);1.296(3.2);1.278(6.7);1.260(3.3);0.008(0.7);0.000 (24.6);-0.008(0.7) |
| I-31 | ¹H-NMR(400.0 MHz, CDCl3): |
| | δ= 7.520(1.5);7.343(1.5);7.322(1.6);7.305(1.7);7.284(1.9);7.261(268.6);7.179(1.2);7.177 (1.2);7.166(5.7);7.164(8.0);7.161(9.1);7.158(8.3);7.147(8.5);7.144(8.8);7.141(7.4);7.139 (5.1);7.129(1.0);6.997(1.5);6.906(1.3);6.900(2.2);6.896(1.6);6.890(2.3);6.884(3.3);6.878 (4.5);6.874(3.2);6.869(4.3);6.863(3.1);6.857(2.3);6.853(1.6);6.847(2.1);6.841(0.9);6.168 (4.3);6.156(4.4);6.141(4.9);6.130(5.1);6.124(5.4);6.113(5.2);6.098(5.5);6.086(5.4);5.544 (7.8);5.543(6.8);5.532(7.5);5.531(6.8);5.501(6.8);5.500(6.0);5.489(6.7);5.355(7.2);5.346 (7.0);5.328(6.8);5.319(6.4);5.299(16.0);4.609(3.9);4.598(7.8);4.588(5.5);4.585(6.1);4.57 5(8.8);4.565(5.7);4.553(2.3);4.546(2.1);4.540(1.5);4.527(1.2);4.380(0.5);4.150(1.0);4.13 2(2.6);4.115(2.7);4.103(3.0);4.097(1.2);4.090(3.0);4.079(7.4);4.066(7.2);4.056(4.9);4.04 2(4.5);4.012(3.1);4.007(3.1);3.988(2.1);3.983(4.6);3.976(3.1);3.958(1.9);3.952(1.8);3.92 6(7.3);3.915(7.6);3.884(8.4);3.872(8.6);3.698(0.6);3.332(7.6);3.325(7.6);3.289(6.6);3.28 2(6.7);2.681(1.5);2.664(1.6);2.657(1.6);2.646(2.1);2.640(3.3);2.629(2.0);2.623(3.4);2.61 6(1.7);2.605(3.7);2.599(1.8);2.588(1.8);2.582(1.8);2.564(1.6);2.226(1.3);2.219(2.1);2.21 0(1.3);2.192(1.2);2.184(3.0);2.176(3.1);2.167(1.4);2.150(1.1);2.141(1.8);2.133(1.1);2.11 6(4.1);2.046(10.8);1.432(3.6);1.304(1.2);1.277(5.0);1.265(5.9);1.259(10.0);1.242(3.6);0. 899(3.2);0.882(11.0);0.864(4.2);0.008(2.5);0.000(95.7);-0.008(2.8) |
| I-32 | ¹H-NMR(400.0 MHz, CDCl3): |
| | δ= 7.487(0.6);7.466(0.7);7.434(0.6);7.399(1.6);7.395(1.9);7.392(1.6);7.388(1.8);7.383(1. 7);7.380(1.8);7.375(5.0);7.372(5.4);7.370(5.0);7.366(5.9);7.360(2.1);7.357(2.4);7.261(5 8.8);7.149(0.5);7.146(0.6);7.139(1.0);7.134(0.9);7.126(1.2);7.122(1.1);7.119(1.2);7.113( 1.0);7.107(0.9);7.102(0.5);5.144(1.2);5.128(1.6);5.112(1.2);5.091(0.5);5.075(1.3);5.060( 1.7);5.044(1.3);5.028(0.5);4.602(0.6);4.595(0.9);4.588(1.1);4.582(1.0);4.577(1.1);4.570( 1.1);4.563(0.9);4.557(0.7);4.550(0.5);4.503(1.1);4.493(1.2);4.478(1.8);4.468(1.9);4.454( 1.3);4.444(1.3);4.082(1.1);4.068(1.1);4.059(1.8);4.045(1.6);4.032(1.1);4.018(1.1);4.008( 1.5);3.995(1.4);3.967(1.1);3.961 (1.1);3.944(0.8);3.937(0.7);3.889(1.0);3.882(1.0);3.865( 0.7);3.859(0.7);3.803(2.8);3.791(2.5);3.760(3.2);3.748(2.9);3.221(2.8);3.212(3.1);3.178( 2.4);3.169(2.7);2.599(0.6);2.581(0.6);2.576(0.6);2.565(0.7);2.558(0.7);2.555(0.7);2.547( 0.7);2.541(0.7);2.536(0.7);2.531(0.7);2.523(0.7);2.520(0.8);2.513(0.7);2.502(0.7);2.497( 0.7);2.478(0.6);2.056(0.7);2.022(0.6);1.958(0.8);1.923(0.7);1.708(16.0);1.685(14.4);1.56 9(9.5);1.327(7.3);1.314(8.2);1.311(8.4);1.299(7.5);1.290(8.2);1.274(8.0);1.262(0.6);1.25 5(0.6);1.247(0.5);1.212(8.0);1.196(7.9);0.008(0.6);0.000(19.7);-0.008(0.8) |
| I-33 | ¹H-NMR(400.0 MHz, CDCl3): |
| | δ= 7.519(0.7);7.387(0.5);7.373(0.8);7.364(1.0);7.352(0.6);7.260(128.7);6.996(0.7);3.797 (0.5);3. 754(0.6);3. 743(0.5);3.220(0.5);3.211(0.6);3.177(0.5);3.168(0.5); 1.918(0.6); 1.704 (3.4);1.682(3.1);1.540(16.0);1.520(9.8);1.465(10.8);1.369(0.6);1.335(0.6);1.255(0.9);1.1 34(0.5);1.106(0.5);0.008(1.6);0.000(47.3);-0.008(1.9) |
| I-34 | ¹H-NMR(400.0 MHz, CDCl3): |
| | δ= 7.483(0.6);7.464(0.6);7.448(0.6);7.427(0.5);7.402(1.0);7.387(4.8);7.384(4.6);7.376(4. 5);7.369(7.4);7.365(7.2);7.352(3.1);7.347(1.8);7.340(1.0);7.331(1.5);7.328(1.1);7.318(1 5.6);7.260(69.1);7.151(0.5);7.142(0.8);7.135(0.8);7.128(1.1);7.123(1.0);7.120(1.4);7.114 (1.1);7.108(0.8);7.103(0.5);7.097(0.7);5.304(1.3);5.274(3.0);5.228(3.0);5.197(1.4);5.182 (5.2);5.179(5.6);4.591(0.6);4.586(1.6);4.576(1.8);4.566(2.2);4.563(2.0);4.556(2.1);4.553 (1.8);4.542(1.6);4.533(1.3);4.091(1.1);4.077(1.1);4.068(1. 7);4.054(1.6);4.040(1.0);4.026 (1.0);4.016(1.4);4.002(1.3);3.968(1.1);3.961(1.1);3.944(0.7);3.938(0.7);3.897(0.9);3.890 (0.9);3.874(0.6);3.866(0.6);3.803(2.8);3.785(2.4);3.760(3.2);3.742(2.7);3.213(2.8);3.210 (3.3);3.170(2.4);3.167(2.8);2.602(0.5);2.584(0.6);2.579(0.5);2.568(0.6);2.561(0.6);2.556 (0.6);2.550(0.6);2.544(0.7);2.538(0.7);2.533(0.7);2.527(0.6);2.522(0.8);2.515(0.7);2.503 (0.7);2.498(0. 7);2.480(0.6);2.079(0.7);2.045(0.6);2.019(0.8);1.984(0.7);1.701(16.0);1.64 7(13.2);1.560(16.4);0.008(0.8);0.000(23.7);-0.008(0.7) |
| I-35 | ¹H-NMR(400.0 MHz, CDCl3): |
| | δ= 7.519(1.7);7.477(1.5);7.452(2.5);7.430(1.8);7.260(283.4);7.228(5.0);7.210(0.7);7.173 (8.0);7.158(8.0);6.996(1.6);6.901(2.3);6.880(4.7);6.858(2.4);6.175(3.6);6.148(4.0);6.132 (4.4);6.105(4.5);5.558(4.5);5.540(5.0);5.515(3.9);5.497(4.4);5.354(4.2);5.335(4.9);5.328 (4.3);5.309(4.5);4.595(2.6);4.546(2.4);4.537(2.8);4.530(2.5);4.522(4.5);4.513(2.8);4.507 (2.6);4.498(2.3);4.292(2.1);4.274(7.8);4.256(11.6);4.238(7.8);4.221(2.2);4.061(1.8);4.04 8(2.0);4.038(3.5);4.024(3.5);4.017(3.5);4.004(3.1);3.947(2.4);3.922(8.1);3.900(6.2);3.87 9(4.8);3.857(5.2);3.320(5.1);3.310(4.6);3.277(4.4);3.267(4.1);2.588(1.1);2.570(1.4);2.56 4(1.6);2.554(1.7);2.547(1.7);2.536(2.5);2.530(2.1);2.520(1.4);2.512(1.7);2.484(1.2);2.07 2(2.0);2.031(2.4);1.990(1.5);1.537(40.6);1.350(8.0);1.332(16.0);1.318(9.5);1.314(9.5);1. 300(14.6);1.282(7.4);1.258(1.3);0.146(0.5);0.000(103.5) |
| I-36 | ¹H-NMR(400.0 MHz, CDCl3): |
| | δ= 7.518(1.5);7.460(2.0);7.435(1.3);7.260(234.7);7.226(0.6);7.211(1.0);7.174(5.8);7.160 (5.8);6.996(1.3);6.900(1.5);6.878(3.1);6.856(1.6);6.176(2.8);6.150(3.0);6.133(3.2);6.106 (3.4);5.560(3.3);5.544(3.5);5.518(2.9);5.501(3.0);5.354(3.2);5.329(5.3);5.303(3.1);5.142 (0.6);5.126(1.5);5.110(2.1);5.102(1.7);5.095(1.8);5.086(2.0);5.071(1.6);5.055(0.6);4.602 (2.0);4.502(1.6);4.493(2.0);4.487(1.9);4.478(3.1);4.470(2.0);4.464(1.9);4.455(1.6);4.064 (1.3);4.050(1.4);4.040(3.4);4.027(3.4);4.016(2.2);4.003(2.0);3.946(1.9);3.923(5.9);3.901 (4.2);3.880(3.4);3.858(3.5);3.320(3.4);3.310(3.4);3.277(3.0);3.267(3.0);2.583(0.8);2.549 (1.2);2.531(1.8);2.501(1.2);2.478(0.8);2.026(1.4);1.985(2.1);1.949(1.2);1.536(39.5);1.31 8(10.2);1.304(16.0);1.298(13.5);1.290(11.4);1.282(11.1);1.256(1.2);1.240(9.5);1.224(9.5 );0.000(88.1) |
| I-37 | ¹H-NMR(400.0 MHz, CDCl3): |
| | δ= 7.469(0.8);7.448(1.4);7.428(0.8);7.385(3.1);7.383(3.4);7.379(3.7);7.372(9.3);7.359(2. 1);7.350(2.5);7.338(3.4);7.332(18.3);7.308(0.6);7.260(75.8);7.192(0.6);7.186(0.7);7.180 (2.2);7.174(4.2);7.169(3.1);7.164(2.5);7.159(3.2);7.154(4.1);7.148(2.1);7.142(0.7);6.902 (0.9);6.897(0.9);6.891(0.9);6.886(1.3);6.880(1.8);6.875(1.7);6.869(1.7);6.864(1.2);6.859 (0.9);6.853(0.9);6.848(0.8);6.168(1.6);6.152(1.6);6.141(1.7);6.125(3.4);6.109(1.9);6.098 (1.9);6.082(1.9);5.552(3.0);5.537(2.9);5.509(2.6);5.494(2.5);5.348(2.8);5.321(3.0);5.318 (3.1);5.291 (2.6);5.274(1.4);5.252(1.5);5.244(4.8);5.220(7.7);5.198(4.8);5.189(1.5);5.167 (1.4);4.618(0.8);4.611(1.0);4.604(1.0);4.598(1.3);4.592(1.3);4.585(2.3);4.576(3.4);4.567 (2.0);4.562(1.8);4.553(3.0);4.544(1.4);4.074(1.3);4.060(1.2);4.050(2.8);4.036(2.5);4.025 (1.9);4.012(1.8);3.954(1.3);3.947(1.3);3.930(2.2);3.921(4.2);3.906(1.0);3.898(3.7);3.878 (3.4);3.855(3.3);3.318(3.1);3.306(3.1);3.275(2.7);3.263(2.7);2.590(0.6);2.573(0.7);2.567 (0.7);2.560(0.8);2.556(0.9);2.549(0.8);2.542(0.9);2.53 8(1.3);2.532(0.9);2.526(0.9);2.519 (0.8);2.515(0.8);2.508(0.8);2.502(0.8);2.484(0.7);2.072(0.6);2.065(1.0);2.058(0.6);2.051 (0.7);2.043(1.1);2.037(1.0);2.022(0.6);2.017(0.6);2.009(0.9);2.002(0.5);1.554(16.0);0.00 8(1.0);0.000(27.3);-0.008(1.0) |
| I-38 | ¹H-NMR(400.0 MHz, CDCl3): |
| | δ= 7.518(16.0);7.454(1.7);7.321(2.2);7.297(3.1);7.260(2792.7);7.230(2.9);7.210(9.5);7.2 01(2.0);7.177(3.3);7.172(3.7);7.166(4.1);7.160(5.6);7.146(5.5);6.996(15.6);6.898(1.8);6. 876(2.8);6.175(3.9);6.148(3.6);6.132(4.2);6.105(3.9);5.559(4.5);5.540(4.0);5.516(4.3);5. 497(3.5);5.349(4.3);5.321(6.3);5.293(3.2);4.610(1.9);4.432(1.7);4.423(2.4);4.418(2.3);4. 409(3.9);4.399(2.0);4.394(2.5);4.385(2.2);4.065(2.1);4.052(2.5);4.042(3.3);4.028(3.7);4. 011(3.4);3.944(2.3);3.915(6.8);3.894(5.0);3.872(6.0);3.852(4.5);3.493(1.9);3.319(4.5);3. 308(5.1);3.276(3.8);3.265(4.4);2.535(1.8);2.517(1.8);2.501(2.1);2.481(1.6);1.949(5.4);1. 915(4.5);1.713(4.1);1.679(5.0);1.617(2.8);1.593(2.8);1.574(3.2);1.537(678.7);1.505(87.4 );1.479(101.8);1.398(2.3);1.369(5.2);1.335(5.1);1.306(3.1);1.254(13.8);1.193(2.0);1.166 (3.2);1.135(4.2);1.105(4.8);1.079(4.0);0.880(1.7);0.146(3.5);0.008(31.6);0.000(1031.9);-0.008(30.7);-0.050(3.7);-0.150(3.8) |
| I-39 | ¹H-NMR(400.0 MHz, CDCl3): |
| | δ= 7.5181 (3.2); 7.3998 (1.1); 7.3867 (2.5); 7.3811 (3.4); 7.3771 (3.1); 7.3707 (3.0); 7.3616 (1.5); 7.3073 (0.9); 7.2880 (0.9); 7.2592 (562.4); 7.2336 (0.7); 7.2088 (1.0); 7.2001 (0.6); 7.1603 (1.1); 7.1383 (0.9); 7.1308 (0.7); 7.1227 (0.8); 6.9953 (3.6); 5.9486 (1.8); 5.9409 (1.8); 5.8861 (1.1); 5.8789 (1.1); 5.2426 (0.8); 5.2314 (0.6); 5.2211 (0.6); 5.2124 (0.5); 4.6185 (0.6); 4.6035 (0.8); 4.5964 (0.6); 4.5766 (1.0); 4.5539 (0.8); 4.3293 (0.6); 4.3195 (0.6); 4.3024 (0.6); 4.2342 (1.1); 4.2240 (1.1); 4.2074 (1.0); 4.1973 (0.9); 3.8522 (13.3); 3.8423 (8.0); 3.8080 (1.2); 3.7891 (1.8); 3.7649 (1.3); 3.7458 (2.1); 3.6913 (0.6); 3.2485 (2.1); 3.2434 (1.4); 3.2052 (1.8); 3.2001 (1.1); 2.4632 (1.1); 2.3297 (1.0); 1.7155 (16.0); 1.7038 (0.8); 1.5291 (89.5); 1.3237 (0.8); 1.3064 (0.7); 1.2559 (0.7); 0.1460 (0.9); 0.0079 (6.9); -0.0002 (200.7); -0.0084 (6.5); -0.1498 (0.9) |
| I-40 | ¹H-NMR(599.8 MHz, CDCl3): |
| | δ= 7.4820 (2.4); 7.4684 (2.5); 7.4487 (2.4); 7.4353 (2.4); 7.4046 (1.0); 7.3998 (1.3); 7.3962 (1.2); 7.3915 (4.1); 7.3866 (6.8); 7.3826 (9.1); 7.3816 (9.1); 7.3780 (12.0); 7.3758 (12.0); 7.3717 (12.8); 7.3672 (14.8); 7.3579 (2.5); 7.3553 (2.1); 7.2849 (5.3); 7.1506 (1.4); 7.1462 (2.9); 7.1436 (2.9); 7.1419 (2.6); 7.1381 (2.7); 7.1367 (2.7); 7.1321 (5.0); 7.1279 (4.7); 7.1212 (3.0); 7.1167 (2.7); 7.1124 (1.3); 7.1078 (0.3); 4.6805 (0.5); 4.6718 (1.5); 4.6672 (2.4); 4.6634 (3.0); 4.6594 (3.4); 4.6550 (3.8); 4.6512 (3.5); 4.6473 (2.9); 4.6429 (2.4); 4.6393 (1.7); 4.6298 (0.6); 4.1557 (3.8); 4.1474 (3.9); 4.1401 (4.8); 4.1318 (4.5); 4.1271 (3.8); 4.1185 (3.8); 4.1113 (4.4); 4.1027 (4.1); 4.0077 (0.3); 3.9767 (3.9); 3.9624 (3.1); 3.9614 (3.1); 3.9010 (3.4); 3.8990 (3.6); 3.8867 (50.0); 3.8552 (49.2); 3.8453 (0.8); 3.8328 (2.0); 3.8078 (0.4); 3.7963 (7.8); 3.7759 (7.7); 3.7675 (8.7); 3.7607 (0.6); 3.7471 (8.3); 3.7291 (0.3); 3.3491 (48.2); 3.3405 (48.2); 3.2449 (0.4); 3.2368 (8.2); 3.2253 (8.6); 3.2186 (0.7); 3.2163 (0.6); 3.2080 (7.5); 3.2021 (0.6); 3.1965 (7.7); 2.5589 (3.2); 2.5468 (3.4); 2.5351 (3.9); 2.5231 (3.7); 2.5024 (3.4); 2.4902 (3.5); 2.4785 (4.0); 2.4664 (3.8); 2.2319 (2.9); 2.2308 (3.0); 2.2264 (3.0); 2.2082 (2.6); 2.2071 (2.6); 2.2027 (2.6); 2.1546 (2.9); 2.1533 (3.0); 2.1493 (3.1); 2.1481 (2.9); 2.1307 (2.6); 2.1294 (2.7); 2.1255 (2.8); 1.8553 (1.3); 1.7169 (40.5); 1.6954 (39.1); 1.3373 (0.8); 1.2871 (1.3); 1.2573 (3.3); 0.8920 (0.4); 0.8808 (0.9); 0.8689 (0.5); 0.8424 (0.3); - 0.0001 (4.5) |
| I-41 | ¹H-NMR(400.0 MHz, CDCl3): |
| | δ= 7.5185 (0.9); 7.3305 (0.6); 7.3101 (0.9); 7.2935 (0.8); 7.2901 (0.8); 7.2596 (159.2); 7.1701 (0.7); 7.1642 (0.6); 7.1577 (3.3); 7.1520 (4.0); 7.1488 (2.3); 7.1411 (2.2); 7.1378 (4.0); 7.1322 (3.3); 7.1197 (0.5); 6.9956 (0.9); 6.8966 (0.8); 6.8885 (0.8); 6.8808 (1.0); 6.8749 (1.6); 6.8668 (1.7); 6.8609 (0.9); 6.8532 (0.8); 6.8451 (0.8); 5.2983 (0.6); 4.6116 (1.2); 4.6013 (1.4); 4.5885 (2.0); 4.5793 (1.9); 4.5660 (1.5); 4.5561 (1.7); 4.5443 (0.8); 4.5381 (0.9); 4.5332 (0.9); 4.5265 (0.9); 4.5204 (0.9); 4.5131 (0.8); 4.5085 (0.5); 4.1133 (1.1); 4.1002 (1.1); 4.0894 (2.0); 4.0763 (1.9); 4.0733 (1.4); 4.0599 (1.2); 4.0492 (1.8); 4.0357 (2.0); 4.0261 (1.2); 4.0094 (0.7); 4.0022 (0.7); 3.9682 (1.1); 3.9624 (1.1); 3.9441 (0.7); 3.9372 (0.7); 3.7796 (2.9); 3.7729 (2.9); 3.7363 (3.3); 3.7296 (3.3); 3.1984 (3.0); 3.1935 (3.0); 3.1551 (2.6); 3.1502 (2.7); 2.6774 (0.6); 2.6601 (0.7); 2.6537 (0.6); 2.6427 (0.8); 2.6365 (0.7); 2.6257 (0.8); 2.6191 (0.8); 2.6112 (0.6); 2.6048 (0.7); 2.6020 (0.8); 2.5939 (0.8); 2.5875 (0.7); 2.5766 (0.7); 2.5703 (0.7); 2.5529 (0.6); 2.2721 (1.0); 2.2378 (0.7); 2.1741 (0.8); 2.1396 (0.7); 2.1109 (7.6); 1.7027 (15.9); 1.6873 (16.0); 1.4319 (4.0); 1.2567 (0.7); 0.8820 (0.9); 0.0080 (1.5); -0.0002 (46.7); -0.0085 (1.4) |
| I-42 | ¹H-NMR(400.0 MHz, CDCl3): |
| | δ= 7.5186 (1.0); 7.5024 (0.6); 7.4829 (0.6); 7.4416 (0.8); 7.4215 (0.8); 7.2598 (167.2); 7.2093 (0.6); 7.1693 (2.1); 7.1639 (2.6); 7.1496 (3.8); 7.1370 (2.2); 7.1315 (1.8); 6.9958 (0.9); 6.8933 (1.0); 6.8888 (1.0); 6.8772 (1.0); 6.8718 (2.0); 6.8669 (2.0); 6.8554 (0.6); 6.8500 (1.0); 6.8454 (0.9); 4.5805 (1.1); 4.4363 (1.2); 4.4274 (1.4); 4.4131 (2.3); 4.4042 (2.4); 4.3899 (1.1); 4.3808 (1.1); 4.0768 (0.9); 4.0631 (0.9); 4.0533 (1.5); 4.0394 (1.7); 4.0231 (1.2); 4.0132 (1.7); 3.9994 (1.6); 3.9694 (1.1); 3.9633 (1.2); 3.9467 (0.8); 3.9395 (0.7); 3.8931 (1.3); 3.8869 (1.4); 3.8694 (0.9); 3.8631 (1.1); 3.7676 (2.2); 3.7564 (2.7); 3.7244 (2.6); 3.7133 (3.1); 3.1860 (2.9); 3.1770 (2.6); 3.1428 (2.6); 3.1338 (2.3); 2.5651 (0.6); 2.5472 (0.7); 2.5417 (0.7); 2.5311 (1.0); 2.5239 (0.8); 2.5127 (1.1); 2.4992 (0.7); 2.4910 (1.0); 2.4807 (0.7); 2.4756 (0.7); 2.4573 (0.6); 2.2528 (0.6); 2.0199 (1.0); 1.9855 (0.9); 1.9132 (0.8); 1.8784 (0.7); 1.7070 (13.3); 1.6833 (16.0); 1.5392 (24.4); 1.5194 (53.5); 1.4680 (43.6); 1.4445 (0.7); 1.4275 (0.7); 1.2552 (0.7); 1.2049 (0.7); 1.1885 (0.8); 1.1827 (2.5); 1.1713 (2.3); 1.1525 (1.0); 1.1368 (1.0); 0.0079 (2.2); -0.0002 (58.9) |
| I-43 | ¹H-NMR(400.6 MHz, CDCl3): |
| | δ= 7.5219 (0.6); 7.5011 (0.7); 7.4886 (0.7); 7.4681 (0.6); 7.2990 (1.4); 7.1903 (0.5); 7.1780 (2.9); 7.1724 (3.6); 7.1691 (2.1); 7.1616 (2.1); 7.1580 (3.6); 7.1525 (2.9); 7.1460 (0.5); 7.1402 (0.5); 6.9069 (0.7); 6.9011 (1.3); 6.8953 (0.7); 6.8852 (1.5); 6.8794 (2.6); 6.8736 (1.3); 6.8634 (0.8); 6.8577 (1.3); 6.8519 (0.6); 4.6076 (0.6); 4.6031 (0.7); 4.6008 (0.6); 4.5967 (0.8); 4.5933 (0.7); 4.5899 (1.0); 4.5867 (0.9); 4.5832 (1.0); 4.5761 (0.8); 4.5727 (0.7); 4.5697 (0.7); 4.5660 (0.6); 4.5590 (0.6); 4.5559 (1.3); 4.5466 (1.4); 4.5320 (2.2); 4.5226 (2.2); 4.5082 (1.3); 4.4989 (1.2); 4.3114 (0.9); 4.3099 (1.0); 4.2936 (2.9); 4.2921 (3.0); 4.2757 (3.0); 4.2745 (3.0); 4.2577 (1.1); 4.2567 (1.1); 4.2531 (1.2); 4.2353 (3.7); 4.2176 (3.9); 4.1998 (1.4); 4.0770 (1.1); 4.0638 (1.1); 4.0535 (1.8); 4.0403 (1.7); 4.0340 (1.2); 4.0207 (1.2); 4.0104 (1.7); 3.9971 (1.6); 3.9801 (1.1); 3.9749 (1.0); 3.9734 (1.0); 3.9566 (0.7); 3.9514 (0.6); 3.9499 (0.6); 3.9089 (0.9); 3.9075 (1.0); 3.9024 (1.0); 3.9009 (0.9); 3.8854 (0.6); 3.8838 (0.7); 3.8788 (0.7); 3.8774 (0.6); 3.8340 (1.0); 3.7866 (2.9); 3.7836 (1.3); 3.7732 (2.6); 3.7435 (3.3); 3.7300 (3.0); 3.2065 (2.8); 3.1990 (3.0); 3.1634 (2.5); 3.1559 (2.6); 2.6102 (0.6); 2.5926 (0.6); 2.5868 (0.6); 2.5758 (0.8); 2.5691 (0.8); 2.5665 (0.7); 2.5582 (0.7); 2.5524 (0.8); 2.5484 (0.7); 2.5430 (0.7); 2.5348 (0.8); 2.5320 (0.9); 2.5251 (0.7); 2.5139 (0.7); 2.5086 (0.7); 2.4906 (0.6); 2.1117 (0.8); 2.1095 (0.5); 2.0773 (0.6); 2.0132 (0.8); 2.0063 (0.5); 1.9969 (0.6); 1.9787 (0.7); 1.7143 (16.0); 1.6904 (14.9); 1.3614 (5.6); 1.3436 (11.6); 1.3257 (5.4); 1.3047 (5.8); 1.2869 (12.0); 1.2691 (5.7); 1.2597 (0.8); -0.0002 (1.0) |
| I-44 | ¹H-NMR(400.6 MHz, CDCl3): |
| | δ= 7.5274 (0.7); 7.5067 (0.8); 7.4922 (0.8); 7.4716 (0.7); 7.3034 (1.2); 7.1783 (2.8); 7.1745 (3.5); 7.1728 (3.3); 7.1603 (3.1); 7.1586 (3.6); 7.1548 (2.9); 7.1532 (2.4); 6.9036 (0.6); 6.8991 (1.0); 6.8979 (1.0); 6.8935 (0.6); 6.8922 (0.6); 6.8832 (1.1); 6.8819 (1.3); 6.8774 (2.0); 6.8762 (2.1); 6.8718 (1.1); 6.8704 (1.1); 6.8614 (0.6); 6.8601 (0.7); 6.8558 (1.0); 6.8544 (1.1); 6.8501 (0.5); 6.8488 (0.5); 5.1433 (1.3); 5.1277 (1.8); 5.1120 (1.3); 5.0961 (0.7); 5.0947 (0.7); 5.0788 (1.4); 5.0631 (1.8); 5.0475 (1.4); 5.0318 (0.5); 4.6095 (0.7); 4.6023 (0.9); 4.5987 (0.9); 4.5952 (1.0); 4.5918 (1.1); 4.5889 (1.0); 4.5846 (1.0); 4.5820 (0.9); 4.5779 (0.8); 4.5748 (0.7); 4.5712 (0.6); 4.5124 (1.2); 4.5030 (1.4); 4.4888 (2.5); 4.4793 (2.6); 4.4651 (1.4); 4.4558 (1.3); 4.0796 (1.1); 4.0663 (1.1); 4.0561 (1.9); 4.0428 (1.7); 4.0321 (1.2); 4.0187 (1.1); 4.0085 (1.7); 3.9951 (1.6); 3.9824 (1.2); 3.9765 (1.2); 3.9589 (0.8); 3.9533 (0.7); 3.9036 (1.1); 3.8980 (1.0); 3.8800 (0.7); 3.8745 (0.7); 3.7882 (2.9); 3.7840 (0.5); 3.7769 (2.7); 3.7451 (3.3); 3.7337 (3.1); 3.2084 (2.9); 3.2001 (3.1); 3.1652 (2.6); 3.1569 (2.7); 2.6063 (0.6); 2.5885 (0.6); 2.5830 (0.6); 2.5720 (0.8); 2.5651 (0.7); 2.5610 (0.7); 2.5542 (0.7); 2.5486 (0.8); 2.5428 (0.7); 2.5376 (0.7); 2.5308 (0.7); 2.5265 (0.9); 2.5195 (0.7); 2.5083 (0.7); 2.5032 (0.7); 2.4850 (0.6); 2.0692 (0.8); 2.0622 (0.5); 2.0443 (0.9); 2.0348 (0.8); 1.9781 (0.5); 1.9712 (0.8); 1.9641 (0.5); 1.9626 (0.5); 1.9367 (0.7); 1.7159 (16.0); 1.6923 (15.1); 1.3290 (8.0); 1.3156 (9.6); 1.3136 (9.8); 1.3002 (8.8); 1.2970 (9.4); 1.2812 (8.3); 1.2594 (0.6); 1.2582 (0.6); 1.2217 (8.2); 1.2060 (8.2); -0.0002 (0.9) |
| I-45 | ¹H-NMR(400.0 MHz, CDCl3): |
| | δ= 7.5182 (1.0); 7.4766 (0.5); 7.4574 (0.6); 7.4411 (0.6); 7.4206 (0.6); 7.4015 (0.8); 7.3897 (2.3); 7.3853 (4.0); 7.3813 (3.4); 7.3750 (1.3); 7.3723 (3.1); 7.3701 (3.5); 7.3646 (1.0); 7.3572 (1.2); 7.3547 (1.7); 7.3514 (1.6); 7.3487 (1.4); 7.3436 (1.0); 7.3313 (2.3); 7.3254 (13.2); 7.3128 (0.7); 7.3062 (0.5); 7.2935 (0.6); 7.2593 (174.4); 7.1696 (1.9); 7.1661 (2.9); 7.1637 (2.9); 7.1604 (3.1); 7.1528 (1.5); 7.1497 (3.0); 7.1463 (2.9); 7.1441 (2.9); 7.1405 (1.8); 6.9953 (1.0); 6.8960 (0.8); 6.8899 (0.8); 6.8839 (0.8); 6.8801 (1.0); 6.8743 (1.6); 6.8682 (1.5); 6.8622 (1.5); 6.8564 (0.9); 6.8526 (0.8); 6.8464 (0.7); 6.8405 (0.8); 5.3028 (1.6); 5.2723 (3.6); 5.2284 (4.1); 5.1973 (5.2); 5.1839 (4.2); 5.1533 (0.8); 4.5944 (0.6); 4.5894 (1.9); 4.5800 (2.0); 4.5765 (1.3); 4.5717 (2.0); 4.5659 (2.1); 4.5626 (2.2); 4.5565 (2.0); 4.5486 (1.6); 4.5391 (1.5); 4.0842 (1.1); 4.0709 (1.1); 4.0607 (1.8); 4.0473 (1.6); 4.0356 (1.2); 4.0222 (1.2); 4.0119 (1.7); 3.9985 (1.5); 3.9693 (1.0); 3.9637 (1.0); 3.9458 (0.7); 3.9399 (0.7); 3.9022 (1.0); 3.8967 (1.0); 3.8785 (0.7); 3.8731 (0.7); 3.7711 (2.8); 3.7541 (2.8); 3.7280 (3.2); 3.7110 (3.2); 3.1777 (3.1); 3.1733 (3.1); 3.1346 (2.7); 3.1302 (2.7); 2.5979 (0.6); 2.5800 (0.6); 2.5746 (0.6); 2.5634 (0.8); 2.5563 (1.0); 2.5456 (0.7); 2.5400 (0.8); 2.5373 (0.8); 2.5321 (0.7); 2.5212 (1.1); 2.5141 (0.7); 2.5027 (0.7); 2.4976 (0.7); 2.4795 (0.6); 2.0772 (0.8); 2.0428 (0.7); 2.0327 (0.7); 2.0231 (0.8); 1.9886 (0.7); 1.7099 (0.6); 1.7009 (16.0); 1.6443 (16.0); 1.5408 (30.6); 1.2557 (0.5); 0.0080 (1.9); -0.0002 (61.3); -0.0085 (1.8) |
| I-46 | ¹H-NMR(400.0 MHz, CDCl3): |
| | δ= 7.5183 (1.2); 7.4000 (1.1); 7.3866 (2.4); 7.3814 (3.3); 7.3772 (3.0); 7.3726 (2.5); 7.3705 (3.1); 7.3650 (1.1); 7.3617 (1.4); 7.2595 (199.8); 7.1662 (0.6); 7.1600 (0.8); 7.1475 (0.6); 7.1451 (0.7); 7.1368 (0.8); 7.1305 (0.6); 7.1224 (0.8); 6.9954 (1.5); 5.9487 (1.6); 5.9411 (1.6); 5.8864 (1.3); 5.8787 (1.3); 5.2429 (0.6); 5.2304 (0.6); 5.2226 (0.6); 5.2204 (0.5); 5.2104 (0.5); 4.6454 (0.6); 4.6231 (0.6); 4.6189 (0.7); 4.6036 (0.7); 4.5963 (0.6); 4.5807 (0.7); 4.5768 (0.9); 4.5542 (0.7); 4.3294 (0.8); 4.3197 (0.8); 4.3027 (0.7); 4.2930 (0.7); 4.2343 (1.0); 4.2243 (1.0); 4.2075 (0.9); 4.1975 (0.9); 3.8592 (0.5); 3.8520 (14.0); 3.8421 (11.8); 3.8082 (1.5); 3.7892 (1.8); 3.7649 (1.7); 3.7459 (2.1); 3.2487 (2.0); 3.2436 (1.7); 3.2054 (1.7); 3.2003 (1.4); 2.0436 (1.1); 1.7157 (16.0); 1.5330 (11.1); 1.2765 (0.5); 1.2586 (1.3); 0.8819 (0.8); 0.0080 (2.3); -0.0002 (73.4); -0.0085 (2.2) |
| I-47 | ¹H-NMR(400.0 MHz, CDCl3): |
| | δ= 7.4477 (0.6); 7.4344 (0.6); 7.4155 (0.7); 7.4110 (0.7); 7.3960 (1.4); 7.3912 (1.9); 7.3889 (1.7); 7.3826 (2.0); 7.3779 (2.5); 7.3722 (3.0); 7.3673 (5.3); 7.3610 (3.5); 7.3520 (0.6); 7.3479 (0.5); 7.2625 (29.8); 7.1554 (0.8); 7.1497 (0.8); 7.1456 (0.5); 7.1421 (0.5); 7.1393 (0.6); 7.1352 (0.9); 7.1310 (1.0); 7.1277 (0.8); 7.1255 (0.8); 7.1172 (0.6); 7.1118 (0.6); 4.6657 (0.6); 4.6604 (0.7); 4.6525 (0.8); 4.6474 (0.8); 4.6402 (0.7); 4.6346 (0.6); 4.1581 (1.0); 4.1458 (1.0); 4.1345 (1.4); 4.1303 (1.1); 4.1222 (1.2); 4.1175 (1.0); 4.1066 (1.2); 4.0938 (1.1); 3.9763 (0.8); 3.9514 (0.7); 3.8892 (15.7); 3.8757 (0.8); 3.8727 (0.8); 3.8703 (0.7); 3.8592 (16.0); 3.8414 (0.8); 3.8008 (2.1); 3.7795 (2.0); 3.7576 (2.3); 3.7363 (2.3); 3.3514 (15.6); 3.3424 (15.2); 3.2389 (2.2); 3.2272 (2.2); 3.1956 (1.9); 3.1839 (1.9); 2.5668 (0.8); 2.5487 (0.9); 2.5311 (1.1); 2.5123 (1.5); 2.4932 (0.9); 2.4755 (1.1); 2.4573 (1.0); 2.2298 (0.7); 2.2275 (0.7); 2.2215 (0.7); 2.2192 (0.7); 2.1941 (0.6); 2.1917 (0.6); 2.1858 (0.6); 2.1834 (0.6); 2.1532 (0.7); 2.1506 (0.7); 2.1453 (0.7); 2.1428 (0.7); 2.1173 (0.6); 2.1148 (0.6); 2.1095 (0.6); 2.1069 (0.6); 1.7164 (11.9); 1.6946 (11.4); -0.0002 (10.0) |
| I-48 | ¹H-NMR(400.0 MHz, CDCl3): |
| | δ= 7.2618 (68.4); 7.1797 (0.5); 7.1715 (2.4); 7.1676 (3.6); 7.1620 (2.6); 7.1571 (2.1); 7.1543 (2.6); 7.1518 (3.5); 7.1480 (3.2); 7.1419 (1.5); 7.1353 (0.5); 6.9978 (0.9); 6.9792 (0.6); 6.9142 (0.8); 6.9086 (0.8); 6.9056 (0.5); 6.9037 (0.6); 6.8982 (1.2); 6.8925 (1.8); 6.8869 (1.5); 6.8839 (0.9); 6.8819 (1.0); 6.8779 (1.3); 6.8766 (1.4); 6.8708 (1.3); 6.8653 (0.8); 6.8603 (0.5); 6.8564 (0.6); 6.8549 (0.6); 4.6416 (0.6); 4.6209 (1.4); 4.6031 (1.4); 4.5801 (1.3); 4.5744 (0.8); 4.5703 (1.2); 4.5610 (0.8); 4.5555 (2.0); 4.5462 (1.7); 4.5369 (0.9); 4.5310 (1.5); 4.5221 (1.0); 4.1650 (0.9); 4.1522 (0.9); 4.1411 (1.1); 4.1359 (1.0); 4.1283 (1.0); 4.1230 (0.9); 4.1119 (1.1); 4.0989 (1.0); 4.0662 (0.7); 4.0532 (0.6); 4.0426 (1.1); 4.0299 (1.6); 4.0172 (0.8); 4.0066 (1.0); 3.9935 (1.0); 3.9680 (0.7); 3.9635 (0.6); 3.9010 (0.6); 3.8965 (0.6); 3.8555 (0.7); 3.8515 (0.6); 3.8489 (0.6); 3.8342 (13.9); 3.8276 (0.9); 3.8252 (0.8); 3.7909 (2.6); 3.7856 (13.6); 3.7738 (2.0); 3.7706 (2.5); 3.7654 (14.3); 3.7570 (2.4); 3.7505 (14.2); 3.7307 (2.1); 3.7273 (2.4); 3.7137 (2.0); 3.2132 (2.0); 3.2095 (2.1); 3.1912 (1.8); 3.1832 (1.8); 3.1699 (1.8); 3.1661 (1.8); 3.1480 (1.6); 3.1400 (1.5); 2.5651 (0.5); 2.5591 (0.7); 2.5418 (0.8); 2.5247 (0.8); 2.4160 (0.7); 2.3990 (0.9); 2.3863 (0.8); 2.3818 (1.2); 2.3692 (0.9); 2.3644 (0.6); 2.3520 (1.0); 2.3345 (0.5); 1.7167 (16.0); 1.7106 (10.7); 1.6878 (9.8); 1.5652 (8.8); 0.0080 (0.7); -0.0002 (24.5); -0.0085 (0.8) |
| I-49 | ¹H-NMR(400.0 MHz, CDCl3): |
| | δ= 7.2603 (79.2); 7.2105 (0.5); 7.1993 (1.7); 7.1949 (2.3); 7.1797 (2.3); 7.1755 (1.7); 6.9331 (0.8); 6.9171 (0.9); 6.9113 (1.6); 6.9056 (0.8); 6.8897 (0.8); 4.6474 (0.7); 4.6423 (0.7); 4.6307 (0.7); 4.6268 (0.7); 4.6214 (0.5); 4.5982 (0.8); 4.5903 (0.9); 4.5751 (1.2); 4.5676 (1.4); 4.5526 (0.9); 4.5445 (0.8); 4.0667 (0.7); 4.0543 (0.8); 4.0430 (2.0); 4.0304 (1.9); 4.0191 (1.3); 4.0063 (1.2); 3.9783 (0.7); 3.9749 (0.8); 3.9636 (0.7); 3.9575 (1.0); 3.8276 (15.4); 3.8151 (0.9); 3.8010 (16.0); 3.7826 (1.4); 3.7706 (1.3); 3.6729 (2.3); 3.6505 (2.1); 3.6285 (1.3); 3.6061 (1.3); 2.5764 (0.5); 2.5591 (0.5); 2.5562 (0.5); 2.5526 (0.5); 2.5354 (0.6); 2.5325 (0.5); 2.5212 (0.5); 2.4976 (0.5); 2.1028 (0.5); 2.0811 (0.6); 2.0730 (0.6); 2.0436 (0.8); 1.8128 (2.2); 1.7968 (2.1); 1.7659 (4.4); 1.7498 (4.2); 1.7190 (2.4); 1.7029 (2.3); 1.5438 (17.9); 0.0080 (1.2); -0.0002 (41.5); -0.0085 (1.2) |
| I-50 | ¹H-NMR(400.0 MHz, CDCl3): |
| | δ= 7.5191 (1.2); 7.4723 (1.2); 7.4535 (1.2); 7.3606 (0.6); 7.3094 (0.5); 7.2779 (0.6); 7.2771 (0.6); 7.2763 (0.7); 7.2755 (0.7); 7.2739 (0.9); 7.2731 (1.0); 7.2723 (1.1); 7.2715 (1.2); 7.2707 (1.3); 7.2699 (1.4); 7.2691 (1.6); 7.2683 (1.8); 7.2675 (2.0); 7.2602 (205.8); 7.2530 (1.5); 7.2521 (1.2); 7.2513 (0.9); 7.2505 (0.8); 7.2497 (0.7); 7.2489 (0.6); 7.2481 (0.5); 7.2095 (0.6); 7.1932 (0.6); 7.1876 (0.6); 7.1801 (2.8); 7.1761 (3.4); 7.1608 (3.7); 7.1568 (2.5); 6.9962 (1.2); 6.9293 (0.9); 6.9228 (1.0); 6.9138 (1.0); 6.9077 (2.0); 6.9011 (2.0); 6.8952 (0.9); 6.8862 (0.9); 6.8795 (1.0); 4.6357 (1.2); 4.6266 (1.5); 4.6216 (0.8); 4.6114 (3.1); 4.6023 (3.8); 4.5877 (2.2); 4.5785 (2.0); 4.5726 (0.7); 4.1498 (1.2); 4.1320 (3.5); 4.1195 (1.2); 4.1141 (3.7); 4.1069 (1.1); 4.0958 (2.9); 4.0884 (1.2); 4.0828 (1.9); 4.0755 (1.0); 4.0641 (1.9); 4.0511 (1.8); 4.0386 (1.3); 4.0335 (1.3); 4.0142 (1.8); 3.9952 (0.6); 3.9901 (0.6); 3.8220 (1.4); 3.8138 (1.3); 3.7775 (2.8); 3.7742 (3.7); 3.7681 (3.2); 3.7638 (2.3); 3.7574 (6.6); 3.7514 (2.2); 3.7471 (1.5); 3.7400 (3.4); 3.7221 (2.1); 3.7046 (0.8); 3.6642 (4.8); 3.6196 (2.8); 2.6928 (0.6); 2.6757 (0.6); 2.6688 (0.6); 2.6580 (0.8); 2.6518 (0.7); 2.6410 (0.7); 2.6342 (0.9); 2.6177 (1.0); 2.6123 (0.7); 2.6012 (0.9); 2.5951 (0.7); 2.5840 (0.8); 2.5775 (0.7); 2.5602 (0.7); 2.2473 (0.5); 2.2406 (0.8); 2.2307 (0.7); 2.2207 (0.9); 2.2137 (0.9); 2.2058 (0.8); 2.1948 (0.6); 2.1859 (0.7); 2.1136 (1.6); 2.0456 (16.0); 1.8760 (2.4); 1.8683 (2.2); 1.8594 (7.1); 1.8546 (1.5); 1.8505 (2.2); 1.8428 (2.4); 1.8042 (3.1); 1.7864 (2.9); 1.7572 (6.5); 1.7392 (5.8); 1.7104 (3.5); 1.6924 (3.1); 1.4320 (1.1); 1.2772 (4.6); 1.2655 (2.4); 1.2593 (9.4); 1.2480 (4.5); 1.2414 (4.9); 1.2391 (2.8); 1.2304 (2.2); 0.0079 (2.0); -0.0002 (64.7); -0.0068 (0.9); -0.0085 (2.1) |
| I-51 | ¹H-NMR(400.0 MHz, CDC13): |
| | δ= 7.7312 (1.0); 7.7094 (1.0); 7.6924 (1.0); 7.6716 (0.9); 7.5189 (1.4); 7.2929 (0.5); 7.2600 (265.2); 7.2108 (1.2); 7.1984 (4.5); 7.1938 (5.8); 7.1788 (5.7); 7.1742 (4.5); 7.1616 (0.8); 6.9960 (1.5); 6.9329 (1.0); 6.9290 (1.6); 6.9271 (1.6); 6.9213 (0.9); 6.9130 (1.9); 6.9112 (2.0); 6.9073 (3.2); 6.9055 (3.2); 6.9016 (1.7); 6.8997 (1.6); 6.8914 (1.0); 6.8896 (1.0); 6.8856 (1.6); 6.8838 (1.6); 6.8799 (0.8); 5.1532 (0.9); 5.1375 (2.3); 5.1218 (3.1); 5.1099 (1.1); 5.1061 (2.4); 5.0943 (2.4); 5.0905 (1.1); 5.0786 (3.2); 5.0629 (2.3); 5.0472 (0.9); 4.6923 (0.5); 4.6866 (0.5); 4.6798 (0.9); 4.6744 (1.0); 4.6690 (1.0); 4.6633 (1.2); 4.6582 (1.3); 4.6518 (1.3); 4.6457 (1.3); 4.6399 (1.2); 4.6247 (0.9); 4.6183 (0.5); 4.6122 (0.5); 4.5188 (2.0); 4.5104 (2.3); 4.5028 (2.2); 4.4948 (4.4); 4.4867 (2.1); 4.4794 (2.2); 4.4711 (2.1); 4.0691 (1.8); 4.0564 (1.8); 4.0453 (3.2); 4.0366 (2.0); 4.0327 (3.1); 4.0236 (1.8); 4.0127 (3.1); 3.9996 (2.9); 3.9768 (1.8); 3.9730 (1.8); 3.9471 (2.8); 3.9426 (1.9); 3.9232 (1.1); 3.9188 (1.0); 3.8237 (2.0); 3.8157 (2.1); 3.7792 (3.4); 3.7713 (3.6); 3.6777 (5.6); 3.6551 (5.5); 3.6332 (3.2); 3.6107 (3.3); 2.6051 (1.0); 2.5872 (1.0); 2.5813 (1.0); 2.5702 (1.3); 2.5635 (1.1); 2.5526 (1.2); 2.5465 (1.3); 2.5431 (1.1); 2.5287 (1.2); 2.5250 (1.2); 2.5195 (1.1); 2.5081 (1.3); 2.5016 (1.1); 2.4902 (1.2); 2.4847 (1.2); 2.4667 (1.0); 2.0339 (1.4); 2.0308 (1.7); 2.0283 (1.8); 2.0257 (1.6); 2.0227 (1.5); 1.9991 (1.3); 1.9934 (1.5); 1.9877 (1.3); 1.8128 (5.2); 1.7970 (5.2); 1.7659 (10.6); 1.7500 (10.5); 1.7191 (5.7); 1.7032 (5.7); 1.5429 (43.8); 1.4474 (0.6); 1.3265 (13.4); 1.3108 (14.3); 1.3062 (16.0); 1.3037 (16.0); 1.2905 (15.3); 1.2880 (15.4); 1.2553 (0.8); 1.2437 (13.2); 1.2280 (13.0); 0.0079 (3.5); -0.0002 (112.0); -0.0085 (3.3) |
| I-52 | ¹H-NMR(400.0 MHz, CDC13): |
| | δ= 7.5190 (1.4); 7.4491 (1.9); 7.4333 (2.6); 7.4159 (1.9); 7.3658 (1.7); 7.3512 (1.9); 7.3461 (3.2); 7.3323 (3.9); 7.3263 (2.8); 7.3188 (2.1); 7.3122 (4.5); 7.2989 (3.4); 7.2938 (2.8); 7.2793 (2.8); 7.2731 (1.4); 7.2690 (2.2); 7.2683 (2.4); 7.2601 (236.0); 7.2109 (0.6); 7.1919 (0.6); 7.1861 (1.2); 7.1777 (1.8); 7.1738 (5.6); 7.1679 (7.8); 7.1653 (10.5); 7.1624 (8.6); 7.1597 (9.8); 7.1570 (6.8); 7.1541 (7.7); 7.1483 (8.9); 7.1456 (8.8); 7.1433 (6.9); 7.1400 (6.9); 7.1275 (1.3); 7.1177 (4.0); 7.1162 (4.0); 7.1138 (3.4); 7.1049 (2.8); 7.1009 (3.7); 7.0998 (3.7); 7.0972 (3.3); 7.0868 (1.8); 7.0815 (2.4); 7.0775 (1.9); 7.0581 (2.8); 7.0557 (2.8); 7.0524 (3.4); 7.0342 (4.3); 7.0303 (5.4); 7.0097 (3.4); 6.9961 (1.7); 6.9883 (1.3); 6.9818 (1.0); 6.9085 (1.3); 6.9027 (2.3); 6.8986 (1.7); 6.8929 (2.5); 6.8868 (3.6); 6.8810 (4.6); 6.8769 (3.4); 6.8754 (3.0); 6.8712 (4.7); 6.8653 (3.4); 6.8593 (2.3); 6.8552 (1.7); 6.8495 (2.3); 6.8436 (1.0); 6.1652 (4.5); 6.1552 (4.5); 6.1384 (5.0); 6.1284 (5.0); 6.1221 (5.5); 6.1120 (5.2); 6.0952 (5.6); 6.0852 (5.4); 5.5477 (7.5); 5.5463 (7.7); 5.5327 (7.0); 5.5311 (7.3); 5.5046 (6.7); 5.5032 (6.7); 5.4895 (6.3); 5.4880 (6.3); 5.3488 (6.9); 5.3476 (6.7); 5.3219 (6.6); 5.3207 (7.2); 5.3186 (7.3); 5.3171 (6.7); 5.2917 (5.9); 5.2903 (5.9); 5.2638 (2.6); 5.2385 (2.7); 5.2325 (8.5); 5.2069 (16.0); 5.1880 (8.9); 5.1749 (2.6); 5.1567 (2.3); 4.7085 (0.8); 4.6437 (0.5); 4.6373 (1.0); 4.6307 (1.2); 4.6238 (2.0); 4.6171 (2.5); 4.6101 (2.4); 4.6034 (3.3); 4.5998 (5.6); 4.5916 (8.0); 4.5832 (5.6); 4.5768 (5.0); 4.5683 (6.3); 4.5598 (3.8); 4.0771 (3.3); 4.0638 (3.4); 4.0532 (6.5); 4.0398 (5.8); 4.0283 (4.9); 4.0149 (4.4); 3.9575 (3.0); 3.9524 (2.9); 3.9328 (4.4); 3.9298 (4.6); 3.9257 (11.9); 3.9068 (2.0); 3.8998 (2.3); 3.8953 (8.0); 3.8827 (9.2); 3.8523 (8.9); 3.3196 (7.7); 3.3057 (7.9); 3.2766 (6.7); 3.2627 (6.9); 2.6071 (1.7); 2.5893 (1.8); 2.5837 (1.8); 2.5727 (2.6); 2.5660 (2.0); 2.5569 (2.4); 2.5549 (2.6); 2.5516 (2.5); 2.5494 (2.5); 2.5404 (2.3); 2.5337 (2.3); 2.5317 (2.2); 2.5226 (2.1); 2.5172 (2.0); 2.4993 (1.8); 2.0840 (1.4); 2.0772 (2.3); 2.0687 (2.5); 2.0602 (2.5); 2.0515 (2.4); 2.0427 (2.1); 2.0340 (2.2); 2.0256 (2.1); 2.0190 (1.2); 1.5534 (22.4); 1.2559 (0.7); 0.0079 (3.2); -0.0002 (99.5); - 0.0068 (1.2); -0.0085 (3.0) |
| I-53 | ¹H-NMR(400.0 MHz, CDCl3): |
| | δ= 7.4651 (0.6); 7.4434 (0.7); 7.3653 (0.5); 7.3508 (0.5); 7.3455 (0.9); 7.3302 (1.3); 7.3257 (0.9); 7.3140 (0.7); 7.3104 (1.7); 7.2949 (1.2); 7.2900 (0.8); 7.2748 (0.9); 7.2601 (81.2); 7.1746 (1.2); 7.1731 (1.2); 7.1706 (1.4); 7.1641 (1.8); 7.1580 (3.8); 7.1547 (2.9); 7.1521 (3.0); 7.1486 (2.0); 7.1441 (2.2); 7.1416 (2.1); 7.1382 (3.4); 7.1322 (2.0); 7.1257 (0.6); 7.1197 (0.9); 7.1065 (1.3); 7.1051 (1.3); 7.0877 (1.3); 7.0861 (1.2); 7.0463 (0.8); 7.0411 (0.9); 7.0345 (0.9); 7.0311 (1.0); 7.0276 (1.2); 7.0244 (1.0); 7.0176 (0.9); 7.0134 (1.0); 7.0071 (1.2); 6.9962 (0.6); 6.8971 (0.7); 6.8919 (0.7); 6.8863 (0.8); 6.8810 (1.1); 6.8753 (1.4); 6.8702 (1.3); 6.8646 (1.6); 6.8590 (1.0); 6.8536 (0.7); 6.8485 (0.6); 6.8429 (0.8); 5.2928 (1.0); 5.2616 (2.3); 5.2160 (2.6); 5.1836 (4.0); 5.1732 (3.4); 4.6044 (1.4); 4.5953 (1.8); 4.5911 (1.0); 4.5879 (1.8); 4.5845 (1.3); 4.5809 (2.1); 4.5786 (2.4); 4.5716 (1.7); 4.5645 (2.0); 4.5554 (1.4); 4.0874 (1.1); 4.0741 (1.1); 4.0638 (1.8); 4.0506 (1.6); 4.0390 (1.0); 4.0257 (1.0); 4.0153 (1.4); 4.0020 (1.3); 3.9744 (1.0); 3.9688 (1.0); 3.9507 (0.7); 3.9453 (0.6); 3.9040 (0.8); 3.8987 (0.8); 3.8803 (0.6); 3.8749 (0.6); 3.7769 (2.8); 3.7520 (2.3); 3.7337 (3.2); 3.7088 (2.7); 3.1824 (2.5); 3.1760 (2.9); 3.1393 (2.2); 3.1328 (2.6); 2.5968 (0.6); 2.5912 (0.5); 2.5801 (0.7); 2.5734 (0.6); 2.5704 (0.7); 2.5624 (0.6); 2.5567 (0.7); 2.5525 (0.7); 2.5472 (0.7); 2.5389 (0.6); 2.5359 (0.8); 2.5292 (0.7); 2.5179 (0.7); 2.5126 (0.7); 2.4947 (0.6); 2.0911 (0.7); 2.0566 (0.6); 2.0479 (0.8); 2.0396 (0.8); 2.0049 (0.7); 1.7017 (16.0); 1.6504 (13.3); 1.5512 (10.8); 1.2584 (0.5); 0.0079 (1.2); - 0.0002 (35.6); -0.0085 (1.0) |
| I-54 | ¹H-NMR(400.0 MHz, CDCl3): |
| | δ= 7.4531 (0.7); 7.3377 (2.3); 7.3325 (0.8); 7.3212 (0.8); 7.3158 (2.5); 7.3089 (0.5); 7.2925 (2.5); 7.2872 (1.0); 7.2760 (1.0); 7.2706 (2.9); 7.2600 (55.9); 7.1855 (2.2); 7.1798 (2.6); 7.1767 (1.5); 7.1689 (1.5); 7.1657 (2.7); 7.1600 (2.2); 6.9096 (0.6); 6.9032 (3.4); 6.8978 (1.2); 6.8949 (0.8); 6.8869 (1.3); 6.8815 (3.4); 6.8760 (1.1); 6.8732 (1.5); 6.8686 (3.3); 6.8634 (1.0); 6.8603 (0.7); 6.8518 (1.3); 6.8467 (2.8); 6.1708 (1.1); 6.1572 (1.0); 6.1440 (1.2); 6.1304 (1.2); 6.1277 (1.4); 6.1141 (1.2); 6.1009 (1.3); 6.0873 (1.2); 5.5589 (1.9); 5.5575 (1.8); 5.5443 (1.7); 5.5428 (1.7); 5.5157 (1.6); 5.5144 (1.6); 5.5013 (1.5); 5.4997 (1.4); 5.3543 (1.7); 5.3532 (1.6); 5.3315 (1.6); 5.3300 (1.7); 5.3276 (1.7); 5.3264 (1.6); 5.3047 (1.4); 5.3033 (1.4); 5.2078 (0.7); 5.1956 (0.9); 5.1781 (2.3); 5.1658 (2.2); 5.1573 (2.3); 5.1308 (2.3); 5.1011 (0.8); 4.6040 (0.6); 4.5971 (0.5); 4.5907 (0.7); 4.5854 (0.6); 4.5790 (0.5); 4.5723 (0.6); 4.5504 (0.9); 4.5407 (1.5); 4.5311 (1.0); 4.5269 (1.0); 4.5173 (1.6); 4.5078 (0.8); 4.0599 (0.8); 4.0465 (0.8); 4.0361 (1.5); 4.0227 (1.4); 4.0111 (1.1); 3.9977 (1.0); 3.9409 (0.8); 3.9337 (0.8); 3.9301 (2.0); 3.9168 (1.1); 3.9100 (1.1); 3.8965 (1.9); 3.8871 (2.6); 3.8536 (2.1); 3.8127 (16.0); 3.7946 (15.8); 3.3214 (1.8); 3.3109 (1.9); 3.2784 (1.6); 3.2679 (1.7); 2.5341 (0.6); 2.5186 (0.6); 2.5162 (0.6); 2.5137 (0.6); 2.5108 (0.6); 2.5023 (0.5); 2.4955 (0.5); 2.0433 (0.6); 2.0343 (0.5); 2.0089 (0.8); 2.0000 (0.8); 1.5548 (7.2); 1.2584 (0.5); 0.0079 (0.7); -0.0002 (24.0); -0.0085 (0.7) |
| I-55 | ¹H-NMR(400.0 MHz, CDCl3): |
| | δ= 7.2610 (30.5); 7.1737 (1.7); 7.1677 (2.8); 7.1643 (2.5); 7.1617 (2.4); 7.1581 (2.3); 7.1539 (2.6); 7.1515 (2.6); 7.1481 (2.8); 7.1421 (1.7); 6.9968 (1.0); 6.9770 (1.0); 6.9141 (0.8); 6.9087 (0.8); 6.8979 (0.8); 6.8925 (1.6); 6.8870 (1.5); 6.8813 (0.8); 6.8764 (0.6); 6.8708 (0.9); 6.8653 (0.8); 4.6416 (0.6); 4.6215 (1.5); 4.6030 (1.6); 4.5825 (0.8); 4.5490 (1.0); 4.5426 (1.1); 4.5372 (1.1); 4.5306 (1.1); 4.1649 (0.8); 4.1522 (0.8); 4.1410 (1.2); 4.1359 (1.1); 4.1283 (1.2); 4.1230 (1.0); 4.1119 (1.1); 4.0990 (1.0); 3.8549 (0.8); 3.8511 (0.8); 3.8337 (0.8); 3.8271 (0.8); 3.7906 (2.4); 3.7654 (11.5); 3.7505 (11.9); 3.7270 (2.4); 3.2127 (2.2); 3.2093 (2.0); 3.1694 (2.0); 3.1660 (1.8); 2.4160 (0.6); 2.3990 (0.9); 2.3862 (1.0); 2.3819 (1.2); 2.3693 (1.0); 2.3646 (0.7); 2.3521 (1.0); 2.3346 (0.5); 2.2651 (0.6); 2.1767 (0.5); 1.7169 (16.0); 1.5519 (8.1); -0.0002 (13.3) |
| I-56 | ¹H-NMR(400.0 MHz, CDC13): |
| | δ= 7.2602 (70.1); 7.1772 (4.1); 7.1582 (4.2); 6.9440 (1.7); 6.9241 (1.5); 6.9183 (1.7); 6.9128 (1.5); 6.8967 (2.4); 6.8913 (2.3); 6.8751 (1.4); 6.8696 (1.3); 6.1887 (1.1); 6.1731 (1.2); 6.1620 (1.3); 6.1460 (2.5); 6.1300 (1.5); 6.1190 (1.4); 6.1032 (1.5); 5.5554 (2.9); 5.5500 (3.0); 5.5122 (2.6); 5.5069 (2.7); 5.3632 (4.1); 5.3365 (3.9); 4.6264 (1.9); 4.6074 (3.2); 4.5884 (2.3); 4.5516 (1.8); 4.1567 (1.2); 4.1438 (1.5); 4.1392 (1.6); 4.1326 (1.8); 4.1263 (1.6); 4.1198 (1.8); 4.1152 (1.8); 4.1025 (1.5); 3.9348 (2.3); 3.9161 (2.4); 3.8917 (2.6); 3.8730 (2.8); 3.8349 (1.3); 3.8153 (2.5); 3.7911 (1.3); 3.7857 (1.3); 3.7620 (15.4); 3.7515 (16.0); 3.3388 (3.9); 3.2956 (3.4); 2.4196 (0.5); 2.4120 (0.6); 2.4024 (1.0); 2.3945 (1.1); 2.3854 (1.5); 2.3775 (1.5); 2.3682 (1.8); 2.3604 (1.8); 2.3507 (1.1); 2.3428 (1.0); 2.2829 (0.7); 2.2757 (0.7); 2.2625 (0.7); 2.2552 (0.8); 2.2273 (1.1); 2.2198 (1.1); 2.2062 (0.8); 2.1984 (0.8); 2.1722 (0.6); 1.5419 (18.5); -0.0002 (29.5) |

In Analogie zu den oben angeführten und an entsprechender Stelle zitierten Herstellungsbeispielen und unter Berücksichtigung der allgemeinen Angaben zur Herstellung von substituierten Isoxazolincarboxamiden kann man die nachfolgend genannten Verbindungen erhalten:
Tabelle 2.1: Erfindungsgemäße Verbindungen 2.1-1 bis 2.1-240 der allgemeinen Formel (I.1), worin Z-(C=W)-O-R⁴ wie nachfolgend definiert ist.

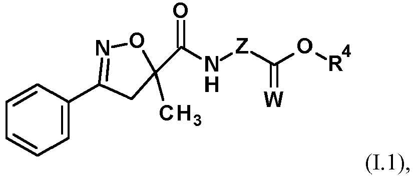

**Tabelle 2.1**

| Nr. | 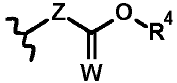 | Nr. | 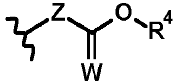 |
|---|---|---|---|
| 2.1-1 | 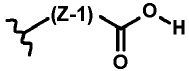 | 2.1-2 | 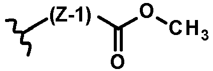 |
| 2.1-3 | 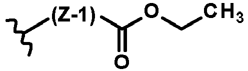 | 2.1-4 | 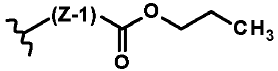 |
| 2.1-5 | 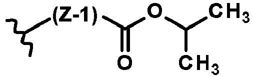 | 2.1-6 | 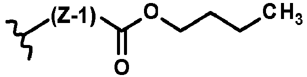 |
| 2.1-7 | 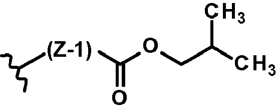 | 2.1-8 | 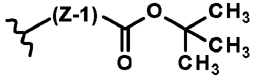 |
| 2.1-9 | 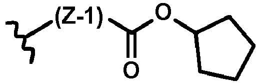 | 2.1-10 | 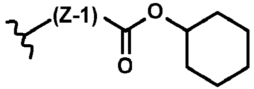 |
| 2.1-11 | 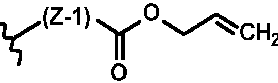 | 2.1-12 | 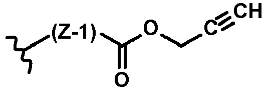 |
| 2.1-13 | 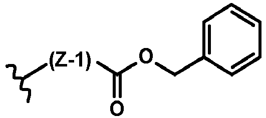 | 2.1-14 | 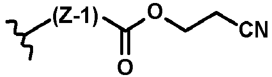 |
| 2.1-15 | 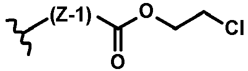 | 2.1-16 | 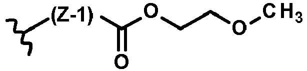 |
| 2.1-17 | 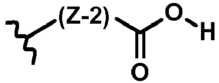 | 2.1-18 | 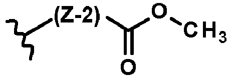 |
| 2.1-19 | 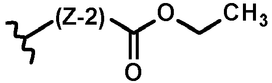 | 2.1-20 | 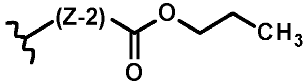 |
| 2.1-21 | 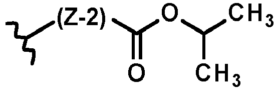 | 2.1-22 | 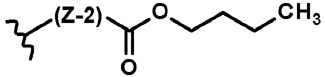 |
| 2.1-23 | 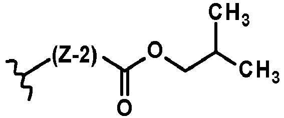 | 2.1-24 | 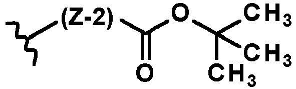 |
| 2.1-25 | 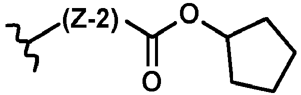 | 2.1-26 | 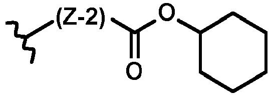 |
| 2.1-27 | 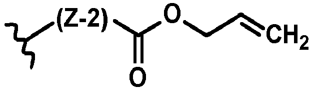 | 2.1-28 | 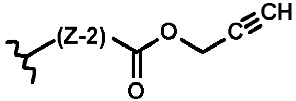 |
| 2.1-29 | 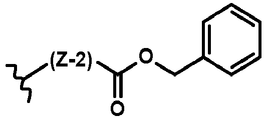 | 2.1-30 | 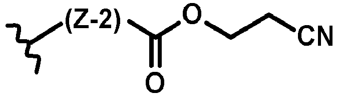 |
| 2.1-31 | 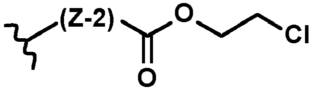 | 2.1-32 | 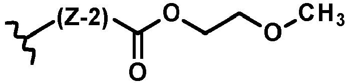 |
| 2.1-33 | 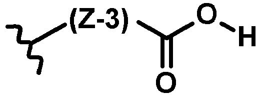 | 2.1-34 | 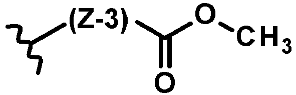 |
| 2.1-35 | 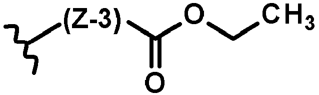 | 2.1-36 | 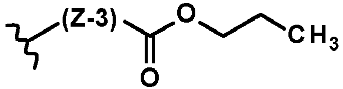 |
| 2.1-37 | 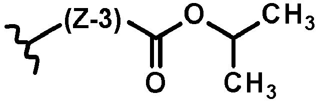 | 2.1-38 | 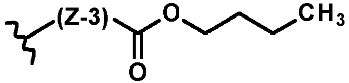 |
| 2.1-39 | 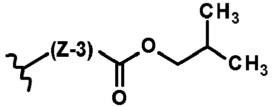 | 2.1-40 | 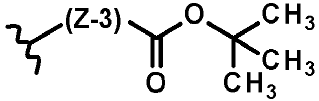 |
| 2.1-41 | 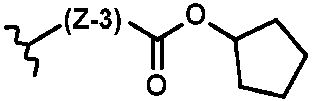 | 2.1-42 | 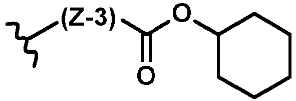 |
| 2.1-43 | 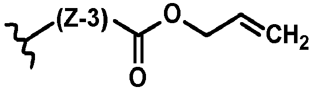 | 2.1-44 | 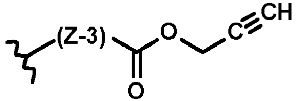 |
| 2.1-45 | 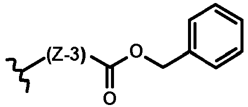 | 2.1-46 | 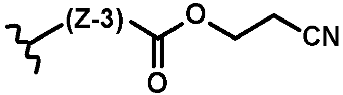 |
| 2.1-47 | 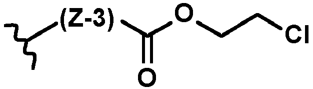 | 2.1-48 | 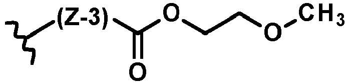 |
| 2.1-49 | 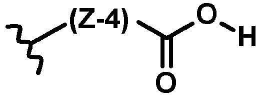 | 2.1-50 | 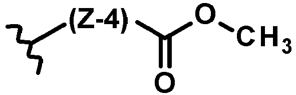 |
| 2.1-51 | 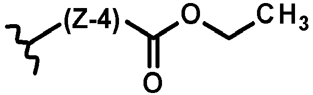 | 2.1-52 | 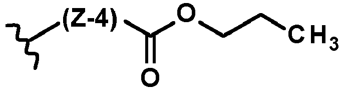 |
| 2.1-53 | 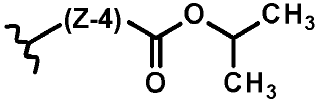 | 2.1-54 | 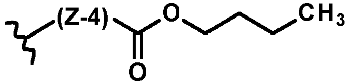 |
| 2.1-55 | 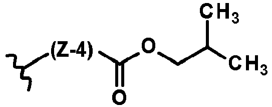 | 2.1-56 | 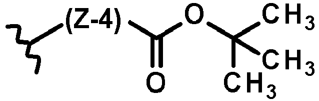 |
| 2.1-57 | 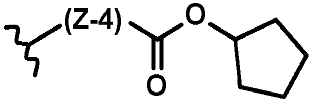 | 2.1-58 | 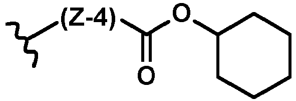 |
| 2.1-59 | 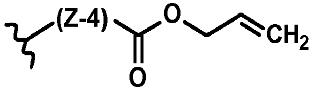 | 2.1-60 | 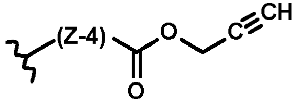 |
| 2.1-61 | 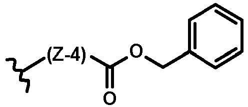 | 2.1-62 | 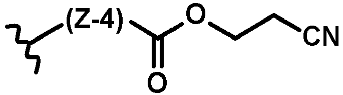 |
| 2.1-63 | 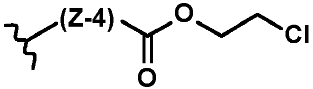 | 2.1-64 | 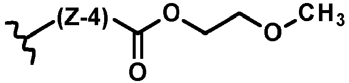 |
| 2.1-65 | 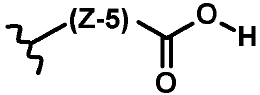 | 2.1-66 | 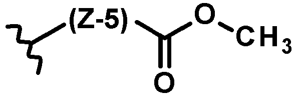 |
| 2.1-67 | 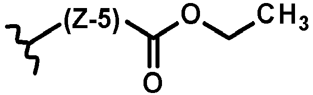 | 2.1-68 | 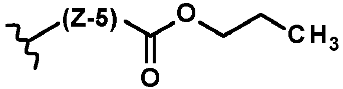 |
| 2.1-69 | 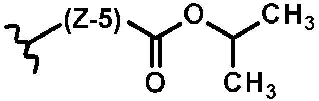 | 2.1-70 | 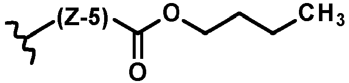 |
| 2.1-71 | 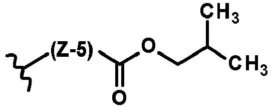 | 2.1-72 | 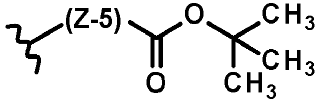 |
| 2.1-73 | 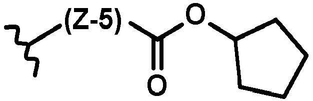 | 2.1-74 | 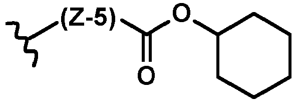 |
| 2.1-75 | 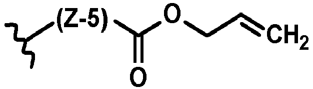 | 2.1-76 | 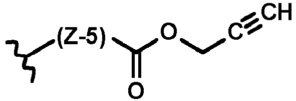 |
| 2.1-77 | 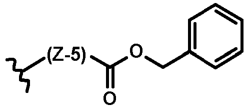 | 2.1-78 | 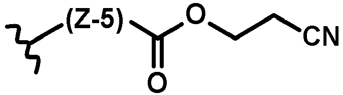 |
| 2.1-79 | 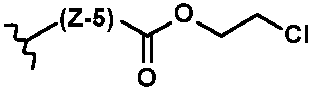 | 2.1-80 | 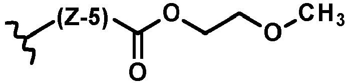 |
| 2.1-81 | 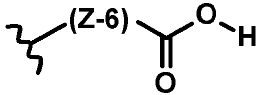 | 2.1-82 | 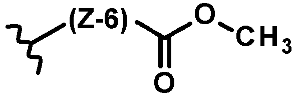 |
| 2.1-83 | 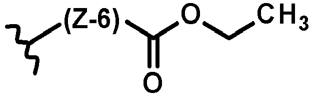 | 2.1-84 | 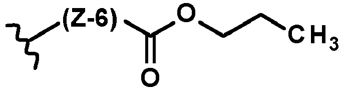 |
| 2.1-85 | 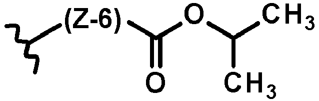 | 2.1-86 | 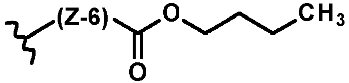 |
| 2.1-87 | 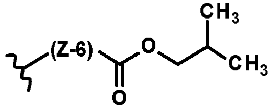 | 2.1-88 | 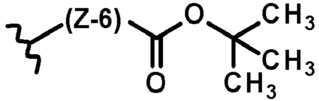 |
| 2.1-89 | 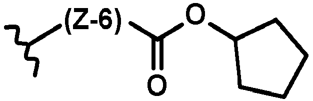 | 2.1-90 | 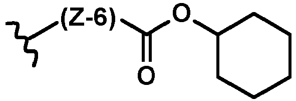 |
| 2.1-91 | 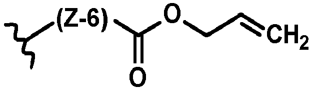 | 2.1-92 | 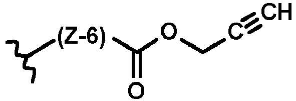 |
| 2.1-93 | 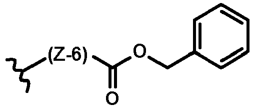 | 2.1-94 | 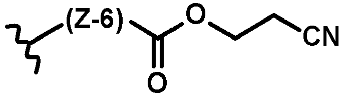 |
| 2.1-95 | 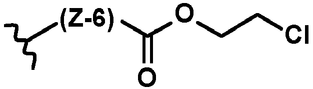 | 2.1-96 | 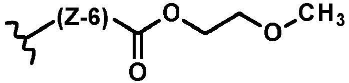 |
| 2.1-97 | 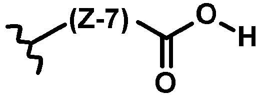 | 2.1-98 | 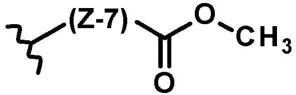 |
| 2.1-99 | 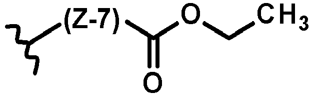 | 2.1-100 | 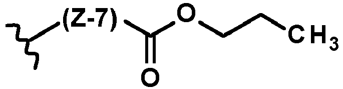 |
| 2.1-101 | 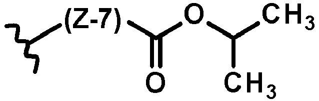 | 2.1-102 | 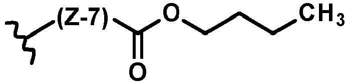 |
| 2.1-103 | 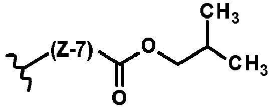 | 2.1-104 | 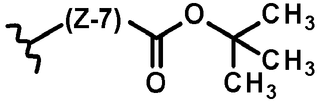 |
| 2.1-105 | 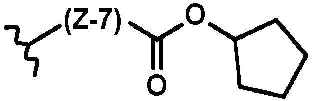 | 2.1-106 | 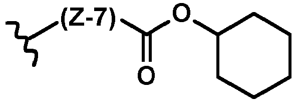 |
| 2.1-107 | 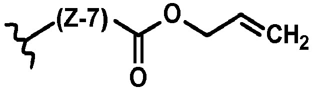 | 2.1-108 | 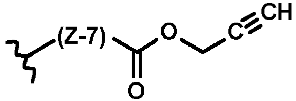 |
| 2.1-109 | 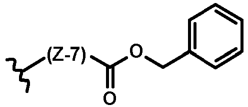 | 2.1-110 | 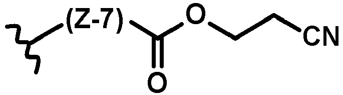 |
| 2.1-111 | 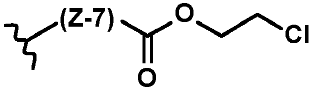 | 2.1-112 | 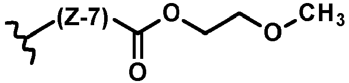 |
| 2.1-113 | 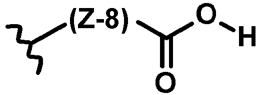 | 2.1-114 | 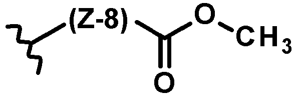 |
| 2.1-115 | 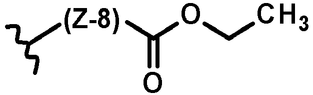 | 2.1-116 | 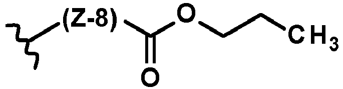 |
| 2.1-117 | 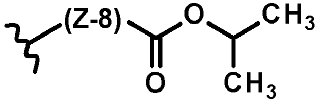 | 2.1-118 | 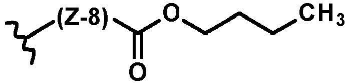 |
| 2.1-119 | 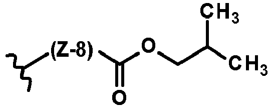 | 2.1-120 | 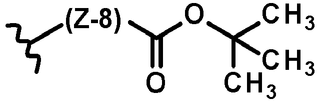 |
| 2.1-121 | 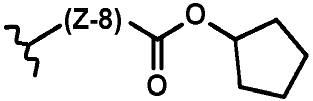 | 2.1-122 | 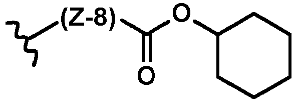 |
| 2.1-123 | 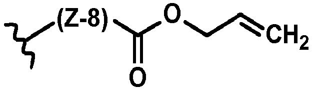 | 2.1-124 | 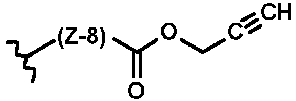 |
| 2.1-125 | 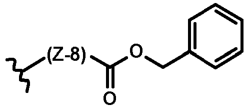 | 2.1-126 | 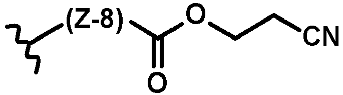 |
| 2.1-127 | 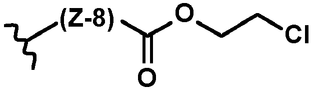 | 2.1-128 | 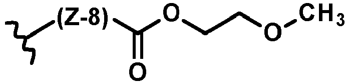 |
| 2.1-129 | 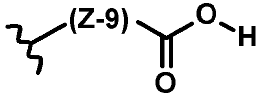 | 2.1-130 | 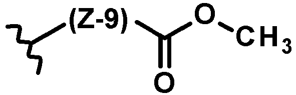 |
| 2.1-131 | 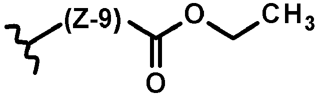 | 2.1-132 | 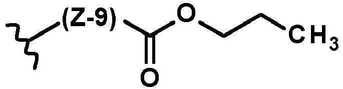 |
| 2.1-133 | 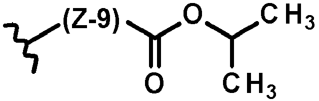 | 2.1-134 | 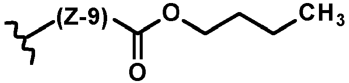 |
| 2.1-135 | 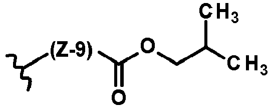 | 2.1-136 | 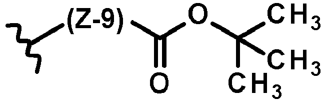 |
| 2.1-137 | 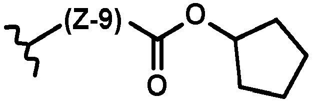 | 2.1-138 | 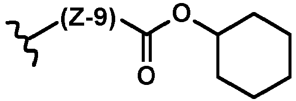 |
| 2.1-139 | 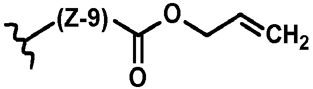 | 2.1-140 | 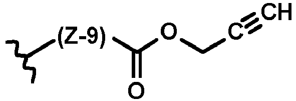 |
| 2.1-141 | 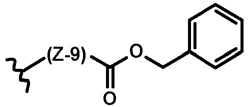 | 2.1-142 | 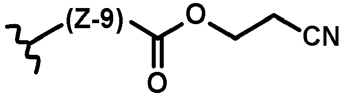 |
| 2.1-143 | 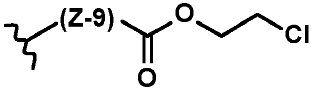 | 2.1-144 | 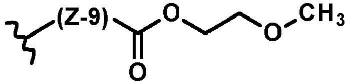 |
| 2.1-145 | 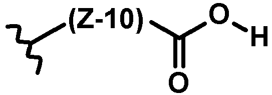 | 2.1-146 | 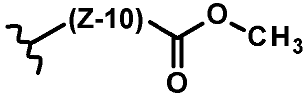 |
| 2.1-147 | 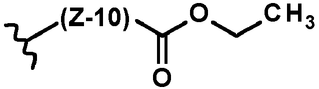 | 2.1-148 | 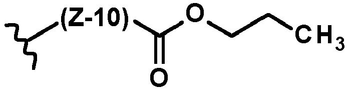 |
| 2.1-149 | 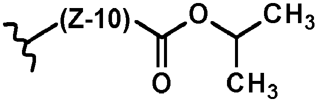 | 2.1-150 | 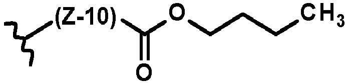 |
| 2.1-151 | 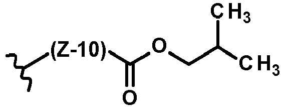 | 2.1-152 | 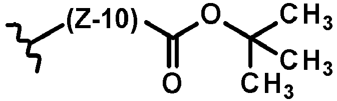 |
| 2.1-153 | 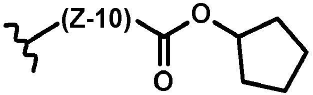 | 2.1-154 | 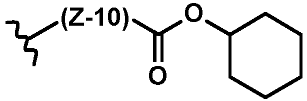 |
| 2.1-155 | 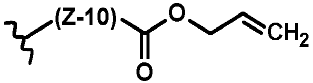 | 2.1-156 | 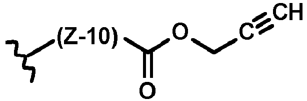 |
| 2.1-157 | 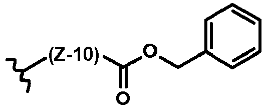 | 2.1-158 | 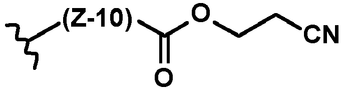 |
| 2.1-159 | 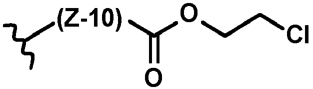 | 2.1-160 | 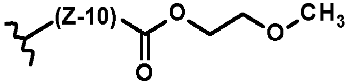 |
| 2.1-161 | 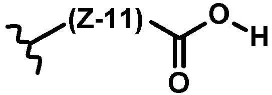 | 2.1-162 | 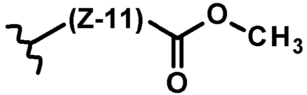 |
| 2.1-163 | 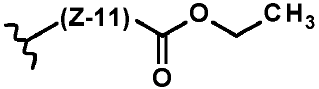 | 2.1-164 | 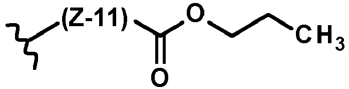 |
| 2.1-165 | 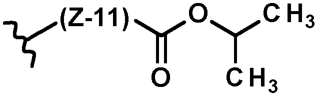 | 2.1-166 | 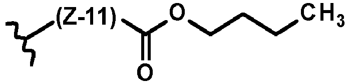 |
| 2.1-167 | 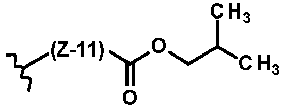 | 2.1-168 | 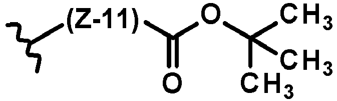 |
| 2.1-169 | 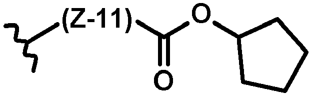 | 2.1-170 | 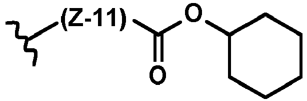 |
| 2.1-171 | 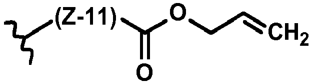 | 2.1-172 | 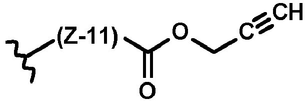 |
| 2.1-173 | 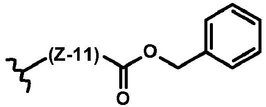 | 2.1-174 | 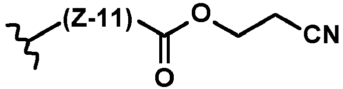 |
| 2.1-175 | 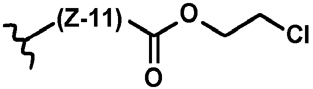 | 2.1-176 | 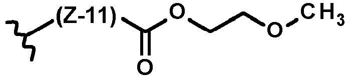 |
| 2.1-177 | 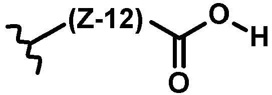 | 2.1-178 | 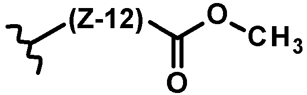 |
| 2.1-179 | 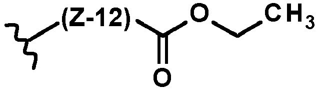 | 2.1-180 | 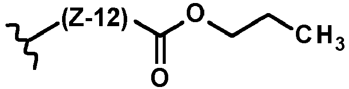 |
| 2.1-181 | 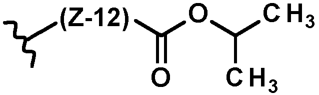 | 2.1-182 | 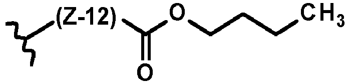 |
| 2.1-183 | 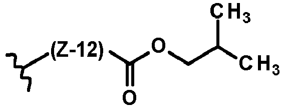 | 2.1-184 | 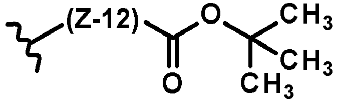 |
| 2.1-185 | 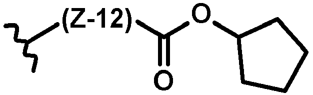 | 2.1-186 | 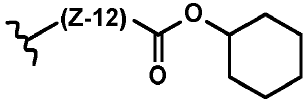 |
| 2.1-187 | 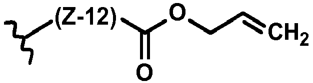 | 2.1-188 | 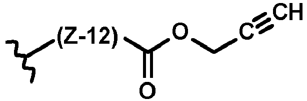 |
| 2.1-189 | 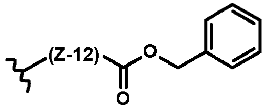 | 2.1-190 | 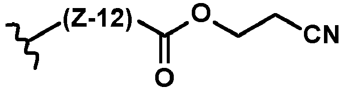 |
| 2.1-191 | 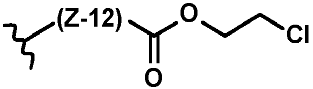 | 2.1-192 | 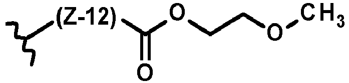 |
| 2.1-193 | 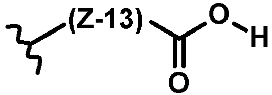 | 2.1-194 | 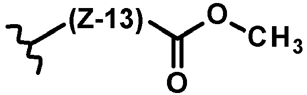 |
| 2.1-195 | 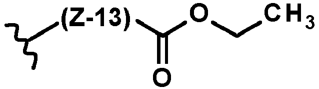 | 2.1-196 | 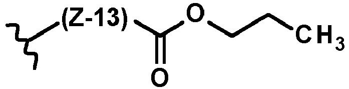 |
| 2.1-197 | 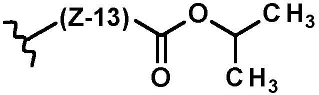 | 2.1-198 | 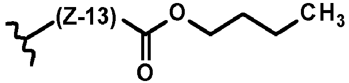 |
| 2.1-199 | 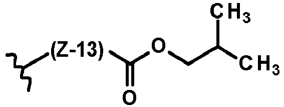 | 2.1-200 | 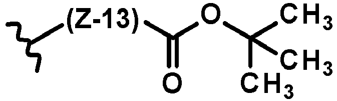 |
| 2.1-201 | 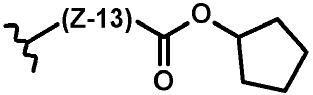 | 2.1-202 | 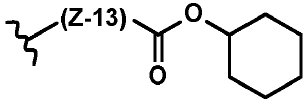 |
| 2.1-203 | 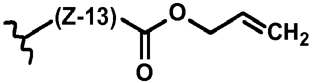 | 2.1-204 | 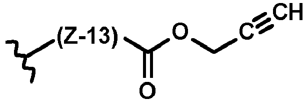 |
| 2.1-205 | 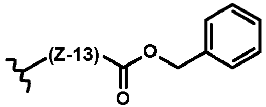 | 2.1-206 | 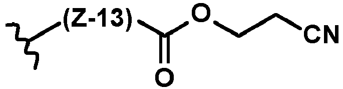 |
| 2.1-207 | 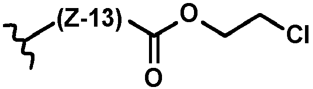 | 2.1-208 | 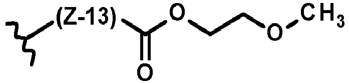 |
| 2.1-209 | 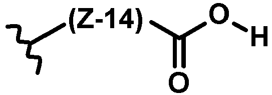 | 2.1-210 | 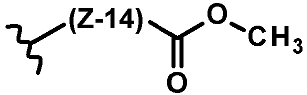 |
| 2.1-211 | 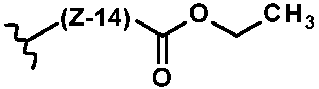 | 2.1-212 | 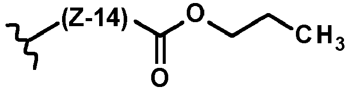 |
| 2.1-213 | 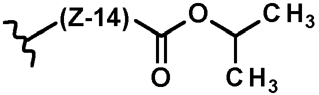 | 2.1-214 | 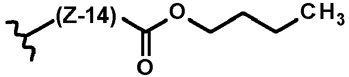 |
| 2.1-215 | 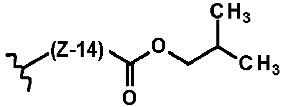 | 2.1-216 | 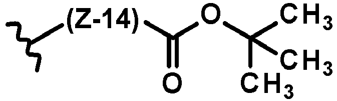 |
| 2.1-217 | 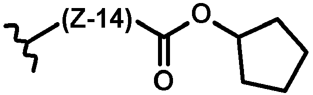 | 2.1-218 | 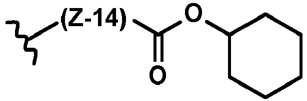 |
| 2.1-219 | 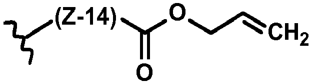 | 2.1-220 | 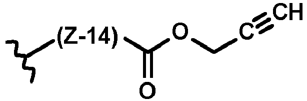 |
| 2.1-221 | 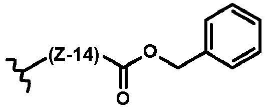 | 2.1-222 | 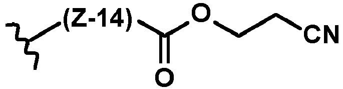 |
| 2.1-223 | 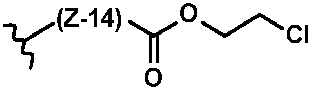 | 2.1-224 | 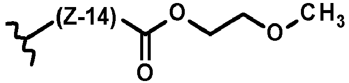 |
| 2.1-225 | 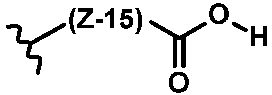 | 2.1-226 | 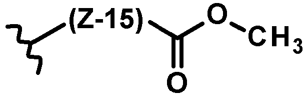 |
| 2.1-227 | 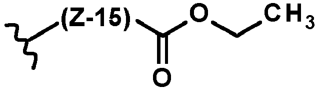 | 2.1-228 | 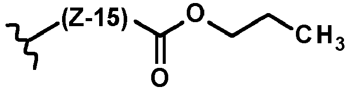 |
| 2.1-229 | 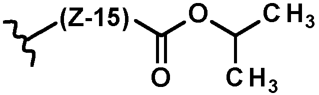 | 2.1-230 | 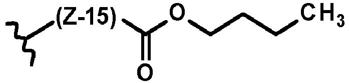 |
| 2.1-231 | 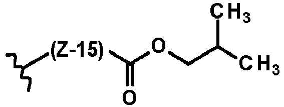 | 2.1-232 | 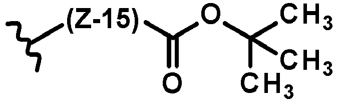 |
| 2.1-233 | 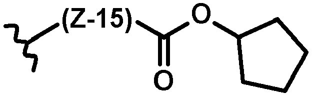 | 2.1-234 | 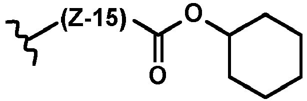 |
| 2.1-235 | 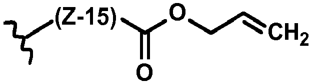 | 2.1-236 | 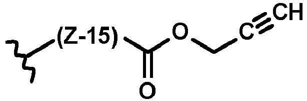 |
| 2.1-237 | 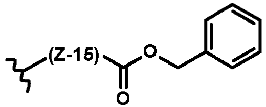 | 2.1-238 | 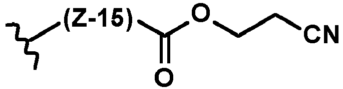 |
| 2.1-239 | 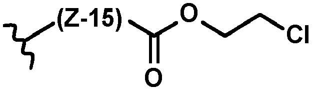 | 2.1-240 | 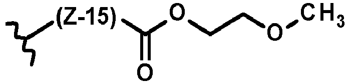 |

Tabelle 2.2: Erfindungsgemäße Verbindungen 2.2-1 bis 2.2-240 der allgemeinen Formel (I.2), worin Z-(C=W)-O-R⁴ wie in Tabelle 2.1 definiert ist. 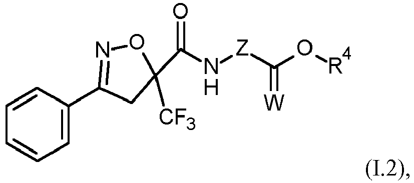
Tabelle 2.3: Erfindungsgemäße Verbindungen 2.3-1 bis 2.3-240 der allgemeinen Formel (I.3), worin Z-(C=W)-O-R⁴ wie in Tabelle 2.1 definiert ist. 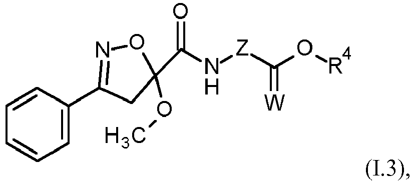
Tabelle 2.4: Erfindungsgemäße Verbindungen 2.4-1 bis 2.4-240 der allgemeinen Formel (I.4), worin Z-(C=W)-O-R⁴ wie in Tabelle 2.1 definiert ist. 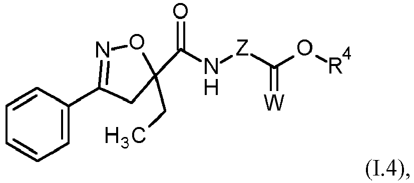
Tabelle 2.5: Erfindungsgemäße Verbindungen 2.5-1 bis 2.5-240 der allgemeinen Formel (I.5), worin Z-(C=W)-O-R⁴ wie in Tabelle 2.1 definiert ist. 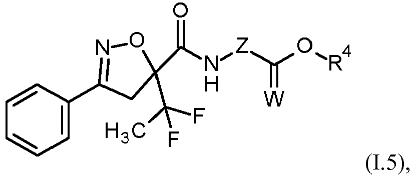
Tabelle 2.6: Erfindungsgemäße Verbindungen 2.6-1 bis 2.6-240 der allgemeinen Formel (I.6), worin Z-(C=W)-O-R⁴ wie in Tabelle 2.1 definiert ist. 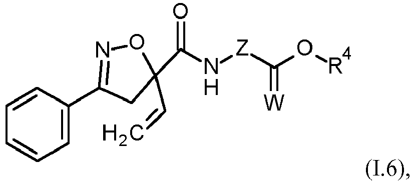
Tabelle 2.7: Erfindungsgemäße Verbindungen 2.7-1 bis 2.7-240 der allgemeinen Formel (I.7), worin Z-(C=W)-O-R⁴ wie in Tabelle 2.1 definiert ist. 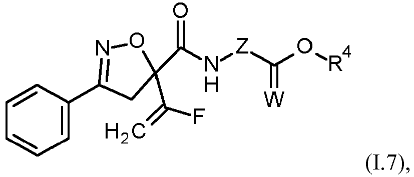
Tabelle 2.8: Erfindungsgemäße Verbindungen 2.8-1 bis 2.8-240 der allgemeinen Formel (I.8), worin Z-(C=W)-O-R⁴ wie in Tabelle 2.1 definiert ist. 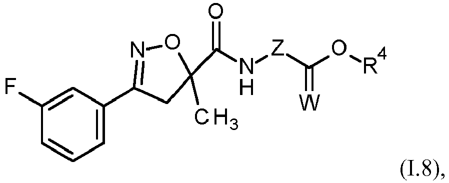
Tabelle 2.9: Erfindungsgemäße Verbindungen 2.9-1 bis 2.9-240 der allgemeinen Formel (I.9), worin Z-(C=W)-O-R⁴ wie in Tabelle 2.1 definiert ist. 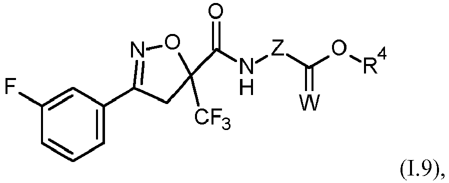
Tabelle 2.10: Erfindungsgemäße Verbindungen 2.10-1 bis 2.10-240 der allgemeinen Formel (I.10), worin Z-(C=W)-O-R⁴ wie in Tabelle 2.1 definiert ist. 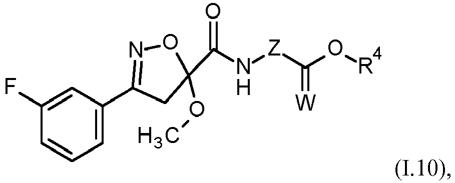
Tabelle 2.11: Erfindungsgemäße Verbindungen 2.11-1 bis 2.11-240 der allgemeinen Formel (I.11), worin Z-(C=W)-O-R⁴ wie in Tabelle 2.1 definiert ist. 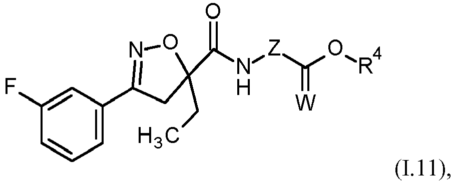
Tabelle 2.12: Erfindungsgemäße Verbindungen 2.12-1 bis 2.12-240 der allgemeinen Formel (I.12), worin Z-(C=W)-O-R⁴ wie in Tabelle 2.1 definiert ist. 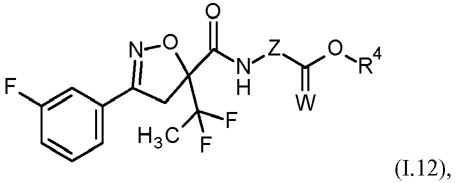
Tabelle 2.13: Erfindungsgemäße Verbindungen 2.13-1 bis 2.13-240 der allgemeinen Formel (I.13), worin Z-(C=W)-O-R⁴ wie in Tabelle 2.1 definiert ist. 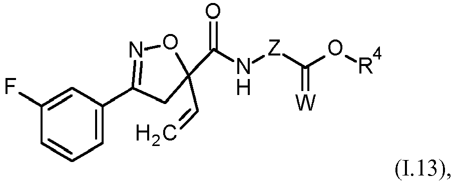
Tabelle 2.14: Erfindungsgemäße Verbindungen 2.14-1 bis 2.14-240 der allgemeinen Formel (I.14), worin Z-(C=W)-O-R⁴ wie in Tabelle 2.1 definiert ist. 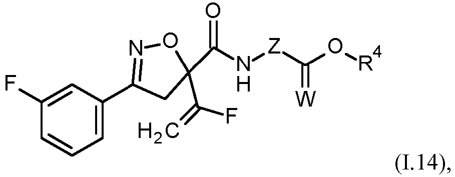
Tabelle 2.15: Erfindungsgemäße Verbindungen 2.15-1 bis 2.15-240 der allgemeinen Formel (I.15), worin Z-(C=W)-O-R⁴ wie in Tabelle 2.1 definiert ist. 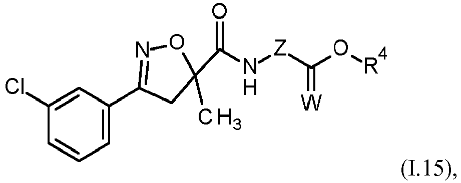
Tabelle 2.16: Erfindungsgemäße Verbindungen 2.16-1 bis 2.16-240 der allgemeinen Formel (I.16), worin Z-(C=W)-O-R⁴ wie in Tabelle 2.1 definiert ist. 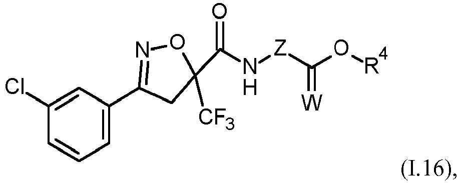
Tabelle 2.17: Erfindungsgemäße Verbindungen 2.17-1 bis 2.17-240 der allgemeinen Formel (I.17), worin Z-(C=W)-O-R⁴ wie in Tabelle 2.1 definiert ist. 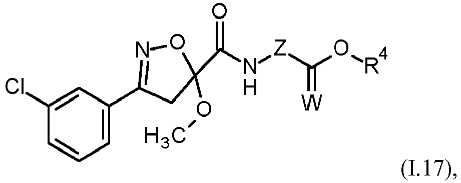
Tabelle 2.18: Erfindungsgemäße Verbindungen 2.18-1 bis 2.18-240 der allgemeinen Formel (I.18), worin Z-(C=W)-O-R⁴ wie in Tabelle 2.1 definiert ist. 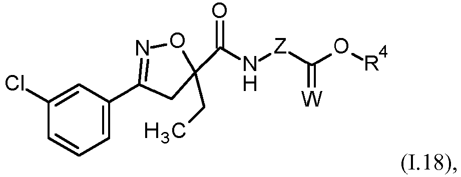
Tabelle 2.19: Erfindungsgemäße Verbindungen 2.19-1 bis 2.19-240 der allgemeinen Formel (I.19), worin Z-(C=W)-O-R⁴ wie in Tabelle 2.1 definiert ist. 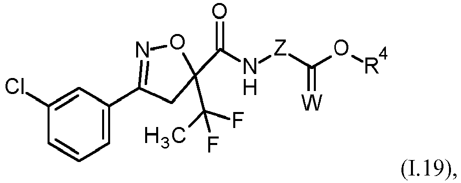
Tabelle 2.20: Erfindungsgemäße Verbindungen 2.20-1 bis 2.20-240 der allgemeinen Formel (I.20), worin Z-(C=W)-O-R⁴ wie in Tabelle 2.1 definiert ist. 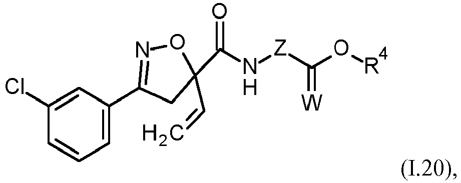
Tabelle 2.21: Erfindungsgemäße Verbindungen 2.21-1 bis 2.21-240 der allgemeinen Formel (I.21), worin Z-(C=W)-O-R⁴ wie in Tabelle 2.1 definiert ist. 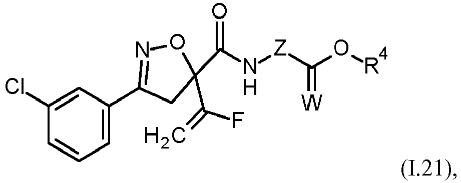
Tabelle 2.22: Erfindungsgemäße Verbindungen 2.22-1 bis 2.22-240 der allgemeinen Formel (I.22), worin Z-(C=W)-O-R⁴ wie in Tabelle 2.1 definiert ist. 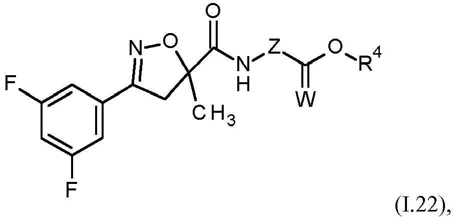
Tabelle 2.23: Erfindungsgemäße Verbindungen 2.23-1 bis 2.23-240 der allgemeinen Formel (I.23), worin Z-(C=W)-O-R⁴ wie in Tabelle 2.1 definiert ist. 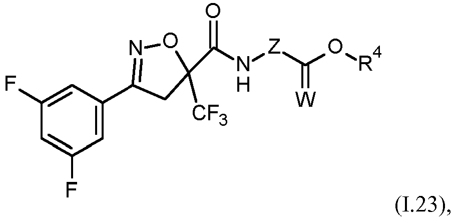
Tabelle 2.24: Erfindungsgemäße Verbindungen 2.24-1 bis 2.24-240 der allgemeinen Formel (I.24), worin Z-(C=W)-O-R⁴ wie in Tabelle 2.1 definiert ist. 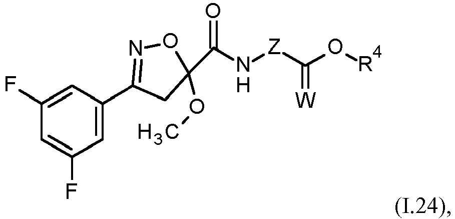
Tabelle 2.25: Erfindungsgemäße Verbindungen 2.25-1 bis 2.25-240 der allgemeinen Formel (I.25), worin Z-(C=W)-O-R⁴ wie in Tabelle 2.1 definiert ist. 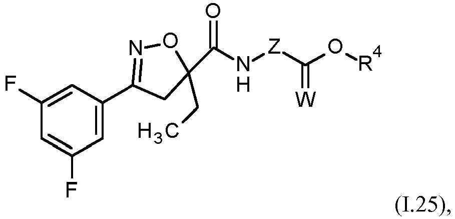
Tabelle 2.26: Erfindungsgemäße Verbindungen 2.26-1 bis 2.26-240 der allgemeinen Formel (I.26), worin Z-(C=W)-O-R⁴ wie in Tabelle 2.1 definiert ist. 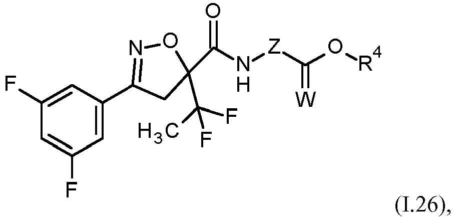
Tabelle 2.27: Erfindungsgemäße Verbindungen 2.27-1 bis 2.27-240 der allgemeinen Formel (I.27), worin Z-(C=W)-O-R⁴ wie in Tabelle 2.1 definiert ist. 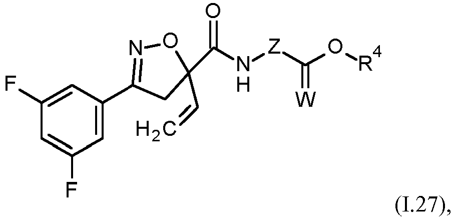
Tabelle 2.28: Erfindungsgemäße Verbindungen 2.28-1 bis 2.28-240 der allgemeinen Formel (I.28), worin Z-(C=W)-O-R⁴ wie in Tabelle 2.1 definiert ist. 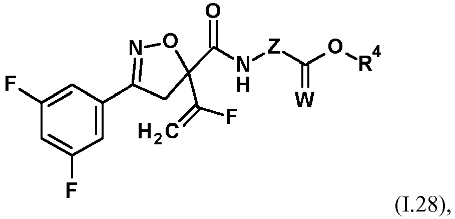
Tabelle 2.29: Erfindungsgemäße Verbindungen 2.29-1 bis 2.29-240 der allgemeinen Formel (I.29), worin Z-(C=W)-O-R⁴ wie in Tabelle 2.1 definiert ist. 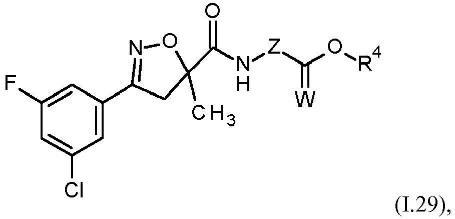
Tabelle 2.30: Erfindungsgemäße Verbindungen 2.30-1 bis 2.30-240 der allgemeinen Formel (I.30), worin Z-(C=W)-O-R⁴ wie in Tabelle 2.1 definiert ist. 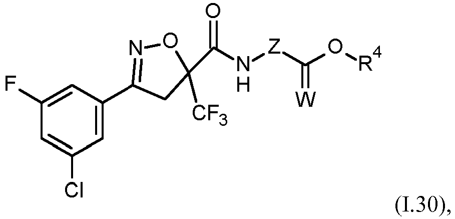
Tabelle 2.31: Erfindungsgemäße Verbindungen 2.31-1 bis 2.31-240 der allgemeinen Formel (I.31), worin Z-(C=W)-O-R⁴ wie in Tabelle 2.1 definiert ist. 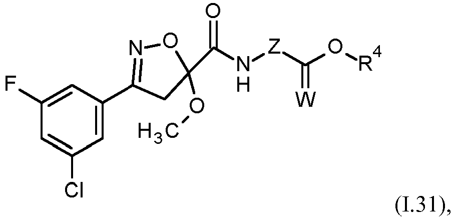
Tabelle 2.32: Erfindungsgemäße Verbindungen 2.32-1 bis 2.32-240 der allgemeinen Formel (I.32), worin Z-(C=W)-O-R⁴ wie in Tabelle 2.1 definiert ist. 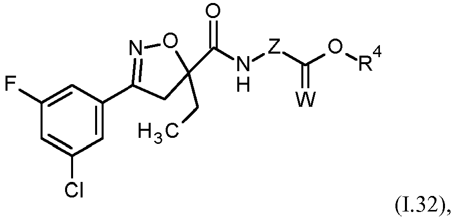
Tabelle 2.33: Erfindungsgemäße Verbindungen 2.33-1 bis 2.33-240 der allgemeinen Formel (I.33), worin Z-(C=W)-O-R⁴ wie in Tabelle 2.1 definiert ist. 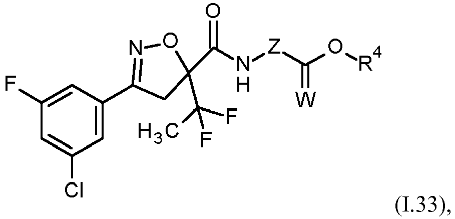
Tabelle 2.34: Erfindungsgemäße Verbindungen 2.34-1 bis 2.34-240 der allgemeinen Formel (I.34), worin Z-(C=W)-O-R⁴ wie in Tabelle 2.1 definiert ist. 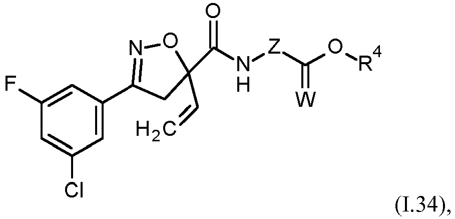
Tabelle 2.35: Erfindungsgemäße Verbindungen 2.35-1 bis 2.35-240 der allgemeinen Formel (I.35), worin Z-(C=W)-O-R⁴ wie in Tabelle 2.1 definiert ist. 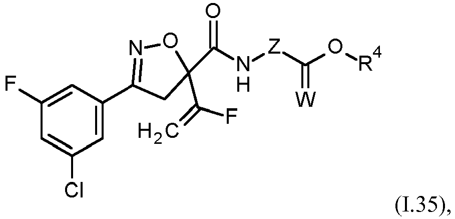
Tabelle 2.36: Erfindungsgemäße Verbindungen 2.36-1 bis 2.36-240 der allgemeinen Formel (I.36), worin Z-(C=W)-O-R⁴ wie in Tabelle 2.1 definiert ist. 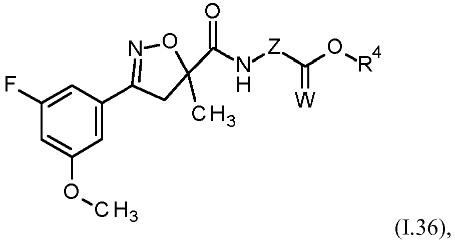
Tabelle 2.37: Erfindungsgemäße Verbindungen 2.37-1 bis 2.37-240 der allgemeinen Formel (I.37), worin Z-(C=W)-O-R⁴ wie in Tabelle 2.1 definiert ist. 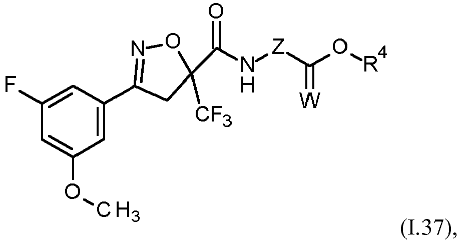
Tabelle 2.38: Erfindungsgemäße Verbindungen 2.38-1 bis 2.38-240 der allgemeinen Formel (I.38), worin Z-(C=W)-O-R⁴ wie in Tabelle 2.1 definiert ist. 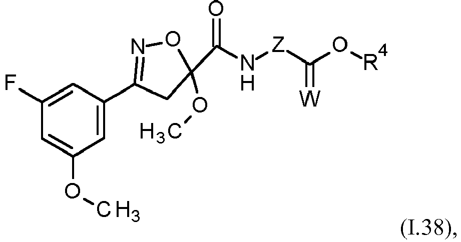
Tabelle 2.39: Erfindungsgemäße Verbindungen 2.39-1 bis 2.39-240 der allgemeinen Formel (I.39), worin Z-(C=W)-O-R⁴ wie in Tabelle 2.1 definiert ist. 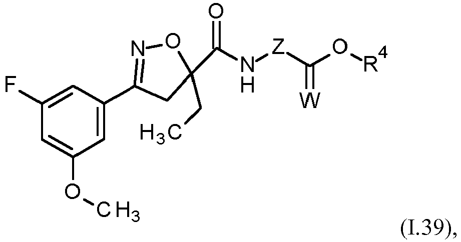
Tabelle 2.40: Erfindungsgemäße Verbindungen 2.40-1 bis 2.40-240 der allgemeinen Formel (I.40), worin Z-(C=W)-O-R⁴ wie in Tabelle 2.1 definiert ist. 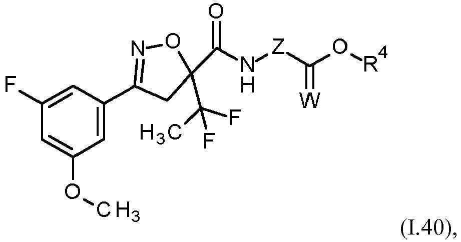
Tabelle 2.41: Erfindungsgemäße Verbindungen 2.41-1 bis 2.41-240 der allgemeinen Formel (I.41), worin Z-(C=W)-O-R⁴ wie in Tabelle 2.1 definiert ist. 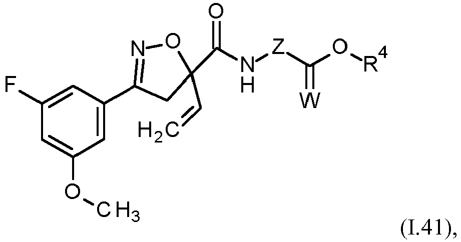
Tabelle 2.42: Erfindungsgemäße Verbindungen 2.42-1 bis 2.42-240 der allgemeinen Formel (I.42), worin Z-(C=W)-O-R⁴ wie in Tabelle 2.1 definiert ist. 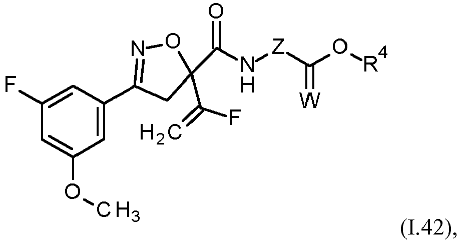
Tabelle 2.43: Erfindungsgemäße Verbindungen 2.43-1 bis 2.43-240 der allgemeinen Formel (I.43), worin Z-(C=W)-O-R⁴ wie in Tabelle 2.1 definiert ist. 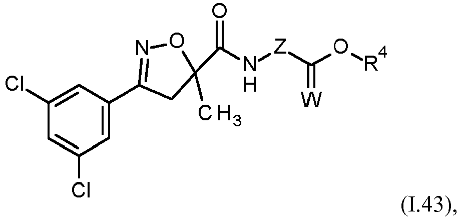
Tabelle 2.44: Erfindungsgemäße Verbindungen 2.44-1 bis 2.44-240 der allgemeinen Formel (I.44), worin Z-(C=W)-O-R⁴ wie in Tabelle 2.1 definiert ist. 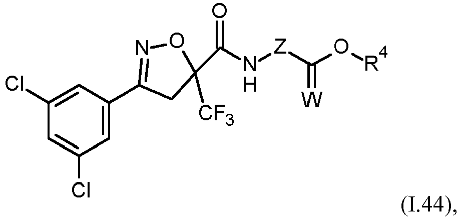
Tabelle 2.45: Erfindungsgemäße Verbindungen 2.45-1 bis 2.45-240 der allgemeinen Formel (I.45), worin Z-(C=W)-O-R⁴ wie in Tabelle 2.1 definiert ist. 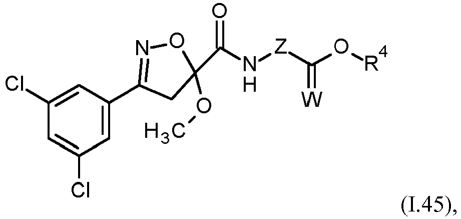
Tabelle 2.46: Erfindungsgemäße Verbindungen 2.46-1 bis 2.46-240 der allgemeinen Formel (I.46), worin Z-(C=W)-O-R⁴ wie in Tabelle 2.1 definiert ist. 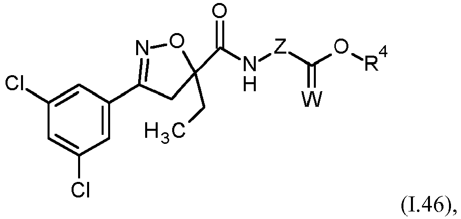
Tabelle 2.47: Erfindungsgemäße Verbindungen 2.47 bis 2.47-240 der allgemeinen Formel (I.47), worin Z-(C=W)-O-R⁴ wie in Tabelle 2.1 definiert ist. 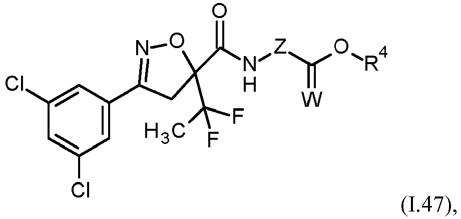
Tabelle 2.48: Erfindungsgemäße Verbindungen 2.48-1 bis 2.48-240 der allgemeinen Formel (I.48), worin Z-(C=W)-O-R⁴ wie in Tabelle 2.1 definiert ist. 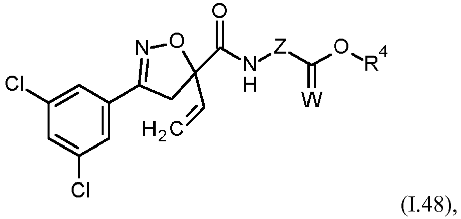
Tabelle 2.49: Erfindungsgemäße Verbindungen 2.49-1 bis 2.49-240 der allgemeinen Formel (I.49), worin Z-(C=W)-O-R⁴ wie in Tabelle 2.1 definiert ist. 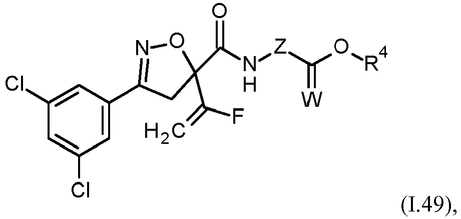
Tabelle 2.50: Erfindungsgemäße Verbindungen 2.50-1 bis 2.50-240 der allgemeinen Formel (I.50), worin Z-(C=W)-O-R⁴ wie in Tabelle 2.1 definiert ist. 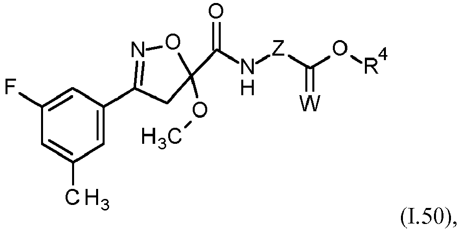
Tabelle 2.51: Erfindungsgemäße Verbindungen 2.51-1 bis 2.51-240 der allgemeinen Formel (I.51), worin Z-(C=W)-O-R⁴ wie in Tabelle 2.1 definiert ist. 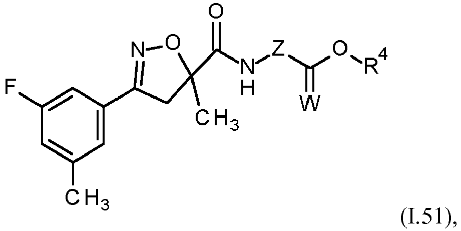
Tabelle 2.52: Erfindungsgemäße Verbindungen 2.52-1 bis 2.52-240 der allgemeinen Formel (I.52), worin Z-(C=W)-O-R⁴ wie in Tabelle 2.1 definiert ist. 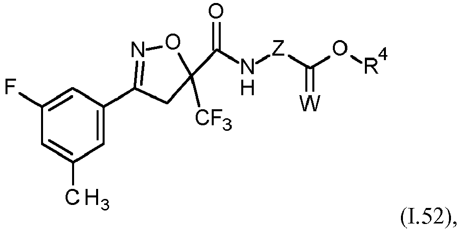
Tabelle 2.53: Erfindungsgemäße Verbindungen 2.53-1 bis 2.53-240 der allgemeinen Formel (I.53), worin Z-(C=W)-O-R⁴ wie in Tabelle 2.1 definiert ist. 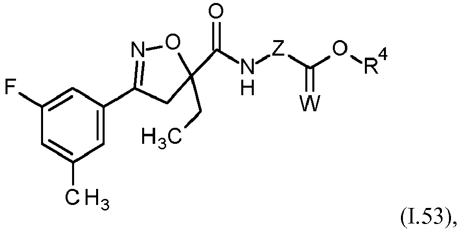
Tabelle 2.54: Erfindungsgemäße Verbindungen 2.54-1 bis 2.54-240 der allgemeinen Formel (I.54), worin Z-(C=W)-O-R⁴ wie in Tabelle 2.1 definiert ist. 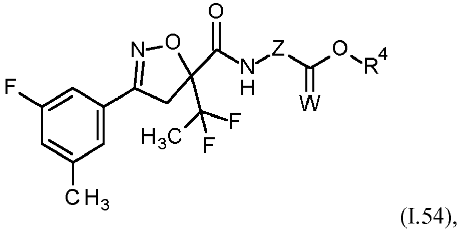
Tabelle 2.55: Erfindungsgemäße Verbindungen 2.55-1 bis 2.55-240 der allgemeinen Formel (I.55), worin Z-(C=W)-O-R⁴ wie in Tabelle 2.1 definiert ist. 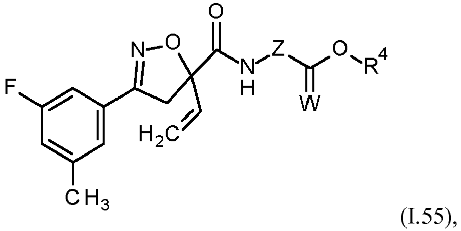
Tabelle 2.56: Erfindungsgemäße Verbindungen 2.56-1 bis 2.56-240 der allgemeinen Formel (I.56), worin Z-(C=W)-O-R⁴ wie in Tabelle 2.1 definiert ist.

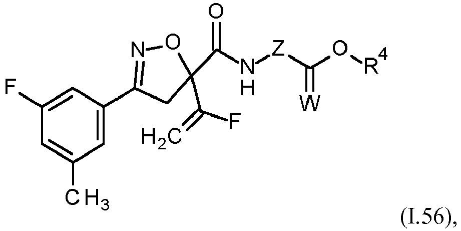

### B. Formulierungsbeispiele

### 1. Stäubemittel

Ein Stäubemittel wird erhalten, indem man 10 Gew.-Teile einer Verbindung der Formel (I) und 90 Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der Formel (I), 6 Gew.-Teile Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man
75 Gew.-Teile einer Verbindung der Formel (I),

| | | |
|---|---|---|
| 10 | " | ligninsulfonsaures Calcium, |
| 5 | " | Natriumlaurylsulfat, |
| 3 | " | Polyvinylalkohol und |
| 7 | " | Kaolin |

mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man
25 Gew.-Teile einer Verbindung der Formel (I),

| | | |
|---|---|---|
| 5 | " | 2,2'-dinaphthylmethan-6,6'-disulfonsaures Natrium, |
| 2 | " | oleoylmethyltaurinsaures Natrium, |
| 1 | " | Polyvinylalkohol, |
| 17 | " | Calciumcarbonat und |
| 50 | " | Wasser |

auf einer Kolloidmühle homogenesiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### Versuchsbeschreibung

### 1. Herbizide Wirkung und Kulturverträglichkeit im Vorauflauf

Samen von mono- bzw. dikotylen Unkraut und Kulturpflanzen werden in Kunststoff- oder Holzfasertöpfen ausgelegt und mit Erde abgedeckt. Die in Form von benetzbaren Pulvern (WP) oder als Emulsionskonzentrate (EC) formulierten erfindungsgemäßen Verbindungen werden dann als wässrige Suspension bzw. Emulsion unter Zusatz von 0,5% Additiv mit einer Wasseraufwandmenge von umgerechnet 600 1/ha auf die Oberfläche der Abdeckerde appliziert. Nach der Behandlung werden die Töpfe im Gewächshaus aufgestellt und unter guten Wachstumsbedingungen für die Testpflanzen gehalten. Nach ca. 3 Wochen wird die Wirkung der Präparate visuell im Vergleich zu unbehandelten Kontrollen in Prozentwerten bonitiert. Beispielsweise bedeutet 100% Wirkung = Pflanzen sind abgestorben, 0% Wirkung = wie Kontrollpflanzen.

In den nachfolgenden Tabellen werden folgende Abkürzungen verwendet:

### Unerwünschte Pflanzen / Weeds:

| | | | |
|---|---|---|---|
| ABUTH: | *Abutilon theophrasti* | ALOMY: | *Alopecurus myosuroides* |
| AMARE: | *Amaranthus retroflexus* | AVEFA: | *Avena fatua* |
| ECHCG: | *Echinochloa crus-galli* | HORMU: | *Hordeum murinum* |
| LOLRI: | *Lolium rigidum* | PHBPU: | *Pharbitis purpurea* |
| POLCO: | *Polygonum convolvulus* | SETVI: | *Setaria viridis* |
| STEME: | *Stellaria media* | VERPE: | *Veronica persica* |
| VIOTR: | *Viola tricolor* | | |

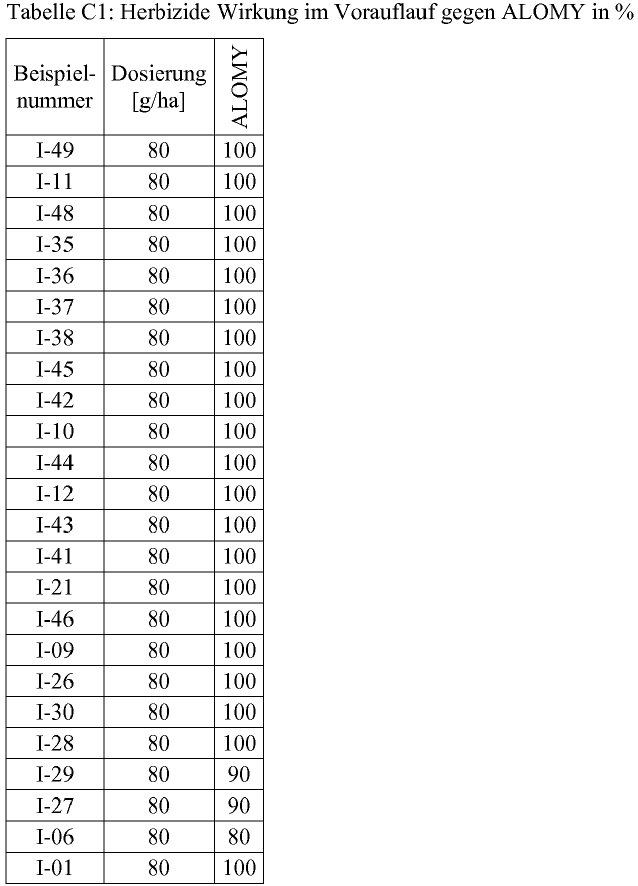

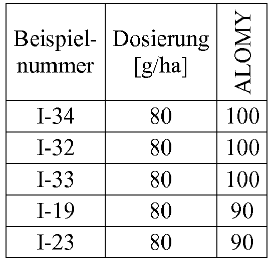

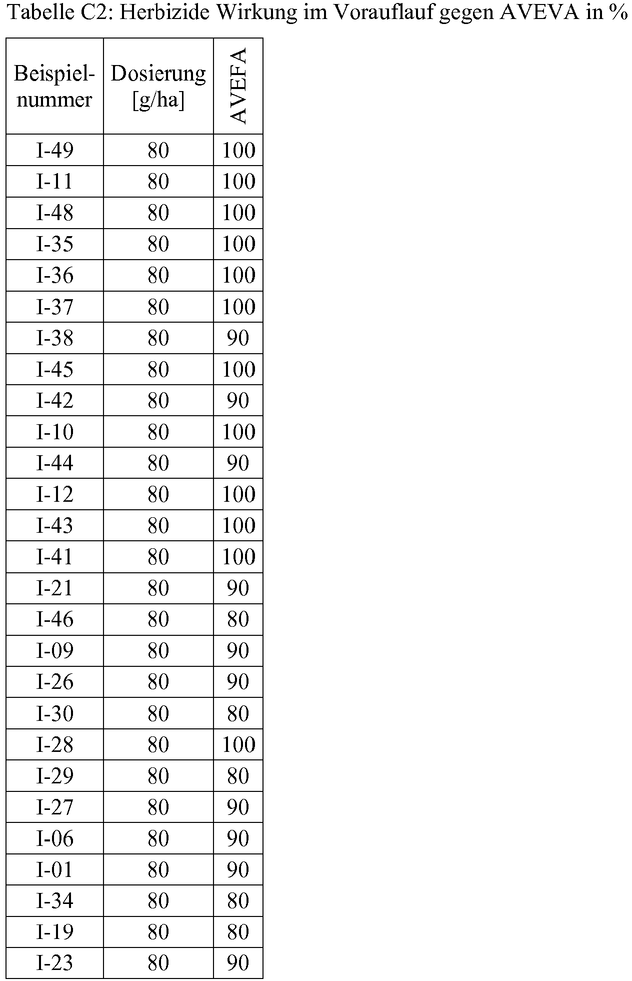

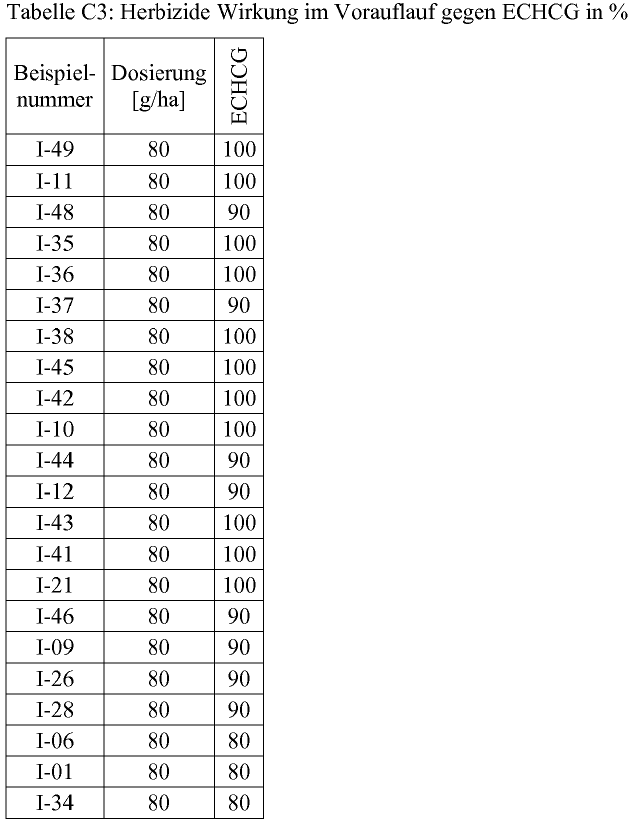

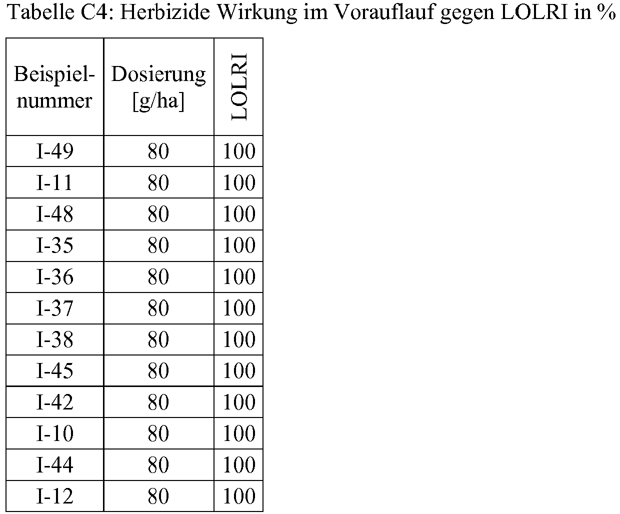

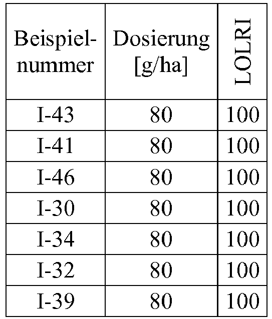

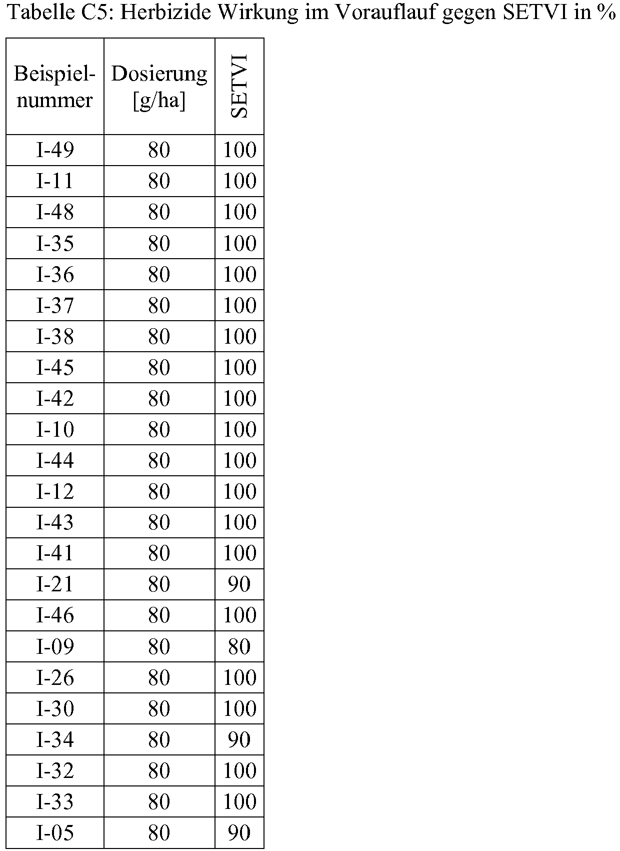

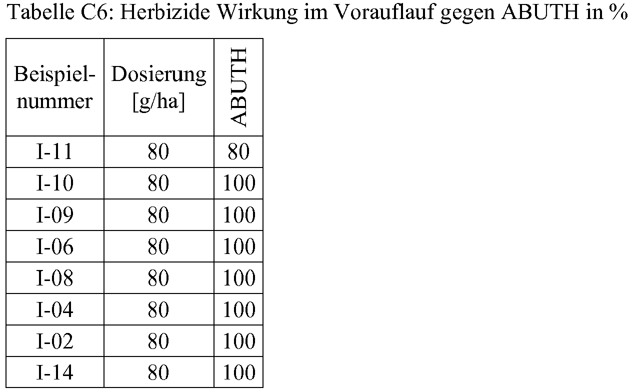

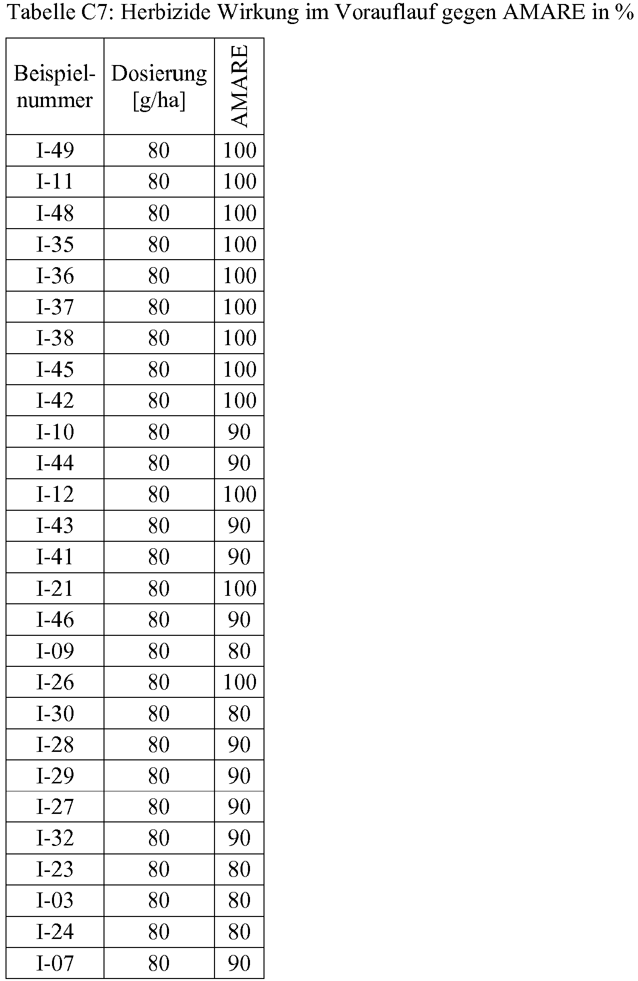

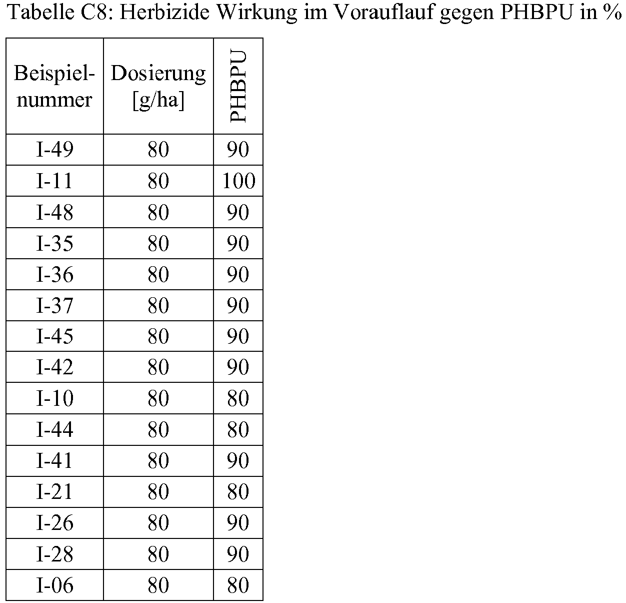

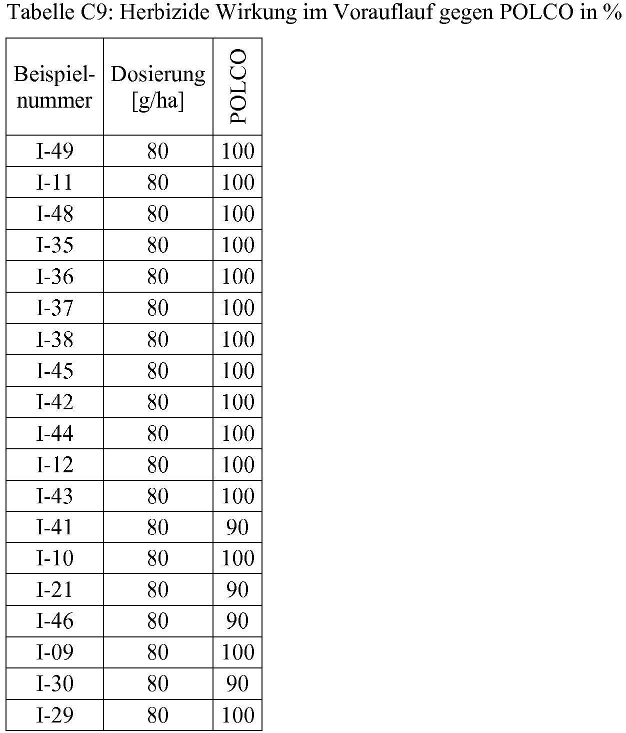

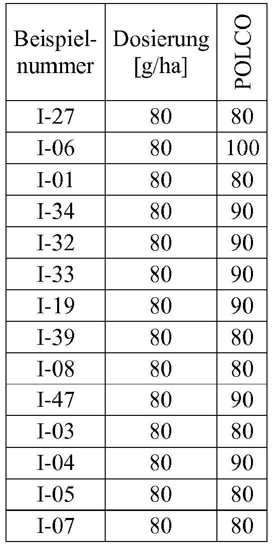

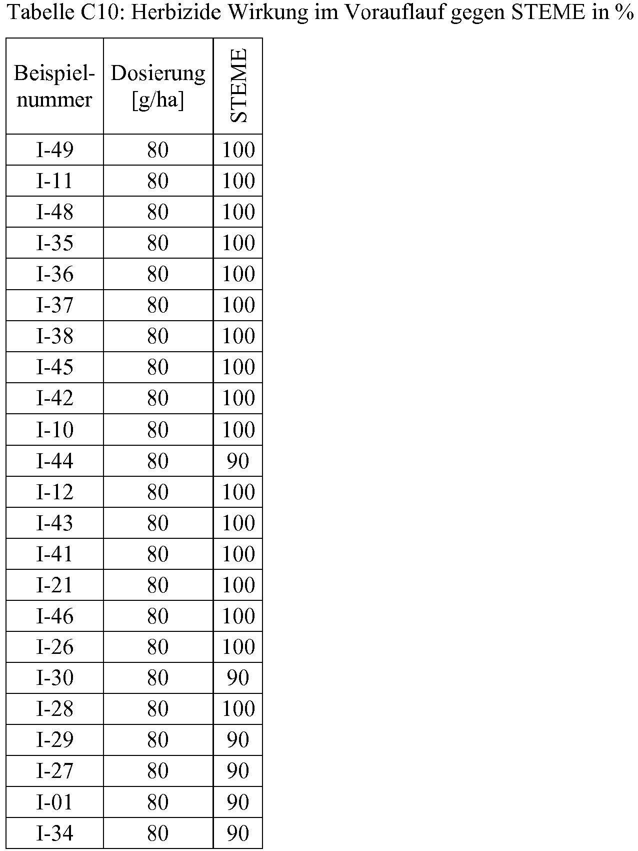

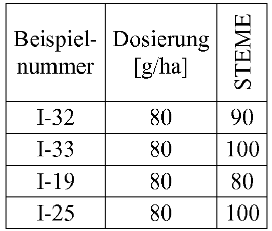

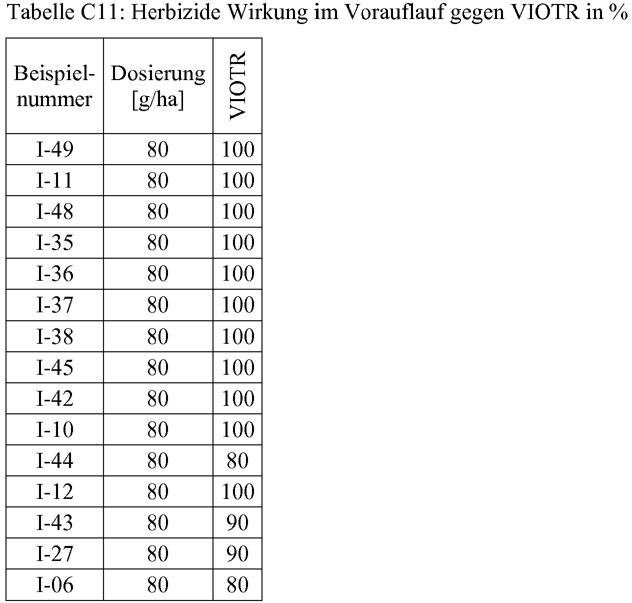

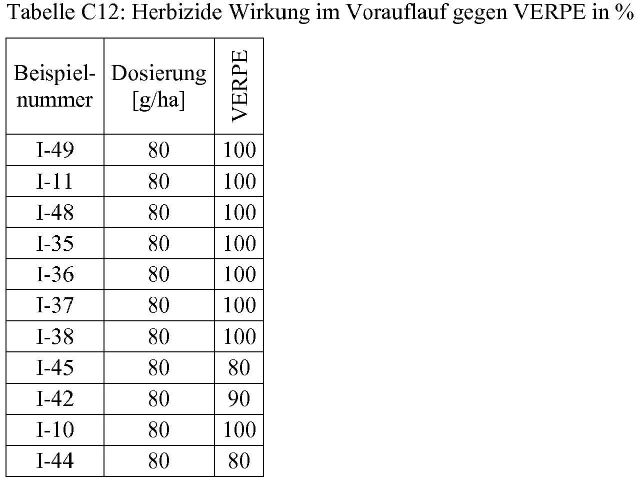

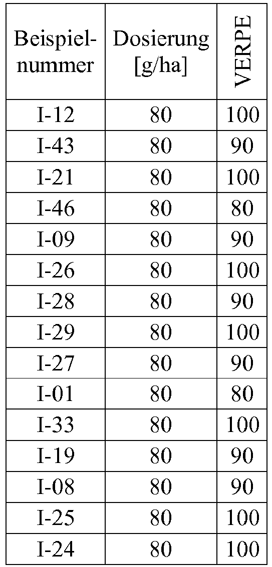

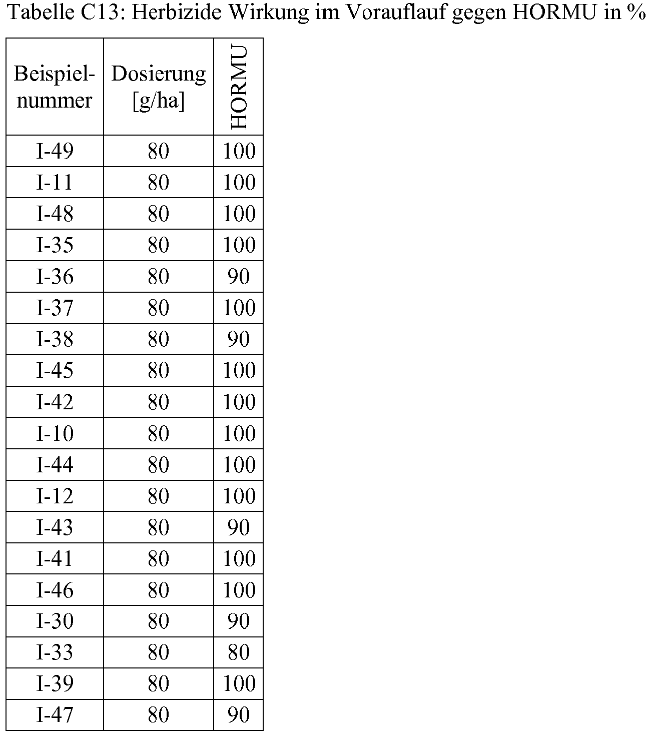

Wie die Ergebnisse zeigen, weisen erfindungsgemäße Verbindungen, wie beispielsweise die Verbindungen Nr. I-21 und andere Verbindungen aus den Tabellen (beispielsweise I-10, I-26, I-09, I-30, I-28, I-29, I-27, I-01, I-06, I-34, I-32, I-33, I-19, I-11, I-12, I-35, I-36, I-37, I-38, I-41, I-42, I-43, I-44, I-46, I-49) bei Behandlung im Vorauflauf eine eine sehr gute Wirkung (80% bis 100% herbizide Wirkung) gegen Schadpflanzen wie *Abutilon theophrasti, Alopecurus myosuroides, Amaranthus retroflexus, Avena fatua, Echinochloa crus-galli, Hordeum murinum, Lolium rigidum, Pharbitis purpurea, Polygonum convolvulus, Setaria viridis, Stellaria media, Veronica persica* und *Viola tricolor* bei einer Aufwandmenge von 0.08 kg Aktivsubstanz oder weniger pro Hektar auf.

### 2. Herbizide Wirkung und Kulturverträglichkeit im Nachauflauf

Samen von mono- bzw. dikotylen Unkraut- bzw. Kulturpflanzen werden in Kunststoff- oder Holzfasertöpfen in sandigem Lehmboden ausgelegt, mit Erde abgedeckt und im Gewächshaus unter kontrollierten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wässrige Suspension bzw. Emulsion unter Zusatz von 0,5% Additiv mit einer Wasseraufwandmenge von umgerechnet 600 1/ha auf die grünen Pflanzenteile gesprüht. Nach ca. 3 Wochen Standzeit der Versuchspflanzen im Gewächshaus, unter optimalen Wachstumsbedingungen, wird die Wirkung der Präparate visuell im Vergleich zu unbehandelten Kontrollen bonitiert. Beispielsweise bedeutet 100% Wirkung = Pflanzen sind abgestorben, 0% Wirkung = wie Kontrollpflanzen.

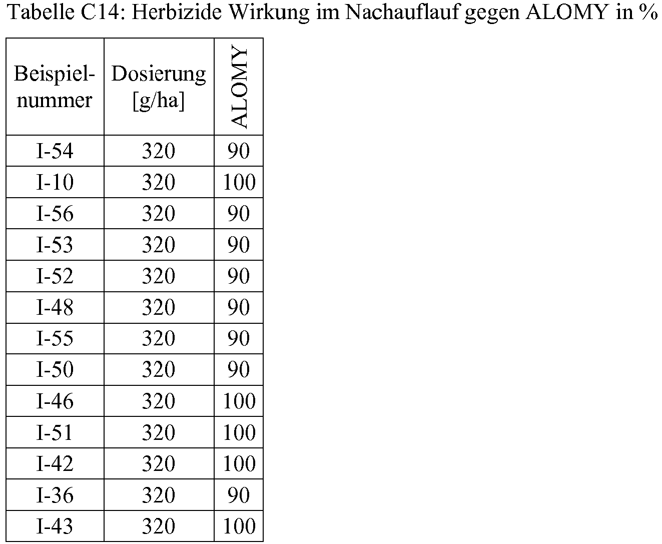

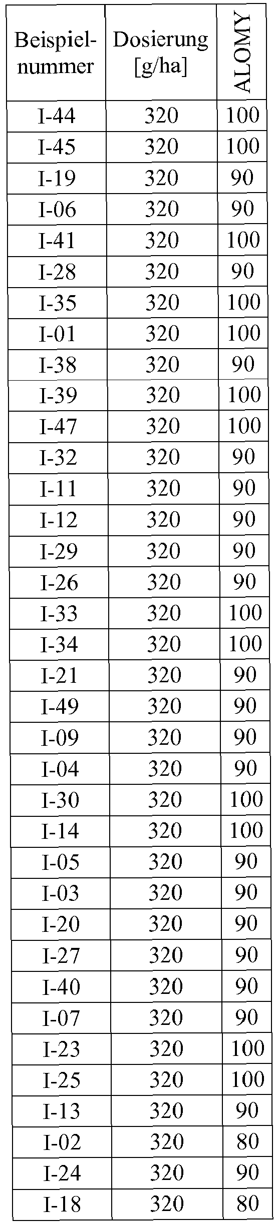

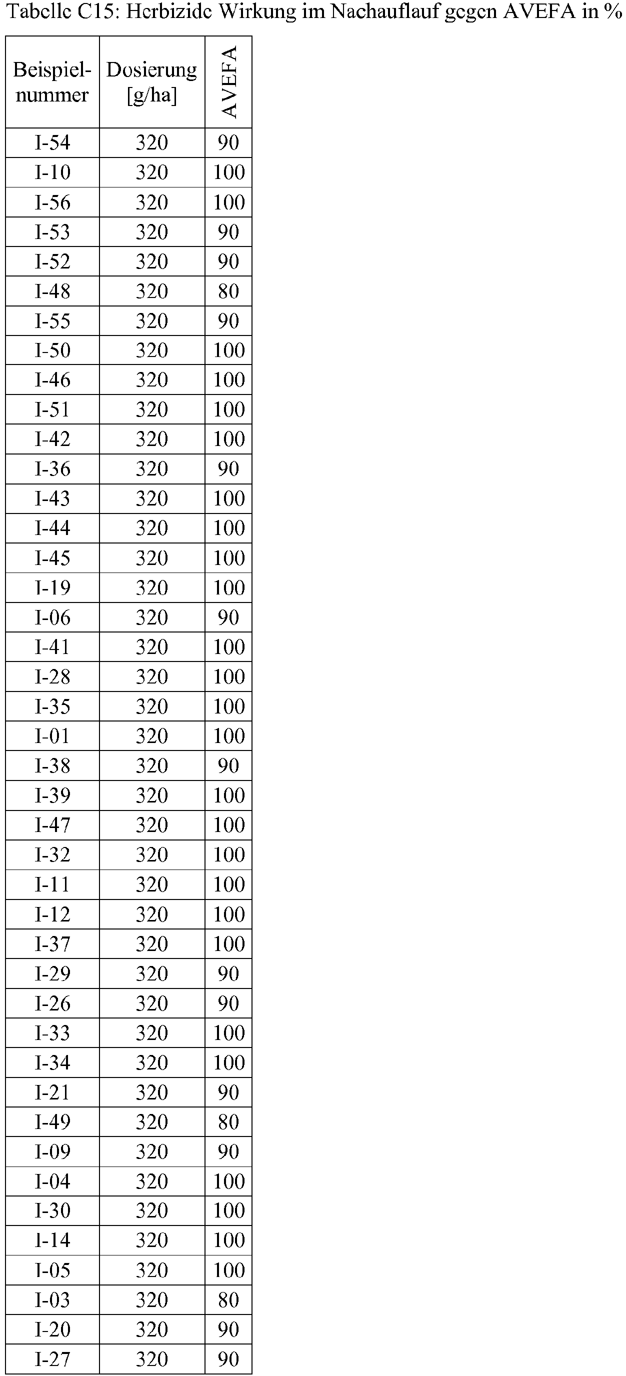

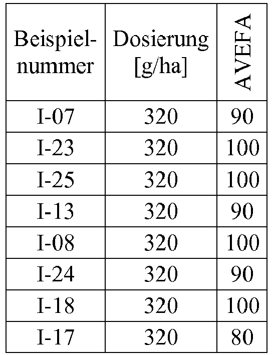

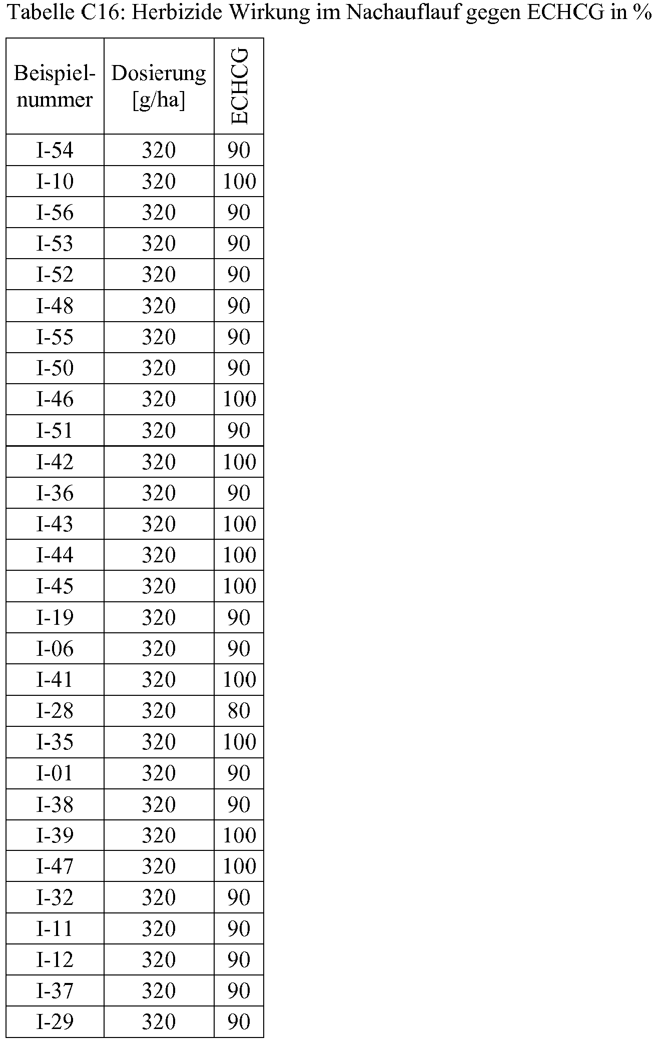

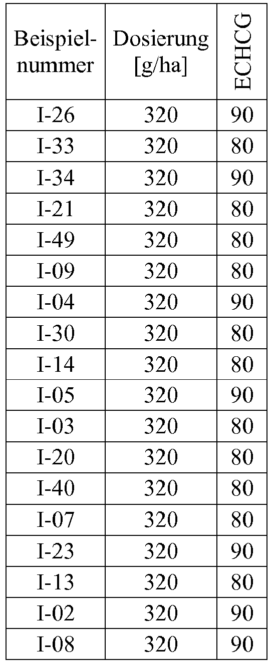

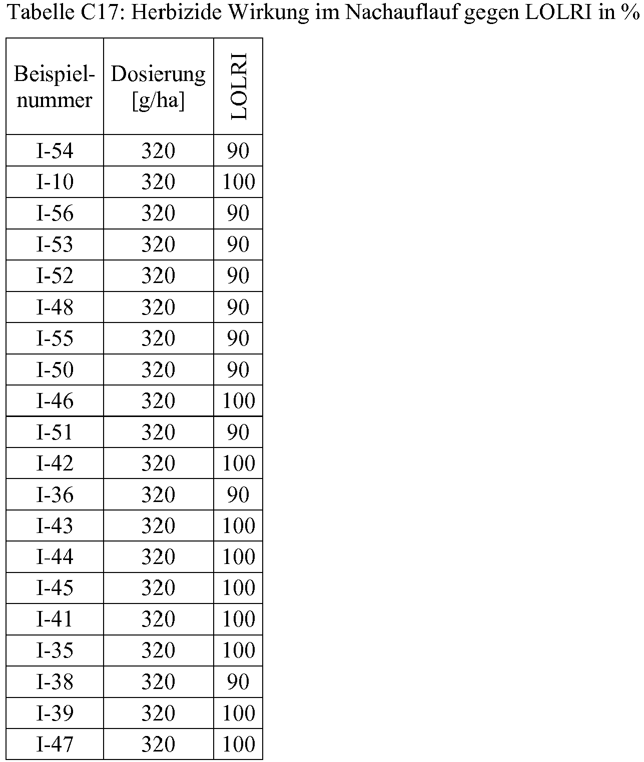

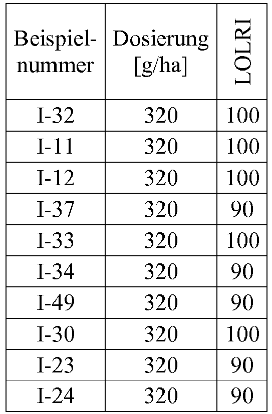

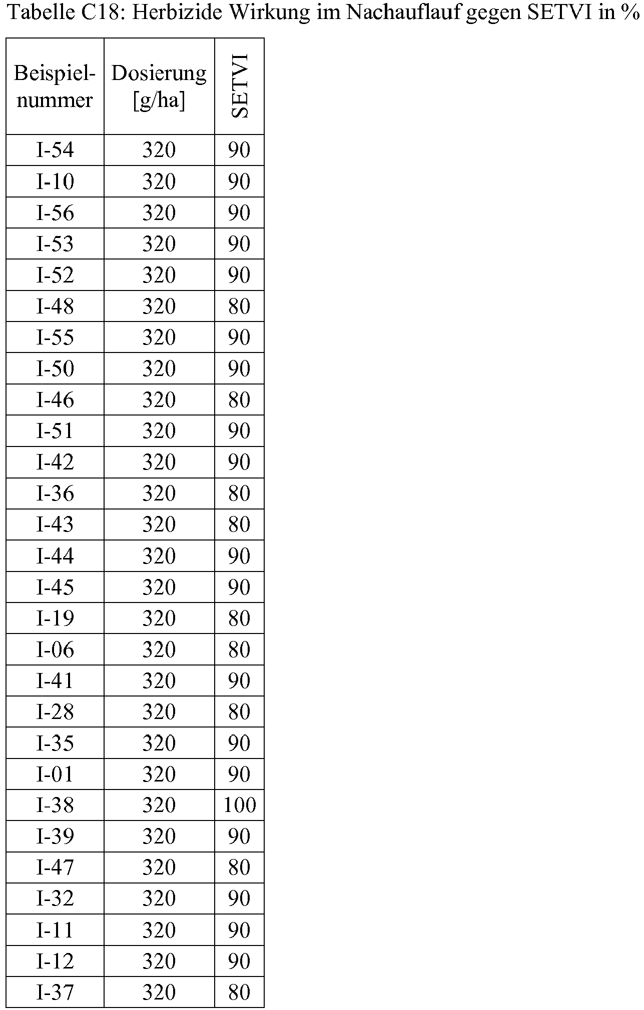

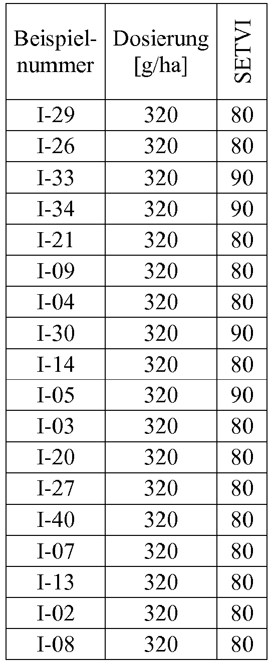

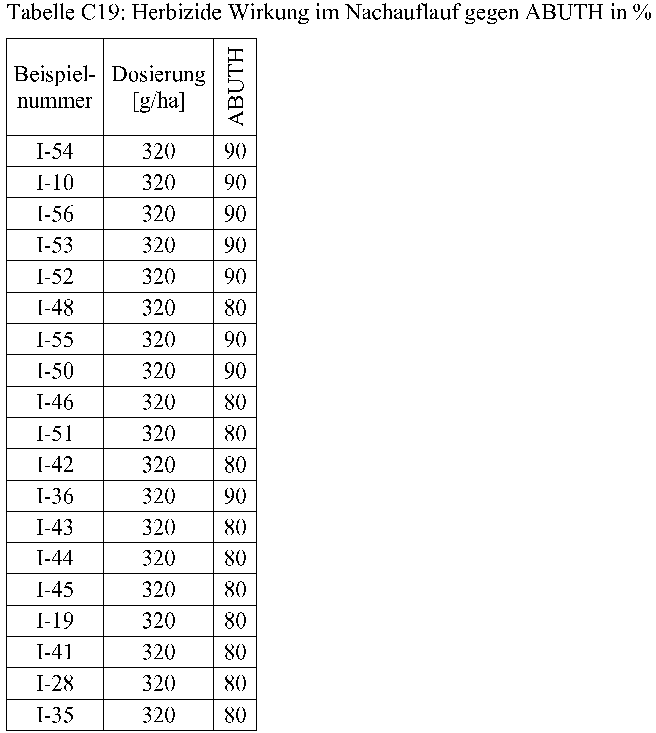

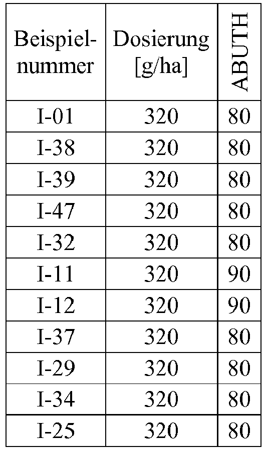

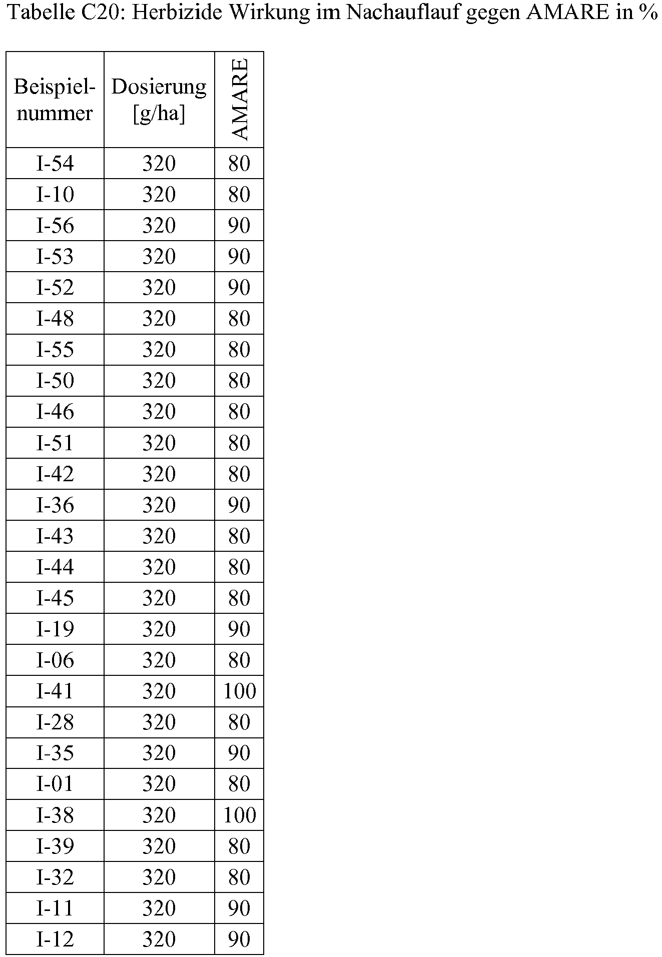

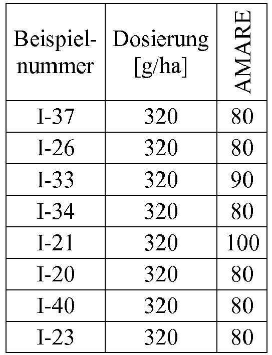

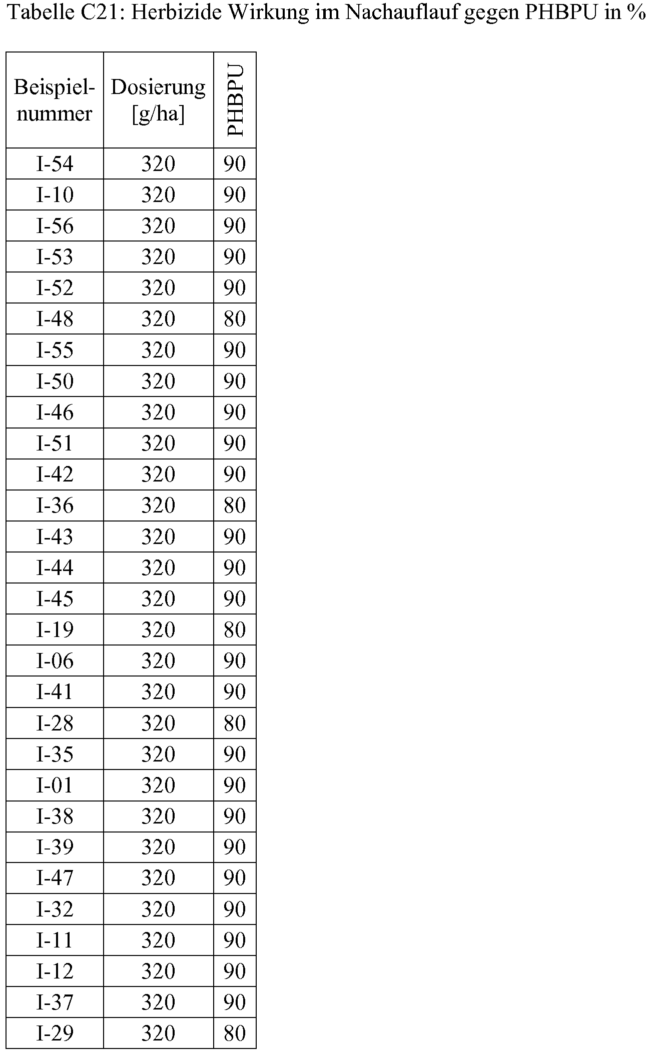

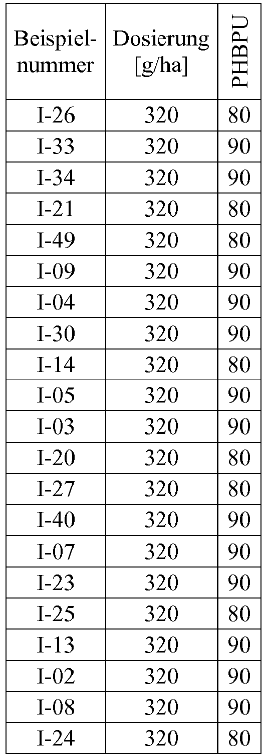

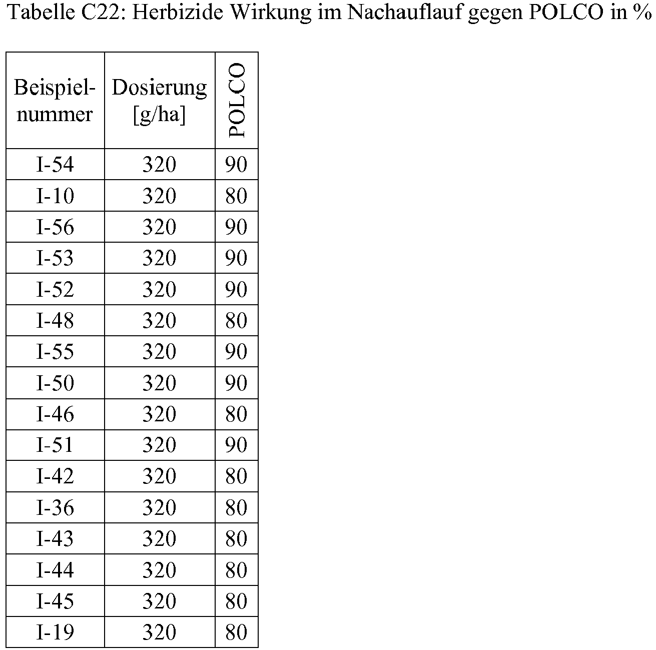

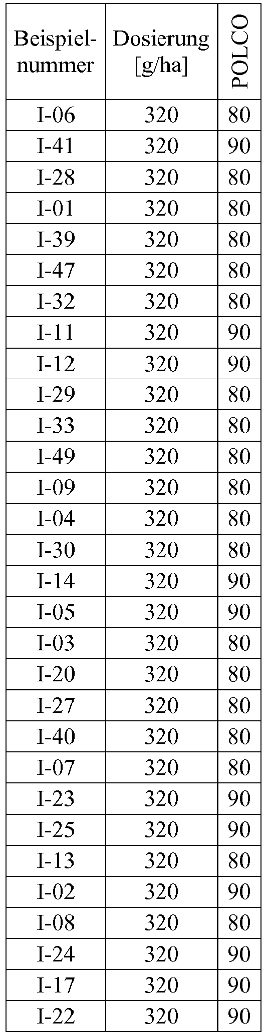

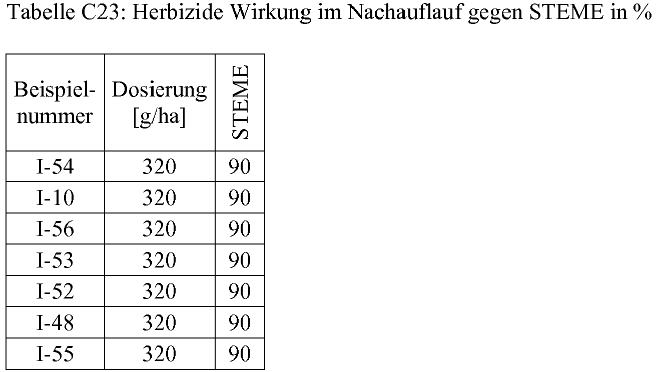

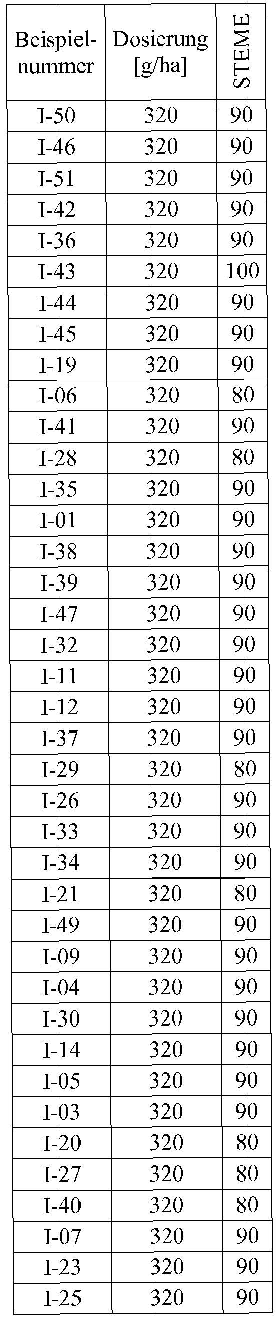

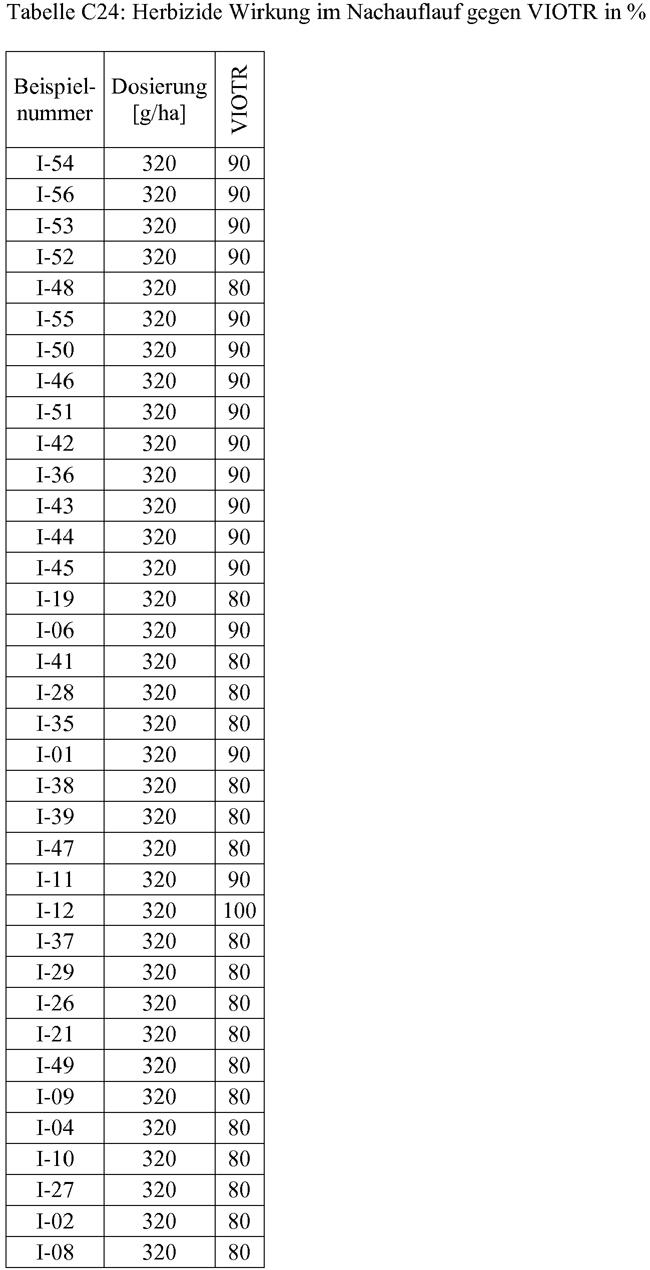

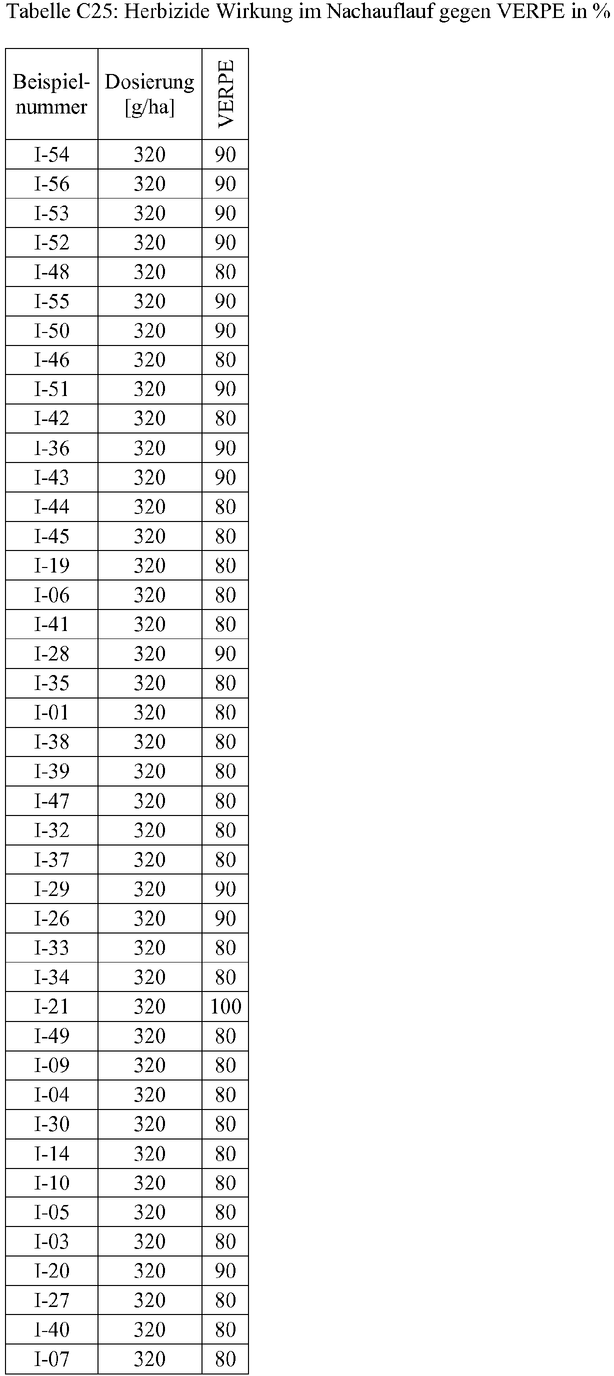

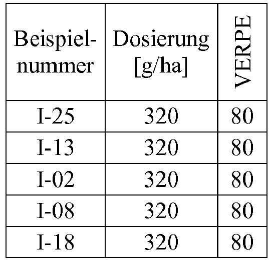

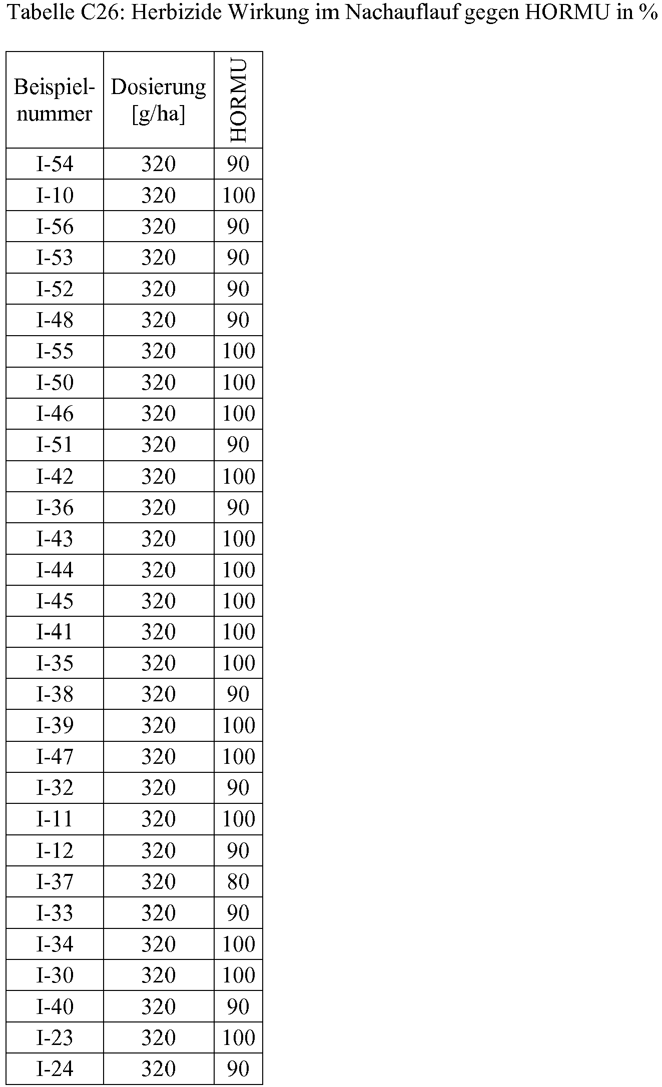

Wie die Ergebnisse zeigen, weisen erfindungsgemäße Verbindungen, wie beispielsweise die Verbindungen Nr. I-35 und andere Verbindungen aus den Tabellen (beispielsweise I-28, I-38, I-01, I-19, I-36, I-29, I-32, I-11, I-12, I-37, I-06, I-09, I-33, I-34, I-13, I-04, I-26, I-21, I-30, I-05, I-03, I-20, I-27, I-14, I-10, I-23, I-07, I-25, I-02, I-08, I-24, I-41, I-42, I-43, I-44, I-46, I-49, I-54, I-55, I-56) bei Behandlung im Nachauflauf eine sehr gute herbizide Wirkung (80% bis 100% herbizide Wirkung) gegen Schadpflanzen wie *Abutilon theophrasti, Alopecurus myosuroides, Amaranthus retroflexus, Avena fatua, Echinochloa crus-galli, Hordeum murinum, Lolium rigidum, Pharbitis purpurea, Polygonum convolvulus, Setaria viridis, Stellaria media, Veronica persica* und *Viola tricolor* bei einer Aufwandmenge von 0.32 kg Aktivsubstanz oder weniger pro Hektar auf.

## Patentansprüche

1. 3-Phenylisoxazolin-5-carboxamide und -5-thioamide der allgemeinen Formel (I), 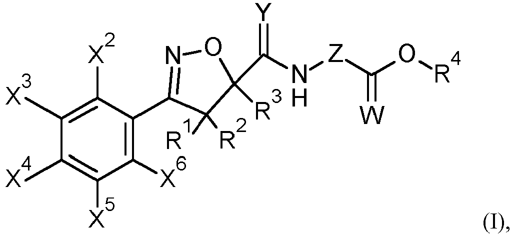 und deren agrochemisch verträglichen Salze, in welchen
R¹ und R² unabhängig voneinander jeweils Wasserstoff, Fluor, Chlor oder Cyano, oder durch jeweils m Reste aus der Gruppe bestehend aus Fluor, Chlor, Brom und Cyano substituiertes (C₁-C₃)-Alkyl oder (C₁-C₃)-Alkoxy bedeuten;
R³ durch jeweils m Reste aus der Gruppe bestehend aus Fluor, Chlor, Brom, Cyano, (C₁-C₄)-Alkoxy und Hydroxy substituiertes (C₁-C₄)-Alkyl, (C₃-C₅)-Cycloalkyl, (C₂-C₄)-Alkenyl, (C₂-C₄)-Alkinyl oder (C₁-C₄)-Alkoxy bedeutet;
R⁴ Wasserstoff, oder durch jeweils m Reste aus der Gruppe bestehend aus Halogen, Cyano, (C₁-C₆)-Alkoxy, Hydroxy und Aryl substituiertes (C₁-C₁₂)-Alkyl, (C₃-C₇)-Cycloalkyl, (C₃-C₇)-Cycloalkyl-(C₁-C₈)-Alkyl, (C₂-C₆)-Alkenyl, (C₅-C₆)-Cycloalkenyl oder (C₂-C₈)-Alkinyl bedeutet;
Y Sauerstoff oder Schwefel bedeutet;
W Sauerstoff oder Schwefel bedeutet;
Z für einen vollständig gesättigten oder teilweise gesättigten Furanring steht, der mit k Resten der Gruppe R⁵ substituiert ist 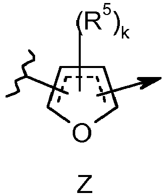 worin der Pfeil jeweils für eine Bindung zur Gruppe C=W der Formel (I) steht;
R⁵ Fluor, Chlor, Cyano oder CO₂R⁷, oder durch jeweils m Reste aus der Gruppe bestehend aus Fluor und Chlor substituiertes (C₁-C₂)-Alkyl oder (C₁-C₂)-Alkoxy bedeutet;
X², X⁴ und X⁶ unabhängig voneinander jeweils Wasserstoff, Fluor, Chlor, Brom oder Cyano, oder jeweils durch m Reste aus der Gruppe bestehend aus Fluor, Chlor, Brom und (C₁-C₂)-Alkoxy substituiertes (C₁-C₂)-Alkyl bedeuten;
X³ und X⁵ unabhängig voneinander Wasserstoff, Fluor, Chlor, Brom, Iod, Hydroxy, Cyano, Nitro, S(O)ₙR⁶ oder CO₂R⁷, oder jeweils durch m Reste aus der Gruppe bestehend aus Fluor, Chlor und Brom substituiertes (C₁-C₃)-Alkyl, (C₁-C₃)-Alkoxy, (C₃-C₄)-Cycloalkyl, (C₂-C₃)-Alkenyl oder (C₂-C₃)-Alkinyl bedeuten;
R⁶ durch jeweils m Reste aus der Gruppe bestehend aus Fluor und Chlor substituiertes (C₁-C₂)-Alkyl oder (C₃-C₄)-Cycloalkyl bedeutet;
R⁷ Wasserstoff, oder durch jeweils m Reste aus der Gruppe bestehend aus Fluor, Chlor und (C₁-C₂)-Alkoxy substituiertes (C₁-C₃)-Alkyl oder (C₃-C₅)-Cycloalkyl bedeutet;
k die Laufzahl 0, 1 oder 2 beträgt;
m die Laufzahl 0, 1, 2, 3, 4 oder 5 beträgt; und
n die Laufzahl 0, 1 oder 2 beträgt.

2. Verbindungen der allgemeinen Formel (I) gemäß Anspruch 1, wobei R¹ und R² unabhängig voneinander jeweils Wasserstoff, Fluor, Chlor oder Cyano,
oder
durch jeweils m Reste aus der Gruppe bestehend aus Fluor und Chlor substituiertes Methyl oder Methoxy bedeuten.

3. Verbindungen der allgemeinen Formel (I) gemäß Anspruch 1 oder 2, wobei R³ durch jeweils m Reste aus der Gruppe bestehend aus Fluor, Chlor, Brom, Cyano, (C₁-C₂)-Alkoxy und Hydroxy substituiertes (C₁-C₃)-Alkyl, (C₃-C₄)-Cycloalkyl, (C₂-C₃)-Alkenyl, (C₂-C₃)-Alkinyl oder (C₁-C₃)-Alkoxy bedeutet.

4. Verbindungen der allgemeinen Formel (I) gemäß einem der Ansprüche 1 bis 3, wobei R⁴ Wasserstoff,
oder
durch jeweils m Reste aus der Gruppe bestehend aus Fluor, Chlor, Brom, Cyano, (C₁-C₄)-Alkoxy, Hydroxy und Aryl substituiertes (C₁-C₆)-Alkyl, (C₃-C₆)-Cycloalkyl, (C₃-C₆)-Cycloalkyl-(C₁-C₆)-Alkyl, (C₂-C₆)-Alkenyl, (C₅-C₆)-Cycloalkenyl oder (C₂-C₆)-Alkinyl bedeutet.

5. Verbindungen der allgemeinen Formel (I) gemäß einem der Ansprüche 1 bis 4, wobei Y und W Sauerstoff bedeutet.

6. Verbindungen der allgemeinen Formel (I) gemäß einem der Ansprüche 1 bis 5, wobei Z für eine Gruppe Z-1 bis Z-33 steht, wobei Z-1 bis Z-33 folgende Bedeutung haben: 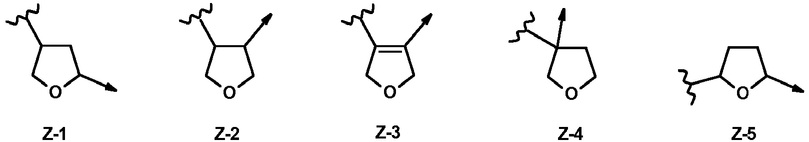 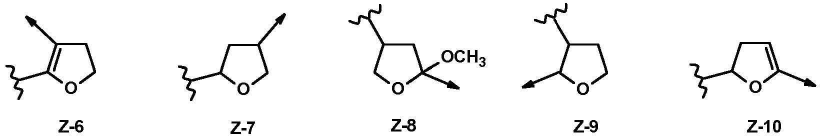 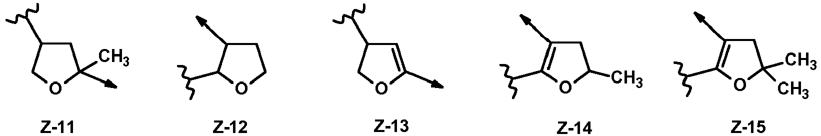 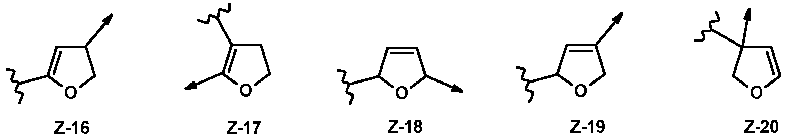 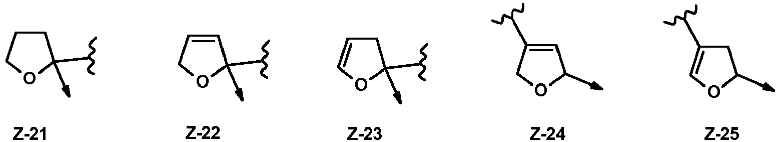 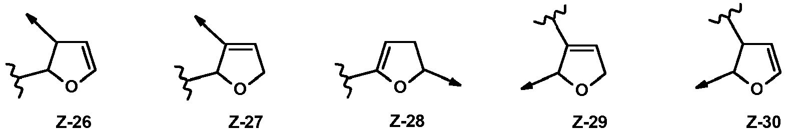 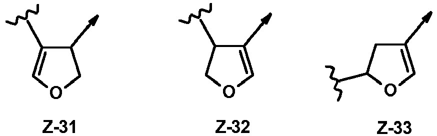 worin der Pfeil jeweils für eine Bindung zur Gruppe C=W der Formel (I) steht.

7. Verbindungen der allgemeinen Formel (I) gemäß einem der Ansprüche 1 bis 6, wobei R⁵ Fluor, Chlor, Cyano, CO₂H, CO₂CH₃ oder CO₂CH₂CH₃,
oder
jeweils durch m Reste aus der Gruppe bestehend aus Fluor und Chlor substituiertes (C₁-C₂)-Alkyl oder (C₁-C₂)-Alkoxy bedeutet.

8. Verbindungen der allgemeinen Formel (I) gemäß einem der Ansprüche 1 bis 7, wobei X², X⁴ und X⁶ unabhängig voneinander jeweils Wasserstoff, Fluor, Chlor, Brom oder Cyano,
oder
jeweils durch m Reste aus der Gruppe bestehend aus Fluor und Chlor substituiertes Methyl oder Methoxy bedeuten.

9. Verbindungen der allgemeinen Formel (I) gemäß einem der Ansprüche 1 bis 8, wobei X³ und X⁵ Wasserstoff, Fluor, Chlor, Brom, Hydroxy oder Cyano,
oder
jeweils durch m Reste aus der Gruppe bestehend aus Fluor, Chlor und Brom substituiertes (C₁-C₃)-Alkyl, (C₁-C₃)-Alkoxy, (C₃-C₄)-Cycloalkyl, (C₂-C₃)-Alkenyl oder (C₂-C₃)-Alkinyl bedeuten.

10. Verbindungen der allgemeinen Formel (I) gemäß einem der Ansprüche 1 bis 9, wobei R⁷ Wasserstoff,
oder
durch jeweils m Reste aus der Gruppe bestehend aus Fluor und Chlor substituiertes (C₁-C₃)-Alkyl bedeutet.

11. Verbindungen der allgemeinen Formel (I) gemäß einem der Ansprüche 1 bis 10, wobei die Laufzahl m 0, 1, 2 oder 3 beträgt.

12. Verbindungen der allgemeinen Formel (I) gemäß einem der Ansprüche 1 bis 11,
in welchen
R¹ und R² jeweils Wasserstoff bedeuten;
R³ durch jeweils m Reste aus der Gruppe bestehend aus Fluor, Chlor, Brom, Cyano, (C₁-C₂)-Alkoxy und Hydroxy substituiertes (C₁-C₃)-Alkyl, (C₃-C₄)-Cycloalkyl, (C₂-C₃)-Alkenyl, (C₂-C₃)-Alkinyl oder (C₁-C₃)-Alkoxy bedeutet;
R⁴ Wasserstoff bedeutet, oder durch jeweils m Reste aus der Gruppe bestehend aus Fluor, Chlor, Brom, Cyano, (C₁-C₄)-Alkoxy, Hydroxy und Aryl substituiertes (C₁-C₆)-Alkyl, (C₃-C₆)-Cycloalkyl, (C₃-C₆)-Cycloalkyl-(C₁-C₆)-Alkyl, (C₂-C₆)-Alkenyl, (C₅-C₆)-Cycloalkenyl oder (C₂-C₆)-Alkinyl bedeutet;
Y bedeutet Sauerstoff;
W bedeutet Sauerstoff;
Z für eine Gruppe Z-1 bis Z-4 steht, wobei Z-1 bis Z-4 folgende Bedeutung haben: 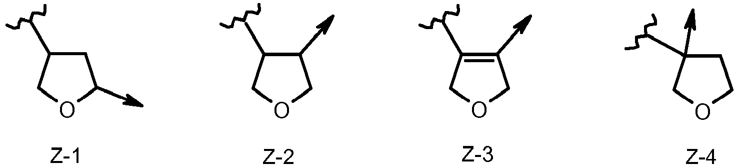 worin der Pfeil jeweils für eine Bindung zur Gruppe C=W der Formel (I) steht;
X², X⁴ und X⁶ unabhängig voneinander Wasserstoff oder Fluor bedeuten;
X³ und X⁵ unabhängig voneinander Wasserstoff, Fluor, Chlor, Brom, Hydroxy oder Cyano,
oder
jeweils durch m Reste aus der Gruppe bestehend aus Fluor, Chlor und Brom substituiertes (C₁-C₃)-Alkyl, (C₁-C₃)-Alkoxy, (C₃-C₄)-Cycloalkyl, (C₂-C₃)-Alkenyl oder (C₂-C₃)-Alkinyl bedeuten; und
m die Laufzahl 0, 1, 2 oder 3 beträgt.

13. Herbizides Mittel oder pflanzenwachstumsregulierendes Mittel, **dadurch gekennzeichnet, dass** es eine oder mehrere Verbindungen der allgemeinen Formel (i) oder deren Salze nach einem der Ansprüche 1 bis 12 enthält.

14. Herbizide Mittel nach Anspruch 13, die ferner ein Formulierungshilfsmitteln enthält.

15. Herbizide Mittel nach Anspruch 13 oder 14 enthaltend mindestens einen weiteren Wirkstoff aus der Gruppe der Insektizide, Akarizide, Herbizide, Fungizide, Safenern und/oder Wachstumsregulatoren.

16. Herbizide Mittel nach Anspruch 14 oder 15 enthaltend einen Safener.

17. Herbizide Mittel nach Anspruch 16, worin der Safener ausgewählt ist aus der Gruppe bestehend aus Mefenpyr-diethyl, Cyprosulfamid, Isoxadifen-ethyl, Cloquintocet-mexyl, Benoxacor und Dichlormid.

18. Verfahren zur Bekämpfung unerwünschter Pflanzen, **dadurch gekennzeichnet, dass** man eine wirksame Menge mindestens einer Verbindung der Formel (I) gemäß einem der Ansprüche 1 bis 12 oder eines herbiziden Mittels nach einem der Ansprüche 13 bis 17 auf die Pflanzen oder auf den Ort des unerwünschten Pflanzenwachstums appliziert.

19. Verwendung von Verbindungen der Formel (I) gemäß einem der Ansprüche 1 bis 12 oder von herbiziden Mitteln nach einem der Ansprüche 13 bis 17 zur Bekämpfung unerwünschter Pflanzen.

20. Verwendung nach Anspruch 19, **dadurch gekennzeichnet, dass** die Verbindungen der Formel (I) zur Bekämpfung unerwünschter Pflanzen in Kulturen von Nutzpflanzen eingesetzt werden.

21. Verwendung nach Anspruch 20, **dadurch gekennzeichnet, dass** die Nutzpflanzen transgene Nutzpflanzen sind.

22. Verbindungen der Formel (II) und deren agrochemisch verträglichen Salze 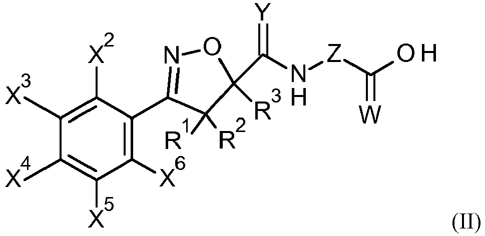 worin die Reste X², X³, X⁴, X⁵, X⁶, R¹, R², R³, Y, Z und W gemäß den Ansprüchen 1 bis 12 definiert sind.

## Claims

1. 3-Phenylisoxazoline-5-carboxamides and -5-thioamides of the general formula (I) 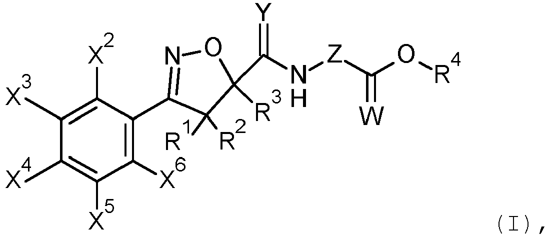 and the agrochemically acceptable salts thereof in which
R¹ and R² independently of one another each represent hydrogen, fluorine, chlorine or cyano,
or
represent (C₁-C₃)-alkyl or (C₁-C₃)-alkoxy, each of which is substituted by m radicals from the group consisting of fluorine, chlorine, bromine and cyano;
R³ represents (C₁-C₄)-alkyl, (C₃-C₅)-cycloalkyl, (C₂-C₄)-alkenyl, (C₂-C₄)-alkynyl or (C₁-C₄)-alkoxy, each of which is substituted by m radicals from the group consisting of fluorine, chlorine, bromine, cyano, (C₁-C₄)-alkoxy and hydroxy;
R⁴ represents hydrogen,
or
represents (C₁-C₁₂)-alkyl, (C₃-C₇)-cycloalkyl, (C₃-C₇)-cycloalkyl- (C₁-C₈)-alkyl, (C₂-C₆)-alkenyl, (C₅-C₆)-cycloalkenyl or (C₂-C₈)-alkynyl, each of which is substituted by m radicals from the group consisting of halogen, cyano, (C₁-C₆)-alkoxy, hydroxy and aryl;
Y represents oxygen or sulfur;
W represents oxygen or sulfur;
Z represents a fully saturated or partially saturated furan ring which is substituted by k radicals from the group R⁵, 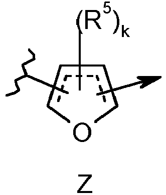 where the arrow in each case denotes a bond to the group C=W of the formula (I);
R⁵ represents fluorine, chlorine, cyano or CO₂R⁷,
or
represents (C₁-C₂)-alkyl or (C₁-C₂)-alkoxy, each of which is substituted by m radicals from the group consisting of fluorine and chlorine;
X², X⁴ and X⁶ independently of one another each represent hydrogen, fluorine, chlorine, bromine or cyano,
or
represent (C₁-C₂)-alkyl, in each case substituted by m radicals from the group consisting of fluorine, chlorine, bromine and (C₁-C₂)-alkoxy;
X³ and X⁵ independently of one another represent hydrogen, fluorine, chlorine, bromine, iodine, hydroxy, cyano, nitro, S(O)ₙ R⁶ or CO₂R⁷,
or
represent (C₁-C₃)-alkyl, (C₁-C₃)-alkoxy, (C₃-C₄)-cycloalkyl, (C₂-C₃)-alkenyl or (C₂-C₃)-alkynyl, each of which is substituted by m radicals from the group consisting of fluorine, chlorine and bromine;
R⁶ represents (C₁-C₂)-alkyl or (C₃-C₄)-cycloalkyl, each of which is substituted by m radicals from the group consisting of fluorine and chlorine;
R⁷ represents hydrogen,
or
represents (C₁-C₃)-alkyl or (C₃-C₅)-cycloalkyl, each of which is substituted by m radicals from the group consisting of fluorine, chlorine and (C₁-C₂)-alkoxy;
k is the running number 0, 1 or 2;
m is the running number 0, 1, 2, 3, 4 or 5; and
n is the running number 0, 1 or 2.

2. Compounds of the general formula (I) according to Claim 1, where R¹ and R² independently of one another each represent hydrogen, fluorine, chlorine or cyano,
or
represent methyl or methoxy, each of which is substituted by m radicals from the group consisting of fluorine and chlorine.

3. Compounds of the general formula (I) according to Claim 1 or 2, where R³ represents (C₁-C₃)-alkyl, (C₃-C₄)-cycloalkyl, (C₂-C₃)-alkenyl, (C₂-C₃)-alkynyl or (C₁-C₃)-alkoxy, each of which is substituted by m radicals from the group consisting of fluorine, chlorine, bromine, cyano, (C₁-C₂)-alkoxy and hydroxy.

4. Compounds of the general formula (I) according to any of Claims 1 to 3, where R⁴ represents hydrogen,
or
represents (C₁-C₆)-alkyl, (C₃-C₆)-cycloalkyl, (C₃-C₆)-cycloalkyl- (C₁-C₆)-alkyl, (C₂-C₆)-alkenyl, (C₅-C₆)-cycloalkenyl or (C₂-C₆)-alkynyl, each of which is substituted by m radicals from the group consisting of fluorine, chlorine, bromine, cyano, (C₁-C₄)-alkoxy, hydroxy and aryl.

5. Compounds of the general formula (I) according to any of Claims 1 to 4, where Y and W represents oxygen.

6. Compounds of the general formula (I) according to any of Claims 1 to 5, where Z represents a group Z-1 to Z-33, where Z-1 to Z-33 have the following meaning: 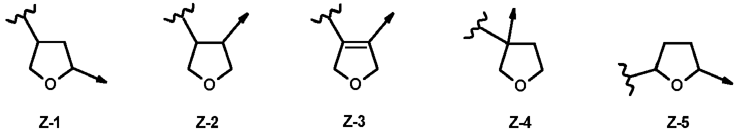 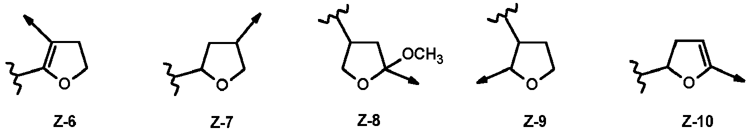 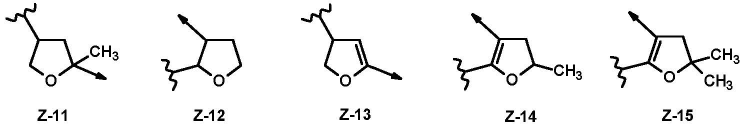 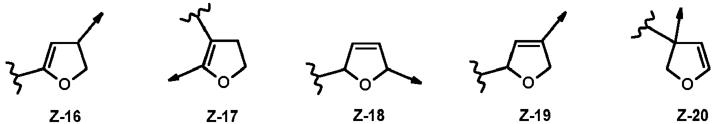 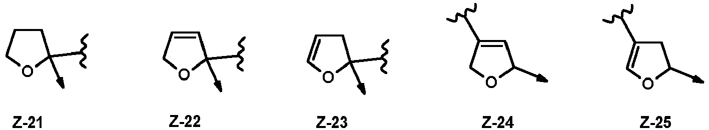 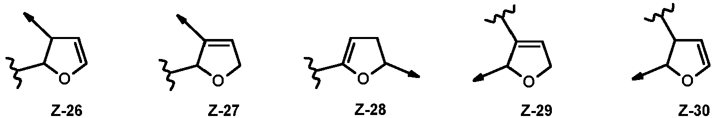 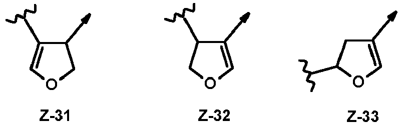 where the arrow in each case denotes a bond to the group C=W of the formula (I).

7. Compounds of the general formula (I) according to any of Claims 1 to 6, where R⁵ represents fluorine, chlorine, cyano, CO₂H, CO₂CH₃ or CO₂CH₂CH₃,
or
represents (C₁-C₂)-alkyl or (C₁-C₂)-alkoxy, each of which is substituted by m radicals from the group consisting of fluorine and chlorine.

8. Compounds of the general formula (I) according to any of Claims 1 to 7, where X², X⁴ and X⁶ independently of one another each represent hydrogen, fluorine, chlorine, bromine or cyano,
or
represent methyl or methoxy, each of which is substituted by m radicals from the group consisting of fluorine and chlorine.

9. Compounds of the general formula (I) according to any of Claims 1 to 8, where X³ and X⁵ represent hydrogen, fluorine, chlorine, bromine, hydroxy or cyano,
or
represent (C₁-C₃)-alkyl, (C₁-C₃)-alkoxy, (C₃-C₄)-cycloalkyl, (C₂-C₃)-alkenyl or (C₂-C₃)-alkynyl, each of which is substituted by m radicals from the group consisting of fluorine, chlorine and bromine.

10. Compounds of the general formula (I) according to any of Claims 1 to 9, where R⁷ represents hydrogen,
or
represents (C₁-C₃)-alkyl each substituted by m radicals from the group consisting of fluorine and chlorine.

11. Compounds of the general formula (I) according to any of Claims 1 to 10, where the running number m is 0, 1, 2 or 3.

12. Compounds of the general formula (I) according to any of Claims 1 to 11,
in which
R¹ and R² each represent hydrogen;
R³ represents (C₁-C₃)-alkyl, (C₃-C₄)-cycloalkyl, (C₂-C₃)-alkenyl, (C₂-C₃)-alkynyl or (C₁-C₃)-alkoxy, each of which is substituted by m radicals from the group consisting of fluorine, chlorine, bromine, cyano, (C₁-C₂)-alkoxy and hydroxy;
R⁴ represents hydrogen,
or
represents (C₁-C₆)-alkyl, (C₃-C₆)-cycloalkyl, (C₃-C₆)-cycloalkyl-(C₁-C₆)-alkyl, (C₂-C₆)-alkenyl, (C₅-C₆)-cycloalkenyl or (C₂-C₆)-alkynyl, each of which is substituted by m radicals from the group consisting of fluorine, chlorine, bromine, cyano, (C₁-C₄)-alkoxy, hydroxy and aryl;
Y represents oxygen;
W represents oxygen;
Z represents a group Z-1 to Z-4, where Z-1 to Z-4 have the following meaning: 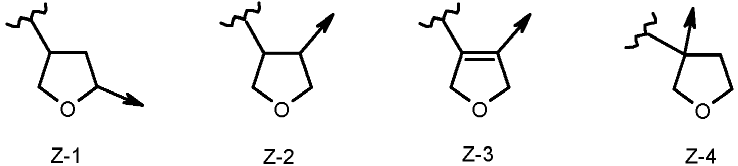 where the arrow in each case denotes a bond to the group C=W of the formula (I);
X², X⁴ and X⁶ independently of one another represent hydrogen or fluorine;
X³ and X⁵ independently of one another represent hydrogen, fluorine, chlorine, bromine, hydroxy or cyano,
or
represent (C₁-C₃)-alkyl, (C₁-C₃)-alkoxy, (C₃-C₄)-cycloalkyl, (C₂-C₃)-alkenyl or (C₂-C₃)-alkynyl, each of which is substituted by m radicals from the group consisting of fluorine, chlorine and bromine; and
m is the running number 0, 1, 2 or 3.

13. Herbicidal composition or plant growth-regulating composition, **characterized in that** it comprises one or more compounds of the general formula (i) or salts thereof according to any of Claims 1 to 12.

14. Herbicidal compositions according to Claim 13, further comprising a formulation auxiliary.

15. Herbicidal compositions according to Claim 13 or 14, comprising at least one further active compound from the group of insecticides, acaricides, herbicides, fungicides, safeners and/or growth regulators.

16. Herbicidal compositions according to Claim 14 or 15, comprising a safener.

17. Herbicidal compositions according to Claim 16, in which the safener is selected from the group consisting of mefenpyr-diethyl, cyprosulfamide, isoxadifen-ethyl, cloquintocet-mexyl, benoxacor and dichlormid.

18. Method of controlling unwanted plants, **characterized in that** an effective amount of at least one compound of the formula (I) according to any of Claims 1 to 12 or of a herbicidal composition according to any of Claims 13 to 17 is applied to the plants or to the site of the unwanted vegetation.

19. Use of compounds of the formula (I) according to any of Claims 1 to 12 or of herbicidal compositions according to any of Claims 13 to 17 for controlling unwanted plants.

20. Use according to Claim 19, **characterized in that** the compounds of the formula (I) are used for controlling unwanted plants in crops of useful plants.

21. Use according to Claim 20, **characterized in that** the useful plants are transgenic useful plants.

22. Compounds of the formula (II) and agrochemically acceptable salts thereof 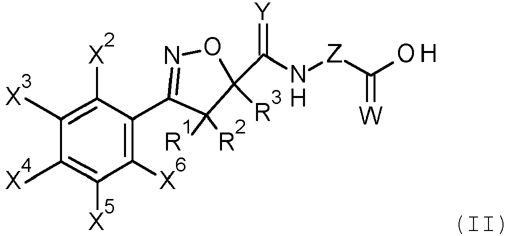 in which the radicals X², X³, X⁴, X⁵, X⁶, R¹, R², R³, Y, Z and W are defined according to Claims 1 to 12.

## Revendications

1. 3-Phénylisoxazoline-5-carboxamides et 3-phénylisoxazoline-5-thioamides de formule générale (I), 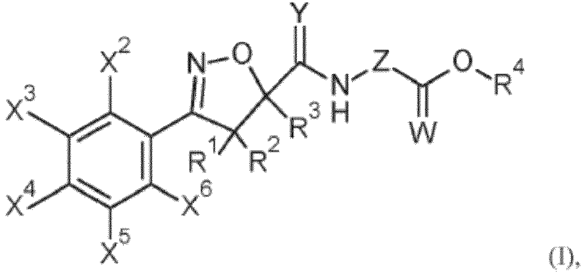 et leurs sels acceptables sur le plan agrochimique, dans lesquels
R¹ et R² signifient indépendamment l'un de l'autre à chaque fois hydrogène, fluor, chlore ou cyano,
ou
(C₁-C₃)-alkyle ou (C₁-C₃)-alcoxy substitué par à chaque fois m radicaux du groupe constitué par fluor, chlore, brome et cyano ;
R³ signifie (C₁-C₄)-alkyle, (C₃-C₅)-cycloalkyle, (C₂-C₄)-alcényle, (C₂-C₄)-alcynyle ou (C₁-C₄)-alcoxy substitué par à chaque fois m radicaux du groupe constitué par fluor, chlore, brome, cyano, (C₁-C₄)-alcoxy et hydroxy ;
R⁴ signifie hydrogène,
ou
(C₁-C₁₂)-alkyle, (C₃-C₇)-cycloalkyle, (C₃-C₇)-cycloalkyl-(C₁-C₈)-alkyle, (C₂-C₆)-alcényle, (C₅-C₆)-cycloalcényle ou (C₂-C₈)-alcynyle substitué par à chaque fois m radicaux du groupe constitué par halogène, cyano, (C₁-C₈)-alcoxy, hydroxy et aryle ;
Y signifie oxygène ou soufre ;
W signifie oxygène ou soufre ;
Z représente un cycle furanne totalement saturé ou partiellement saturé, qui est substitué par k radicaux du groupe R⁵ 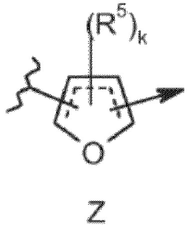 dans lequel la flèche représente à chaque fois une liaison au groupe C=W de la formule (I) ;
R⁵ signifie fluor, chlore, cyano ou CO₂R⁷,
ou
(C₁-C₂)-alkyle ou (C₁-C₂)-alcoxy substitué par à chaque fois m radicaux du groupe constitué par fluor et chlore;
X², X⁴ et X⁶ signifient indépendamment les uns des autres à chaque fois hydrogène, fluor, chlore, brome ou cyano, ou
(C₁-C₂)-alkyle substitué à chaque fois par m radicaux du groupe constitué par fluor, chlore, brome et (C₁-C₂)-alcoxy;
X³ et X⁵ signifient indépendamment l'un de l'autre hydrogène, fluor, chlore, brome, iode, hydroxy, cyano, nitro, S(O)ₙR⁶ ou CO₂R⁷,
ou
(C₁-C₃)-alkyle, (C₁-C₃)-alcoxy, (C₃-C₄)-cycloalkyle, (C₂-C₃)-alcényle ou (C₂-C₃)-alcynyle substitué à chaque fois par m radicaux du groupe constitué par fluor, chlore et brome ;
R⁶ signifie (C₁-C₂)-alkyle ou (C₃-C₄)-cycloalkyle substitué par à chaque fois m radicaux du groupe constitué par fluor et chlore ;
R⁷ signifie hydrogène,
ou
(C₁-C₃)-alkyle ou (C₃-C₅)-cycloalkyle substitué par à chaque fois m radicaux du groupe constitué par fluor, chlore et (C₁-C₂)-alcoxy ;
k est l'indice 0, 1 ou 2 ;
m est l'indice 0, 1, 2, 3, 4 ou 5 ; et
n est l'indice 0, 1 ou 2.

2. Composés de formule générale (I) selon la revendication 1, R¹ et R² signifiant indépendamment l'un de l'autre à chaque fois hydrogène, fluor, chlore ou cyano,
ou
méthyle ou méthoxy substitué par à chaque fois m radicaux du groupe constitué par fluor et chlore.

3. Composés de formule générale (I) selon la revendication 1 ou 2, R³ signifiant (C₁-C₃)-alkyle, (C₃-C₄)-cycloalkyle, (C₂-C₃)-alcényle, (C₂-C₃)-alcynyle ou (C₁-C₃)-alcoxy substitué par à chaque fois m radicaux du groupe constitué par fluor, chlore, brome, cyano, (C₁-C₂)-alcoxy et hydroxy.

4. Composés de formule générale (I) selon l'une quelconque des revendications 1 à 3, R⁴ signifiant hydrogène,
ou
(C₁-C₆)-alkyle, (C₃-C₆)-cycloalkyle, (C₃-C₆)- cycloalkyl-(C₁-C₈)-alkyle, (C₂-C₆)-alcényle, (C₅-C₆)- cycloalcényle ou (C₂-C₆)-alcynyle substitué par à chaque fois m radicaux du groupe constitué par fluor, chlore, brome, cyano, (C₁-C₄)-alcoxy, hydroxy et aryle.

5. Composés de formule générale (I) selon l'une quelconque des revendications 1 à 4, Y et W signifiant oxygène.

6. Composés de formule générale (I) selon l'une quelconque des revendications 1 à 5, Z représentant un groupe Z-1 à Z-33, Z-1 à Z-33 possédant la signification suivante : 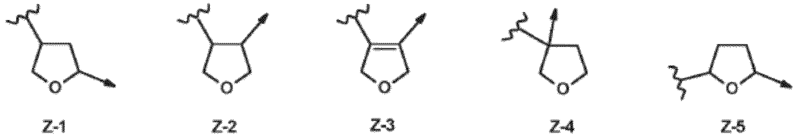 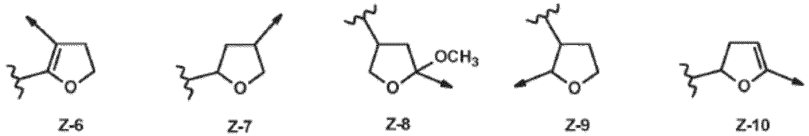 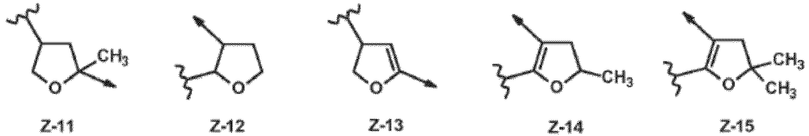 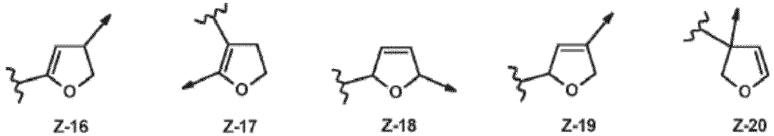 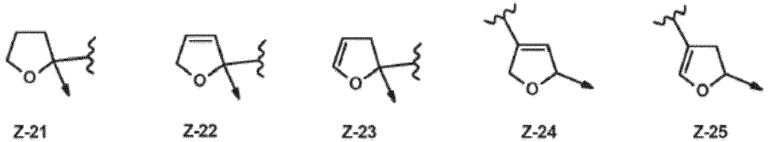 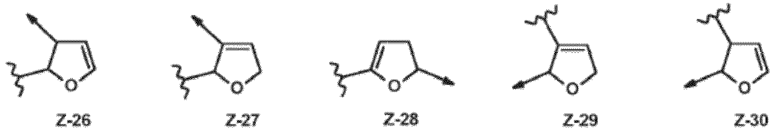 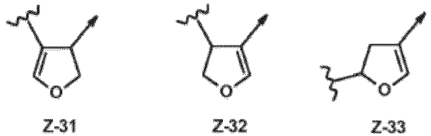 dans lesquels la flèche représente à chaque fois une liaison au groupe C=W de la formule (I).

7. Composés de formule générale (I) selon l'une quelconque des revendications 1 à 6, R⁵ signifiant fluor, chlore, cyano, CO₂H, CO₂CH₃ ou CO₂CH₂CH₃,
ou
(C₁-C₂)-alkyle ou (C₁-C₂)-alcoxy substitué à chaque fois par m radicaux du groupe constitué par fluor et chlore.

8. Composés de formule générale (I) selon l'une quelconque des revendications 1 à 7, X², X⁴ et X⁶ signifiant indépendamment les uns des autres à chaque fois hydrogène, fluor, chlore, brome ou cyano,
ou
méthyle ou méthoxy substitué à chaque fois par m radicaux du groupe constitué par fluor et chlore.

9. Composés de formule générale (I) selon l'une quelconque des revendications 1 à 8, X³ et X⁵ signifiant hydrogène, fluor, chlore, brome, hydroxy ou cyano,
ou
(C₁-C₃)-alkyle, (C₁-C₃)-alcoxy, (C₃-C₄)-cycloalkyle, (C₂-C₃)-alcényle ou (C₂-C₃)-alcynyle substitué à chaque fois par m radicaux du groupe constitué par fluor, chlore et brome.

10. Composés de formule générale (I) selon l'une quelconque des revendications 1 à 9, R⁷ signifiant hydrogène,
ou
(C₁-C₃)-alkyle substitué par à chaque fois m radicaux du groupe constitué par fluor et chlore.

11. Composés de formule générale (I) selon l'une quelconque des revendications 1 à 10, l'indice m étant 0, 1, 2 ou 3.

12. Composés de formule générale (I) selon l'une quelconque des revendications 1 à 11,
dans lesquels
R¹ et R² signifient à chaque fois hydrogène ;
R³ signifie (C₁-C₃)-alkyle, (C₃-C₄)-cycloalkyle, (C₂-C₃)-alcényle, (C₂-C₃)-alcynyle ou (C₁-C₃)-alcoxy substitué par à chaque fois m radicaux du groupe constitué par fluor, chlore, brome, cyano, (C₁-C₂)-alcoxy et hydroxy ;
R⁴ signifie hydrogène,
ou
(C₁-C₆)-alkyle, (C₃-C₆)-cycloalkyle, (C₃-C₆)- cycloalkyl- (C₁-C₈)-alkyle, (C₂-C₆)-alcényle, (C₅-C₆)-cycloalcényle ou (C₂-C₆)-alcynyle substitué par à chaque fois m radicaux du groupe constitué par fluor, chlore, brome, cyano, (C₁-C₄)-alcoxy, hydroxy et aryle ;
Y signifie oxygène ;
W signifie oxygène ;
Z représente un groupe Z-1 à Z-4, Z-1 à Z-4 possédant la signification suivante : 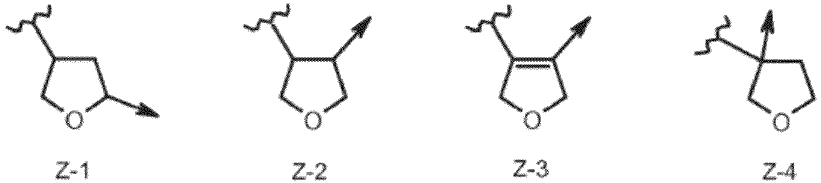 dans lesquels la flèche représente à chaque fois une liaison au groupe C=W de la formule (I) ;
X², X⁴ et X⁶ signifient indépendamment les uns des autres hydrogène ou fluor ;
X³ et X⁵ signifient indépendamment l'un de l'autre hydrogène, fluor, chlore, brome, hydroxy ou cyano,
ou
(C₁-C₃)-alkyle, (C₁-C₃)-alcoxy, (C₃-C₄)-cycloalkyle, (C₂-C₃)-alcényle ou (C₂-C₃)-alcynyle substitué à chaque fois par m radicaux du groupe constitué par fluor, chlore et brome ; et
m est l'indice 0, 1, 2 ou 3.

13. Agent herbicide ou agent de régulation de la croissance végétale, **caractérisé en ce qu'**il contient un ou plusieurs composés de formule générale (i) ou leurs sels selon l'une quelconque des revendications 1 à 12.

14. Agent herbicide selon la revendication 13, qui contient en outre un auxiliaire de formulation.

15. Agent herbicide selon la revendication 13 ou 14 contenant au moins un principe actif supplémentaire du groupe des insecticides, des acaricides, des herbicides, des fongicides, des phytoprotecteurs et/ou des régulateurs de croissance.

16. Agent herbicide selon la revendication 14 ou 15 contenant un phytoprotecteur.

17. Agent herbicide selon la revendication 16, le phytoprotecteur étant choisi dans le groupe constitué par le méfenpyr-diéthyle, le cyprosulfamide, l'isoxadifen-éthyle, le cloquintocet-mexyle, le bénoxacor et le dichlormide.

18. Procédé pour la lutte contre des végétaux indésirables, **caractérisé en ce qu'**on applique une quantité efficace d'au moins un composé de formule (I) selon l'une quelconque des revendications 1 à 12 ou d'un agent herbicide selon l'une quelconque des revendications 13 à 17 sur les végétaux ou sur le lieu de croissance végétale indésirable.

19. Utilisation de composés de formule (I) selon l'une quelconque des revendications 1 à 12 ou d'agents herbicides selon l'une quelconque des revendications 13 à 17 pour la lutte contre des végétaux indésirables.

20. Utilisation selon la revendication 19, **caractérisée en ce que** les composés de formule (I) sont utilisés pour la lutte contre des végétaux indésirables dans des cultures de végétaux utiles.

21. Utilisation selon la revendication 20, **caractérisée en ce que** les végétaux utiles sont des végétaux utiles transgéniques.

22. Composés de formule (II) et leurs sels acceptables sur le plan agrochimique 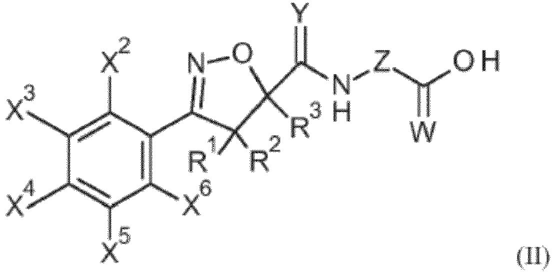 les radicaux X², X³, X⁴, X⁵, X⁶, R¹, R², R³, Y, Z et W étant définis selon les revendications 1 à 12.
